(19) Europäisches Patentamt
European Patent Office
Office européen des brevets

(11) EP 0 934 940 A1

(12) **EUROPEAN PATENT APPLICATION**
published in accordance with Art. 158(3) EPC

(43) Date of publication:
**11.08.1999 Bulletin 1999/32**

(21) Application number: **97926224.3**

(22) Date of filing: **11.06.1997**

(51) Int. Cl.[6]: **C07D 487/04**, C07D 487/14, C07D 495/12, A61K 31/55

(86) International application number:
**PCT/JP97/02016**

(87) International publication number:
**WO 97/47622 (18.12.1997 Gazette 1997/54)**

(84) Designated Contracting States:
**AT BE CH DE DK ES FI FR GB GR IE IT LI LU MC NL PT SE**

(30) Priority: **12.06.1996 JP 17426896**
**28.03.1997 JP 9523797**

(71) Applicant: **Japan Tobacco Inc.**
**Minato-Ku Tokyo 105 (JP)**

(72) Inventors:
- **YAMAMOTO, Yoshihisa,**
**Central Pharm. Res. Inst**
**Takatsuki-shi, Osaka 569-11 (JP)**
- **SHINDO, Msanori,**
**Central Pharm. Res. Inst.**
**Takatsuki-shi, Osaka 569-11 (JP)**
- **NAKAMURA, Takeshi,**
**Central Pharm. Res. Inst.**
**Takatsuki-shi, Osaka 569-11 (JP)**

(74) Representative:
**von Kreisler, Alek, Dipl.-Chem. et al**
**Patentanwälte,**
**von Kreisler-Selting-Werner,**
**Bahnhofsvorplatz 1 (Deichmannhaus)**
**50667 Köln (DE)**

(54) **CYTOKINE PRODUCTION INHIBITORS, TRIAZEPINE COMPOUNDS, AND INTERMEDIATES THEREOF**

(57) The present invention relates to a cytokine production inhibitor comprising a compound of the formula [I]

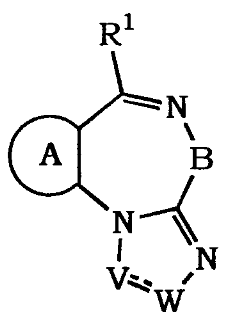

[I]

wherein $R^1$ is aryl or heteroaryl; B is a group of the formula (a) or (b)

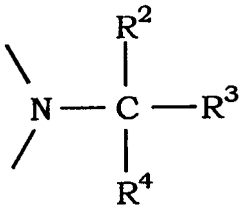

(a)

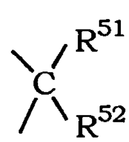

(b)

wherein $R^2$ is hydrogen atom, hydroxy, halogen atom and the like, $R^4$ is hydrogen atom or halogen atom, or $R^2$ and $R^4$ in combination form carbonyl; $R^3$ is lower alkoxy, cycloalkyl, aryl, heteroaryl, -X-Y and the like, wherein X is $-(CH_2)_m-$, -

EP 0 934 940 A1

CO-, $-COCH_2-$, $-NHCH_2-$, $-CH_2NH-$, $-OCH_2-$ and the like, Y is halogen atom, cycloalkyl, aryl or heteroaryl; $R^{51}$ is -NHCONHR$^{57}$ and the like; $R^{52}$ is hydrogen atom or - COOR$^{53}$; A is benzene ring or thiophene ring, novel triazepine compound and intermediates for the production of those triazepine compounds. The compounds of the formula [I] suppress production of cytokine such as IL-6, TNF-$\alpha$, IL-8, IFN$\gamma$, IL-2, GM-CSF and the like, and are useful as cytokine production inhibitors or antiinflammatory drugs.

## Description

### Technical Field

The present invention relates to a cytokine production inhibitor containing a triazepine compound or diazepine compound as an active ingredient, particularly to a medicament that inhibits production of interleukin-6 (IL-6), tumor necrosis factor α (TNF-α), interleukin-8 (IL-8), interleukin-2 (IL-2), interferon γ (IFNγ) and granulocyte macrophage colony stimulating factor (GM-CSF). The present invention also relates to a novel triazepine compound having a cytokine production inhibitory action and an intermediate compound used for producing said triazepine compound.

### Background Art

Cytokine acts on various cells and exerts various physiological activities. For example, cytokine plays an important role in biophylaxis such as immune responses, inflammatory responses and hematopoietic responses.
On the other hand, an excessive production of cytokine has been reported to be closely related to the pathogenesis or formation of a disease state of inflammatory diseases, autoimmune diseases, viral diseases and cancer.
For example, reports have documented that, in various inflammatory diseases, IFNγ or IL-2 activates T cell; IL-8 leads neutrophil to inflammation site; TNF-α, IL-6 or GM-CSF activates inflammatory cells; and these phenomena are solely or integrally involved in the progress of the disease state.
Likewise, many cytokines inclusive of IFNγ and IL-2 are responsible for the progression of symptoms in autoimmune diseases.
It is therefore considered that an effective cytokine production inhibitor is critical in the treatment and prophylaxis of inflammatory diseases and autoimmune diseases.
Under the circumstances, Japanese Patent Unexamined Publication No. 6-192094 discloses a carbostyryl derivative of the following formula as a compound having an inhibitory action on IL-6 and TNF-α production.

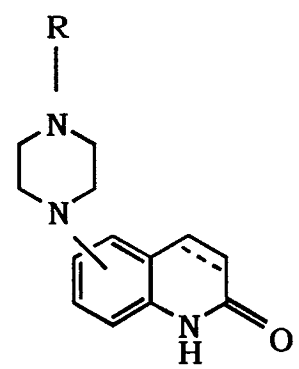

wherein R is benzoyl optionally having lower alkoxy on the phenyl ring and the bond between the 3-position carbon and 4-position carbon of the carbostyryl skeleton is a single or double bond.
In addition, Japanese Patent Unexamined Publication No. 8-73453 discloses oxoquinoline derivative of the following formula as a compound having an ihhibitory action on the production of TNF-α, IL-6, IL-8 and IFNγ.

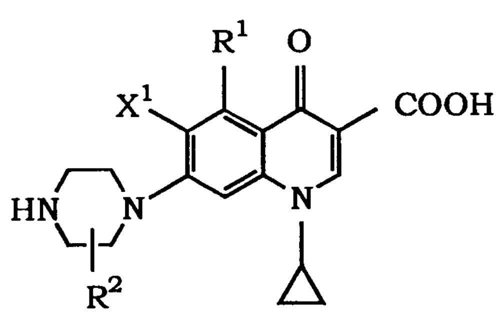

wherein $R^1$ and $R^2$ are each lower alkyl and $X^1$ is halogen atom.

Nevertheless, these publications do not disclose a triazepine compound or a diazepine compound of the present invention. These compounds are not sufficient in the activity to strongly and widely suppress various kinds of cytokines.

Meanwhile, the present inventors have filed a patent application directed to a medicament for treating osteoporosis, which comprises, as an active ingredient, a triazepine compound of the following formula:

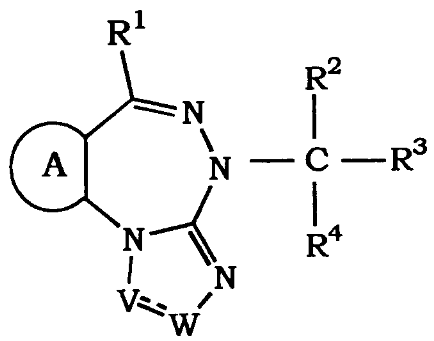

[I']

wherein $R^1$, $R^2$, $R^3$, $R^4$, A, V and W are as defined below, or a salt thereof (Japanese Patent Application No. 7-324080).

Certain compounds having a triazepine skeleton have been reported to have an anticonvulsive action, muscle relaxing action, sedation, antianxiety action and ataractic action (US Patent No. 4,144,233, US Patent No. 5,091,381, US Patent No. 3,891,666 and US Patent No. 3,880,878).

However, these publications do not disclose that the triazepine compound of the present invention has a cytokine production inhibitory action.

Also, the present inventors previously filed a patent application directed to a medicament for treating osteoporosis, which comprises, as an active ingredient, a diazepine compound embracing the compounds of the following formula:

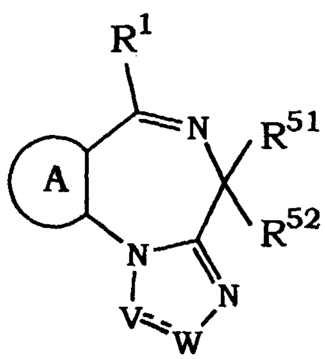

[I"]

wherein $R^1$, $R^{51}$, $R^{52}$, A, V and W are as defined below (WO 93/07129).

It has also been reported that certain triazolodiazepine compound has a cell adhesion inhibitory action (Japanese Patent Unexamined Publication No. 7-179417, PAF antagonistic action (Japanese Patent Unexamined Publication No. 5-86067, Japanese Patent Unexamined Publication No. 2-49787, Japanese Patent Unexamined Publication No. 2-256681) or coronary vasodilating action (Japanese Patent Unexamined Publication No. 64-79185).

These publications, however, do not disclose that a diazepine compound as the one in the present invention has a cytokine production inhibitory action.

The benzodiazepine compound of the following formula has been reported to have a cytokine (IL-1,IL-6, TNF-α) production inhibitory activity (F. Zavala et al., The Journal of Pharmacology and Experimental Therapeutics, Vol. 255, No. 2, 442-450 (1990)).

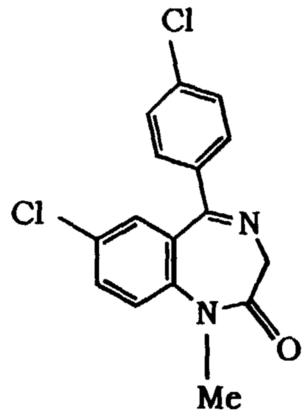

This publication do not disclose a diazepine compound as in the present invention, nor does the benzodiazepine compound disclosed therein show sufficient activity.

At present, steroids such as adrenocortical hormone, nonsteroidal antiinflammatory agents such as indomethacin, gold preparations such as aurothiomalate, antirheumatic agents such as D-penicillamine, antipodagric agents such as colchicine, immunosuppressive agents such as cylophosphamide and the like have been used for drug treatment of inflammatory diseases. Autoimmune disease have been mainly treated with steroids and immunosuppressive agents. Cancers have been mainly treated with anticancer drugs such as 5-FU and adriamycin.

In terms of the cytokine inhibitory activity, steroids have a broad range of inhibitory activity against cytokine production. Nevertheless, steroids show strong toxicity and do not permit a long-term use due to a rebound phenomenon that occurs after interruption of the use.

Other drugs mentioned above lack an inhibitory activity against several kinds of cytokines as mentioned in the present invention.

Ther is a strong demand, therefore, for the development of a cytokine production inhibitor showing dependable effect high safety, easy use of the method, and superior effects of prophylaxis and treatment.

**Disclosure of the Invention**

The present invention aims at providing a novel cytokine production inhibitor and an antiinflammatory drug.

The present invention also aims at providing a novel compound useful as an active ingredient of a cytokine production inhibitor.

The present invention further aims at providing an intermediate compound for producing a compound useful as an active ingredient of a cytokine production inhibitor.

The present invention moreover aims at providing a method for the prophylaxis or treatment of diseases caused by abnormal production of cytokine and a method for the prophylaxis or treatment of inflammation.

The present invention yet aims at providing a novel use of a triazepine compound or diazepine compound.

In view of the above-mentioned problems, the present inventors have conducted intensive studies in an attempt to seek a useful cytokine production inhibitor, and found that a triazepine compound or diazepine compound of the following formula [I] has a remarkable cytokine production inhibitory action, which resulted in the completion of the present invention.

Accordingly, the present invention relates to a cytokine production inhibitor containing a compound of the following formula [I] or a pharmaceutically acceptable salt thereof as an active ingredient. The present invention also relates to a novel triazepine compound having a cytokine production inhibitory action. Further, the present invention relates to an intermediate compound useful for producing said triazepine compound. Moreover, the present invention relates to a method for the prophylaxis or treatment of the diseases caused by abnormal production of cytokine or inflammation, which comprises administering a compound of the following formula [I] or a pharmaceutically acceptable salt thereof. The present invention also relates to a use of a compound of the following formula [I] for producing a cytokine production inhibitor or an antiinflammatory drug.

More particularly, the present invention provides the following (1)-(38).

(1) A cytokine production inhibitor comprising, as an active ingredient, a compound of the formula [I]

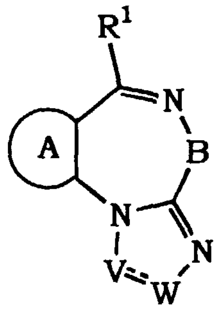

[I]

wherein

$R^1$ is an optionally substituted aryl or an optionally substituted heteroaryl;

B is a group of the formula

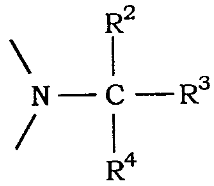 or 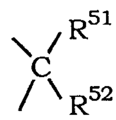

wherein,

$R^2$ is hydrogen atom, hydroxy, halogen atom or lower alkyl,

$R^4$ is hydrogen atom or halogen atom,

or $R^2$ and $R^4$ in combination form carbonyl with the carbon atom they bind with,

$R^3$ is hydrogen atom, lower alkyl, lower alkoxy, cycloalkyl, optionally substituted aryl, optionally substituted heteroaryl or a group of the formula:

$-CR^5=CR^6R^7$, $-OR^{18}$, 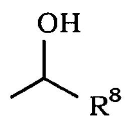 ,

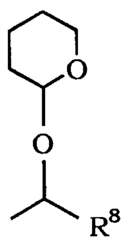 , $-COOR^8$, $-CONHR^8$, 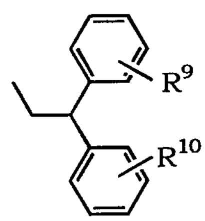

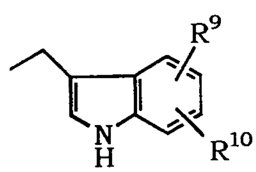 or -X-Y

wherein $R^5$, $R^6$ and $R^7$ are the same or different and each is hydrogen atom, halogen atom, lower alkyl, or optionally substituted aryl, $R^8$ is hydrogen atom, lower alkyl, cycloalkyl, optionally substituted aryl, aralkyl or optionally substituted heteroaryl, $R^9$ and $R^{10}$ are the same or different and each is hydrogen atom, lower alkyl, lower alkoxy, hydroxy, halogen atom, nitro or amino, $R^{18}$ is optionally substituted aryl, X is $-(CH_2)_m-$, -CO-, $-COCH_2-$, -NH-, $-NHCH_2-$, $-CH_2NH-$, $-CH_2NHCO-$,

$-OCH_2-$, $-(CH_2)_nO-$ or $-CH_2S-$, Y is halogen atom, cycloalkyl, optionally substituted aryl or optionally substituted heteroaryl, m is an integer of 1 to 4 and n is an integer of 1 to 4,

$R^{51}$ is hydrogen atom, lower alkyl, haloalkyl, optionally substituted aryl, optionally substituted heteroaryl, aralkyl, heteroarylalkyl or a group selected from the groups of the following formulas:

| | |
|---|---|
| $—(CH_2)_bN(R^{53})(R^{54})$ | (1) |
| $—(CH_2)_bOR^{55}$ | (2) |
| $—(CH_2)_bN(R^{55})\overset{Z}{\overset{\|}{C}}N(R^{56})(R^{57})$ | (3) |
| $—(CH_2)_bN(R^{55})CORa^{56}$ | (4) |
| $—(CH_2)_bN(R^{55})SO_2R^{58}$ | (5) |
| $—(CH_2)_bN(R^{55})COOR^{59}$ | (6) |
| $—(CH_2)_bO\overset{Z}{\overset{\|}{C}}N(R^{56})(R^{57})$ | (7) |
| $—(CH_2)_bOCOR^{60}$ | (8) |
| $—(CH_2)_bCON(R^{61})(R^{62})$ | (9) |
| $—(CH_2)_bOSO_2R^{58}$ | (10) |
| $—(CH_2)_bCOR^{63}$ | (11) |
| $—(CH_2)_bS(O)_pR^{56}$ | (12) |
| $—CON(R^{55})OR^{53}$ | (13) |
| $—CON(R^{55})N(R^{55})\overset{Z}{\overset{\|}{C}}Ra^{56}$ | (14) |
| $—CON(R^{55})N(R^{55})SO_2Ra^{56}$ | (15) |
| $—N(R^{55})\overset{Z}{\overset{\|}{C}}N(R^{55})CORa^{56}$ | (16) |
| $—N(R^{55})\overset{Z}{\overset{\|}{C}}N(R^{55})SO_2Ra^{56}$ | (17) |
| $—CON(R^{55})N(R^{55})(R^{56})$ | (18) |
| $—(CH_2)_bN(R^{55})COCON(R^{56})(R^{57})$ | (19) and |
| $—(CH_2)_aCOOR^{64}$ | (20) |

wherein b is 0 or an integer of 1 to 6, Z is oxygen atom or sulfur atom, $R^{53}$ and $R^{54}$ are the same or different and each is hydrogen atom, lower alkyl, optionally substituted aryl or aralkyl, $R^{55}$ is hydrogen atom, lower alkyl or aralkyl, $R^{56}$ and $R^{57}$ are the same or different and each is hydrogen atom, lower alkyl, cycloalkyl, cycloalkylalkyl, optionally substituted aryl, aralkyl, optionally substituted heteroaryl or heteroarylalkyl, $Ra^{56}$ is lower alkyl, cycloalkyl, cycloalkylalkyl, optionally substituted aryl, aralkyl, optionally substituted heteroaryl or heteroarylalkyl, $R^{58}$ is lower alkyl, optionally substituted aryl, aralkyl, cycloalkyl, or optionally substituted heteroaryl, $R^{59}$ is lower alkyl, optionally substituted aryl or aralkyl, $R^{60}$ is lower alkyl, lower alkenyl, lower alkynyl, cycloalkyl, cycloalkylalkyl, optionally substituted aryl, aralkyl, optionally substituted heteroaryl or heteroarylalkyl, $R^{61}$ and $R^{62}$ are the same or different and each is hydrogen atom, lower alkyl, acyl, optionally substituted aryl or aralkyl, $R^{63}$ is lower alkyl, optionally substituted aryl, aralkyl, optionally substituted heteroaryl or heteroarylalkyl, p is 0, 1 or 2, a is an integer of 1 to 6, and $R^{64}$ is hydrogen atom, lower alkyl, optionally substituted aryl or aralkyl, and

$R^{52}$ is hydrogen atom or $-COOR^{53}$ wherein $R^{53}$ is hydrogen atom, lower alkyl, optionally substituted aryl or aralkyl, or $R^{51}$ and $R^{52}$ in combination form, together with the carbon atom they bind with, a spiro ring of the formula:

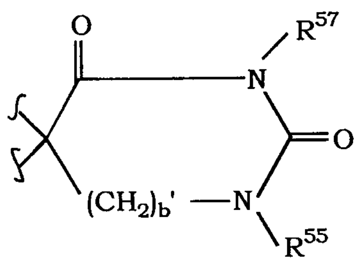

wherein b' is 0 or 1, $R^{55}$ is hydrogen atom, lower alkyl or aralkyl, and $R^{57}$ is hydrogen atom, lower alkyl, cycloalkyl, cycloalkylalkyl, optionally substituted aryl, aralkyl, optionally substituted heteroaryl or heteroarylalkyl; and

ring A is a ring selected from the following rings:

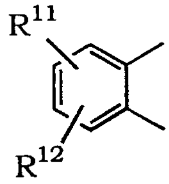 , 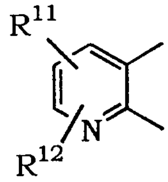 , 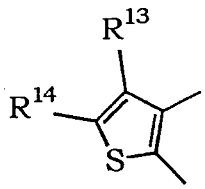 and 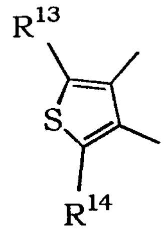

wherein $R^{11}$ and $R^{12}$ are the same or different and each is hydrogen atom, halogen atom, lower alkyl, said lower alkyl being optionally substituted by halogen atom, lower alkoxy, nitro, amino, amino substituted by lower alkyl, cyclic amino, hydroxy, acyloxy, cyano, carbamoyl, carbamoyl substituted by lower alkyl, cyclic aminocarbonyl, carboxy, lower alkoxycarbonyl or aralkyloxycarbonyl, lower alkenyl, aralkyl, aralkyl substituted by lower alkyl, lower alkoxy, nitro, amino, amino substituted by lower alkyl, cyclic amino, hydroxy, acyloxy, cyano, carbamoyl, carbamoyl substituted by lower alkyl, cyclic aminocarbonyl, carboxy, lower alkoxycarbonyl or aralkyloxycarbonyl, and $R^{13}$ and $R^{14}$ are the same or different and each is hydrogen atom, halogen atom, lower alkyl, lower alkenyl, lower alkynyl, haloalkyl, lower alkoxy, nitro, amino, amino substituted by lower alkyl, cyclic amino, hydroxy, acyloxy, cyano, carbamoyl, carbamoyl substituted by lower alkyl, cyclic aminocarbonyl, carboxy, lower alkoxycarbonyl, aralkyloxycarbonyl, cycloalkyl or lower alkylcarbonyl; and

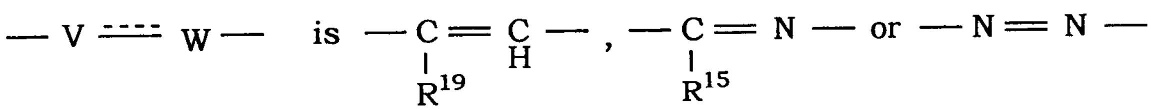
is , or

wherein $R^{15}$ is lower alkyl and $R^{19}$ is hydrogen atom or lower alkyl,

or a pharmaceutically acceptable salt thereof.

(2) The cytokine production inhibitor of (1), comprising, as an active ingredient, a compound of the formula [I] wherein B is

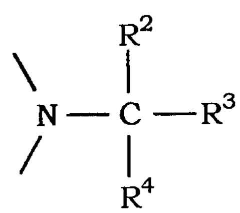

namely, a compound of the formula [I']

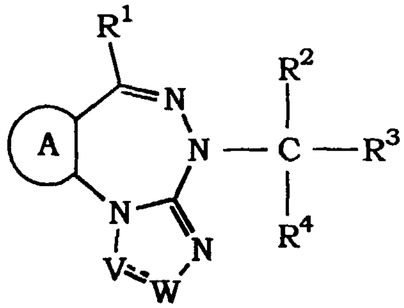

[I']

wherein $R^1$, $R^2$, $R^3$, $R^4$, A, V and W are as defined in (1),
or a pharmaceutically acceptable salt thereof.

(3) The cytokine production inhibitor of (2), wherein, in the formula [I'], the ring A is

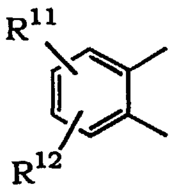

or

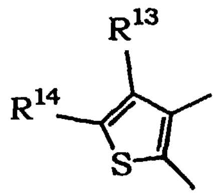

wherein $R^{11}$, $R^{12}$, $R^{13}$ and $R^{14}$ are as defined in (1),

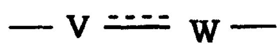

is

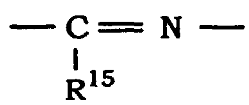

wherein $R^{15}$ is lower alkyl,
$R^1$ is optionally substituted phenyl, and
$R^2$ and $R^4$ are both hydrogen atoms.

(4) The cytokine production inhibitor of (2), comprising, as an active ingredient, a compound selected from the group consisting of:

6-(4-chlorophenyl)-4-(4-methoxybenzyl)-1-methyl-4H-2,3,4,5,10b-pentaazabenz[e]azulene,
6-(4-chlorophenyl)-4-(3,4-dimethoxybenzyl)-1-methyl-4H-2,3,4,5,10b-pentaazabenz[e]azulene,
4-(4-methoxybenzyl)-1-methyl-6-phenyl-4H-2,3,4,5,10b-pentaazabenz[e]azulene,
4-(4-methoxybenzyl)-1,9-dimethyl-6-phenyl-4H-2,3,4,5,10b-pentaazabenz[e]azulene,
8-chloro-4-(4-methoxybenzyl)-1-methyl-6-phenyl-4H-2,3,4,5,10b-pentaazabenz[e]azulene,
4-(4-methoxybenzyl)-1-methyl-8-nitro-6-phenyl-4H-2,3,4,5,10b-pentaazabenz[e]azulene,
4-(4-methoxybenzyl)-1-methyl-6-(4-methylphenyl)-4H-2,3,4,5,10b-pentaazabenz[e]azulene,
8-chloro-6-(2-chlorophenyl)-4-(4-methoxybenzyl)-1-methyl-4H-2,3,4,5,10b-pentaazabenz[e]azulene,
6-(4-chlorophenyl)-4-(4-methoxybenzyl)-1,9-dimethyl-4H-2,3,4,5,10b-pentaazabenz[e]azulene,
6-(4-bromophenyl)-4-(4-methoxybenzyl)-1-methyl-4H-2,3,4,5,10b-pentaazabenz[e]azulene,
tert-butyl 4-[4-(4-methoxybenzyl)-1-methyl-4H-2,3,4,5,10b-pentaazabenz[e]azulen-6-yl]benzoate,
6-(4-chlorophenyl)-4-(4-methoxybenzyl)-1-methyl-4H-2,3,4,5,10,10b-hexaazabenz[e]azulene,
4-(4-chlorophenyl)-6-(4-methoxybenzyl)-2,3,9-trimethyl-6H-5,6,7,8,9a-pentaazathieno[2,3-e]azulene,
4-(4-chlorophenyl)-2-ethyl-6-(4-methoxybenzyl)-9-methyl-6H-5,6,7,8,9a-pentaazathieno[2,3-e]azulene,
6-(4-methoxybenzyl)-2,9-dimethyl-4-phenyl-6H-5,6,7,8,9a-pentaazathieno[2,3-e]azulene,
6-(4-methoxybenzyl)-2,3,9-trimethyl-4-phenyl-6H-5,6,7,8,9a-pentaazathieno[2,3-e]azulene,
2-ethyl-6-(4-methoxybenzyl)-9-methyl-4-phenyl-6H-5,6,7,8,9a-pentaazathieno[2,3-e]azulene,

6-(4-methoxybenzyl)-4-(4-methoxyphenyl)-2,3,9-trimethyl-6H-5,6,7,8,9a-pentaazathieno[2,3-e]azulene,
6-(4-chlorophenyl)-1-methyl-4-(pyridin-3-ylmethyl)-4H-2,3,4,5,10b-pentaazabenz[e]azulene,
4-(2-chlorophenyl)-2,3,9-trimethyl-6-(pyridin-4-ylmethyl)-6H-5,6,7,8,9a-pentaazathieno[2,3-e]azulene,
6-(4-chlorophenyl)-4-(3-cyanobenzyl)-1-methyl-4H-2,3,4,5,10b-pentaazabenz[e]azulene,
6-(4-chlorophenyl)-4-(2-fluorobenzyl)-1-methyl-4H-2,3,4,5,10b-pentaazabenz[e]azulene,
6-(4-chlorophenyl)-4-(3-fluorobenzyl)-1-methyl-4H-2,3,4,5,10b-pentaazabenz[e]azulene,
6-(4-chlorophenyl)-4-(4-fluorobenzyl)-1-methyl-4H-2,3,4,5,10b-pentaazabenz[e]azulene,
6-(4-chlorophenyl)-4-(2,4-difluorobenzyl)-1-methyl-4H-2,3,4,5,10b-pentaazabenz[e]azulene,
6-(4-chlorophenyl)-4-(2,5-difluorobenzyl)-1-methyl-4H-2,3,4,5,10b-pentaazabenz[e]azulene,
6-(4-chlorophenyl)-4-(3,5-difluorobenzyl)-1-methyl-4H-2,3,4,5,10b-pentaazabenz[e]azulene,
6-(4-chlorophenyl)-4-(3,4-difluorobenzyl)-1-methyl-4H-2,3,4,5,10b-pentaazabenz[e]azulene,
6-(4-chlorophenyl)-1-methyl-4-(2-trifluoromethylbenzyl)-4H-2,3,4,5,10b-pentaazabenz[e]azulene,
6-(4-chlorophenyl)-1-methyl-4-(4-trifluoromethylbenzyl)-4H-2,3,4,5,10b-pentaazabenz[e]azulene,
6-(4-chlorophenyl)-1-methyl-4-(3-trifluoromethylbenzyl)-4H-2,3,4,5,10b-pentaazabenz[e]azulene,
6-(4-chlorophenyl)-1-methyl-4-(4-trifluoromethoxybenzyl)-4H-2,3,4,5,10b-pentaazabenz[e]azulene,
6-(4-chlorophenyl)-1-methyl-4-(3-nitrobenzyl)-4H-2,3,4,5,10b-pentaazabenz[e]azulene,
4-(2-chlorobenzyl)-6-(4-chlorophenyl)-1-methyl-4H-2,3,4,5,10b-pentaazabenz[e]azulene,
4-(3-chlorobenzyl)-6-(4-chlorophenyl)-1-methyl-4H-2,3,4,5,10b-pentaazabenz[e]azulene,
4-(4-chlorobenzyl)-6-(4-chlorophenyl)-1-methyl-4H-2,3,4,5,10b-pentaazabenz[e]azulene,
6-(4-chlorophenyl)-4-(2-cyanobenzyl)-1-methyl-4H-2,3,4,5,10b-pentaazabenz[e]azulene,
6-(4-chlorophenyl)-4-(4-cyanobenzyl)-1-methyl-4H-2,3,4,5,10b-pentaazabenz[e]azulene,
6-(4-chlorophenyl)-4-(2-methoxybenzyl)-1-methyl-4H-2,3,4,5,10b-pentaazabenz[e]azulene,
6-(4-chlorophenyl)-4-(3-methoxybenzyl)-1-methyl-4H-2,3,4,5,10b-pentaazabenz[e]azulene,
6-(4-chlorophenyl)-4-(4-methoxybenzyl)-1-methyl-4H-2,3,4,5,10b-pentaazabenz[e]azulene,
6-(4-chlorophenyl)-4-(2,5-dimethoxybenzyl)-1-methyl-4H-2,3,4,5,10b-pentaazabenz[e]azulene,
6-(4-chlorophenyl)-1-methyl-4-(3,4,5-trimethoxybenzyl)-4H-2,3,4,5,10b-pentaazabenz[e]azulene,
4-(5-acetyl-2-methoxybenzyl)-6-(4-chlorophenyl)-1-methyl-4H-2,3,4,5,10b-pentaazabenz[e]azulene,
6-(4-chlorophenyl)-1-methyl-4-(3,4-methylenedioxybenzyl)-4H-2,3,4,5,10b-pentaazabenz[e]azulene,
4-(2-chloro-4,5-methylenedioxybenzyl)-6-(4-chlorophenyl)-1-methyl-4H-2,3,4,5,10b-pentaazabenz[e]azulene,
6-(4-chlorophenyl)-4-(2-methoxy-5-nitrobenzyl)-1-methyl-4H-2,3,4,5,10b-pentaazabenz[e]azulene,
6-(4-chlorophenyl)-4-(4-methoxy-3-nitrobenzyl)-1-methyl-4H-2,3,4,5,10b-pentaazabenz[e]azulene,
4-(3-chloro-4-methoxybenzyl)-6-(4-chlorophenyl)-1-methyl-4H-2,3,4,5,10b-pentaazabenz[e]azulene,
6-(4-chlorophenyl)-4-(3,5-dichloro-4-methoxybenzyl)-1-methyl-4H-2,3,4,5,10b-pentaazabenz[e]azulene,
6-(4-chlorophenyl)-1-methyl-4-(2-methylbenzyl)-4H-2,3,4,5,10b-pentaazabenz[e]azulene,
6-(4-chlorophenyl)-1-methyl-4-(3-methylbenzyl)-4H-2,3,4,5,10b-pentaazabenz[e]azulene,
6-(4-chlorophenyl)-1-methyl-4-(4-methylbenzyl)-4H-2,3,4,5,10b-pentaazabenz[e]azulene,
4-(4-tert-butylbenzyl)-6-(4-chlorophenyl)-1-methyl-4H-2,3,4,5,10b-pentaazabenz[e]azulene,
6-(4-chlorophenyl)-1-methyl-4-(naphthalen-1-ylmethyl)-4H-2,3,4,5,10b-pentaazabenz[e]azulene,
6-(4-chlorophenyl)-1-methyl-4-(naphthalen-2-ylmethyl)-4H-2,3,4,5,10b-pentaazabenz[e]azulene,
4-(4-benzyloxybenzyl)-6-(4-chlorophenyl)-1-methyl-4H-2,3,4,5,10b-pentaazabenz[e]azulene,
4-benzyl-6-(4-chlorophenyl)-1-methyl-4H-2,3,4,5,10b-pentaazabenz[e]azulene,
6-(4-chlorophenyl)-1-methyl-4-(4-phenylbenzyl)-4H-2,3,4,5,10b-pentaazabenz[e]azulene,
4-(4-chlorophenoxymethyl)-6-(4-chorophenyl)-1-methyl-4H-2,3,4,5,10b-pentaazabenz[e]azulene,
6-(4-chlorophenyl)-1-methyl-4-(pyridin-2-ylmethyl)-4H-2,3,4,5,10b-pentaazabenz[e]azulene,
6-(4-chlorophenyl)-4-[2-(indol-3-yl)ethyl]-1-methyl-4H-2,3,4,5,10b-pentaazabenz[e]azulene,
6-(4-chlorophenyl)-4-(2-methyl-1,3-thiazol-4-ylmethyl)-1-methyl-4H-2,3,4,5,10b-pentaazabenz[e]azulene,
6-(4-chlorophenyl)-4-(5-chlorothiophen-2-ylmethyl)-1-methyl-4H-2,3,4,5,10b-pentaazabenz[e]azulene,
6-(4-chlorophenyl)-1-methyl-4-(3,5-dimethylisoxazol-4-ylmethyl)-4H-2,3,4,5,10b-pentaazabenz[e]azulene,
6-(4-chlorophenyl)-1-methyl-4-phenethyl-4H-2,3,4,5,10b-pentaazabenz[e]azulene,
6-(4-chlorophenyl)-1-methyl-4-(3-phenylpropyl)-4H-2,3,4,5,10b-pentaazabenz[e]azulene,
6-(4-chlorophenyl)-4-(3,3-diphenylpropyl)-1-methyl-4H-2,3,4,5,10b-pentaazabenz[e]azulene,
6-(4-chlorophenyl)-4-cyclopropylmethyl-1-methyl-4H-2,3,4,5,10b-pentaazabenz[e]azulene,
6-(4-chlorophenyl)-4-cyclohexylmethyl-1-methyl-4H-2,3,4,5,10b-pentaazabenz[e]azulene,
6-(4-chlorophenyl)-4-(2-cyclohexylethyl)-1-methyl-4H-2,3,4,5,10b-pentaazabenz[e]azulene,
6-(4-chlorophenyl)-1-methyl-4-(3-phenyl-2-propenyl)-4H-2,3,4,5,10b-pentaazabenz[e]azulene,
4-allyl-6-(4-chlorophenyl)-1-methyl-4H-2,3,4,5,10b-pentaazabenz[e]azulene,
6-(4-chlorophenyl)-1-methyl-4-(2-methyl-2-propenyl)-4H-2,3,4,5,10b-pentaazabenz[e]azulene,
6-(4-chlorophenyl)-4-(2-chloro-2-propenyl)-1-methyl-4H-2,3,4,5,10b-pentaazabenz[e]azulene,

4-(2-bromo-2-propenyl)-6-(4-chlorophenyl)-1-methyl-4H-2,3,4,5,10b-pentaazabenz[e]azulene,
6-(4-chlorophenyl)-4-(2,3-dichloro-2-propenyl)-1-methyl-4H-2,3,4,5,10b-pentaazabenz[e]azulene,
6-(4-chlorophenyl)-4-(3,4-dibenzyloxybenzyl)-1-methyl-4H-2,3,4,5,10b-pentaazabenz[e]azulene,
4-benzyloxymethyl-6-(4-chlorophenyl)-1-methyl-4H-2,3,4,5,10b-pentaazabenz[e]azulene,
6-(4-chlorophenyl)-1-methyl-4-(3-phenoxypropyl)-4H-2,3,4,5,10b-pentaazabenz[e]azulene,
6-(4-chlorophenyl)-4-(3,3-dichloro-2-propenyl)-1-methyl-4H-2,3,4,5,10b-pentaazabenz[e]azulene,
6-(4-chlorophenyl)-4-(4-methoxy-3-methylbenzyl)-1-methyl-4H-2,3,4,5,10b-pentaazabenz[e]azulene,
6-(4-chlorophenyl)-4-(3,4-dichlorobenzyl)-1-methyl-4H-2,3,4,5,10b-pentaazabenz[e]azulene,
6-(4-chlorophenyl)-1-methyl-4-(pyridin-4-ylmethyl)-4H-2,3,4,5,10b-pentaazabenz[e]azulene,
6-(4-chlorophenyl)-1-methyl-4-(4-methylsulfonylbenzyl)-4H-2,3,4,5,10b-pentaazabenz[e]azulene,
6-(4-chlorophenyl)-1-methyl-4-(4-nitrobenzyl)-4H-2,3,4,5,10b-pentaazabenz[e]azulene,
6-(4-chlorophenyl)-4-(2,6-dichloropyridin-4 ylmethyl)-1-methyl-4H-2,3,4,5,10b-pentaazabenz[e]azulene,
6-(4-chlorophenyl)-4-(2,2,2-trifluoroethyl)-1-methyl-4H-2,3,4,5,10b-pentaazabenz[e]azulene,
6-(4-chlorophenyl)-4-(3,5-dinitrobenzyl)-1-methyl-4H-2,3,4,5,10b-pentaazabenz[e]azulene,
8-chloro-1-methyl-6-phenyl-4-(pyridin-4-ylmethyl)-4H-2,3,4,5,10b-pentaazabenz[e]azulene,
8-chloro-1-methyl-6-phenyl-4-(pyridin-3-ylmethyl)-4H-2,3,4,5,10b-pentaazabenz[e]azulene,
1-methyl-6-phenyl-4-(pyridin-3-ylmethyl)-4H-2,3,4,5,10b-pentaazabenz[e]azulene,
1-methyl-6-phenyl-4-(pyridin-4-ylmethyl)-4H-2,3,4,5,10b-pentaazabenz[e]azulene,
1,9-dimethyl-6-phenyl-4-(pyridin-3-ylmethyl)-4H-2,3,4,5,10b-pentaazabenz[e]azulene,
1,9-dimethyl-6-phenyl-4-(pyridin-4-ylmethyl)-4H-2,3,4,5,10b-pentaazabenz[e]azulene,
4-(3-cyanobenzyl)-1,9-dimethyl-6-phenyl-4H-2,3,4,5,10b-pentaazabenz[e]azulene,
4-(4-cyanobenzyl)-1,9-dimethyl-6-phenyl-4H-2,3,4,5,10b-pentaazabenz[e]azulene,
4-(3,4-dichlorobenzyl)-1,9-dimethyl-6-phenyl-4H-2,3,4,5,10b-pentaazabenz[e]azulene,
1-methyl-8-nitro-6-phenyl-4-(pyridin-3-ylmethyl)-4H-2,3,4,5,10b-pentaazabenz[e]azulene,
1-methyl-8-nitro-6-phenyl-4-(pyridin-4-ylmethyl)-4H-2,3,4,5,10b-pentaazabenz[e]azulene,
1-methyl-6-(4-methylphenyl)-4-(pyridin-4-ylmethyl)-4H-2,3,4,5,10b-pentaazabenz[e]azulene,
4-(3-cyanobenzyl)-1-methyl-6-(4-methylphenyl)-4H-2,3,4,5,10b-pentaazabenz[e]azulene,
8-chloro-6-(2-chlorophenyl)-1-methyl-4-(pyridin-3-ylmethyl)-4H-2,3,4,5,10b-pentaazabenz[e]azulene,
8-chloro-6-(2-chlorophenyl)-1-methyl-4-(pyridin-4-ylmethyl)-4H-2,3,4,5,10b-pentaazabenz[e]azulene,
methyl 4-[6-(4-chlorophenyl)-1-methyl-4H-2,3,4,5,10b-pentaazabenz[e]azulen-4-ylmethyl]benzoate,
6-(4-chlorophenyl)-4-(4-cyanobenzyl)-1,9-dimethyl-4H-2,3,4,5,10b-pentaazabenz[e]azulene,
4-(4-bromobenzyl)-6-(4-bromophenyl)-1-methyl-4H-2,3,4,5,10b-pentaazabenz[e]azulene,
4-(4-cyanobenzyl)-1-methyl-6-phenyl-4H-2,3,4,5,10b-pentaazabenz[e]azulene,
4-[6-(4-chlorophenyl)-1-methyl-4H-2,3,4,5,10b-pentaazabenz[e]azulen-4-ylmethyl]benzoic acid,
4-(4-chlorophenyl)-2,3,9-trimethyl-6-(pyridin-3-ylmethyl)-6H-5,6,7,8,9a-pentaazathieno[2,3-e]azulene,
4-(4-chlorophenyl)-2,3,9-trimethyl-6-(pyridin-4-ylmethyl)-6H-5,6,7,8,9a-pentaazathieno[2,3-e]azulene,
4-(4-chlorophenyl)-6-(4-cyanobenzyl)-2,3,9-trimethyl-6H-5,6,7,8,9a-pentaazathieno[2,3-e]azulene,
4-(4-chlorophenyl)-6-(3,4-difluorobenzyl)-2,3,9-trimethyl-6H-5,6,7,8,9a-pentaazathieno[2,3-e]azulene,
4-(4-chlorophenyl)-2-ethyl-9-methyl-6-(pyridin-3-ylmethyl)-6H-5,6,7,8,9a-pentaazathieno[2,3-e]azulene,
4-(4-chlorophenyl)-2-ethyl-9-methyl-6-(pyridin-4-ylmethyl)-6H-5,6,7,8,9a-pentaazathieno[2,3-e]azulene,
4-(4-chlorophenyl)-6-(4-cyanobenzyl)-2-ethyl-9-methyl-6H-5,6,7,8,9a-pentaazathieno[2,3-e]azulene,
2,9-dimethyl-4-phenyl-6-(pyridin-3-ylmethyl)-6H-5,6,7,8,9a-pentaazathieno[2,3-e]azulene,
2,9-dimethyl-4-phenyl-6-(pyridin-4-ylmethyl)-6H-5,6,7,8,9a-pentaazathieno[2,3-e]azulene,
6-(4-cyanobenzyl)-2,9-dimethyl-4-phenyl-6H-5,6,7,8,9a-pentaazathieno[2,3-e]azulene,
6-(4-chlorobenzyl)-2,9-dimethyl-4-phenyl-6H-5,6,7,8,9a-pentaazathieno[2,3-e]azulene,
2,3,9-trimethyl-4-phenyl-6-(pyridin-3-ylmethyl)-6H-5,6,7,8,9a-pentaazathieno[2,3-e]azulene,
2,3,9-trimethyl-4-phenyl-6-(pyridin-4-ylmethyl)-6H-5,6,7,8,9a-pentaazathieno[2,3-e]azulene,
2-ethyl-9-methyl-4-phenyl-6-(pyridin-3-ylmethyl)-6H-5,6,7,8,9a-pentaazathieno[2,3-e]azulene,
2-ethyl-9-methyl-4-phenyl-6-(pyridin-4-ylmethyl)-6H-5,6,7,8,9a-pentaazathieno[2,3-e]azulene,
4-(4-methoxyphenyl)-2,3,9-trimethyl-6-(pyridin-3-ylmethyl)-6H-5,6,7,8,9a-pentaazathieno[2,3-e]azulene,
4-(4-methoxyphenyl)-2,3,9-trimethyl-6-(pyridin-4-ylmethyl)-6H-5,6,7,8,9a-pentaazathieno[2,3-e]azulene,
6-(4-cyanobenzyl)-4-(4-methoxyphenyl)-2,3,9-trimethyl-6H-5,6,7,8,9a-pentaazathieno[2,3-e]azulene,
4-(4-chlorophenyl)-6-(4-fluorobenzyl)-2,3,9-trimethyl-6H-5,6,7,8,9a-pentaazathieno[2,3-e]azulene,
6-(4-chlorobenzyl)-4-(4-chlorophenyl)-2,3,9-trimethyl-6H-5,6,7,8,9a-pentaazathieno[2,3-e]azulene,
4-(4-chlorophenyl)-6-(3,4-dichlorobenzyl)-2,3,9-trimethyl-6H-5,6,7,8,9a-pentaazathieno[2,3-e]azulene,
4-(4-chlorophenyl)-6-(3,4-dichlorobenzyl)-2-ethyl-9-methyl-6H-5,6,7,8,9a-pentaazathieno[2,3-e]azulene,
6-(4-chlorophenyl)-1-methyl-4-(2-nitrobenzyl)-4H-2,3,4,5,10b-pentaazabenz[e]azulene,
6-(4-chlorophenyl)-4-ethoxycarbonylmethyl-1-methyl-4H-2,3,4,5,10b-pentaazabenz[e]azulene,

[6-(4-chlorophenyl)-1-methyl-4H-2,3,4,5,10b-pentaazabenz[e]azulen-4-yl]acetic acid,
6-(4-chlorophenyl)-1-methyl-4-phenylcarbamoylmethyl-4H-2,3,4,5,10b-pentaazabenz[e]azulene,
6-(4-chlorophenyl)-1-methyl-4-(4-methylphenylcarbamoylmethyl)-4H-2,3,4,5,10b-pentaazabenz[e]azulene,
6-(4-chlorophenyl)-4-(2-methoxyphenylcarbamoylmethyl)-1-methyl-4H-2,3,4,5,10b-pentaazabenz[e]azulene,
6-(4-chlorophenyl)-4-(2,5-dimethoxyphenylecarbamoylmethyl)-1-methyl-4H-2,3,4,5,10b-pentaazabenz[e]azulene,
4-(4-chloro-2,5-dimethoxyphenylcarbamoylmethyl)-6-(4-chlorophenyl)-1-methyl-4H-2,3,4,5,10b-pentaazabenz[e]azulene,
6-(4-chlorophenyl)-1-methyl-4-(naphthalen-1-ylcarbamoylmethyl)-4H-2,3,4,5,10b-pentaazabenz[e]azulene,
6-(4-chlorophenyl)-1-methyl-4-(pyridin-3-ylcarbamoylmethyl)-4H-2,3,4,5,10b-pentaazabenz[e]azulene,
6-(4-chlorophenyl)-4-(cyclohexylcarbamoylmethyl)-1-methyl-4H-2,3,4,5,10b-pentaazabenz[e]azulene,
6-(4-chlorophenyl)-1-methyl-4-n-propylcarbamoylmethyl-4H-2,3,4,5,10b-pentaazabenz[e]azulene,
4-bromoacetyl-6-(4-chlorophenyl)-1-methyl-4H-2,3,4,5,10b-pentaazabenz[e]azulene,
6-(4-chlorophenyl)-4-(2-methoxyphenylaminoacetyl)-1-methyl-4H-2,3,4,5,10b-pentaazabenz[e]azulene,
6-(4-chlorophenyl)-1-methyl-4-phenylaminoacetyl-4H-2,3,4,5,10b-pentaazabenz[e]azulene,
6-(4-chlorophenyl)-1-methyl-4-(4-methylphenylaminoacetyl)-4H-2,3,4,5,10b-pentaazabenz[e]azulene,
6-(4-chlorophenyl)-4-(3-fluorophenylaminoacetyl)-1-methyl-4H-2,3,4,5,10b-pentaazabenz[e]azulene,
6-(4-chlorophenyl)-4-(2,5-dimethoxyphenylaminoacetyl)-1-methyl-4H-2,3,4,5,10b-pentaazabenz[e]azulene,
6-(4-chlorophenyl)-1-methyl-4-phenylthioacetyl-4H-2,3,4,5,10b-pentaazabenz[e]azulene,
6-(4-chlorophenyl)-1-methyl-4-phenylacetyl-4H-2,3,4,5,10b-pentaazabenz[e]azulene,
6-(4-chlorophenyl)-1-methyl-4-phenyloxalyl-4H-2,3,4,5,10b-pentaazabenz[e]azulene,
6-(4-chlorophenyl)-4-ethoxymethyl-1-methyl-4H-2,3,4,5,10b-pentaazabenz[e]azulene,
N-[6-(4-chlorophenyl)-1-methyl-4H-2,3,4,5,10b-pentaazabenz[e]azulen-4-ylmethyl]-N-phenyl-amine,
4-benzylcarbamoyl-6-(4-chlorophenyl)-1-methyl-4H-2,3,4,5,10b-pentaazabenz[e]azulene,
6-(4-chlorophenyl)-1-methyl-4-(3-methylphenylcarbamoyl)-4H-2,3,4,5,10b-pentaazabenz[e]azulene,
6-(4-chlorophenyl)-(4-hydroxybenzyl)-1-methyl-4H-2,3,4,5,10b-pentaazabenz[e]azulene,
6-(4-chlorophenyl)-4-(3,4-dihydroxybenzyl)-1-methyl-4H-2,3,4,5,10b-pentaazabenz[e]azulene,
6-(4-chlorophenyl)-4-(4-ethoxybenzyl)-1-methyl-4H-2,3,4,5,10b-pentaazabenz[e]azulene,
6-(4-chlorophenyl)-1-methyl-4-(4-methylsulfonylphenyl)hydroxymethyl-4H-2,3,4,5,10b-pentaazabenz[e]azulene,
4-(4-aminobenzyl)-6-(4-chlorophenyl)-1-methyl-4H-2,3,4,5,10b-pentaazabenz[e]azulene,
6-(4-chlorophenyl)-4-(4-formylaminobenzyl)-1-methyl-4H-2,3,4,5,10b-pentaazabenz[e]azulene,
4-(4-acetylananobenzyl)-6-(4-chlorophenyl)-1-methyl-4H-2,3,4,5,10b-pentaazabenz[e]azulene,
6-(4-chlorophenyl)-4-(4-methylsulfonylaminobenzyl)-1-methyl-4H-2,3,4,5,10b-pentaazabenz[e]azulene,
4-[4-bis(methylsulfonyl)aminobenzyl]-6-(4-chlorophenyl)-4H-2,3,4,5,10b-pentaazabenz[e]azulene,
6-(4-chlorophenyl)-4-(4-dimethylaminobenzyl)-1-methyl-4H-2,3,4,5,10b-pentaazabenz[e]azulene,
6-(4-chlorophenyl)-4-(2-hydroxy-2-phenylethyl)-1-methyl-4H-2,3,4,5,10b-pentaazabenz[e]azulene,
6-(4-chlorophenyl)-4-(2-oxo-2-phenylethyl)-1-methyl-4H-2,3,4,5,10b-pentaazabenz[e]azulene,
6-(4-chlorophenyl)-1-methyl-4-[3-phenyl-2-(tetrahydropyan-2-yloxy)propyl]-4H-2,3,4,5,10b-pentaazabenz[e]azulene,
6-(4-chlorophenyl)-4-[2(2-methoxyphenyl)-2-(tetrahydropyran-2-yloxy)ethyl]-1-methyl-4H-2,3,4,5,10b-pentaazabenz[e]azulene,
6-(4-chlorophenyl)-4-(2-hydroxy-3-phenylpropyl)-1-methyl-4H-2,3,4,5,10b-pentaazabenz[e]azulene,
6-(4-chlorophenyl)-4-(2-oxo-3-phenylpropyl)-1-methyl-4H-2,3,4,5,10b-pentaazabenz[e]azulene,
6-(4-chlorophenyl)-4-[2-(4-chlorophenyl)-2-oxoethyl]-1-methyl-4H-2,3,4,5,10b-pentaazabenz[e]azulene,
6-(4-chlorophenyl)-1-methyl-4-[2-(4-methylphenyl)-2-oxoethyl]-4H-2,3,4,5,10b-pentaazabenz[e]azulene,
6-(4-chlorophenyl)-4-[2-(2-methoxyphenyl)-2-oxoethyl]-1-methyl-4H-2,3,4,5,10b-pentaazabenz[e]azulene,
6-(4-chlorophenyl)-4-[2-(2,5-dimethoxyphenyl)-2-oxoethyl]-1-methyl-4H-2,3,4,5,10b-pentaazabenz[e]azulene,
6-(4-chlomphenyl)-4-[3-(2-methoxyphenyl)-2-oxopropyl]-1-methyl-4H-2,3,4,5,10b-pentaazabenz[e]azulene,
6-(4-chlorophenyl)-4-[3(2,5-dimethoxyphenyl)-2-oxopropyl]-1-methyl-4H-2,3,4,5,10b-pentaazabenz[e]azulene,
4-benzyl-6-(4-chlorophenyl)-1-methyl-4H-2,3,4,5,10b-pentaazabenz[e]azulene,
6-(4-chlorophenyl)-4-(4-methoxybenzyl)-4H-3,4,5,10b-tetraazabenz[e]azulene,
3-[6-(4-chlorophenyl)-1-methyl-4H-2,3,4,5,10b-pentaazabenz[e]azulen-4-ylmethyl]-pyridine-1-oxide,
3-[8-chloro-6-(2-chlorophenyl)-1-methyl-4H-2,3,4,5,10b-pentaazabenz[e]azulen-4-ylmethyl]-pyridine-1-oxide,
4-[4-(4-chlorophenyl)-2,3,9-trimethyl-6H-1-thia-5,6,7,8,9a-pentaazacyclopent[e]azulen-6-ylmethyl]-pyridine-1-oxide,
4-[4(4-chlorophenyl)-2-ethyl-9-methyl-6H-1-thia-5,6,7,8,9a-pentaazacyclopent[e]azulen-6-ylmethyl]-pyridine-1-oxide,
4-[2,9-dimethyl-4-phenyl-6H-1-thia-5,6,7,8,9a-pentaazacyclopent[e]azulen-6-ylmethyl]-pyridine-1-oxide,
methyl 4-[4-(4-chlorobenzyl)-1-methyl-4H-2,3,4,5,10b-pentaazabenz[e]azulen-6-yl]benzoate,
methyl 4-[4-(4-cyanobenzyl)-1-methyl-4H-2,3,4,5,10b-pentaazabenz[e]azulen-6-yl]benzoate,
methyl 4-[1-methyl-4-(4-nitrobenzyl)-4H-2,3,4,5,10b-pentaazabenz[e]azulen-6-yl]benzoate,
6-(4-chlorophenyl)-1-methyl-4-(4-nitrobenzyl)-4H-2,3,4,5,10,10b-hexaazabenz[e]azulene,
[4-(4-chlorobenzyl)-1-methyl-4H-2,3,4,5,10b-pentaazabenz[e]azulen-6-yl]benzoic acid,
[4-(4-cyanobenzyl)-1-methyl-4H-2,3,4,5,10b-pentaazabenz[e]azulen-6-yl]benzoic acid,
4-[1-methyl-4-(4-nitrobenzyl)-4H-2,3,4,5,10b-pentaazabenz[e]azulen-6-yl]benzoic acid,
tert-butyl 4-[4-(4-chlorobenzyl)-1-methyl-4H-2,3,4,5,10b-pentaazabenz[e]azulen-6-yl]phenylcarbamate,
tert-butyl 4-[4-(4-cyanobenzyl)-1-methyl-4H-2,3,4,5,10b-pentaazabenz[e]azulen-6-yl]phenylcarbamate,
tert-butyl 4-[1-methyl-4-(4-nitrobenzyl)-4H-2,3,4,5,10b-pentaazabenz[e]azulen-6-yl]phenylcarbamate,
4-[4-(4-chlorobenzyl)-1-methyl-4H-2,3,4,5,10b-pentaazabenz[e]azulen-6-yl]phenylamine,
4-[4-(4-cyanobenzyl)-1-methyl-4H-2,3,4,5,10b-pentaazabenz[e]azulen-6-yl]phenylamine,
4-[1-methyl-4-(4-nitrobenzyl)-4H-2,3,4,5,10b-pentaazabenz[e]azulen-6-yl]phenylamine,
4-(4-chlorobenzyl)-1-methyl-6-(4-nitrophenyl)-4H-2,3,4,5,10b-pentaazabenz[e]azulene,
2,9-dimethyl-4-phenyl-6-[4-(1H-tetrazol-5-yl)benzyl]-6H-5,6,7,8,9a-pentaazathieno[2,3-e]azulene,
2,9-dimethyl-4-phenyl-6-[4-(1-methyl-1H-tetrazol-5-yl)benzyl]-6H-5,6,7,8,9a-pentaazathieno[2,3-e]azulene,
and
2,9-dimethyl-4-phenyl-6-[4-(2-methyl-2H-tetrazol-5-yl)benzyl]-6H-5,6,7,8,9a-pentaazathieno[2,3-e]azulene,

or a pharmaceutically acceptable salt thereof.
(5) The cytokine production inhibitor of (1), comprising as an active ingredient, a compound of the formula [I] wherein B is

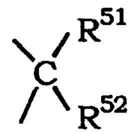

namely, a compound of the formula [I"]

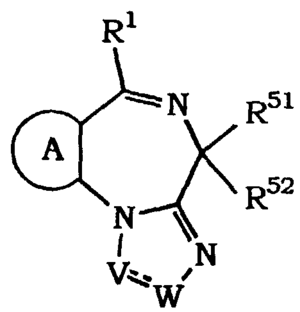

[I"]

wherein $R^1$, $R^{51}$, $R^{52}$, A, V and W are as defined in (1),
or a pharmaceutically acceptable salt thereof.
(6) The cytokine production inhibitor of (5), wherein, in the formula [I"], the ring A is

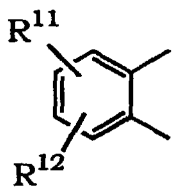

or

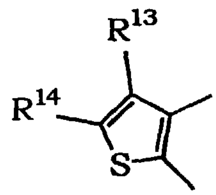

wherein $R^{11}$, $R^{12}$, $R^{13}$ and $R^{14}$ are as defined in (1),

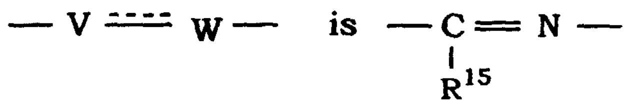

wherein $R^{15}$ is lower alkyl,
$R^{51}$ is hydrogen atom, optionally substituted aryl, optionally substituted heteroaryl, aralkyl, heteroarylalkyl or a group of the formula:

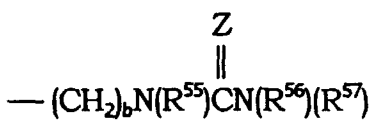 (3)

$-(CH_2)_bN(R^{55})CORa^{56}$ (4)

$-(CH_2)_bN(R^{55})COOR^{59}$ (6) or

$-(CH_2)_bCON(R^{61})(R^{62})$ (9)

wherein b, Z, $R^{55}$, $R^{56}$, $Ra^{56}$,$R^{57}$,$R^{59}$, $R^{61}$, and $R^{62}$ are as defined in (1), and $R^{52}$ is hydrogen atom or -COOR$^{53}$ wherein $R^{53}$ is as defined in (1), or $R^{51}$ and $R^{52}$ in combination form, together with the carbon atom they bind with, a spiro ring of the formula

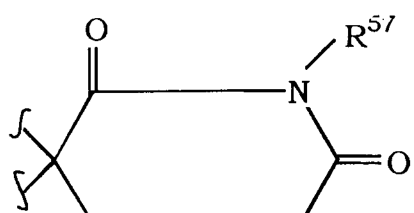

wherein b', $R^{55}$ and $R^{57}$ are as defined in (1).

(7) The cytokine production inhibitor of (5), comprising, as an active ingredient, a compound selected from the group consisting of:

2-[6-(4-chlorophenyl)-1-methyl-4H-[1,2,4]triazolo[4,3-a][1,4]benzodiazepin-4-yl]-N-phenyl-acetamide,
6-(4-chlorophenyl)-1-methyl-4H-[1,2,4]triazolo[4,3-a][1,4]benzodiazepine-4-spiro-5'-[3'-(2,5-dimethoxyphenyl)-2',4'-dioxoimidazolidine],
6-(4-chlorophenyl)-1-methyl-4H-[1,2,4]triazolo[4,3-a][1,4]benzodiazepine-4-spiro-5'-(3'-phenyl-2',4'-dioxoimidazolidine),
1-(3-methylphenyl)-3-[1-methyl-6-(thiophen-2-yl)-4H-[1,2,4]thiazolo[4,3-a][1,4]benzodiazepin-4-yl]urea,
1-[6-(4-chlorophenyl)-4-ethoxycarbonyl-1-methyl-4H-[1,2,4]triazolo[4,3-a][1,4]benzodiazepin-4-yl]-3-(2,5-dimethoxyphenyl)urea,
benzyl [6-(4-chlorophenyl)-1-methyl-4H-[1,2,4]triazolo[4,3-a][1,4]benzodiazepin-4-yl]carbamate,
1-[6-(4-chlorophenyl)-1-methyl-4H-[1,2,4]triazolo[4,3-a][1,4]benzodiazepin-4-yl]methyl-3-(3-methylphenyl)urea,
1-[6-(4-chlorophenyl)-1-methyl-4H-[1,2,4]triazolo[4,3-a][1,4]benzodiazepin-4-yl]-3-(3-pyridyl)urea,
1-[6-(4-chlorophenyl)-1-methyl-4H-[1,2,4]triazolo[4,3-a][1,4]benzodiazepin-4-yl]-3-cyclohexylurea,
1-[6-(4-chlorophenyl)-1-methyl-4H-[1,2,4]triazolo[4,3-a][1,4]benzodiazepin-4-yl]-3-(2,5-dimethoxyhenyl)urea,
N-[6-(4-chlorophenyl)-1-methyl-4H-[1,2,4]triazolo[4,3-a][1,4]benzodiazepin-4-yl]indole-2-carboxamide,
6-(4-chlorophenyl)-4-(indol-3-ylmethyl)-1-methyl-4H-[1,2,4]triazolo[4,3-a][1,4]benzodiazepine,
2-[6-(4-chlorophenyl)-1-methyl-4H-[1,2,4]triazolo[4,3-a][1,4]benzodiazepin-4-yl]-N-pyridin-2-yl-acetamide,
2-[6-(4-chlorophenyl)-1-methyl-4H-[1,2,4]triazolo[4,3-a][1,4]benzodiazepin-4-yl]-N-pyridin-3-yl-acetamide,
2-[6-(4-chlorophenyl)-1-methyl-4H-[1,2,4]triazolo[4,3-a][1,4]benzodiazepin-4-yl]-N-pyridin-4-yl-acetamide, and
2-[6-(4-chlorophenyl)-1-methyl-4H-[1,2,4]triazolo[4,3-a][1,4]benzodiazepin-4-yl]-N-(2,5-dimethoxyphenyl)-acetamide,

or a pharmaceutically acceptable salt thereof.
(8) of cytokine production inhibitor of any of (1) to (7), wherein the cytokine is interleukin 6.
(9) The cytokine production inhibitor of any of (1) to (7), when the cytokine is TNF-$\alpha$.
(10) The cytokine production inhibitor of any of (1) to (7), wherein the cytokine is interleukin 8.
(11) The cytokine production inhibitor of any of (1) to (7), wherein the cytokine is interferon $\gamma$.
(12) The cytokine production inhibitor of any of (1) to (7), wherein the cytokine is interleukin 2.
(13) The cytokine production inhibitor of any of (1) to (7), wherein the cytokine is GM-CSF.
(14) An antiinflammatory agent comprising a compound of any of (1) to (7), or a pharmaceutically acceptable salt thereof, as an active ingredient.
(15) A triazepine compound of the formula [I''']

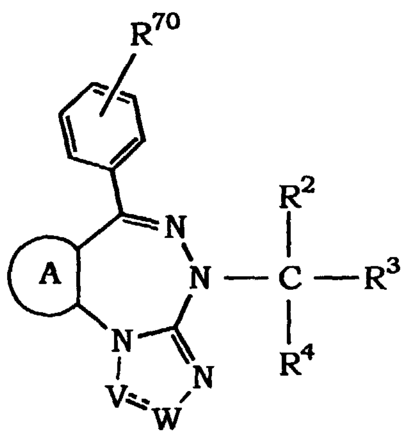

[I''']

wherein $R^{70}$ is -COOR$^{71}$ wherein $R^{71}$ is hydrogen atom or lower alkyl, or -NHCOOR$^{72}$ wherein $R^{72}$ is lower alkyl, and $R^2$, $R^3$, $R^4$, ring A, V and W are as defined in (1),
or a pharmaceutically acceptable salt thereof.
(16) The triazepine compound of (15), wherein, in the formula [I'''], the ring A is

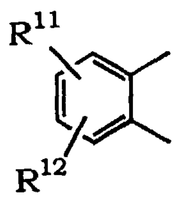

or

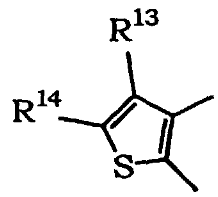

wherein, $R^{11}$, $R^{12}$, $R^{13}$ and $R^{14}$ are as defined in (1),

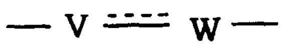

is

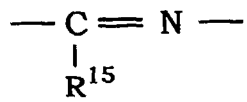

wherein $R^{15}$ is lower alkyl, and $R^2$ and $R^4$ are both hydrogen atoms, or a pharmaceutically acceptable salt thereof.

(17) The triazepine compound of (15),which is a member selected from the group consisting of:

tert-butyl 4-[4-(4-methoxybenzyl]-1-methyl-4H-2,3,4,5,10b-pentaazabenz[e]azulen-6-yl]benzoate,
methyl 4-[4-(4-chlorobenzyl)-1-methyl-4H-2,3,4,5,10b-pentaazabenz[e]azulen-6-yl]benzoate,
methyl 4-[4-(4-cyanobenzyl)-1-methyl-4H-2,3,4,5,10b-pentaazabenz[e]azulen-6-yl]benzoate,
methyl 4-[1-methyl-4-(4-nitrobenzyl)-4H-2,3,4,5,10b-pentaazabenz[e]azulen-6-yl]benzoate,
[4-(4-chlorobenzyl)-1-methyl-4H-2,3,4,5,10b-pentaazabenz[e]azulen-6-yl]benzoic acid,
[4-(4-cyanobenzyl)-1-methyl-4H-2,3,4,5,10b-pentaazabenz[e]azulen-6-yl]benzoic acid,
4-[1-methyl-4-(4-nitrobenzyl)-4H-2,3,4,5,10b-pentaazabenz[e]azulen-6-yl]benzoic acid,
tert-butyl 4-[4-(4-chlorobenzyl)-1-methyl-4H-2,3,4,5,10b-pentaazabenz[e]azulen-6-yl]phenylcarbamate,
tert-butyl 4-[4-(4-cyanobenzyl)-1-methyl-4H-2,3,4,5,10b-pentaazabenz[e]azulen-6-yl]phenylcarbamate, and
tert-butyl 4-[1-methyl-4-(4-nitrobenzyl)-4H-2,3,4,5,10b-pentaazabenz[e]azulen-6-yl]phenylcarbamate,

or a pharmaceutically acceptable salt thereof.

(18) A triazepine compound of the formula [I""]

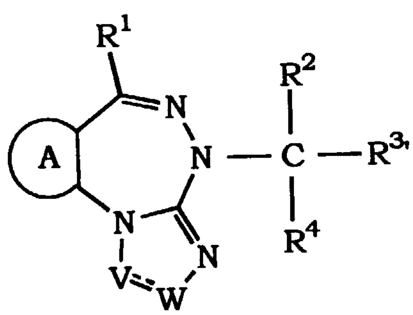

[I""]

wherein $R^{3'}$ is pyridine-1-oxide or phenyl substituted by tetrazolyl or alkyltetrazolyl, and $R^1$, $R^2$, $R^4$, ring A, V and W are as defined in (1), or a pharmaceutically acceptable salt thereof.

(19) The triazepine compound of (18), wherein, in the formula [I""], the ring A is

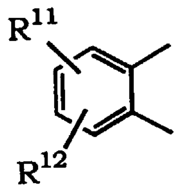

or

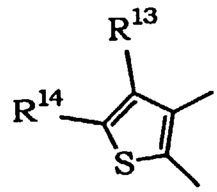

wherein, $R^{11}$, $R^{12}$, $R^{13}$ and $R^{14}$ are as defined in (1),

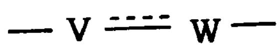 is 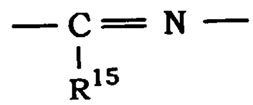

wherein $R^{15}$ is lower alkyl, and $R^2$ and $R^4$ are both hydrogen atoms,
or a pharmaceutically acceptable salt thereof.
(20) The triazepine compound of (18), which is a member selected from the group consisting of:

3-[6-(4-chlorophenyl)-1-methyl-4H-2,3,4,5,10b-pentaazabenz[e]azulen-4-ylmethyl]-pyridine-1-oxide,
3-[8-chloro-6-(2-chlorophenyl)-1-methyl-4H-2,3,4,5,10b-pentaazabenz[e]azulen-4-ylmethyl]-pyridine-1-oxide,
4-[4-(4-chlorophenyl)-2,3,9-trimethyl-6H-1-thia-5,6,7,8,9a-pentaazacyclopent[e]azulen-6-ylmethyl]-pyridine-1-oxide,
4-[4-(4-chlorophenyl)-2-ethyl-9-methyl-6H-1-thia-5,6,7,8,9a-pentaazacyclopent[e]azulen-6-ylmethyl]-pyridine-1-oxide,
4-[2,9-dimethyl-4-phenyl-6H-1-thia-5,6,7,8,9a-pentaazacyclopent[e]azulen-6-ylmethyl]-pyridine-1-oxide,
2,9-dimethyl-4-phenyl-6-[4-(1H-tetrazol-5-yl)benzyl]-6H-5,6,7,8,9a-pentaazathieno[2,3-e]azulene,
2,9-dimethyl-4-phenyl-6-[4-(1-methyl-1H-tetrazol-5-yl)benzyl]-6H-5,6,7,8,9a-pentaazathieno[2,3-e]azulene and
2,9-dimethyl-4-phenyl-6-[4-(2-methyl-2H-tetrazol-5-yl)benzyl]-6H-5,6,7,8,9a-pentaazathieno[2,3-e]azulene,

or a pharmaceutically acceptable salt thereof.
(21) A pharmaceutical composition comprising the triazepine compound of any of (15) to (17) or a pharmaceutically acceptable salt thereof, and a pharmaceutically acceptable carrier.
(22) A pharmaceutical composition comprising the triazepine compound of any of (18) to (20) or a pharmaceutically acceptable salt thereof, and a pharmaceutically acceptable carrier.
(23) A triazepinethione compound of the formula [II']

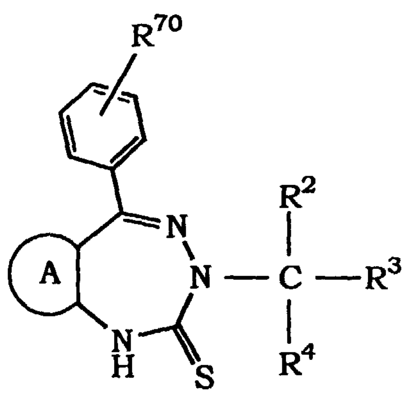

[II']

wherein $R^{70}$ is $-COOR^{71}$ wherein $R^{71}$ is hydrogen atom or lower alkyl, or $-NHCOOR^{72}$ wherein $R^{72}$ is lower alkyl, and $R^2$, $R^3$, $R^4$ and ring A are as defined in (1),

or a salt thereof.

(24) The triazepinethione compound of (23), wherein the ring A is

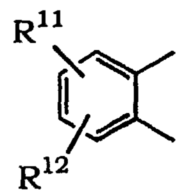

or

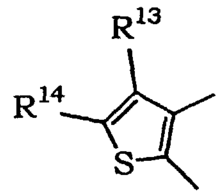

wherein, $R^{11}$, $R^{12}$, $R^{13}$ and $R^{14}$ are as defined in (1), and $R^2$ and $R^4$ are both hydrogen atoms, or a salt thereof.

(25) An alkylthiotriazepine compound of the formula [III']

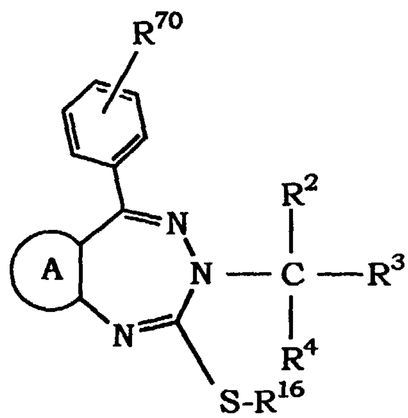

[III']

wherein $R^{16}$ is lower alkyl, $R^{70}$ is-$COOR^{71}$ wherein $R^{71}$ is hydrogen atom or lower alkyl, or -$NHCOOR^{72}$ wherein $R^{72}$ is lower alkyl, and $R^2$, $R^3$, $R^4$ and ring A are as defined in (1), or a salt thereof.

(26) The alkylthiotriazepine compound of (25), wherein the ring A is

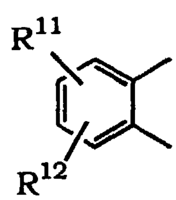

or

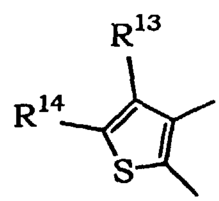

wherein, $R^{11}$, $R^{12}$, $R^{13}$ and $R^{14}$ are as defined in (1), and $R^2$ and $R^4$ are both hydrogen atoms, or a salt thereof.

(27) A triazepinethione compound of the formula [II'']

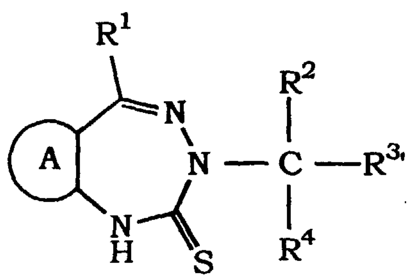

[II'']

wherein $R^{3'}$ is pyridine-1-oxide or phenyl substituted by tetrazolyl or alkyltetrazolyl, and $R^1$, $R^2$, $R^4$ and ring A are

as defined in (1),
or a salt thereof.

(28) The triazepinethione compound of (27), wherein the ring A is

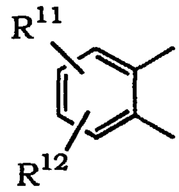

or

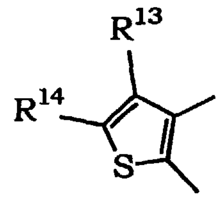

wherein, $R^{11}$, $R^{12}$, $R^{13}$ and $R^{14}$ are as defined in (1), and $R^2$ and $R^4$ are both hydrogen atoms,
or a salt thereof.

(29) An alkylthiotriazepine compound of the formula [III"]

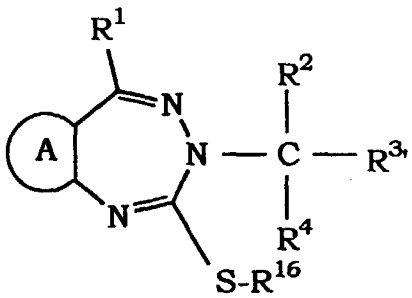

[III"]

wherein $R^{3'}$ is pyridine-1-oxide or phenyl substituted by tetrazolyl or alkyltetrazolyl, $R^{16}$ is lower alkyl, and $R^1$, $R^2$, $R^4$ and ring A are as defined in (1),
or a salt thereof.

(30) The alkylthiotriazepine compound of (29), wherein the ring A is

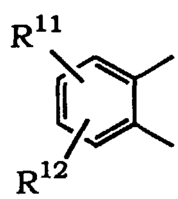

or

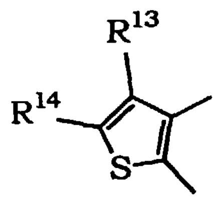

wherein, $R^{11}$, $R^{12}$, $R^{13}$ and $R^{14}$ are as defined in (1), and $R^2$ and $R^4$ are both hydrogen atoms,
or a salt thereof.

(31) A method for the prophylaxis or treatment of diseases caused by abnormal production of cytokine, comprising administering an effective amount of the compound of the formula [I] of (1) or a pharmaceutically acceptable salt thereof.

(32) A method for the prophylaxis or treatment of inflammation, comprising administering an effective amount of the compound of the formula [I] of (1) or a pharmaceutically acceptable salt thereof.

(33) A use of the compound of the formula [I] of (1) or a pharmaceutically acceptable salt thereof for the production of a cytokine production inhibitor.

(34) A use of the compound of the formula [I] of (1) or a pharmaceutically acceptable salt thereof for the production of an antiinflammatory drug.

(35) A pharmaceutical composition for inhibiting cytokine production, comprising an effective amount of the compound of the formula [I] of (1) or a pharmaceutically acceptable salt thereof and a pharmaceutically acceptable carrier.

(36) A commercial package comprising the pharmaceutical composition of (35) and a written matter associated therewith, the written matter stating that said composition can or should be used for suppressing the production of

cytokine.

(37) A pharmaceutical composition for treating inflammation, comprising an effective amount of the compound of the formula [I] of (1) or a pharmaceutically acceptable salt thereof and a pharmaceutically acceptable carrier.

(38) A commercial package comprising the pharmaceutical composition of (37) and a written matter associated therewith, the written matter stating that said composition can or should be used for treating inflammation.

The definitions of the respective substituents to be used in the present specification are as follows.

Optionally substituted aryl means phenyl, naphthyl or biphenylyl which may have 1 to 3 substituents on the ring. Examples of the substituent include halogen atom (e.g., chlorine, bromine, fluorine and iodine), lower alkyl (e.g., alkyl having 1 to 6 carbon atoms, such as methyl, ethyl, propyl, isopropyl, butyl, tert-butyl, isobutyl, pentyl, isopentyl, hexyl and the like), lower alkoxy (e.g., alkoxy having 1 to 6 carbon atoms, such as methoxy, ethoxy, propoxy, isopropoxy, butoxy, tert-butoxy, hexyloxy and the like), aralkyloxy (e.g., benzyloxy and the like), carboxy, lower alkoxycarbonyl (methoxycarbonyl, ethoxycarbonyl, tert-butoxycarbonyl and the like, wherein the alkoxy moiety has 1 to 6 carbon atoms), methylenedioxy, haloalkyl (e.g. haloalkyl wherein alkyl moiety has 1 to 4 carbon atoms, such as chloromethyl, bromomethyl, fluoromethyl, trifluoromethyl, trifluoroethyl, pentafluoropropyl, chlorobutyl and the like), haloalkyloxy (e.g., trifluoromethoxy, trifluoroethoxy and the like), haloalkylsulfonylamino (e.g., trifluoromethanesulfonylamino and the like), hydroxy, nitro, amino, mono- or di-substituted amino such as alkylamino (e.g., methylamino, dimethylamino and the like), acylamino (e.g, acetylamino, formylamino and the like), alkoxycarbonylamino (methoxycarbonylamino, ethoxycarbonylamino, tert-butoxycarbonylamino and the like, wherein the alkoxy moiety has 1 to 6 carbon atoms), alkylsulfonylamino (e.g., methanesulfonylamino and the like) and bisalkylsulfonylamino (e.g., bismethanesulfonylamino and the like), cyano, alkylsulfonyl (e.g., methanesulfonyl and the like), acyl (e.g., acetyl, propionyl, butyryl, pivaloyl and the like), acyloxy wherein acyl moiety is alkanoyl having 2 to 5 carbon atoms (e.g., acetyl, propionyl, butyryl, pivaloyl and the like) or aroyl such as benzoyl optionally having, on the ring, 1 to 3 substituents selected from halogen atom (same as above), lower alkyl (same as above), lower alkoxy (same as above), haloalkyl (same as above) and hydroxy, which is exemplified by benzoyl, chlorobenzoyl, methylbenzoyl, methoxybenzoyl and the like, tetrazolyl, alkyltetrazolyl (1-methyl-1H-tetrazolyl, 2-methyl-2H-tetrazolyl, and the like), aralkyl such as benzyl, 2-phenylethyl, 3-phenylpropyl and the like, wherein alkyl moiety has 1 to 6 carbon atoms and which may have, on the ring, 1 to 3 substituents selected from halogen atom (same as above), lower alkryl (same as above), lower (same as above) and hydroxy, and the like. Preferred are phenyl, biphenylyl, naphthyl, phenyl having, as substituent, halogen atom (same as above), lower alkyl (same as above), lower alkoxy (same as above), aralkyloxy (same as above), carboxy, lower alkoxycarbonyl (same as above), haloalkyl (same as above), haloalkyloxy (same as above), haloalkylsulfonylamino (same as above), nitro, amino, mono- or di-substituted amino (particularly, alkoxycarbonylamino which, is same as above), alkylsulfonyl (same as above), acyloxy (same as above), cyano, tetrazolyl, alkyltetrazolyl, hydroxy or the like, and the like. Particularly preferred are phenyl, 2-methylphenyl, 3-methylphenyl, 4-methylphenyl, 2-methoxyphenyl, 3-methoxyphenyl, 4-methoyxphenyl, 2,5-dimethoxyphenyl, 3,4-dimethoxyphenyl, 3,4,5-trimethoxyphenyl, 5-acetyl-2-methoxyphenyl, 4-ethoxyphenyl, 2-carboxyphenyl, 3-carboxyphenyl, 4-carboxyphenyl, 2-methoxycarbonylphenyl, 3-ethoxycarbonylphenyl, 4-methoxycarbonylphenyl, 3,4-methylenedioxyphenyl, 2-chloro-4,5-methylenedioxyphenyl, 2-fluorophenyl, 3-fluorophenyl, 4-fluorophenyl, 2,4-difluorophenyl, 2,5-difluorophenyl, 3,4-difluorophenyl, 3,5-difluorophenyl, 2-chlorophenyl, 3-chlorophenyl, 4-chlorophenyl, 2-bromophenyl, 3-bromophenyl, 4-bromophenyl, 3-chloro-4-methoxyphenyl, 3,5-dichloro-4-methoxyphenyl, 4-chloro-2,5-dimethoxyphenyl, 2-trifluoromethylphenyl, 3-trifluoromethylphenyl, 4-trifluoromethylphenyl, 4-trilfuoromethoxyphenyl, 2-nitrophenyl, 3-nitrophenyl, 4-nitrophenyl, 2-(1H-tetrazol-5-yl)phenyl, 3-(1H-tetrazol-5-yl)phenyl, 4-(1H-tetrazol-5-yl)phenyl, 4-(1-methyl-1H-tetrazol-5-yl)phenyl, 3-(1-methyl-1H-tetrazol-5-yl)phenyl, 2-(1-methyl-1H-tetrazol-5-yl)phenyl, 4-(2-methyl-2H-tetrazol-5-yl)phenyl, 3-(2-methyl-2H-tetrazol-5-yl)phenyl, 2-(2-methyl-2H-tetrazol-5-yl)phenyl, 2-methoxy-5-nitrophenyl, 4-methoxy-3-nitrophenyl, 2-cyanophenyl, 3-cyanophenyl, 4-cyanophenyl, 2-aminophenyl, 3-aminophenyl, 4-aminophenyl, 2-methoxycarbonlaminophenyl, 3-ethoxcarbonylaminophenyl, 4-methoxycarbonylaminophenyl, 4-formylaminophenyl, 4-acetylaminophenyl, 4-dimethylaminophenyl, 4-methanesulfonylaminophenyl, 4-bismethanesulfonylaminophenyl, 4-trifluoromethanesulfonylaminophenyl, 4-methylsulfonylphenyl, 4-hydroxyphenyl, 3,4-dihydroxyphenyl, 4-benzyloxyphenyl 3,4-dibenzyloxyphenyl, 3-benzyloxy-4-methoxyphenyl, 4-hydroxy-3-methoxyphenyl, 4-benzyloxy-3-hydroxyphenyl, 3-benzyloxy-4-hydroxyphenyl, 4-benzyloxy-3-methoxyphenyl, biphenylyl, 1-naphthyl, 2-naphthyl and the like.

Optionally substituted heteroaryl means, for example, pyridyl, pyridine-1-oxide, thienyl, thiazoyl, isoxazolyl, indolyl, quinolyl, furyl, benzofuryl, 1H-benzimidazol-2-yl, 2-benzothiazolyl or the like, which may have 1 to 3 substituents on the ring. Examples of the substituent include halogen atom (e.g., chlorine, bromine, fluorine and iodine), lower alkyl (e.g., alkyl having 1 to 6 carbon atoms, such as methyl, ethyl, propyl, isopropyl, butyl, tert-butyl, isobutyl, pentyl, isopentyl, hexyl and the like), lower alkoxy (e.g., alkoxy having 1 to 6 carbon atoms, such as methoxy, ethoxy, propoxy, isopropoxy, butoxy, tert-butoxy, hexyloxy and the like), aralkyloxy (e.g., benzyloxy and the like), methylenedioxy, haloalkyl (e.g., haloalkyl wherein alkyl moiety has 1 to 4 carbon atoms, such as chloromethyl, bromomethyl, fluoromethyl, trifluoromethyl, trifluoroethyl, pentafluoropropyl, chlorobutyl and the like), haloalkyloxy (e.g., trifluoromethoxy, trifluoroethoxy

and the like), haloalkysulfonylamino (e.g., trifluoromethanesulfonylamino and the like), hydroxy, nitro, amino, monk or di-substituted amino such as alkylamino (e.g., methylamino, dimethylamino and the like), acylamino (e.g., acetylamino, formylamino and the like), alkylsulfonylamino (e.g., methanesulfonylamino and the like) and bisalkaylsulfonylamino (e.g., bismethanesulfonylamino and the like), cyano, alkylsulfonyl (e.g., methanesulfonyl and the like), acyl (e.g., acetyl, propionyl, butyryl, pivaloyl and the like), acyloxy wherein acyl moiety is alkanoyl having 2 to 5 carbon atoms (e.g., acetyl, propionyl, butyryl, pivaloyl and the like) or aroyl such as benzoyl optionally having, on the ring, 1 to 3 substituents selected from halogen atom (same as above), lower alkyl (same as above), lower alkoxy (same as above), haloalkyl (same as above) and hydroxy, which is exemplified by benzoyl, chlorobenzoyl, methylbenzoyl, methoxybenzoyl and the like, aralkyl such as benzyl, 2-phenylethyl, 3-phenylpropyl and the like, wherein alkyl moiety has 1 to 6 carbon atoms and optionally having, on the ring, 1 to 3 substituents selected from halogen atom (same as above), lowers alkyl (same as above), lower alkyl (same as above) and hydroxy, and the like. Preferred are pyridyl, pyridine-1-oxide, thienyl, thiazolyl, isoxazolyl, indolyl and the like, and these heteroaryl groups may have 1 to 3 substituents selected from halogen atom (same as above), lowers alkyl (same as above), lower alkyl (same as above), aralkyloxy (same as above), haloalkyl (same as above), haloalkyloxy (same as above), haloalkylsulfonylamino (same as above), nitro, amino, mono-or-di-substituted amino (same as above), alkylsulfonylamino (same as above), bisalkylsulfonylamino (same as above), alkylsulfonyl (same as above), acyloxy (same as above), cyano, hydroxy and the like. More preferred are pyridyl, pyridine-1-oxide, 2-methylpyridyl, 3-methylpyridyl, 4-methylpyridyl, 2,5-dimethylpyridyl, 2,6-dimethylpyridyl, 3,5-dimethylpyridyl, 2-methoxypyridyl, 3-methoxypyridyl, 4-methoxypyridyl, 2,5-dimethoxypyridyl, 2,6-dimethoxypyridyl, 3,5-dimethoxypyridyl, 2-fluoropyridyl, 3-fluoropyridyl, 4-fluoropyridyl, 2,5-difluoropyridyl, 2,6-difluoropyridyl, 3,5-difluoropyridyl, 2-chloropyridyl, 3-chloropyridyl, 4-chloropyridyl, 2,5-dichloropyridyl, 2,6-dichloropyridyl, 3,5-dichloropyridyl, 4-trifluoromethylpyridyl, 4-trifluoromethoxypyridyl, 2-nitropyridyl, 3-nitropyridyl, 4-nitropyridyl, 2-cyanopyridyl, 3-cyanopyridyl, 4-cyanopyridyl, 4-amino-pyridyl, 4-formylaminopyridyl, 4-acetylaminopyridyl, 4-dimethylaminopyridyl, 4-methanesulfonylaminopyridyl, 4-bismethanesulfonylaminopyridyl, 4-trifluoromethanesulfonylaminopyridyl, 2-hydroxypyridyl, 3-hydroxypyridyl, 4-hydroxypyridyl, 2-benzyloxypyridyl, 3-benzyloxypyridyl, 4-benzyloxypyridyl, thienyl, 2-methylthienyl, 3-methylthienyl, 2, 3-dimethylthienyl, 3, 4-dimethylthienyl, 2-methoxythienyl, 3-methoxythienyl, 2-fluorothienyl, 3-fluorothienyl, 2, 3-difluorothienyl, 3,4-difluorothienyl, 2-chlorothienyl, 3-chlorothienyl, 2,3-dichlorothienyl, 3,4-dichlorothienyl, 2-trifluoromethylthienyl, 3-trifluoromethylthienyl, 2-trifluoromethoxythienyl, 3-trifluoromethoxythienyl, 2-nitrothienyl, 3-nitrothienyl, 2-cyanothienyl, 3-cyanothienyl, 2-aminothienyl, 3-aminothienyl, 2-formylaminothienyl, 3-formylaminothienyl, 2-acetylaminothienyl, 3-acetylaminothienyl, 2-dimethylaminothienyl, 3-dimethylaminothienyl, 2-methanesulfonylaminothienyl, 3-methanesulfonylaminothienyl, 2-bismethanesulfonylaminothienyl, 3-bismethanesulfonylaminothienyl, 2-trifluoromethanesulfonylaminothienyl, 3-trifluoromethanesulfonylaminothienyl, 2-hydroxythienyl, 3-hydroxythienyl, 2-benzyloxythienyl, 3-benzyloxythienyl, isoxazolyl, 3-methylisoxazolyl, 4-methylisoxazolyl, 5-methylisoxazolyl, 3, 4-dimethylisoxazolyl, 3, 5-dimethylisoxazolyl, 4, 5-dimethylisoxazolyl, 3-chloroisoxazolyl, 4chloroisoxazolyl, 5-chloroisoxazolyl, 3,4-dichloroisoxazolyl, 3,5-dichloroisoxazolyl, 4,5-dichloroisooxazolyl, thiazolyl, 2-methylthiazolyl, 4-methylthiazolyl, 5-methylthiazolyl, 2,4-dimethylthiazolyl, 2, 5-dimethylthiazolyl, 4,5-dimethylthiazolyl, 2-chlorothiazolyl, 4-chlorothiazolyl, 5-chlorothiazolyl, 2,4-dichlorothiazolyl, 2,5-dichlorothiazolyl, 4,5-dichlorothiazolyl, 2-indolyl, 3-indolyl and the like.

Lower alkyl means linear or branched alkyl having 1 to 6 carbon atoms, such as methyl, ethyl, propyl, isopropyl, butyl, isobutyl, tert-butyl, pentyl, tert-pentyl, hexyl and the like, with preference given to alkyl having 1 to 4 carbon atoms, such as methyl, ethyl, propyl, isopropyl, butyl, isobutyl, tert-butyl and the like.

Halogen atom is chlorine, bromine, fluorine or iodine, with preference given to chlorine and bromine.

Aralkyl is arylalkyl wherein aryl is phenyl and alkyl moiety has 1 to 6 carbon atoms, which is exemplified by benzyl, phenylethyl, phenylpropyl, phenylbutyl, phenylhexyl and the like. It may have, on the phenyl ring, 1 to 3 substituents selected from, for example, halogen atom (e.g., chlorine, bromine, fluorine and the like), alkyl (e.g., alkyl having 1 to 6 carbon atoms; such as methyl, ethyl, propyl, isopropyl, butyl, tert-butyl, isobutyl, pentyl, isopentyl, hexyl and the like), alkoxy (e.g., alkoxy having 1 to 6 carbon atoms, such as methoxy, ethoxy, propoxy, isopropoxy, butoxy, tert-butoxy, hexyloxy and the like), haloalkyl (e.g., haloalkyl wherein alkyl moiety has 1 to 4 carbon atoms, such as chloromethyl, bromomethyl, fluoromethyl, trifluoromethyl, trifluoroethyl, pentafluoropropyl, chlorobutyl and the like), hydroxy, nitro, amino, cyano, acyloxy wherein acyl moiety is alkanoyl having 2 to 5 carbon atoms (e.g., acetyl, propionyl, butyryl, pivaloyl and the like) or aroyl such as benzoyl optionally having, on the ring, 1 to 3 substituents selected from halogen atom (same as above), alkyl (same as above), alkoxy (same as above), haloalkyl (same as above) and hydroxy, which is exemplified by benzoyl, chlorobenzoyl, methylbenzoyl, methoxybenzoyl and the like, and the like. Preferred is aralkyl which has phenyl or phenyl having substituent selected from halogen atom (same as above), alkyl (same as above), alkoxy (same as above), haloalkyl (same as above), hydroxy, nitro, amino, cyano and the like, and alkyl moiety having 1 to 4 carbon atoms. Particularly preferred is aralkyl which has phenyl or phenyl having substituent selected from halogen atom (same as above), alkyl (same as above), alkoxy (same as above) and the like, and alkyl moiety having 1 to 4 carbon atoms.

Lower alkoxy means linear or branched alkoxy having 1 to 6 carbon atoms, such as methoxy, ethoxy, propoxy,

isopropoxy, butoxy, tertbutoxy, pentyloxy, tertpentyloxy, hexyloxy and the like, with preference given to alkoxy having 1 to 4 carbon atoms, such as methoxy, ethoxy, propoxy, isopropoxy, butoxy, tert-butoxy and the like, and particular preference given to methoxy, ethoxy, propoxy, butoxy and the like.

Cycloalkyl has 3 to 10 carbon atoms, and is exemplified by cyclopropyl, 2,3-dimethylcyclopropyl, cyclobutyl, 3-methylcyclobutyl, cyclopentyl, 3,4-dimethylcyclopentyl, cyclohexyl, 4-methylcyclohexyl, cycloheptyl, cyclooctyl, norbornyl, adamantyl, bicyclo [3.3.0]octan-1-yl, bicyclo[3.3.1]nonan-9-yl and the like. Preferred are, for example, cyclopropyl, cyclobutyl, cyclopentyl, cyclohexyl, cycloheptyl and the like, and particularly preferred are, for example, cyclopropyl, cyclobutyl, cyclopentyl, cyclohexyl and the like.

Amino substituted by lower alkyl is alkylamino mono- or di-substituted by alkyl having 1 to 5 carbon atoms, and is exemplified by methylamino, dimethylamino, ethylamino, diethylamino, propylamino, dipropylamino and the like. Preferred are methylamino, dimethylamino, ethylamino, diethylamino and the like.

Cyclic amino is exemplified by pyrrolidinyl, piperidino, and morpholino, thiomorpholino, piperazinyl and the like having, as a hetero atom, oxygen, sulfur or nitrogen atom, wherein lower alkyl, aralkyl or the like may substitute the 4-position nitrogen atom of piperazinyl.

Acyl means, for example, alkanoyl having 2 to 5 carbon atoms such as acetyl, propionyl, butyryl, pivaloyl and the like, or benzoyl. Preferred are formyl, acetyl, propionyl, benzoyl and the like.

Acyloxy is, for example, alkanoyloxy having 2 to 5 carbon atoms such as acetyloxy, propionyloxy, butyryloxy, pivaloyloxy and the like, or benzoyloxy. Preferred are acetyloxy, propionyloxy, benzoyloxyl and the like.

Carbamoyl substituted by lower alkyl means alkylcarbamoyl mono- or di-substituted by alkyl having 1 to 5 carbon atoms such as methylcarbamoyl, dimethylcarbamoyl, ethylcarbamoyl, diethylcarbamoyl, propylcarbamoyl, dipropylcarbamoyl and the like. Preferred are methylcarbamoyl, dimethylcarbamoyl, ethylcarbamoyl, diethylcarbamoyl and the like.

Cyclic aminocarbonyl means that having the above-mentioned cyclic amino moiety and is exemplified by pyrrolidinylcarbonyl, piperidinocarbonyl, morpholinocarbonyl, thiomorpholincarbonyl, piperazinylcarbonyl, 4-methyl-1-piperazinylcarbonyl and the like. Preferred are pyrrolidinylcarbonyl, piperidinocarbonyl, morpholinocarbonyl and piperazinylcarbonyl.

Lower alkoxycarbonyl is alkoxycarbonyl wherein alkoxy moiety has 1 to 5 carbon atoms and is exemplified by methoxycarbonyl, ethoxycarbonyl, propoxycarbonyl, isopropoxycarbonyl, butoxycarbonyl, isobutoxycarbonyl, tert-butoxycarbonyl and the like. Preferred are methoxycarbonyl, ethoxycarbonyl, propoxycarbonyl and the like.

Aralkyloxycarbonyl is phenylalkoxycarbonyl wherein alkoxy moiety has 1 to 5 carbon atoms, such as benzyloxycarbonyl, 2-phenylethoxycarbonyl, 3-phenylpropoxycarbonyl and the like, which may have, as substituent, halogen atom, nitro, alkyl, alkoxy, trifluoromethyl and the like.

Lower alkenyl means alkenyl having 2 to 6 carbon atoms, such as ethenyl, 1-propenyl, 1-butenyl, 2-butenyl, 3-butenyl, 2-methyl-1-propenyl, 1-pentenyl, 2-pentenyl, 3-pentenyl, 2-methyl-1-butenyl, 3-methyl-1-butenyl, 1-hexenyl, 2-hexenyl, 3-hexenyl, 4-hexenyl, 5-hexenyl, 2-methyl-1-pentenyl, 3-methyl-1-pentenyl, 4-methyl-1-pentenyl, 2,3-dimethyl-1-butenyl, 3,3-dimethyl-1-butenyl and the like, with preference given to alkenyl having 2 to 4 carbon atoms, such as ethenyl, 1-propenyl, 1-butenyl, 2-butenyl, 3-butenyl, 2-methyl-1-propenyl and the like.

Aralkyl substitued by lower alkyl is that wherein the above-mentioned aralkyl is substituted by alkyl having 1 to 6 carbon atoms, and is exemplified by 4-methylbenzyl, 4-ethylbenzyl, 4-propylbenzyl, 4-isopropylbenzyl, 4-methylphenylethyl, 4-ethylphenylethyl, 4-propylphenylethyl and the like. Preferred are 4-methylbenzyl, 4-ethylbenzyl, 4-isopropylbenzyl and the like.

Lower alkynyl means alkynyl having 2 to 6 carbon atoms, such as ethynyl, 1-propynyl, 1-butynyl, 2-butynyl, 3-butynyl, 1-pentynyl, 2-pentynyl, 3-pentynyl, 3-methyl-1-butynyl, 1-hexynyl, 2-hexynyl, 3-hexynyl, 4-hexynyl, 5-hexynyl, 3-methyl-1-pentynyl, 4-methyl-1-pentynyl, 3,3-dimethyl-1-butynyl and the like, with preference given to alkynyl having 2 to 4 carbon atoms, such as ethynyl, 1-propynyl, 1-butynyl, 2-butynyl, 3-butynyl and the like.

Haloalkyl is that wherein alkyl moiety has 1 to 4 carbon atoms, such as chloromethyl, bromomethyl, fluoromethyl, trifluoromethyl, trichloromethyl, trifluoroethyl, trichloroethyl, pentafluoropropyl, chlorobutyl and the like, with preference given to chloromethyl, bromomethyl, fluoromethyl, trifluoromethyl, trichchloromethyl and the like.

Lower alkylcarbonyl is alkylcarbonyl wherein alkyl moiety has 1 to 5 carbon atoms, and is exemplified by acetyl, propionyl, butyryl, isobutyryl, pivaloyl and the like.

Heteroarylalkyl may have, on its ring, 1 to 3 substituents selected from halogen, alkyl having 1 to 6 carbon atoms, alkoxy having 1 to 6 carbon atoms, trifluoromethyl, nitro, amino, cyano and hydroxy, wherein the alkyl moiety has 1 to 4, preferably 1 or 2, carbon atoms and the hetero atom constituting the ring is nitrogen, oxygen or sulfur. For example, pyridylmethyl (e.g., 2-pyridylmethyl, 3-pyridylmethyl, 4-pyridylmethyl), quinolylmethyl (e.g., 2-quinolylmethyl, 3-quinolylmethyl and the like), indolylmethyl (e.g., 2-indolylmethyl, 3-indolylmethyl and the like), thienylmethyl (e.g., 2-thienylmethyl, 3-thienylmethyl), furylmethyl (e.g., 2-furylmethyl, 3-furylmethyl), benzofurylmethyl (e.g., 2-benzofurylmethyl, 3-benzofurylmethyl and the like), 1H-benzimidazol-2-ylmethyl, 2-benzothiazolylmethyl, 2-(2-thienyl)ethyl, 2-(2-furyl)ethyl and the like.

Cycloalkylakyl is that wherein the cycloalkyl moiety has 3 to 10 carbon atoms and the alkyl moiety has 1 to 6 carbon atoms, preferably 1 to 3 carbon atoms, and is exemplified by cyclopropylmethyl, 2,3-dimethylcyclopropylmethyl, cyclobutylmethyl, 3-methylcyclobutylmethyl, cyclopentylmethyl, 3,4-dimethylcyclopentylmethyl, cyclohexylmethyl, 4-methylcyclohexylmethyl, cycloheptylmethyl, cyclooctymethyl, 2-cyclohexylethyl, 3-cyclohexylpropyl, norbornylmethyl, 1-adamantylmethyl, bicyclo[3.3.0]octan-1-ylmethyl, bicyclo[3.3.1]nonan-9-ylmethyl and the like.

Alkyltetrazolyl is tetrazolyl substituted by alkyl having 1 to 6 carbon atoms, and is exemplified by 1-methyl-1H-tetrazolyl, 1-ethyl-1H-tetrazolyl, 2-methyl-2H-tetrazolyl and 2-ethyl-2H-tetrazolyl, with preference given to 1-methyl-1H-tetrazolyl and 2-methyl-2H-tetrazolyl.

Pyridine-1-oxide is a group selected from the following.

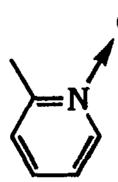 , 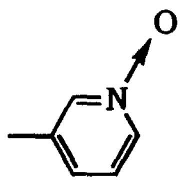 , 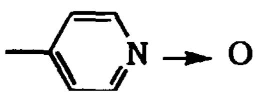

Examples of pharmaceutically acceptable salt include, but not limited to, various inorganic acid addition salts, such as hydrochloride, hydrobromide, sulfate, phosphate, nitrate and the like; various organic acid addition salts, such as acetate, propionate, succinate, glycolate, lactate, malate, oxalate, tartrate, citrate, maleate, fumarate, methanesulfonate, benzenesulfonate, p-toluenesulfonate, ascorbate and the like, and salts with various amino acids, such as aspartate, glutamate and the like. It may be a hydrate where necessary.

Now, various substituents are described in more detail in the following.

$R^1$ means optionally substituted aryl or optionally substituted heteroaryl, wherein preferred are phenyl, 2-methylphenyl, 3-methylphenyl, 4-methylphenyl, 2-methoxyphenyl, 3-methoxyphenyl, 4-methoxyphenyl, 2-carboxyphenyl, 3-carboxyphenyl, 4-carboxyphenyl, 2-methoxycarbonylphenyl, 3-ethoxycarbonylphenyl, 4-methoxycarbonylphenyl, 2-fluorophenyl, 3-fluorophenyl, 4-fluorophenyl, 2-chlorophenyl, 3-chlorophenyl, 4-chlorophenyl, 2-bromophenyl, 3-bromophenyl, 4-bromophenyl, 2-trifluoromethylphenyl, 3-trifluoromethylphenyl, 4-trifluoromethylphenyl, 2-nitrophenyl, 3-nitrophenyl, 4-nitrophenyl, 2-hydroxyphenyl, 3-hydroxyphenyl, 4-hydroxyphenyl, 2-benzyloxyphenyl, 3-benzyloxyphenyl, 4-benzyloxyphenyl, 2-cyanophenyl, 3-cyanophenyl, 4-cyanophenyl, 2-aminophenyl, 3-aminophenyl, 4-aminophenyl, 2-dimethylaminophenyl, 3-dimethylaminophenyl, 4-dimethylaminophenyl, 2-methoxycarbonylaminophenyl, 3-ethoxycarbonylaminophenyl, 4-methoxycarbonylaminophenyl, pyridyl, 2-methylpyridyl, 3-methylpyridyl, 4-methylpyridyl, 2-methoxypyridyl, 3-methoxypyridyl, 4-methoxypyridyl, 2-fluoropyridyl, 3-fluoropyridyl, 4-fluoropyridyl, 2-chloropridyl, 3-chloropyridyl, 4-chloropyridyl, 2-trifluoromethylpyridyl, 3-trifluoromethylpyridyl, 4-trifluoromethylpyridyl, 2-nitropyridyl, 3-nitropyridyl, 4-nitropyridyl, 2-hydroxypyridyl, 3-hydroxypyridyl, 4-hydroxypyridyl, 2-benzyloxypyridyl, 3-benzyloxypyridyl, 4-benzyloxypyridyl, 2-cyanopyridyl, 3-cyanopyridyl, 4-cyanopyridyl, 2-aminopyridyl, 3-aminopyridyl, 4-aminopyridyl, 2-dimethylaminopyridyl, 3-dimethylaminopyridyl, 4-dimethyaminopyridyl, 2-thienyl and the like; more preferred are phenyl, 2-methylphenyl, 3-methylphenyl, 4-methylphenyl, 2-methoxyphenyl, 3-methoxyphenyl, 4-methoxyphenyl, 2-carboxyphenyl, 3-carboxyphenyl, 4-carboxyphenyl, 2-methoxycarbonylphenyl, 3-ethoxycarbonylphenyl, 4-methoxycarbonylphenyl, 2-fluorophenyl, 3-fluorophenyl, 4-fluorophenyl, 2-chlorophenyl, 3-chlorophenyl, 4-chlorophenyl, 2-bromophenyl, 3-bromophenyl, 4-bromophenyl, 2-trifluoromethylphenyl, 3-trifluoromethylphenyl, 4-trifluoromethylpnenyl, 2-nitrophenyl, 3-nitrophenyl, 4-nitrophenyl, 2-hydroxyphenyl, 3-hydroxyphenyl, 4-hydroxyphenyl, 2-aminophenyl, 3-aminophenyl, 4-aminophenyl, 2-methoxycarbonylaminophenyl, 3-ethoxycarbonylaminophenyl, 4-methoxycarbonylaminophenyl, 2-thienyl and the like, and particularly preferred are phenyl, 2-methylphenyl, 3-methylphenyl, 4-methylphenyl, 2-carboxyphenyl, 3-carboxyphenyl, 4-carboxyphenyl, 2-methoxycarbonylphenyl, 3-ethoxycarbonylphenyl, 4-methoxycarbonylphenyl, 2-chlorophenyl, 3-chlorophenyl, 4-chlorophenyl, 2-bromophenyl, 3-bromophenyl, 4-bromophenyl, 2-methoxyphenyl, 3-methoxyphenyl, 4-methoxyphenyl, 2-aminophenyl, 3-aminophenyl, 4-aminophenyl, 2-methoxycarbonylaminophenyl, 3-ethoxycarbonylaminophenyl, 4-methoxycarbonylaminophenyl, 2-thienyl and the like.

$R^2$ means hydrogen atom, hydroxy, halogen atom or lower alkyl, or $R^2$ and $R^4$ combinedly together form carbonyl with the carbon atom to which they are bonded. $R^2$ is preferably hydrogen atom or hydroxy, or $R^2$ and $R^4$ combinedly together form carbonyl with the carbon atom to which they are bonded. $R^2$ is more preferably hydrogen atom or $R^2$ and $R^4$ combinedly together form carbonyl with the carbon atom to which they are bonded, and particularly preferably, $R^2$ is hydrogen atom.

$R^3$ is hydrogen atom, lower alkyl, lower alkoxy, cycloalkyl, optionally substituted aryl, optionally substituted heteroaryl or a group of the formula

$-CR^5=CR^6R^7$, $-OR^{18}$, 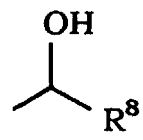 ,

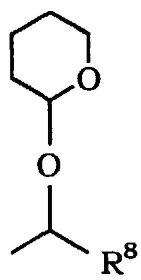 , $-COOR^8$, $-CONHR^8$, 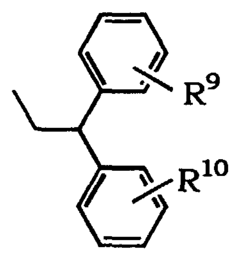

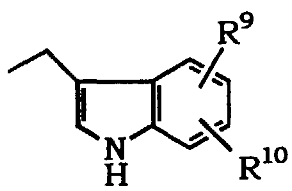 or -X-Y

wherein $R^5$, $R^6$ and $R^7$ are the same or different and each is hydrogen atom, halogen atom, lower alkyl or optionally substituted aryl, $R^8$ is hydrogen atom, lower alkyl, cycloalkyl, optionally substituted aryl, aralkyl or optionally substituted heteroaryl, $R^9$ and $R^{10}$ are the same or different and each is hydrogen atom, lower alkyl, lower alkoxy, hydroxy, halogen atom, nitro or amino, $R^{18}$ is optionally substituted aryl, X is - $(CH_2)_m$-, -CO-, $-COCH_2-$, -NH-, $-NHCH_2-$, $-CH_2NH-$, - $CH_2NHCO-$, $-OCH_2-$, $-(CH_2)_nO-$ or - $CH_2S-$, Y is halogen atom, cycloalkyl, optionally substituted aryl or optionally substituted heteroaryl, m is an integer of 1 to 4 and n is an integer of 1 to 4, with preference given to phenyl, 2-methylphenyl, 3-methylphenyl, 4-methylphenyl, 2-ethylphenyl, 3-ethylphenyl, 4-ethylphenyl, 2-tert-butylphenyl, 3-tert-butylphenyl, 4-tert-butylphenyl, 2-methoxyphenyl, 3-methoxyphenyl, 4-methoxyphenyl, 2,4-dimethoxyphenyl, 2,5-dimethoxyphenyl, 3,4-dimethoxyphenyl, 3,5-dimethoxyphenyl, 3,4,5-trimethoxyphenyl, 2-ethoxyphenyl, 3-ethoxyphenyl, 4-ethoxyphenyl, 2-hydroxyphenyl, 3-hydroxyphenyl, 4-hydroxyphenyl, 2,4-dihydroxyphenyl, 2,5-dihydroxyphenyl, 3,4-dihydroxyphenyl, 3,5-dihydroxyphenyl, 2-carboxyphenyl, 3-carboxyphenyl, 4-carboxyphenyl, 2-methoxycarbonylphenyl, 3-methoxycarbonylphenyl, 4-methoxycarbonylphenyl), 2-fluorophenyl, 3-fluorophenyl, 4-fluorophenyl, 2,4-difluorophenyl, 2,5-difluorophenyl, 3,4-difluorophenyl, 3,5-difluorophenyl, 2-chlorophenyl, 3-chlorophenyl, 4-chlorophenyl, 2,4-dichlorophenyl, 2,5-dichlorophenyl, 3,4-dichlorophenyl, 3,5-dichlorophenyl, 2-bromophenyl, 3-bromophenyl, 4-bromophenyl, 2-trifluoromethylphenyl, 3-trifluoromethylphenyl, 4-trifluoromethylphenyl, 2-trifluoromethoxyphenyl, 3-trifluoromethoxyphenyl, 4-trifluoromethoxyphenyl, 2-aminophenyl, 3-aminophenyl, 4-aminophenyl, 2-methylaminophenyl, 3-methylaminophenyl, 4-methylaminophenyl, 2-dimethylaminophenyl, 3-dimethylaminophenyl, 4-dimethylaminophenyl, 2-cyanophenyl, 3-cyanophenyl, 4-cyanophenyl, 2-nitrophenyl, 3-nitrophenyl, 4-nitrophenyl, 3-chloro-4-methoxyphenyl, 3-chloro-5-methoxyphenyl, 4-chloro-5-methoxyphenyl, 3,5-dichloro-4-methoxyphenyl, 4-chloro-2,5,-dimethoxyphenyl, 2-methoxy-3-nitrophenyl, 2-methoxy-4-nitrophenyl, 2-methoxy-5-nitrophenyl, 3-methoxy-4-nitrophenyl, 3-methoxy-5-nitrophenyl, 4-methoxy-2-nitrophenyl, 4-methoxy-3-nitrophenyl, 2-acetyl-3-methoxyphenyl, 2-acetyl-4-methoxyphenyl, 5-acetyl-2-methoxyphenyl, 3-acetyl-4-methoxyphenyl, 3-acetyl-5-methoxyphenyl, 3,4-methylenedioxyphenyl, 2-chloro-4,5-methylenedioxyphenyl, 3-chloro-4,5-methylenedioxyphenyl, 2-benzloxyphenyl, 3-benzyloxyphenyl, 4-benzyloxyphenyl, 3,4-dibenzyloxyphenyl, 3,5-dibenzloxyphenyl, 2-formylaminophenyl, 3-formylaminophenyl, 4-formylaminophenyl, 2-acetylaminophenyl, 3-acetylaminophenyl, 4-acetylaminophenyl, 2-methylsulfonylphenyl, 3-methylsulfonylphenyl, 4-methylsulfonylphenyl, 2-methylsulfonylaminophenyl, 3-methylsulfonylaminophenyl, 4-methylsulfonylaminophenyl, 2-bis-(methylsulfonyl)aminophenyl, 3-bis(methysulfonyl)aminophenyl, 4-bis(methylsulfonyl)aminophenyl, 2-(1H-tetrazol-5-yl)phenyl, 3-(1H-tetrazol-5-yl)phenyl, 4-(1H-tetrazol-5-yl)phenyl, 2-(1-methyl-1H-tetrazol-5-yl)phenyl, 3-(1-methyl-1H-tetrazol-5-yl)phenyl, 4-(1-methyl-1H-tetrazol-5-yl)phenyl, 2-(1-ethyl-1H-tetrazol-5-yl)phenyl, 3-(1-ethyl-1H-tetrazol-5-yl)phenyl, 4-(1-ethyl-1H-tetrazol-5-yl)phenyl, 2-(2-methyl-2H-tetrazol-5-yl)phenyl, 3-(2-methyl-2H-tetrazol-5-yl)phenyl, 4-(2-methyl-2H-tetrazol-5-yl)phenyl, 2-(2-ethyl-2H-tetrazol-5-yl)phenyl, 3-(2-ethyl-2H-tetrazol-5-yl)phenyl, 4-(2-ethyl-2H-tetrazol-5-yl)phenyl, 2-phenylphenyl, 3-phenylphenyl, 4-phenylphenyl, 1-naphthyl, 2-naphthyl, 2-pyridyl, 3-pyridyl, 4-pyridyl, pyridine-1-oxide, 2-methylpyridyl, 3-methylpyridyl, 4-methylpyridyl, 2-methoxypyridyl, 3-methoxypyridyl, 4-methoxypyridyl, 2-hydroxypyridyl, 3-hydroxypyridyl, 4-hydroxypyridyl, 2-fluoropyridyl, 3-fluoropyridyl, 4-fluoropyridyl, 2-chloropyridyl, 3-chloropyridyl, 4-chloropyridyl, 2,5-dichloropyridyl, 2,6-dichchloropyridyl, 3,5-dichloropyridyl, 4-trifluoromethylpyridyl, 4-trifluoromethoxypyridyl, 2-nitropyridyl, 3-nitropyridyl, 4-nitropyridyl, 2-cyanopyridyl, 3-cyanopyridyl, 4-cyanopyridyl, 4-aminopyridyl, 4-dimethylaminopyridyl, 4-formylaminopyridyl, 4-acetylaminopyridyl, 4-methanesulfonylaminopyridyl, 4-bismethanesulfonylaminopyridyl, thienyl, 2-methylthienyl, 3-methylthienyl, 2,3-dimethylthienyl, 3,4-dimethylthienyl, 2-chlorothienyl, 3-chlorothienyl, 2,3-dichlorothienyl, 3,4-dichlorothienyl, isoxazolyl, 3-methylisoxazolyl, 4-methylisoxazolyl, 5-methylisoxazolyl, 3,4-dimethylisoxazolyl, 3,5-dimethylisoxazolyl, 4,5-dimethylisoxazolyl, 3-chloroisoxazolyl, 4-chloroisoxazolyl, 5-chloroisoxazolyl, 3,4-dichloroisoxazolyl, 3,5-dichloroisoxazolyl, 4,5-dichloroisoxazolyl, thiazolyl, 2-methylthiazolyl, 4-methylthiazolyl, 5-methylthiazolyl, 2,4-dimethylthiazolyl, 2,5-dimethylthiazolyl, 4,5-dimethylthiazolyl, 2-chlorothiazolyl, 4-chlorothiazolyl, 5-chlorothiazolyl, 2,4-dichlorothiazolyl, 2,5-dichlorothiazolyl, 4,5-dichlorothiazolyl, ethenyl, 1-methylethenyl, propenyl, 2-phenylethenyl, 1-chloroethenyl, 2-chloroethenyl, 1,2-dichloroethenyl, 2,2-dichloroethenyl, 1,2,2-trichloroethenyl, 1-bromoethenyl, 2-bromoethenyl, phenylhydroxymethyl, 2-phenyl-1-hydroxyethyl, 2-(2-methoxyphenyl)-1-hydroxyethyl, 2-(3-methoxyphenyl)-1-hydroxyethyl, 2-(4-methoxyphenyl)-1-hydroxyethyl, benzoyl, 2-methylbenzoyl, 3-methylbonzoyl, 4-methylbenzoyl, 2-methoxybenzoyl, 3-methoxybenzoyl, 4-methyoxybenzoyl, 2,3-dimethoxybenzoyl, 2,4-dimethoxybenzoyl ,2,5-dimethoxybenzoyl, 3,5-dimethoxybenzoyl, 2-fluorobenzoyl, 3-fluorobenzoyl, 4-fluorobenzoyl, 2-chlorobenzoyl, 3-chlorobenzoyl, 4-chlorobenzoyl, 2-phenyl-1-oxoethyl, 2-(2-methoxyphenyl)-1-oxoethyl, 2-(3-methoxyphenyl)-1-oxoethyl, 2-(4-methoxyphenyl)-1-oxoethyl, 2-(2,3-dimethoxyphenyl)-1-oxoethyl, 2-(2,4-dimethoxyphenyl)-1-oxoethyl, 2-(2,5-dimethoxylphenyl)-1-oxoethyl, 2-(3,5-dimethoxyphenyl)-1-oxoethyl, (2-methoxyphenyl) (2-tetrahydropyranyloxy) methyl, (3-methoxyphenyl) (2-tetrahydropyranyloxy)methyl, (4-methoxyphenyl)(2-tetrahydropyranyloxy)methyl, 2-phenyl-1-(2-tetrahydropyranyloxy)ethyl, 2,2-diphenylethyl, 2,2-bis(2-methylphenyl)ethyl, 2,2-bis(3-methylphenyl)ethyl, 2,2-bis(4-methylphenyl)ethyl, 2,2-bis (2-methoxphenyl)ethyl, 2,2-bis(3-methoxyphenyl)ethyl, 2,2-bis(4-methoxyphenyl)ethyl, 3-indolylmethyl, 4-methoxyindol-3-ylmethyl, 5-methoxyindol-3-ylmethyl, 6-methoxyindol-3-ylmethyl, cyclopropyl, cyclobutyl, cyclopentyl, cyclohexyl, cyclopropylmethyl, cyclobutylmethyl, cyclopentylmethyl, cyclohexymethyl, benzyl, phenethyl, methoxy, ethoxy, propoxy, butoxy, tert-butoxy, 2-chlorophenoxy, 3-chlorophenoxy, 4-chlorophenoxy, benzyloxy, fluoromethyl, chloromethyl, bromomethyl, carboxy, methoxycarbonyl, ethoxycarbonyl, propoxycarbonyl, butoxycarbonyl, tert-butoxycarbonyl, phenylaminocarbonyl, 2-methylphenylaminocarbonyl, 3-methylphenylaminocarbonyl, 4-methylphenylaminocarbonyl, 2-methoxyphenylaminocarbonyl, 3-methoxyphenylaminocarbonyl, 4-methoxyphenylaminocarbonyl, 2,3-dimethoxyphenylaminocarbonyl, 2,4-dimethoxyphenylaminocarbonyl, 2,5-dimethoxyphenylaminocarbonyl, 3,4,5-trimethoxyphenylaminocarbonyl, 1-naphthylaminocarbonyl, 2-naphthylaminocarbonyl, 2-pyridylaminocarbonyl, 3-pyridylaminocarbonyl, 4-pyridylaminocarbonyl, cyclopropylaminocarbonyl, cyclobutylaminocarbonyl, cyclopentylaminocarbonyl, cyclohexylaminocarbonyl, methylaminocarbonyl, ethylaminocarbonyl, propylaminocarbonyl, butylaminocarbonyl, tert-butylaminocarbonyl, phenylamino, benzylamino, phenylaminomethyl, 2-methylphenylaminomethyl, 3-methylphenylaminomethyl, 4-methylphenylaminomethyl, 2-methoxyphenylamninomethy, 3-methoxyphenylaminomethyl, 4-methoxyphenylaminomethyl, 2,3-dimethoxyphenylaminomethyl, 2,4-dimethoxyphenylaminomethyl, 2,5-dimethoxypherylaminomethyl, 2-fluorophenylaminomethyl, 3-fluorophenylaminomethyl, 4-fluorophenylaminomethyl, 2-chlorophenylaminomethyl, 3-chloromphenylaminomethyl, 4-chlorophenylaminomethyl, 2-bromophenylaminomethyl, 3-bromophenylaminomethyl, 4-bromophenylaminomethyl, 2-phenoxyethyl, 3-phenoxyethyl, 4-phenoxyethyl and phenylthiomethyl, with more preference given to phenyl, 2-methylphenyl, 3-methylphenyl, 4-methylphenyl, 4-tert-butylphenyl, 4-methoxyphenyl, 2,4-dimethoxyphenyl, 2,5-dimethoxyphenyl, 3,4-dimethoxyphenyl, 3,4,5-trimethoxyphenyl, 4-ethoxyphenyl, 4-hydroxphenyl, 3,4-dihydroxyphenyl, 2-fluorophenyl, 3-fluorophenyl, 4-flurophenyl, 2,4-difluorophenyl, 2,5-difluorophenyl, 3,4-difluorophenyl, 3,5-difluorophenyl, 2-chlorophenyl, 3-chlorophenyl, 4-chlorophenyl, 2-bromophenyl, 3-bromophenyl, 4-bromophenyl, 2-trifluromethylphenyl, 3-trifluoromethylphenyl, 4-trifluromethylphenyl, 4-trifluoromethoxyphenyl, 4-aminophenyl, 4-dimethylaminophenyl, 2-cyanophenyl, 3-cyanophenyl, 4-cyanophenyl, 2-nitrophenyl, 3-nitrophenyl, 4-nitrophenyl, 3-chloro-4-methoxyphenyl, 3,5-dichloro-4-methoxyphenyl, 2-methoxy-5-nitrophenyl,4-methoxy-3-nitrophenyl, 5-acetyl-2-methoxyphenyl, 3,4-methylenedioxyphenyl, 2-chloro-4,5 methylenedioxyphenyl, 4-benzyloxyphenyl, 3,4-dibenzyloxyphenyl, 4-formylaminophenyl, 4-acetylaminophenyl , 4-methylsulfonylphenyl, 4-methylsulfonylaminophenyl, 4-bis(methylsulfonyl)aminophenyl, 2-(1H-tetrazol-5-yl)phenyl, 3-(1H-tetrazol-5-yl)phenyl, 4-(1H-tetrazol-5-yl)phenyl, 2-(1-methyl-1H-tetrazol-5yl)phenyl, 3-(1-methyl- 1H-tetrazol-5-yl)phenyl, 4-(1-methyl-1H-tetrazol-5-yl)phenyl, 2-(1-ethyl-1H-tetrazol-5-yl)phenyl, 3-(1-ethyl-1H-tetrazol-5-yl)phenyl, 4-(1-ethyl-1H-tetrazol-5yl)phenyl, 2-(2-methyl-2H-tetrazol-5-yl)phenyl, 3-(2-methyl-2H-tetrazol-5-yl)phenyl, 4-(2-methyl-2H-tetrazol-5-yl)phenyl, 2-(2-ethyl-2H-tetrazol-5 yl)phenyl, 3-(2-ethyl-2H-tetrazol-5-yl)phenyl, 4-(2-ethyl-2H-tetrazol-5-yl)phenyl, 4-phenylphenyl, 1-naphthyl, 2-naphthyl, 2-pyridyl, 3-pyridyl, 4-pyridyl, pyridine-1-oxide, 2,6-dichloropyridin-4-yl, 3,5-dimethylisoxazolin-3-yl, 2-methyl-1, 3-thiazolin-3-yl, 2-chloro-4-thienyl, ethenyl, 1-methylethenyl, 2-phenylethenyl, 1-chloroethenyl, 1,2-dichloroethenyl, 2,2-dichloro-ethenyl, 1-bromoethenyl, phenylhydroxymethyl, 2-phenyl-1-hydroxyethyl, 2-(2-methoxyphenyl)-1-hydroxyethyl, benzoyl, 2-methoxybenzoyl, 4-methoxybenzoyl, 4-methylbenzoyl, 2,5-dimethoxybenzoyl, 4-chlorobenzoyl, 2-phenyl-1-oxoethyl, 2-(2-methoxyphenyl)-1-oxoethyl, 2-(2,5-dimethoxyphenyl)-1-oxoethyl, (2-methoxyphenyl)(2-tetrahydropyranyloxy)methyl, 2-phenyl-1-(2-tetrahydropyranyloxy)ethyl, 2,2-diphenylethyl, 3-indolymethyl, cyclopropyl, cyclohexyl, cyclohexylmethyl, benzyl, phenethyl, ethoxy, 4-chlorophenoxy, benzyloxy, bromomethyl, carboxy, ethoxycarbonyl, phenylaminocarbonyl, 4-methylphenylaminocarbonyl, 2-methoxyphenylaminocarbonyl, 2,5-dimethoxyphenylaminocarbonyl, 3,4,5-trimethoxyphenylaminocarbonyl, 1-naphthylaminocarbonyl, 3-pyridylaminocarbonyl, cyclohexylaminocarbonyl, propylaminocarbonyl, phenylamino, benzylamino, phenylaminomethyl, 4-methylphenylaminomethyl, 2-methoxyphenylaminomethyl, 2,5-dimethoxyphenyl-aminomethyl, 3-fluomphenylaminomethyl, 2-phenoxyethyl and phenylthiomethyl, and particular preference given to phenyl, 2-methylphenyl, 3-methylphenyl, 4-methylphenyl, 4-methoxyphenyl, 2,4-dimethoxyphenyl, 3,4-dimethoxyphenyl, 3,4,5-trimethoxyphenyl, 4-ethoxyphenyl, 4-hydroxyphenyl, 3,4-dihydroxyphenyl, 2-fluorophenyl, 3-fluorophenyl, 4-fluorophenyl, 2,4-difluorophenyl, 2,5-difluorophenyl, 3,4-diflurophenyl, 3,5-difluorophenyl, 2-chlorophenyl, 3-chlorophenyl, 4-chlorophenyl, 2-bromophenyl, 3-bromophenyl, 4-bromophenyl, 4-aminophenyl, 4-dimethylaminophenyl, 2-cyanophenyl, 3-cyanophenyl, 4-cyanophenyl, 2-nitrophenyl, 3-nitrophenyl, 4-nitrophenyl, 4-formylaminophenyl, 4-acetylaminophenyl, 4-methylsulfonylphenyl, 4-methylsulfonylaminophenyl, 4-bis(methylsulfonyl)aminophenyl, 2-(1H-tetrazol-5-yl)phenyl, 3-(1H-tetrazol-5-yl)phenyl, 4-(1H-tetrazol-5-yl)phenyl, 2-(1-methyl-1H-tetrazol-5-yl)phenyl, 3-(1-methyl-1H-tetrazol-5-yl)phenyl, 4-(1-methyl-1H-tetrazol-5-yl)phenyl, 2-(2-methyl-2H-tetrazol-5-yl)phenyl, 3-(2-methyl-2H-tetrazol-5yl)phenyl, 4-(2-methyl-2H-tetrazol-5-yl)phenyl, 2-pyridyl, 3-pyridyl, 4-pyridyl, pyridine-1-oxide, 2,6-dichloropyridin-4-yl, ethenyl, 1-methylethenyl, 2-phenylethenyl, 1-chloroethenyl, 1-bromoethenyl, 1,2-dichloroethenyl, 2,2-dichloroethenyl, phenhydroxymethyl, 2-phenyl-1-hydroxyethyl, 2-(2-methoxyphenyl)-1-hydroxyethyl, benzoyl, 2-methoxybenzoyl, 4-methoxybenzoyl, 2,5-dimethoxybenzoyl, 4-chlorobenzoyl, 2-phenyl-1-oxoethyl, 2-(2-methoxyphenyl)-1-oxoethyl, 2-(2,5-dimethoxyphenyl)-1-oxoethyl, 2,2-diphenylethyl, 3-indolylmethyl, cyclopropyl, cyclohexyl, cyclohexylmethyl, benzyl, phenethyl, ethoxy, 4-chlorophenoxy, benzyloxy, bromomethyl, carboxy, ethoxycarbonyl, phenylaminocarbonyl, 4-methylphenylaminocarbonyl, 2-methoxyphenylaminocarbonyl, 2,5-dimethoxyphenylaminocarbonyl, 3,4,5-trimethoxyphenylaminocarbonyl, phenylamino, benzylamino, phenylaminomethyl, 2-methoxyphenylaminomethyl, 2,5-dimethoxyphenylaminomethyl, 3-fluorophenylaminomethyl and phenylthiomethyl.

$R^4$ means hydrogen atom or halogen atom, with preference given to hydrogen atom.

Ring A is selected from the following:

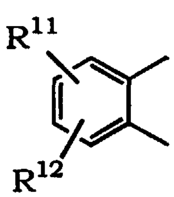 , 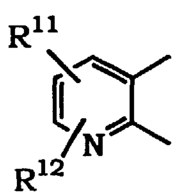 , 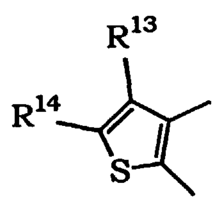 and 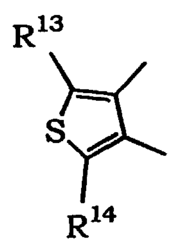

wherein $R^{11}$ and $R^{12}$ are the same or different and each is hydrogen atom, halogen atom, lower alkyl (said lower alkyl may be substituted by halogen atom, lower alkoxy, nitro, amino, amino substituted by lower alkyl, cyclic amino hydroxy, acyloxy, cyano, carbamoyl, carbamoyl, substituted by lower alkyl, cyclic aminocarbonyl, carboxy, lower alkoxycarbonyl or aralkyloxycarbonyl), lower alkenyl, aralkyl, aralkyl substituted by lower alkyl, lower alkoxy, nitro, amino, amino substituted by lower alkyl, cyclic amino, hydroxy, acyloxy, cyano, carbamoyl, carbamoyl substituted by lower alkyl, cyclic aminocarbonyl, carboxy, lower alkoxycarbonyl or aralkyloxycarbonyl, $R^{13}$ and $R^{14}$ are the same or different and each is hydrogen atom, halogen atom, lower alkyl, lower alkenyl, lower alkynyl, haloalkyl, lower alkoxy, nitro, amino, amino substituted by lower alkyl, cyclic amino, hydroxy, acyloxy, cyano, carbamoyl, carbamoyl substituted by lower alkyl, cyclic aminocarbonyl, carboxy, lower alkoxycarbonyl, aralkyloxycarbonyl, cycloalkyl or lower alkylcarbonyl, with preference given to the following:

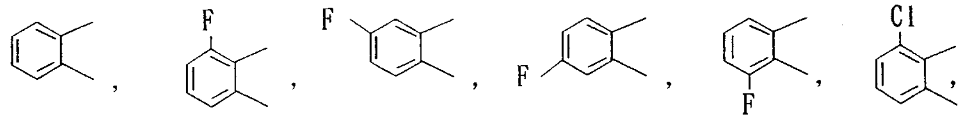,

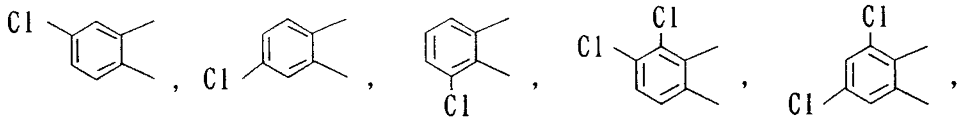,

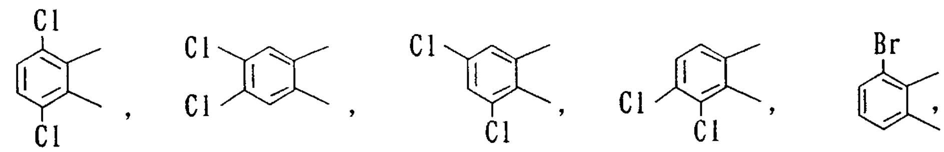,

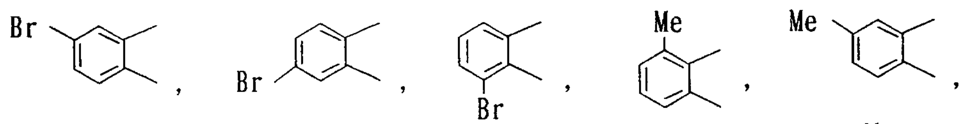,

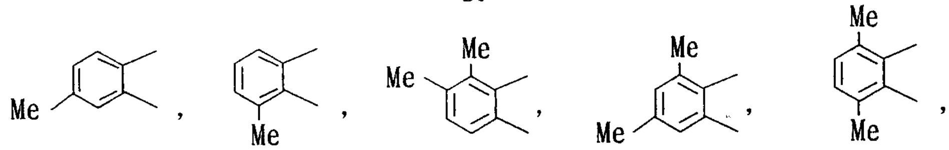,

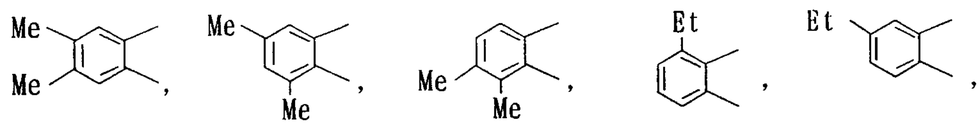,

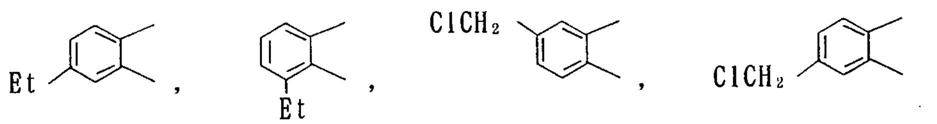,

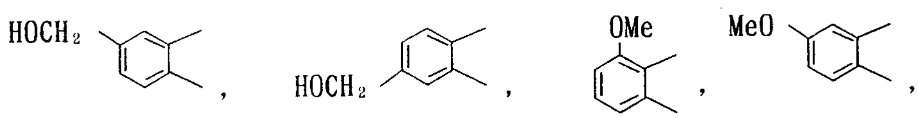,

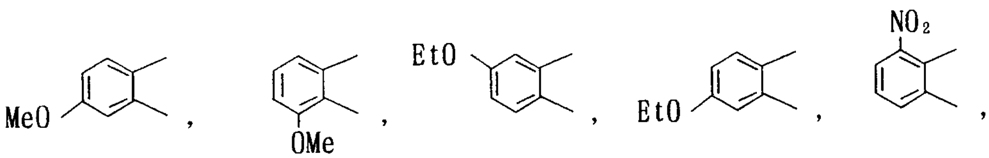,

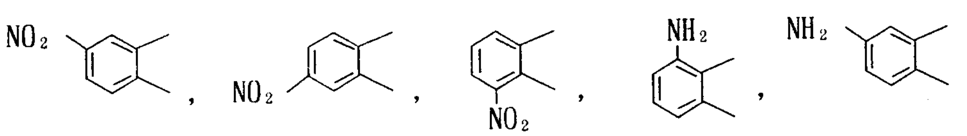,

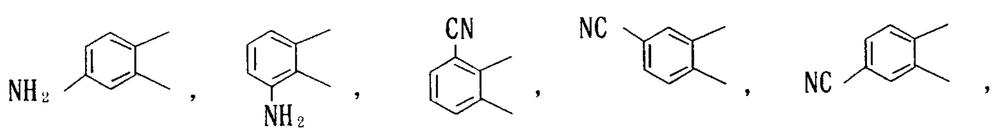,

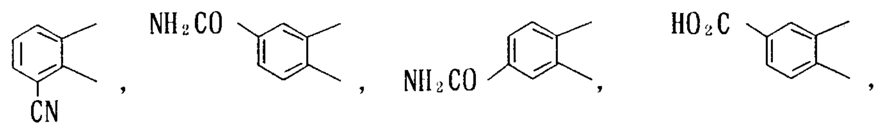
, , , ,

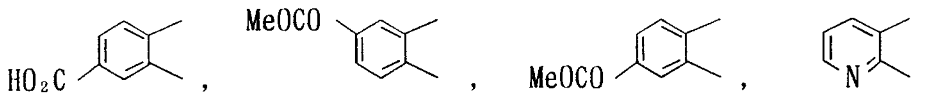
, , , ,

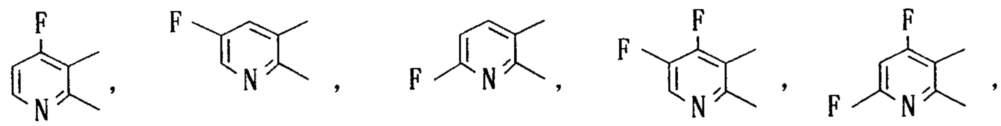
, , , , ,

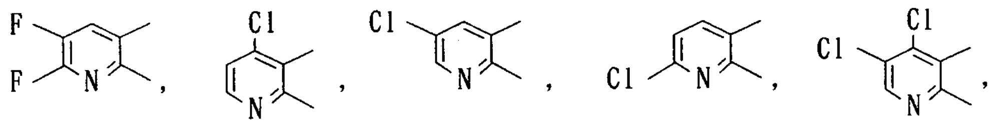
, , , , ,

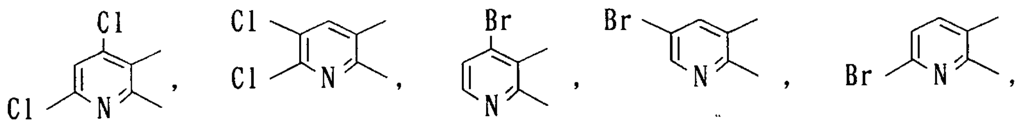
, , , , ,

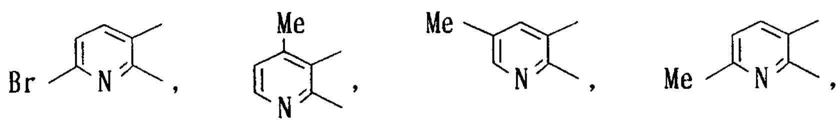
, , , ,

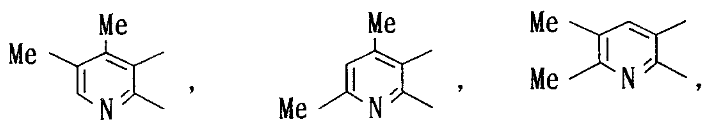
, , ,

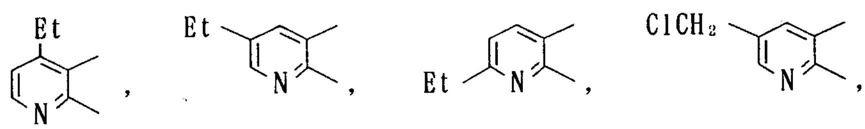
, , , ,

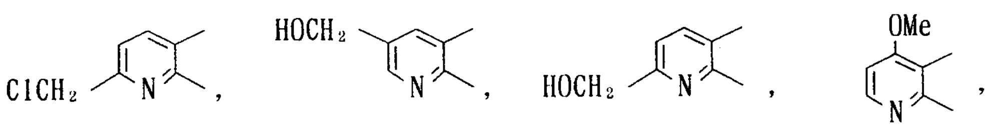
, , , ,

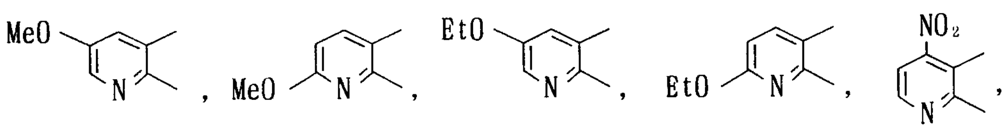
, , , , ,

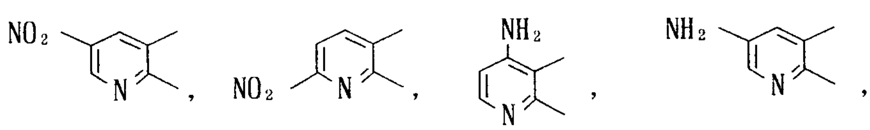
, , , ,

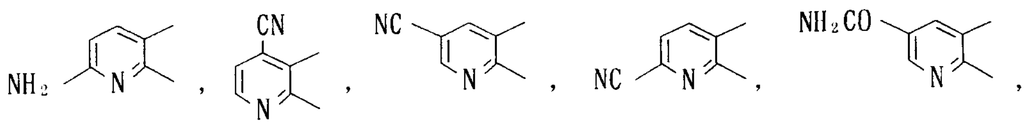

, , , , ,

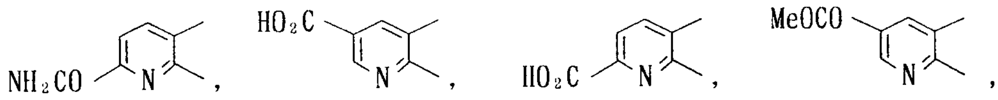

, , , ,

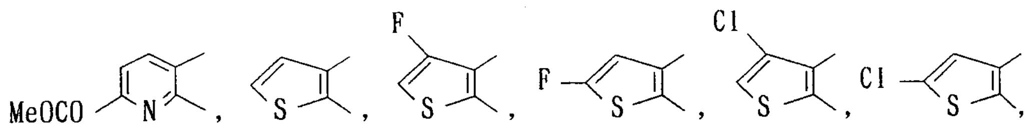

, , , , , ,

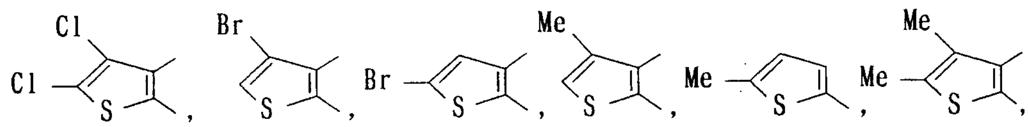

, , , , , , ,

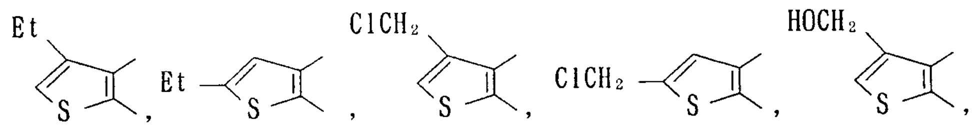

, , , , ,

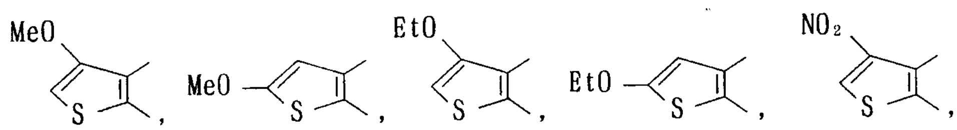

, , , , ,

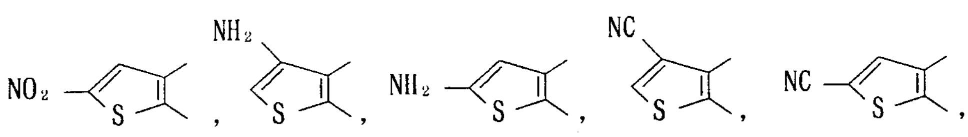

, , , , ,

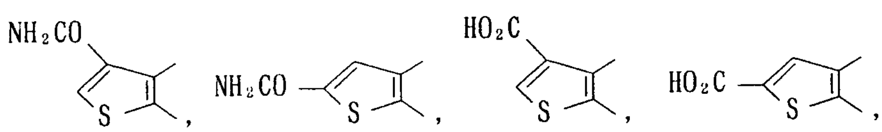

, , , ,

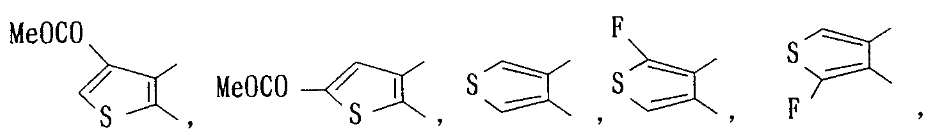

, , , , ,

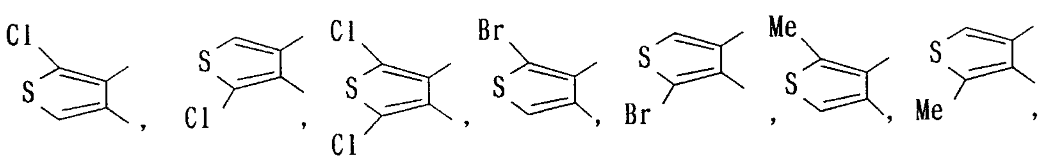

, , , , , , ,

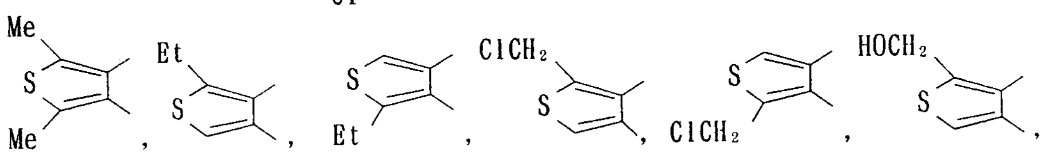

, , , , , ,

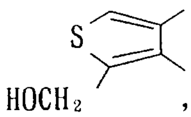 , 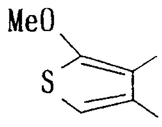 , 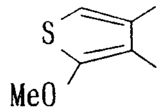 , 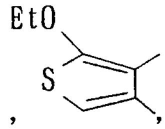 , 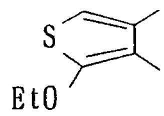 , 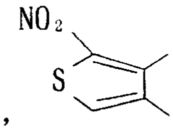 ,

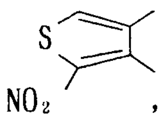 , 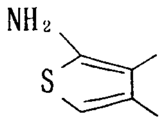 , 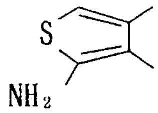 , 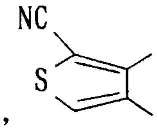 , 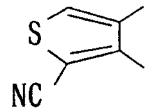 , 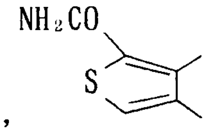 ,

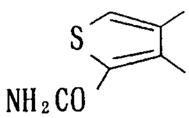 , 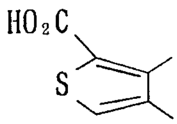 , 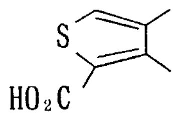 , 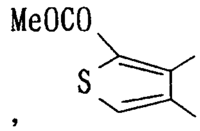 and 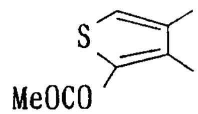

with more preference given to the following:

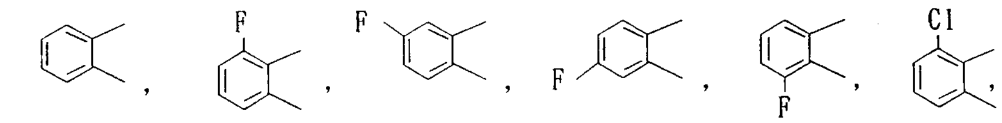

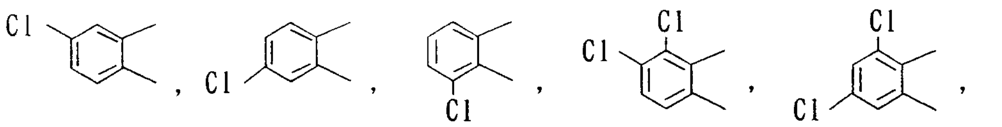

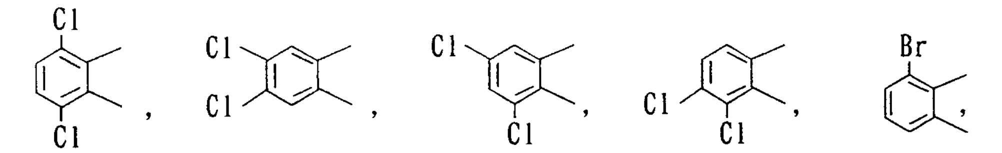

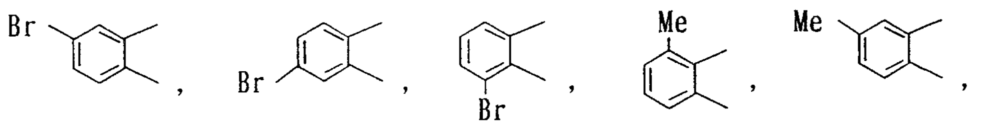

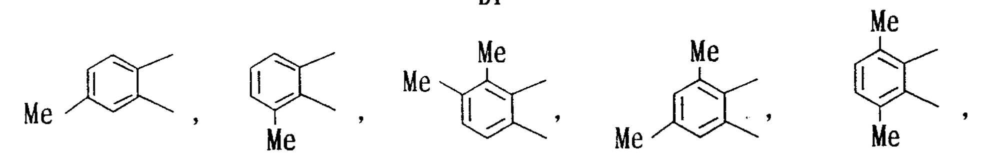

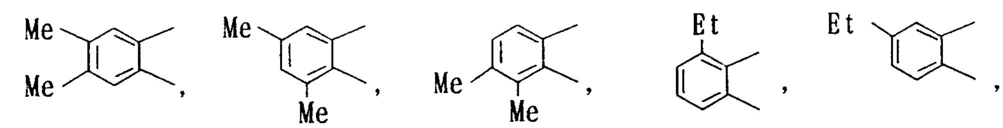

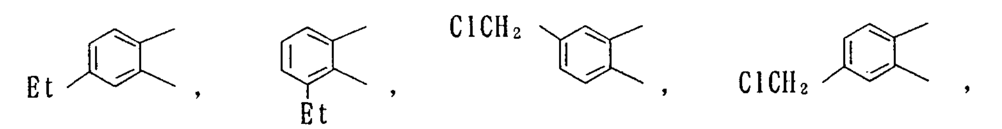

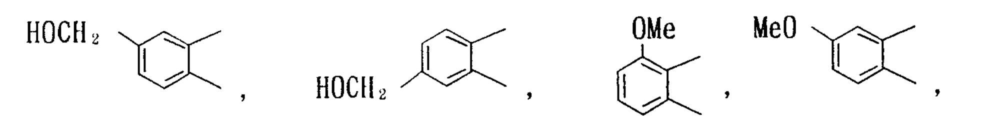

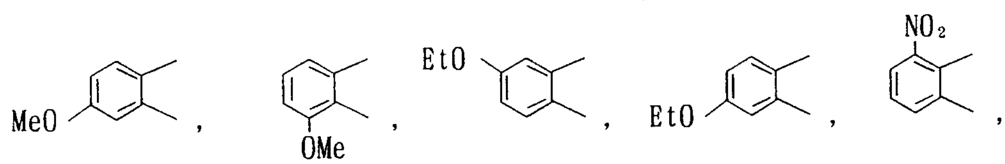

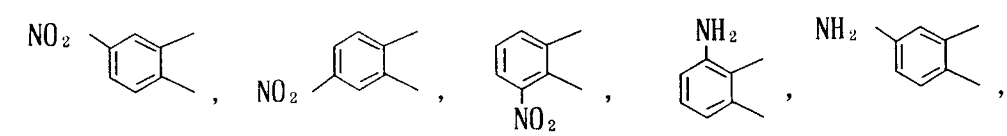

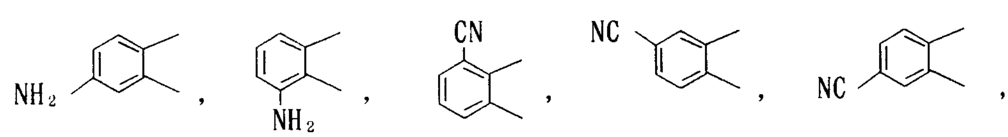

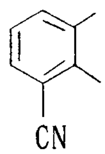, 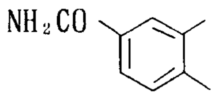, 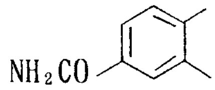, 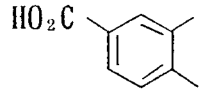,

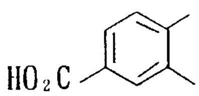, 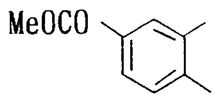, 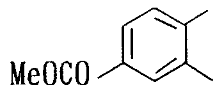, 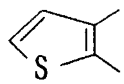, 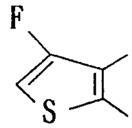,

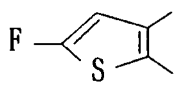, 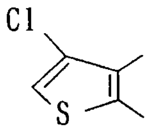, 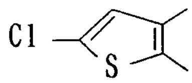, 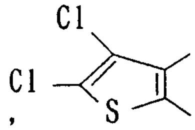, 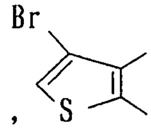, 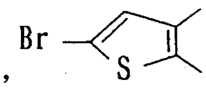,

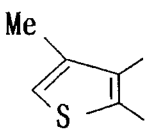, 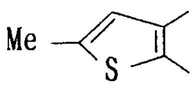, 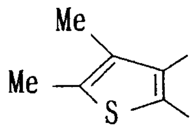, 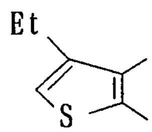, 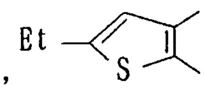, 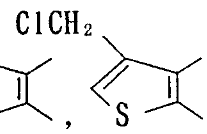,

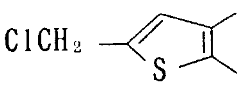, 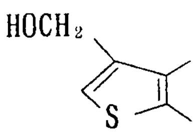, 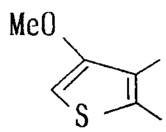, 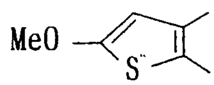, 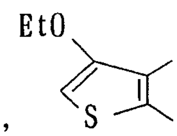,

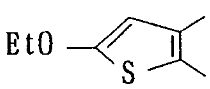, 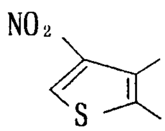, 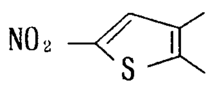, 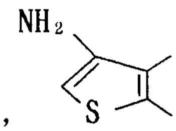, 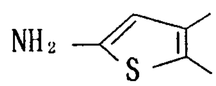,

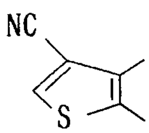, 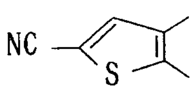, 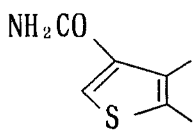, 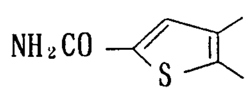, 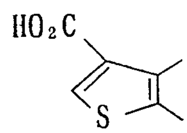,

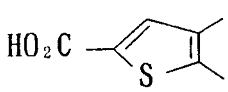, 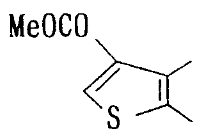 and 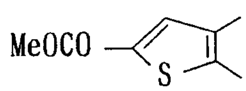

Particularly preferred are the following:

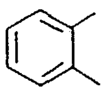, 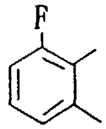, 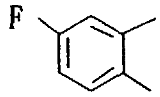, 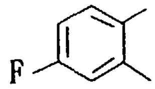, 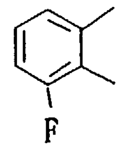, 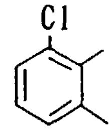,

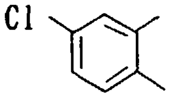, 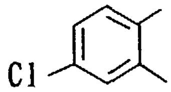, 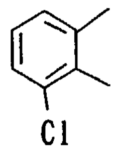, 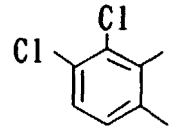, 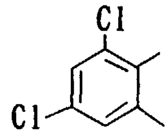,

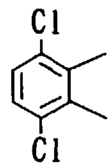, 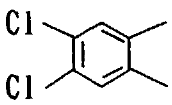, 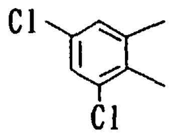, 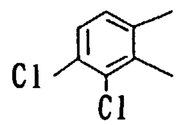, 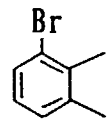,

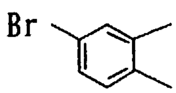, 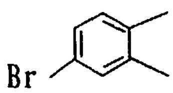, 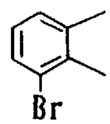, 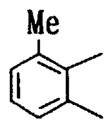, 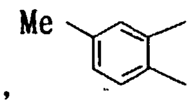, 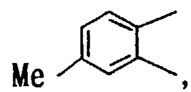,

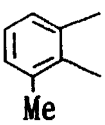, 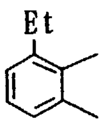, 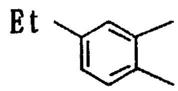, 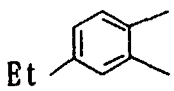, 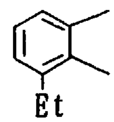, 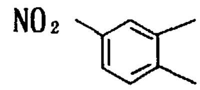,

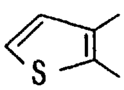, 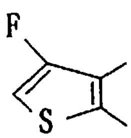, 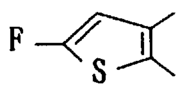, 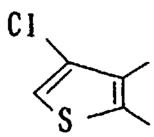, 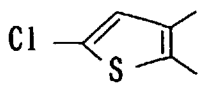,

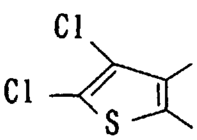, 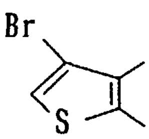, 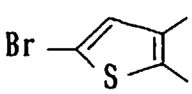, 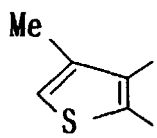, 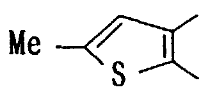,

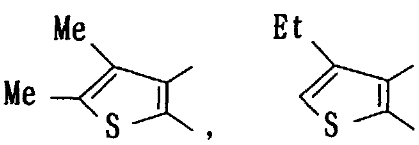, and 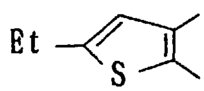

$$-V \overset{\text{----}}{=} W- \quad \text{is} \quad -\underset{R^{19}}{\underset{|}{C}}=\underset{H}{C}-, \quad -\underset{R^{15}}{\underset{|}{C}}=N- \quad \text{or} \quad -N=N-$$

wherein $R^{15}$ is lower alkyl and $R^{19}$ is hydrogen atom or lower alkyl, and particularly preferred is

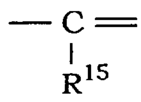
or
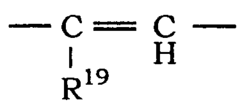

wherein $R^{15}$ and $R^{19}$ are as defined above, and particularly preferred is

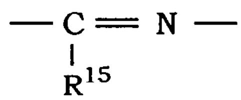

wherein $R^{15}$ is as defined above.

With regard to $R^{51}$ and $R^{52}$, preferred $R^{51}$ is hydrogen atom, optionally substituted aryl, optionally substituted heteroaryl, aralkyl, heteroarylalkyl or a group of the formula

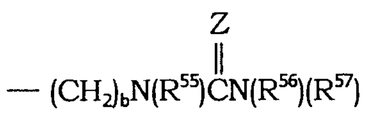 (3)

$$—(CH_2)_bN(R^{55})CORa^{56} \quad (4)$$

$$—(CH_2)_bN(R^{55})COOR^{59} \quad (6) \text{ or}$$

$$—(CH_2)_bCON(R^{61})(R^{62}) \quad (9)$$

wherein b, z, $R^{55}$, $R^{56}$, $Ra^{56}$, $R^{57}$, $R^{59}$, $R^{61}$ and $R^{62}$ are as defined above, $R^{52}$ is hydrogen atom or $-COOR^{53}$ wherein $R^{53}$ is as defined above, or $R^{51}$ and $R^{52}$ in combination form, together with the carbon atom they bind with, a spiro ring of the formula

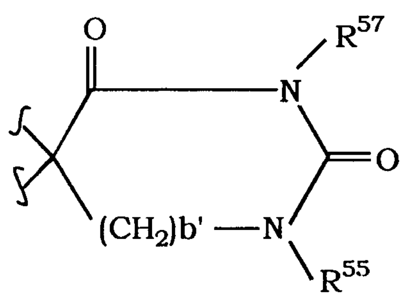

wherein b', $R^{55}$ and $R^{57}$ are as defined above.

The methods for producing the triazepine compounds of the formula [I'] of the present invention are explained in the following.

## Production 1

The method for producing, of the compounds of the formula [I'], the objective compound [I'-1] wherein

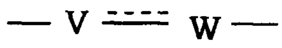 is 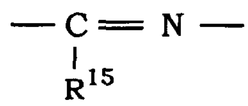

wherein $R^{15}$ is as defined above, and $R^2$ and $R^4$are both hydrogen atoms, is shown below.

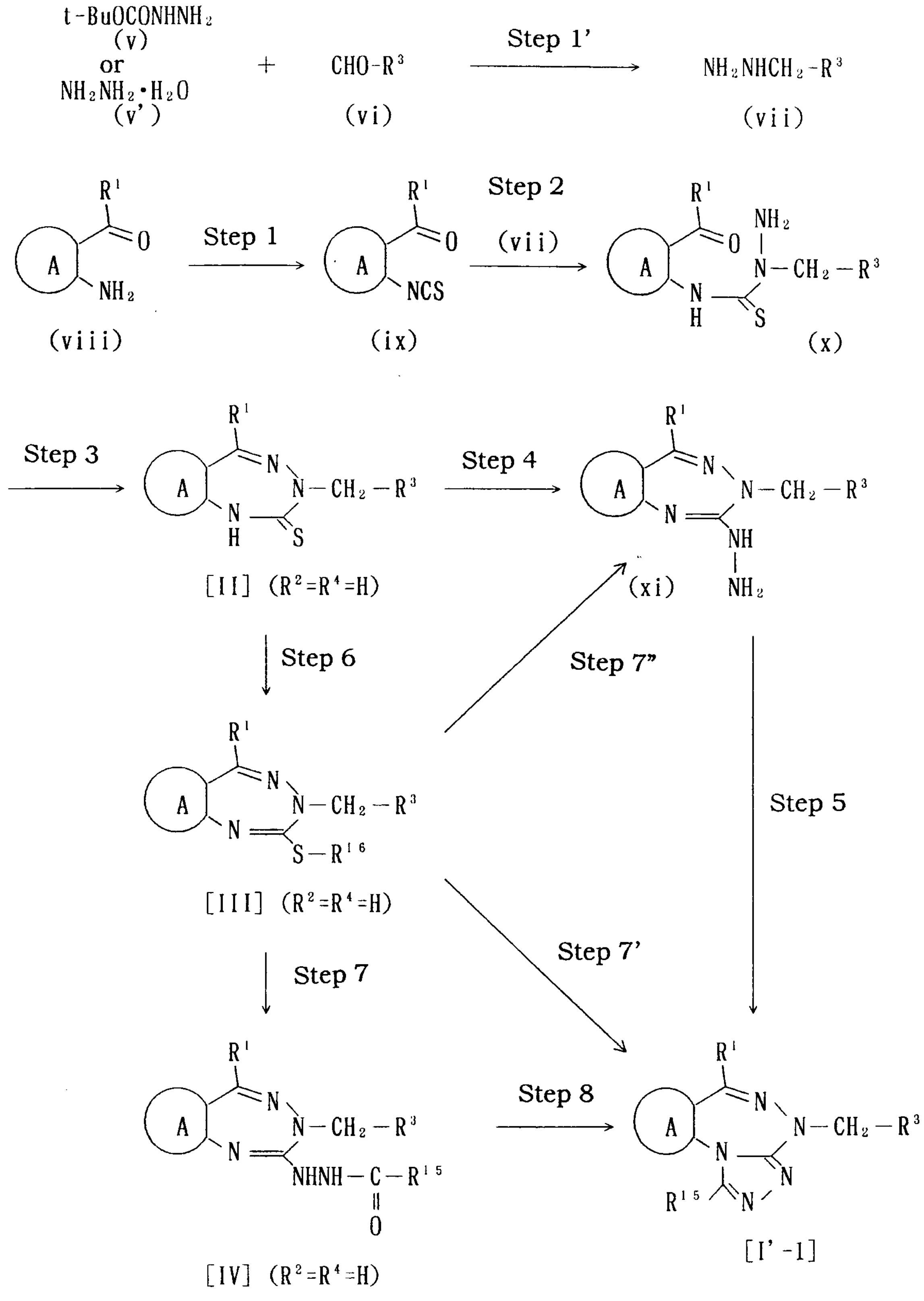


(v) or (v') + (vi) → (vii)

(viii) → (ix) → (x)

[II] ($R^2=R^4=H$)

(xi)

[III] ($R^2=R^4=H$)

[IV] ($R^2=R^4=H$)

[I'-1]

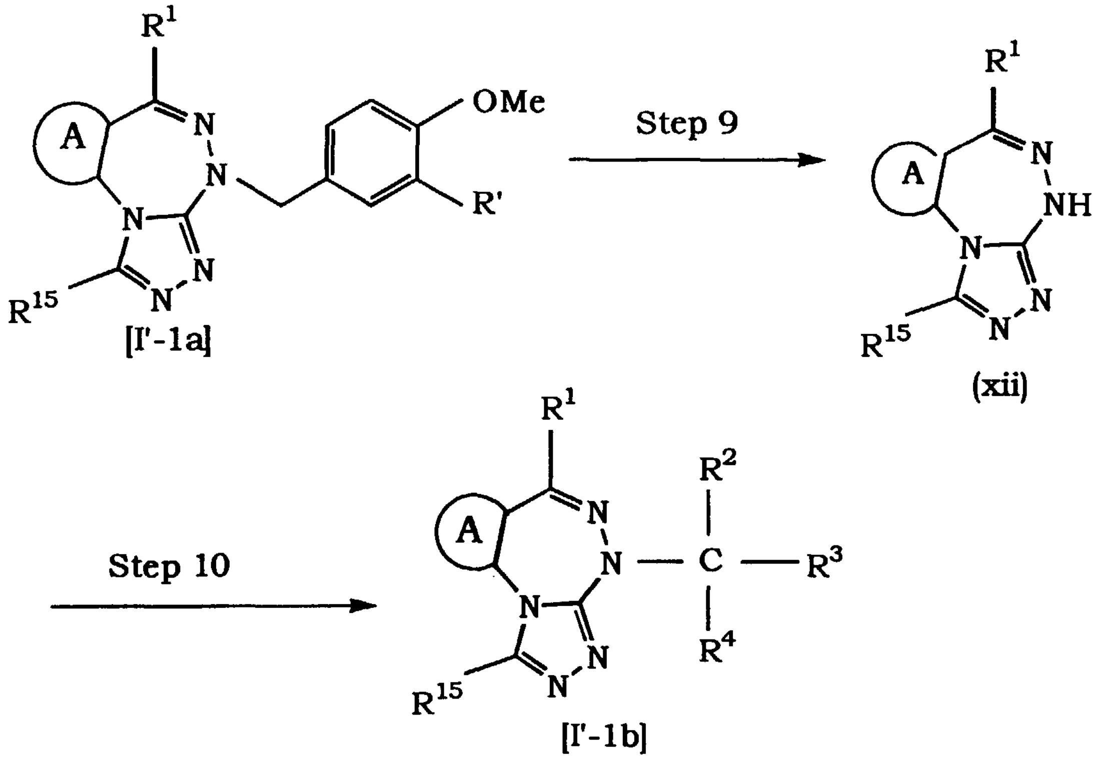


[I'-1a] (xii) [I'-1b]

Step 1

A ketone compound (viii) wherein $R^1$ and A are respectively as defined above, which is obtained by the method known or described m Japanese Patent Unexamined Publication No. 2-256681, US Patent No. 4,144,233, Journal of Organometallic Chemistry, 215, 139-150 (1981), J. Org. Chem., *56*, 3750 (1991), Synthesis, 677 (1980) and publication cited therein, Japanese Patent Unexamined Publication No. 53-121791, Heterocycles, 31, 1241 (1990), Japanese Patent Unexamined Publication No. 64-85978, is reacted with thiophosgene, thiocarbonyldiimidazole, di-2-pyridyl thiocarbonate, diethylthiocarbamyl chloride or carbon disulfide in a solvent such as dichloromethane, chloroform, tetrahydrofuran, 1,4-dioxane, methanol ethanol, n-propanol, isopropanol ethyl acetate, acetone, acetonitrile, toluene and water, or a mixed solvent thereof to give compound (ix) wherein $R^1$ and A are respectively as defined above. The reaction carried out here may be replaced for isothiocyanate synthesis generally employed.

Step 1'

This step is directed to the preparation of hydrazine compound (vii) to be used in Step 2.

A Schiff's base prepared by reacting tert-butyl carbazate (tert-butoxycarbonylhydrazine) (v) and aldehyde compound (vi) wherein $R^3$ is as defined above, is subjected to catalytic reduction using a catalyst such as palladium-carbon, palladium black palladium hydroxide-carbon and Raney nickel. Then, tert-butoxycarbonyl is deprotected using an acid such as hydrochloric acid to give desired hydrazine compound (vii) wherein $R^3$ is as defined above, or its salt The solvent to be used for these reactions may be any as long as it does not participate in the reaction, and is exemplified by methanol, ethanol, n-propanol, isopropanol, water, acetic acid and a mixed solvent thereof. The protecting group may be any, besides tert-butoxycarbonyl, as long as it can be conventionally used as a protecting group for amino, and the method for deprotection may be one conventionally used for deprotecting said protecting group. In addition, hydrazine monohydrate (v') may be used instead of protected hydrazine such as tert-butoxycarbonylhydrazine. In this case, deprotection is not necessary.

Step 2

The compound (ix) obtained in Step 1 is reacted with hydrazine compound (vii) obtained in Step 1' or its salt

in a solvent such as dichloromethane, chloroform, methanol ethanol, n-propanol isopropanol, tetrahydrofuran, 1,4-dioxane, acetone, ethyl acetate and water, or a mixed solvent thereof, under ice-cooling to under heating preferably under ice-cooling to room temperature to give compound (x) wherein $R^1$, $R^3$ and A are respectively as defined above. When a salt of hydrazine compound is used, an organic base such as triethylamine and N,N-diisopropylethylamine or an inorganic base such as sodium hydrogencarbonate is preferably added.

Step 3

The compound (x) obtained in Step 2 is heated in a solvent such as methanol, ethanol, n-propanol, isopropanol, tetrahydrofuran, 1,4-dioxane, benzene, toluene, or a mixed solvent thereof in the presence of an inorganic acid such as hydrochloric acid, sulfuric acid and hydrobromic acid or an organic acid such as p-toluenesulfonic acid and trifluoroacetic acid to give compound [II] wherein $R^1$, $R^3$ and A are as defined above, or its salt.
Alternatively, the compound obtained in Step 2 may be subjected to Step 3 without isolation.

Step 4

The compound [II] obtained in Step 3 is reacted with hydrazine or its hydrate in a solvent such as tetrahydrofuran, N,N-dimethylformamide, methanol, ethanol, n-propanol and isopropanol, or a mixed solvent thereof at room temperature to under heating to give compound (xi) wherein $R^1$, $R^3$ and A are respectively as defined above.

Step 5

The compound (xi) obtained in Step 4 is reacted with orthoester of the formula : $R^{15}$-C(OEt)$_3$ wherein $R^{15}$ is as defined above, in a solvent such as benzene, toluene and N,N-dimethylformamide, or a mixed solvent thereof under heating, preferably with reflux under heating to give objective compound [I'-1] wherein $R^1$, $R^3$, $R^{15}$and A are respectively as defined above. In this reaction, addition of an acid such as acetic acid and p-toluenesulfonic acid or silica gel is sometimes preferable.

Step 6

This step and the next step are directed to the preparation of objective compound [I'-1] from compound [II] via a different route.
The compound [II] obtained in Step 3 is dissolved or suspended in a solvent such as N,N-dimethylformamide and tetrahydrofuran and sodium hydride is added. Then, the mixture is reacted with alkyl halide of the formula : $R^{16}$-Hal wherein $R^{16}$ is lower alkyl and Hal is halogen atom, to give compound [III] wherein $R^1$, $R^3$, $R^{16}$ and A are respectively as delined above. Alternatively, the compound [II] is reacted with

$R^{16}$-Hal or 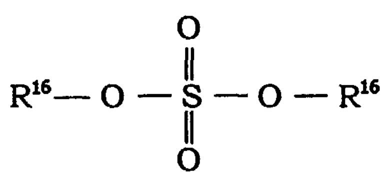

wherein $R^{16}$and Hal are respectively as defined above, in a solvent such as acetone, methyl ethyl ketone and toluene, or in a solvent sub as methanol and ethanol or a mixture of methanol or ethanol and water, in the presence of a base such as an aqueous solution of sodium carbonate, potassium carbonate, sodium hydroxide.

Step 7

The compound [III] obtained in Step 6 is dissolved or suspended in a solvent such as ethanol, n-propanol isopropanol n-butanol, toluene or a mixed solvent thereof and added with

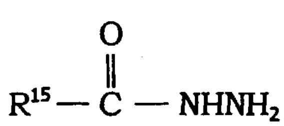

wherein $R^{15}$ is as defined above. The mixture is heated, preferably refluxed under heating to give compound [IV] wherein $R^1$, $R^3$, $R^{15}$ and A are respectively as defined above. In this case, addition of an acid such as acetic acid, p-toluenesulfonic acid and trifluoroacetic add is sometimes preferable.

Step 7'

The compound [III] obtained in Step 6 is dissolved or suspended in a solvent such as ethanol, n-propanol, isopropanol, n-butanol, toluene and the like and added with

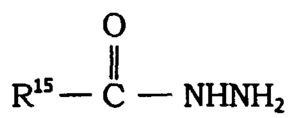

wherein $R^{15}$ is as defined above, which is followed by heating preferably at 90°C - 110°C or at a temperature higher than that to give objective compound [I'-1] wherein $R^1$, $R^3$, $R^{15}$and A are respectively as defined above. In this case again, addition of an acid such as acetic acid, p-toluenesulfonic acid and trifluoroacetic acid is sometimes preferable. When these acids are added in not less than one equivalent relative to the compound [III], a salt of compound [I'-1] can be directly obtained.

Step 7"

This step is directed to the preparation of compound (xi) by a different method.
The compound [III] is reacted with hydrazine instead of

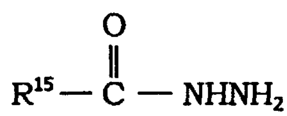

shown in Step 7, in a solvent such as methanol, ethanol, n-propanol, isopropanol and n-butanol, or a mixed solvent thereof to give compound (xi).

Step 8

The compound [IV] obtained in Step 7 is heated or preferably refluxed under heating in a solvent such as benzene and toluene to give objective compound [I'-1]. In this reaction, addition of an acid such as acetic acid, p-toluenesulfonic acid and hydrochloric acid is sometimes preferable.

Step 9

This step and the next step are directed to the substitution of a moiety

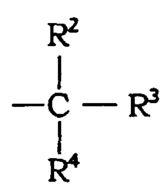

of a compound wherein $R^2$ and $R^4$ are hydrogen atoms and $R^3$ is 4-methoxyphenyl or 3,4-dimethoxyphenyl.
Of the compounds [I'-1], a compound [I'-1a] (in the reaction flow, R' is hydrogen atom or methoxy, and $R^1$, $R^{15}$ and A are as defined above) wherein $R^2$ and $R^4$ are hydrogen atoms and $R^3$ is 4-methoxyphenyl or 3,4-dimethoxyphenyl is reacted in a solvent such as chloroform, 1,4-dioxane, acetic acid and trifluoroacetic acid, or a mixed solvent thereof, in the presence of a strong acid such as methanesulfonic acid, sulfuric acid, hydrochloric acid and hydrobromic acid to give compound (xii) wherein $R^1$, $R^{15}$ and A are as defined above. In this reaction, addition of a benzyl cation

trapping agent such as phenol anisole and thioanisole is sometimes preferable. In this step, a protecting group unstable to acid can be concurrently removed. For example, tert-butyl carboxylate compound, wherein the substituent on aryl group $R^1$ is tert-butoxycarbonyl, can be converted to a carboxylic acid compound wherein the substituent on aryl group $R^1$ is carboxy. Addition of alcohol in this step results in conversion of the resulting carboxylic acid compound to an ester with alcohol used for the reaction.

Step 10-1

Compound (xii) obtained in Step 9 is reacted with

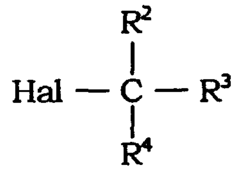

wherein Hal is halogen atom, and $R^2$, $R^3$ and $R^4$are respectively as defined above, in a solvent such as N,N-dimethylformamide, dimethyl sulfoxide, tetrahydrofuran, acetone, methyl ethyl ketone, dichloromethane, chloroform and water, or a mixed solvent thereof, in the presence of a base such as sodium hydride, potassium hydride, sodium hydroxide, potassium hydroxide, sodium carbonate, potassium carbonate, triethylamine, N,N-diisopropylethylamine, pyridine, N,N-dimethylaminopyridine, lithium bis(trimethylsilyl)amide, lithium diisopropylamide and sodium amide under ice-cooling to under heating, preferably under ice-cooling to room temperature to give objective compound [I'-1b] wherein $R^1$, $R^2$, $R^3$, $R^4$, $R^{15}$and A are respectively as defined above. In this reaction, the base to be used is appropriately selected depending on the reactivity and stability of the compound

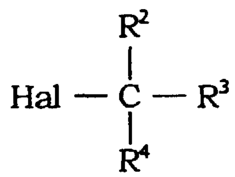

to be reacted. These bases may be used in an appropriate combination as the case demands.

Step 10-2

When a reaction is carried out using a compound

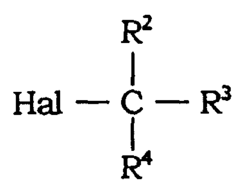

as shown in Step 10-1 wherein $R^2$ and $R^4$ are hydrogen atoms, the use of the same solvent and the base as in Step 10-1 in the presence of the air (oxygen) may result in the production of objective compound [I'-1b] wherein $R^2$ is hydroxy and $R^4$ is hydrogen atom.

Step 10-3

According to the method of Edward et al [Tetrahedron Lett., *31*, 3417(1990)], compound (xii) is reacted with an alcohol compound of the formula : $R^3R^4CHOH$ wherein $R^3$ and $R^4$ are as defined above, and triphenylphosphine in a solvent such as tetrahydrofuran, diethyl ether, 1,4-dioxane and N,N-dimethylformamide. Then, dialkyl azodicarboxylate is added to the reaction system and the mixture is reacted under ice-cooling to 40°C, preferably at room temperature or 25°C to give objective compound [I'-1b] wherein $R^2$ is hydrogen atom.

Step 10-4

The compound (xii) is reacted in a solvent such as methanol, ethanol, n-propanol and isopropanol using an aqueous solution of formaldehyde and the solvent used ($R^{3}$"$CH_2OH$ wherein $R^{3}$" is lower alkyl) as reagents, or with an aqueous solution of formaldehyde and Y'$NH_2$wherein Y' is an optionally substituted aryl or an optionally substituted heteroaryl, at room temperature to heat-refluxing temperature to give objective compound [I'-1b] wherein $R^2$ and $R^4$ are both hydrogen atoms and $R^3$ is a lower alkoxy or -NHY' wherein Y' is as defined above.

Step 10-5

The compound (xii) is reacted with an isocyanate compound of the formula : Y'NCO wherein Y' is as defined above, in a solvent such as dichloromethane, chloroform, N,N-dimethylformamide, tetrahydrofuran and anhydrous acetonitrile in the presence of, where necessary, an inorganic base such as sodium hydroxide and potassium hydroxide or organic base such as triethylamine and N,N-diisopropylethylamine to give objective compound [I'-1b] wherein $R^2$ and $R^4$ form carbonyl together with the carbon atom to which $R^2$ and $R^4$ bind and $R^3$ is -NHY' wherein Y' is as defined above.

Step 10-6

The compound (xii) is reacted with an oxirane compound of the formula

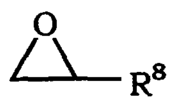

wherein $R^8$ is as defined above, in a solvent such as N,N-dimethylformamide, dimethyl sulfoxide and tetrahydrofuran, in the presence of a base such as sodium hydride and potassium hydride to give objective compound [I'-1b] wherein both $R^2$ and $R^4$ are hydrogen atoms and $R^3$ is of the formula

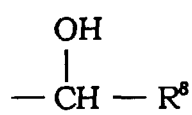

wherein $R^8$is as defined above.

Step 10-7

Of the compounds obtained in Step 10-1, a compound wherein $R^3$ is expressed by the formula

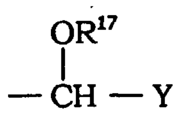

or

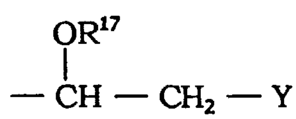

wherein $R^{17}$is a protecting group for hydroxy and Y is as defined above, is deprotected to give objective compound [I'-1b] wherein $R^3$ is

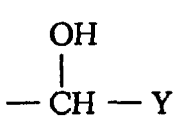

or

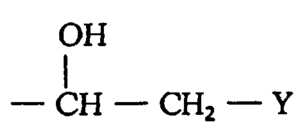

wherein Y is as defined above. The protecting group for hydroxy may be any as long as it is conventionally used for this end, and when tetrahydropyranyl is used for example, the solvent may be any as long as it does not participate in the

reaction, such as methanol ethanol, n-propanol, isopropanol, tetrahydrofuran, 1,4-dioxane, dichloromethane and chloroform, and the reagent may be an organic acid such as p-toluenesulfonic acid, pyridinium-p-tolenesulfonic acid and trifluoroacetic acid, or an inorganic acid such as hydrochloric acid, sulfuric acid and hydrobromic acid.

Step 10-8

The compound obtained in Step 10-6 or 10-7 wherein $R^3$ is

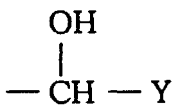

or

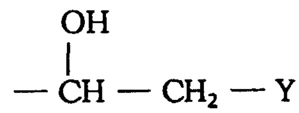

is subjected to an oxidation with an oxidizing agent such as chromium trioxide, pyridinium chlorochromate, pyridinium chromate, Jones reagent (mixture of chromium trioxide and sulfuric acid) and an oxidizing agent prepared from dimethyl sulfoxide and oxalyl chloride in a solvent such as acetic acid, pyridine, dichloromethane and water, or a mixed solvent thereof to give objective compound [I'-1b] wherein $R^3$ is

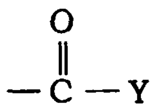

or

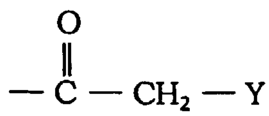

wherein Y is as defined above.

Step 10-9

Of the compounds obtained in Step 10-1, a compound wherein $R^3$ is $-CO_2Et$ is reacted with a base such as sodium hydroxide and potassium hydroxide, in a solvent such as methanol, ethanol, n-propanol, isopropanol, tetrahydrofuran, 1,4-dioxane and water, or a mixed solvent thereof to give objective compound [I'-1b] wherein $R^3$ is $-CO_2H$.

Step 10-10

The compound obtained in Step 10-9 wherein $R^3$ is $-CO_2H$ is reacted with alkyl halocarbonate such as ethyl chlorocarbonate and isobutyl chlorocarbonate, and $R^8NH_2$ wherein $R^8$ is as defined above, in a solvent sub as N,N-dimethylformamide, dimethyl sulfoxide, tetrahydrofuran, acetone and dichloromethane, or a mixed solvent thereof, in the presence of a base such as triethylamine and N,N-diisopropylethylamine to give objective compound [I'-1b] wherein $R^3$ is $-CONHR^8$ wherein $R^8$ is as defined above. Alternatively, conventional peptide linkage formation is followed to give objective compound [I'-1b] wherein $R^3$ is $-CONHR^8$ [Nobuo Izumiya, *Pepuchido Goseino Kiso to Jikken,* Maruzen (1985)].

Step 10-11

Of the compounds obtained in Step 10-1, a compound wherein $R^3$ is $-CH_2$-Hal wherein Hal is halogen atom, is reacted with $Y'NH_2$ wherein Y' is as defined above, or Y'SH wherein Y' is as defined above, in a solvent such as ethanol, dichloromethane, N,N-dimethylformamide, dimethyl sulfoxide, tetrahydrofuran and water, or a mixed solvent thereof, in the presence or absence of a base such as sodium hydride, potassium hydride, sodium hydroxide, potassium hydroxide, potassium carbonate, triethylamine, N,N-diisopropylethylamine, anisidine and pyridine to give objective compound [I'-1b] wherein $R^3$ is $-CH_2NHY'$wherein Y' is as defined above or $-CH_2SY'$ wherein Y' is as defined above.

Step 10-12

Of the compounds obtained in Step 10-1, a compound wherein $R^3$ is substituted aryl or substituted heteroaryl and the substituent is nitro is subjected to catalytic reduction using hydrogen, in the presence of a catalyst such as palladium-carbon, palladium hydroxide-carbon, palladium black and Raney nickel, or catalytic reduction using formic acid, ammonium formate, cyclohexene or cyclohexadiene in the presence of the above-mentioned catalyst, in a solvent such

as methanol, ethanol, n-propanol, isopropanol, 1,4-dioxane, acetic acid and water, or a mixed solvent thereof. Alternatively, reduction using a reducing agent such as sodium borohydride and lithium borohydride in the above-mentioned solvent gives objective compound [I'-1b] wherein $R^3$ is substituted aryl or substituted heteroaryl and the substituent is amino, or objective compound [I'-1b] wherein $R^3$ is substituted aryl or substituted heteroaryl and the substituent is formylamino. A mixture thereof may be obtained, which is separated by a conventional method.

Step 10-13

The compound obtained in Step 10-12 wherein $R^3$ is substituted aryl or substituted heteroaryl and the substituent is amino is reacted with an acylating agent such as acetic anhydride, or acetic anhydride and formic add, or acetyl chloride, or alkylsulfonyl halide such as methanesulfonyl chloride, in a solvent such as dichloromethane, chloroform, pyridine, ethanol acetone and tetrahydrofuran to, give objective compound [I'-1b] wherein $R^3$ is substituted aryl or substituted heteroaryl and the substituent is acylamino, alkylsulfonylamino or bis(alkylsulfonyl)amino. Addition of a base such as triethylamine and N,N-diisopropylethylamine may be preferable as the case demands.

Step 10-14

The compound obtained in Step 10-12 wherein $R^3$ is substituted aryl or substituted heteroaryl and the substituent is amino is subjected to reduction under a hydrogen atmosphere using a catalyst such as palladium-carbon, palladium hydroxide-carbon and palladium black, in a solvent such as methanol, ethanol, n-propanol, isopropanol and water, or a mixed solvent thereof in the presence of an aqueous solution of formaldehyde to give objective compound [I'-1b] wherein $R^3$ is substituted aryl or substituted heteroaryl and the substituent is methylamino or dimethylamino. A mixture thereof may be obtained, which is separated by a conventional method.

Step 10-15

Of the compounds obtained in Step 10-1, a compound wherein $R^3$ is substituted aryl or substituted heteroaryl and the substituent is benzyloxy is subjected to reduction under a hydrogen atmosphere using a catalyst such as palladium-carbon, palladium hydroxide-carbon and palladium black, Raney nickel or catalytic reduction using formic acid, ammonium formate, cyclohexene or cyclohexadiene in the presence of a catalyst such as palladium-carbon, palladium hydroxide-carbon, palladium black and Raney nickel, in a solvent such as methanol, ethanol, n-propanol, isopropanol, 1,4-dioxane and acetic acid, or a mixed solvent thereof to give objective compound [I'-1b] wherein $R^3$ is substituted aryl or substituted heteroaryl and the substituent is hydroxy. When the substituent is dibenzyloxy, a corresponding catechol compound is obtained by the above reaction.

Step 10-16

The compound obtained in Step 10-15 wherein $R^3$ is substituted aryl or substituted heteroaryl and the substituent is hydroxy is reacted with diazoalkane such as diazomethane and diazoethane, in a solvent such as tetrahydrofuran, diethyl ether and 1,4-dioxane to give objective compound [I'-1b] wherein $R^3$ is substituted aryl or substituted heteroaryl and the substituent is lower alkoxy.

Step 10-17

Of the compounds obtained in Step 10-1, a compound wherein $R^3$ is pyridyl is subjected to oxidation in a solvent such as dichloromethane, benzene, toluene, chloroform, dichloroethane, ethyl acetate, hexane and dioxane or a mixed solvent thereof with a peracid such as m-chloroperbenzoic acid and peracetic acid or hydrogen peroxide to give objective compound [I'-1b] wherein $R^3$ is pyridine-1-oxide.

Step 10-18

Of the compounds obtained in Step 10-1, a compound wherein $R^1$ is substituted aryl and said substituent is lower alkoxycarbonyl is reacted for deprotection in a solvent such as 1,4-dioxane, diethyl ether, 1,2-dimethoxyethane, tetrahydrofuran, N,N-dimethylformamide, dimethyl sulfoxide, acetonitrile, acetone, methanol, ethanol, propanol and butanol in the presence of a base such as sodium hydroxide, potassium hydroxide and lithium hydoxide at 0°C- 100°C, preferably from 0°C to room temprature, according to the method disclosed in Protective Groups in Organic Synthesis (John Wiley & Sons Inc. (1991)), whereby to convert lower alkoxycarbonyl to carboxy, to give objective compound [I'-1b] wherein $R^1$ is substituted aryl and said substituent is carboxy.

Alternatively, the compound can be deprotected using an inorganic acid such as hydrochloric acid and sulfuric acid in the above-mentioned solvents, according to the method disclosed in the above-mentioned publication.

Step 10-19

The compound obtained in Step 10-18 wherein $R^1$ is substituted aryl and said sustituent is carboxy is subjected to an improved Curtius reaction in a solvent such as methanol, ethanol, propanol and tert-butanol in the presence of an organic base such as trimethylamine by the use of diphenylphosphoryl azide according to the method of T. Shioiri [Journal of American Chemical Society, *94*, 6203(1972)] to give objective compound [I'-1b] wherein $R^1$ is substituted aryl and said sustituent is alkoxycarbonylamino.

Step 10-20

The compound obtained in Step 10-19 wherein $R^1$ is substituted aryl and said sustituent is alkoxycarbonylamino is subjected to deprotection in a solvent such as methanol, ethanol, propanol, butanol, 1,4-dioxane, diethyl ether, 1,2-dimethoxyethane and tetrahydrofuran or a mixed solvent thereof with water, in the presence of an acid such as hydrochloric acid, hydrobromic acid and p-toluenesulfonic acid at room temprature to under heating according to the method disclosed in Protective Groups in Organic Synthesis (John Wiley & Sons Inc. (1991)), to give objective compound [I'-1b] wherein $R^1$ is substituted aryl and said sustituent is amino.

Step 10-21

The compound obtained in Step 10-20 wherein $R^1$ is substituted aryl and said sustituent is amino is subjected to oxidation in a solvent such as dichloromethane, chloroform, carbon tetrachloride, 1,2-dichloroethane and acetic acid, using an oxidizing agent such as sodium peroxoborate, according to the method disclosed in oxidations in Organic Chemistry [American Chemical Society (1990)] to give objective compound [I'-1b] wherein $R^1$ is substituted aryl and said sustituent is nitro.

Step 10-22

Of the compounds obtained in Step 10-1, a compound wherein $R^2$ and $R^4$ are hydrogen atoms, $R^3$ is substituted phenyl and said substituent is cyano is reacted with sodium azide in a solvent such as 1,4-dioxane, diethyl ether, 1,2-dimethoxyethane, tetrahydrofuran, N,N-dimethylformamide, dimethyl sulfoxide, acetonitrile, acetone, methanol ethanol, propanol and butanol, in the presence of an acid such as ammonium chloride, hydrochloric acid and acetic acid, according to the method disclosed in Journal of Medicinal Chemistry, *27,* 1565 (1984) or the method of K. Raman [J. Heterocycl. Chem. *17*, 1137 (1980) to give objective compound [I'-1b] wherein $R^3$ is substituted phenyl and said sustituent is tetrazolyl.

Step 10-23

The compound obtained in Step 10-22 wherein $R^3$ is substituted phenyl and said sustituent is tetrazolyl is reacted with diazoalkane such as diazomethane in a solvent such as diethyl ether, tetrahydrofuran, methanol, ethanol and propanol, to give objective compound [I'-1b] wherein $R^3$ is substituted phenyl and said sustituent is alkyltetrazolyl.

Step 10-24

Of the compounds obtained in Step 10-1, a compound wherein $R^2$ and $R^4$ are hydrogen atoms and $R^3$ is phenyl substituted by lower alkoxycarbonyl (e.g., methoxycarbonyl, ethoxycarbonyl, propoxycarbonyl and butoxycarbonyl) is reacted with a base such as sodium hydroxide, potassium hydroxide and lithium hydroxide in a solvent such as methanol, ethanol, n-propanol, isopropanol tetrahydrofuran, 1,4-dioxane and water or a mixed solvent thereof to give objective compound [I'-1b] wherein $R^2$ and $R^4$ are hydrogen atoms and $R^3$ is phenyl substituted by carboxy.

Production 2

In this section, a method for producing objective compound [1'-2] by a production method different from Production 1 is shown.

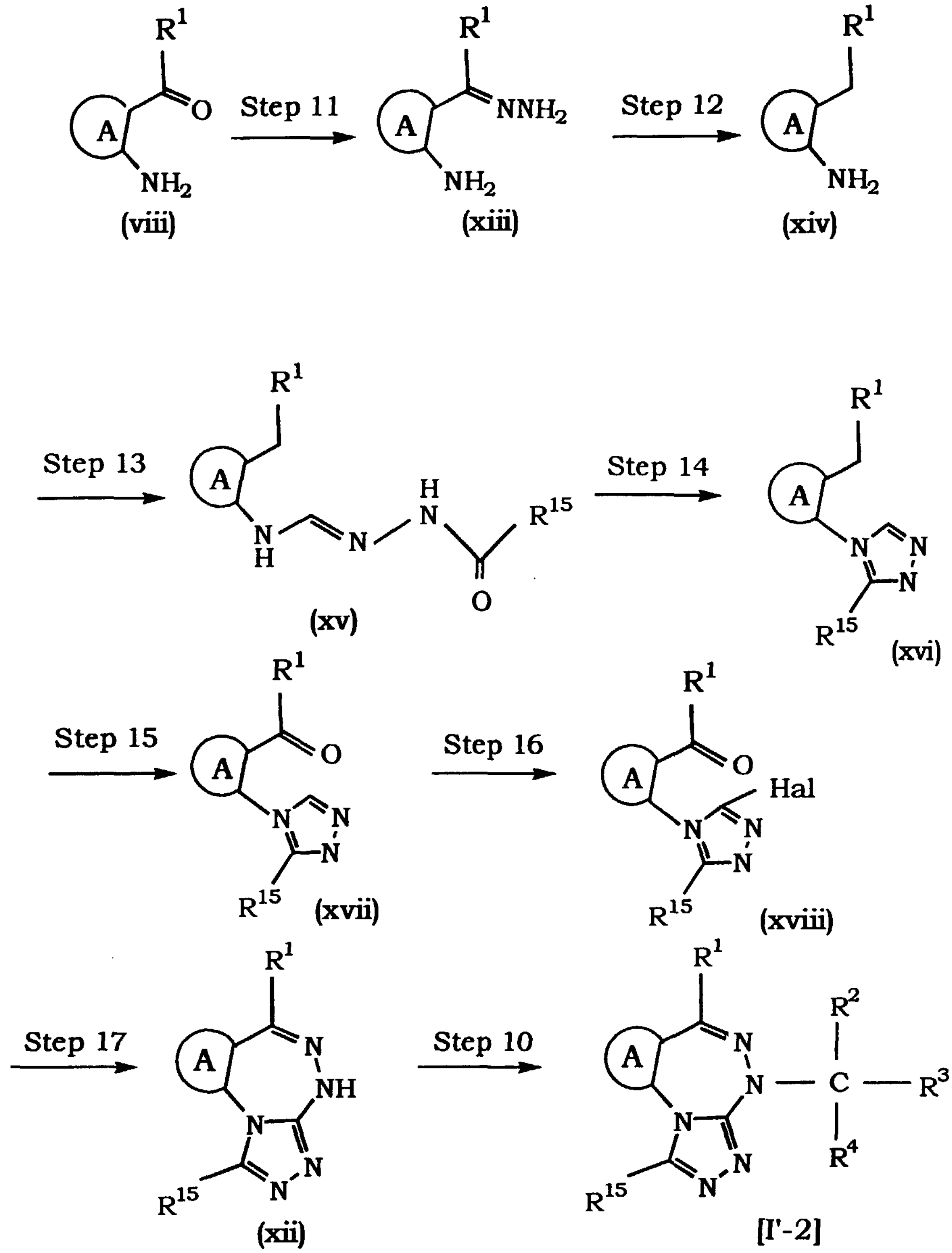


(viii) (xiii) (xiv) (xv) (xvi) (xvii) (xviii) (xii) [I'-2]

Step 11

The compound (viii) is reacted with hydrazine in a solvent such as diethylene glycol, ethylene glycol monomethyl ether, dimethyl sulfoxide and N,N-dimethylformamide under heating, preferably at a temperature not less than 150°C to give compound (xiii) wherein $R^1$ and A are respectively as defined above.

Step 12

The compound (xiii) obtained in Step 11 is reacted in a solvent such as diethylene glycol, ethylene glycol monomethyl ether, dimethyl sulfoxide and N,N-dimethylformamide in the presence of a base such as sodium hydroxide, potassium hydroxide and lithium hydroxide under heating, preferably from 80°C to 150°C, to give compound (xiv) wherein $R^1$ and A are respectively as defined above.

Step 13

The compound (xiv) obtained in Step 12 and tri(lower)alkyl orthoformate such as thiethyl orthoformate are reacted under heating, preferably from 100°C to 150°C. Then, the mixture is reacted with an aryl hydrazide compound of the formula : $R^{15}CONHNH_2$ wherein $R^{15}$ is as defined above, in a solvent such as methanol, ethanol, n-propanol and isopropanol, preferably at room temperature to give compound (xv) wherein $R^1$, $R^{15}$ and A are respectively as defined above.

Step 14

The compound (xv) obtained in Step 13 is reacted in a solvent such as diethylene glycol dimethyl ether, ethylene glycol monomethyl ether, dimethyl sulfoxide, N,N-dimethylformamide, pyridine and water, or a mixed solvent thereof under heating, preferably with reflux under heating to give compound (xvi) wherein $R^1$, $R^{15}$ and A are respectively as defined above.

Step 15

The compound (xvi) obtained in Step 14 is subjected to oxidation with an oxidizing agent such as chromium trioxide, pyridinium chlorochromate and Jones reagent in a solvent such as acetic add, pyridine, dichloromethane and water, or a mixed solvent thereof to give compound (xvii) wherein $R^1$, $R^{15}$ and A are respectively as defined above.

Step 16

The compound (xvii) is halogenated with a halogenating agent such as N-bromosuccinimide, N-chlorosuccinimide, N-iodosuccinimide and carbon tetrabromide in a solvent such as carbon tetrachloride, chloroform, dichloromethane and 1,2-dichloroethane under heating, preferably with reflux under heating to give compound (xviii) wherein $R^1$, $R^{15}$ and A are respectively as defined above, and Hal means halogen.

Step 17

The compound (xviii) is reacted with hydrazine sulfate in a solvent such as methanol, ethanol, n-propanol and isopropanol, or a mixed solvent thereof in the presence of a weak base such as sodium acetate, potassium acetate and lithium acetate under heating, preferably with reflux under heating to give compound (xii) wherein $R^1$, $R^{15}$ and A are respectively as defined above. This compound can be introduced into the objective compound [1'-2] wherein $R^1$, $R^2$, $R^3$, $R^4$, $R^{15}$and A are respectively as defined above, by the method shown in Step 10.

Production 3

A novel method for producing the objective compound [1'-3] by a production method different from the production method shown in Production 1 and Production 2 is shown the the following.

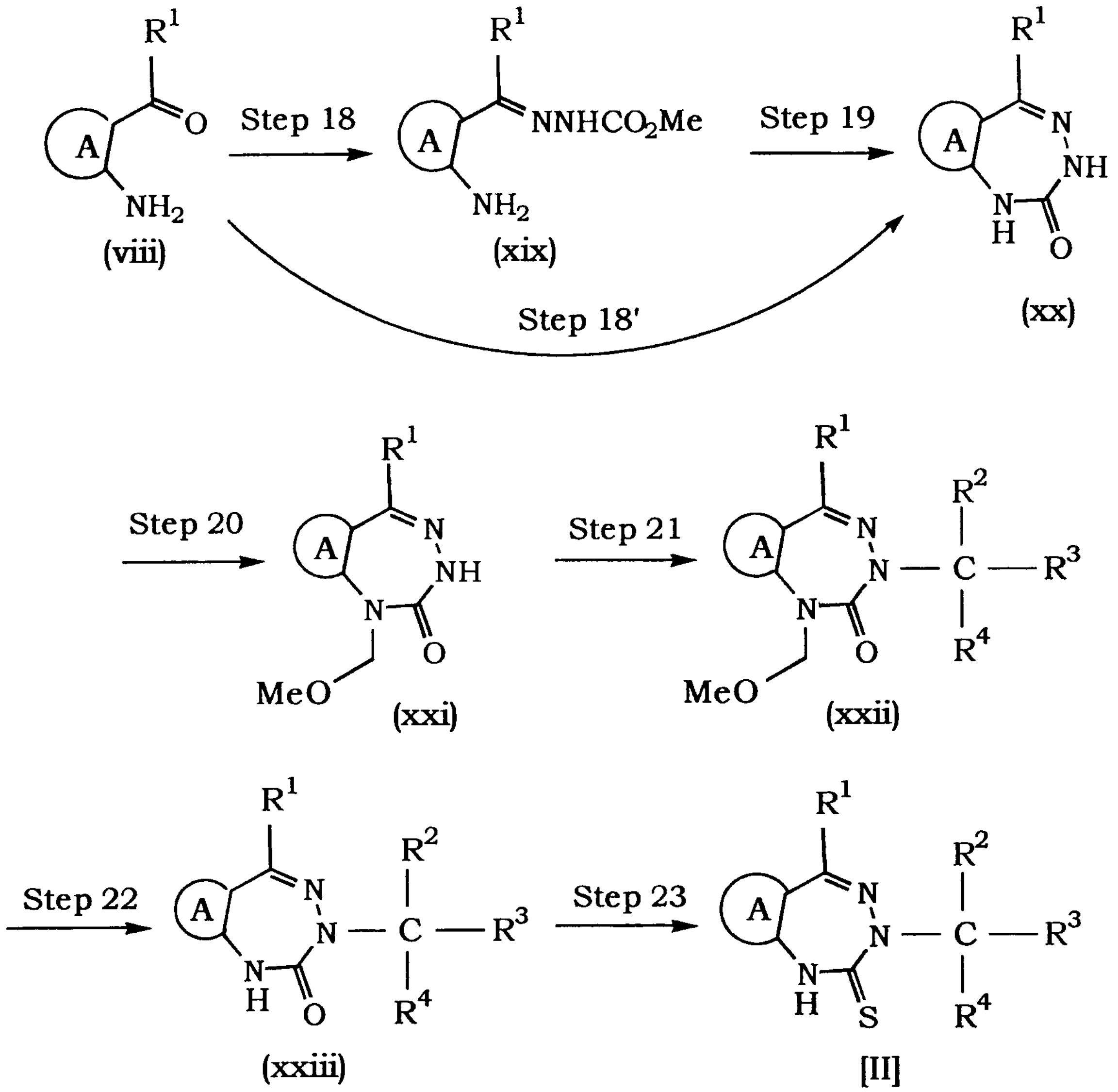


(viii) (xix) (xx) (xxi) (xxii) (xxiii) [II]

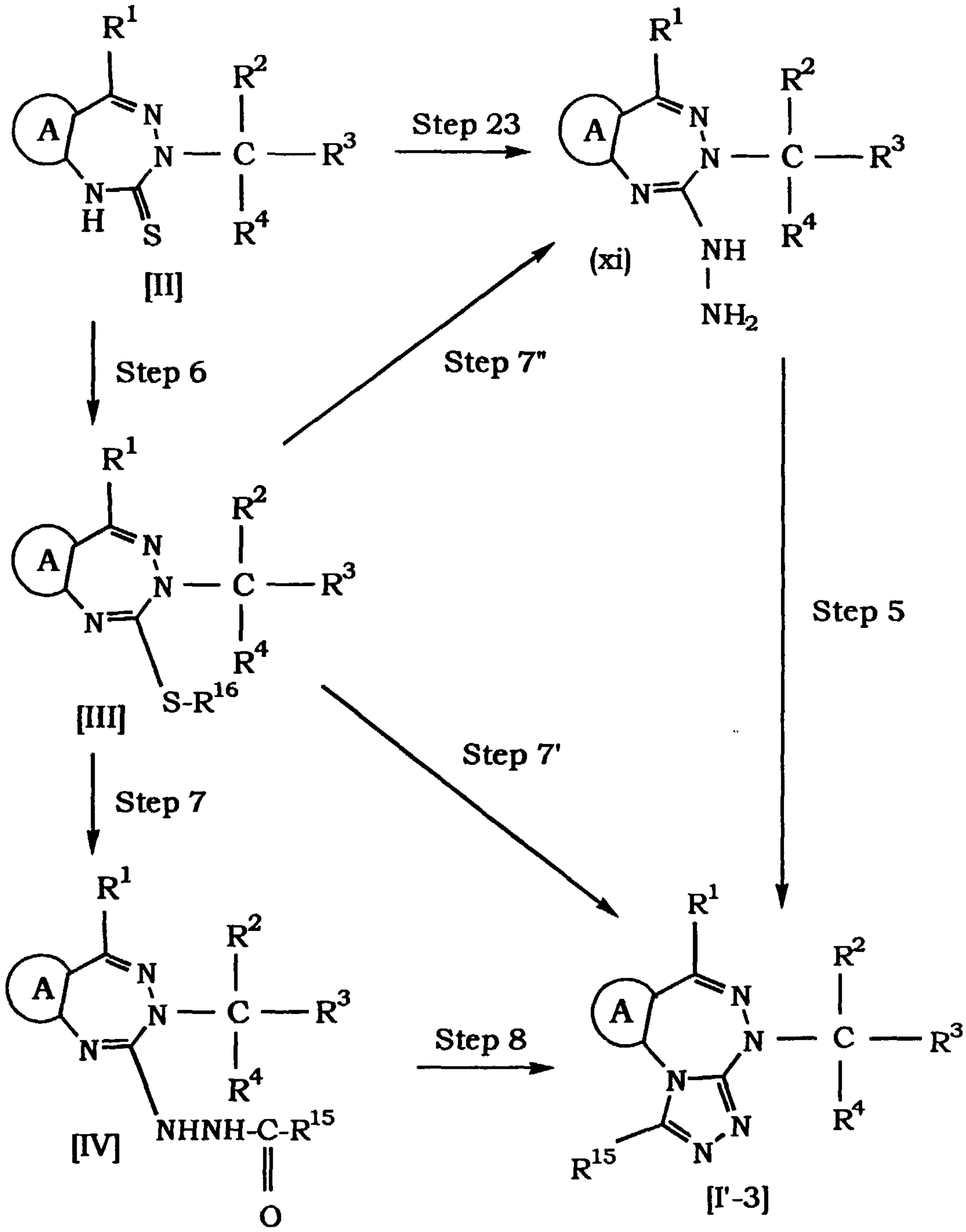


Step 18

The compound (viii) is reacted with methyl carbazate in a solvent such as methanol ethanol, n-propanol and isopropanol, or a mixed solvent thereof in the presence of p-toluenesulfonic acid, under heating, preferably with reflux under heating to give compound (xix) wherein $R^1$ and A are respectively as defined above.

Step 19

The compound (xix) obtained in Step 18 is reacted in a solvent such as dimethyl sulfoxide and N,N-dimethyl-

formamide, under heating, preferably with reflux under heating to give compound (xx) wherein $R^1$ and A are respectively as defined above.

Step 18'

This step is for performing Step 18 and Step 19 in a single step.

The compound (viii) is reacted with methyl carbazate in a solvent such as dimethyl sulfoxide and N,N-dimethylformamide, under heating, preferably with reflux under heating to give compound (xx).

Step 20

This step is for selective protection of the 1-position nitrogen atom of triazepine ring of compound (xx) wherein the protecting group may be any as long as it can selectively protect, and is exemplified by methoxymethyl, which is explained in the following.

The compound (xx) obtained in Step 19 or Step 18' is reacted with chloromethyl methyl ether in a solvent such as N,N-dimethylformamide and tetrahydrofuran in the presence of a base such as sodium hydroxide, potassium hydroxide, lithium hydroxide, sodium carbonate and potassium carbonate to give compound (xxi) wherein $R^1$ and A are respectively as defined above.

Step 21

The compound (xxi) obtained in Step 20 is reacted with

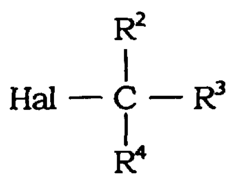

wherein $R^2$, $R^3$, $R^4$ and Hal are respectively as defined above, according to the method of Step 10-1 to give compound (xxii) wherein $R^1$, $R^2$, $R^3$, $R^4$ and A are respectively as defined above.

Step 22

This step is directed to removing the protecting group of the 1-position nitrogen atom of the triazepine ring of compound (xxii), which is carried out by a conventional method used for removing said protecting group. Examples thereof include deprotection of methoxymethyl which is described in the following.

The compound (xxii) obtained in Step 21 is reacted in the presence of an acid such as hydrochloric acid, sulfuric acid, hydrobromic acid, trifluoroacetic acid and trifluoromethanesulfonic acid in a solvent such as methanol, ethanol, n-propanol, isopropanol, tetrahydrofuran, 1,4-dioxane and water, or a mixed solvent thereof to give compound (xxiii) wherein $R^1$, $R^2$, $R^3$, $R^4$ and A are respectively as defined above.

Step 23

The compound (xxiii) obtained in Step 22 is reacted with an agent for converting the compound to thione, such as diphosphorus pentasulfide and Lawesson's reagent, in a solvent such as diethylene glycol, diethylene glycol dimethyl ether, ethylene glycol monomethyl ether, dimethyl sulfoxide and N,N-dimethylformamide, in the presence or absence of sodium carbonate and potassium hydrogencarbonate to give compound [II] wherein $R^1$, $R^2$, $R^3$, $R^4$ and A are respectively as defined above. This compound [II] can be introduced into the objective compound [I'-3] wherein $R^1$, $R^2$ $R^3$, $R^4$, $R^{15}$and A are respectively as defined above, according to the method of Step 4 and Step 5, or Step 6, Step 7 and Step 8, and where necessary, Step 9 and Step 10.

Production 4

A production method of, of the compounds of the formula [I'], the objective compound [I'-4] wherein

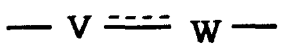 is —N=N—

is shown in the following.

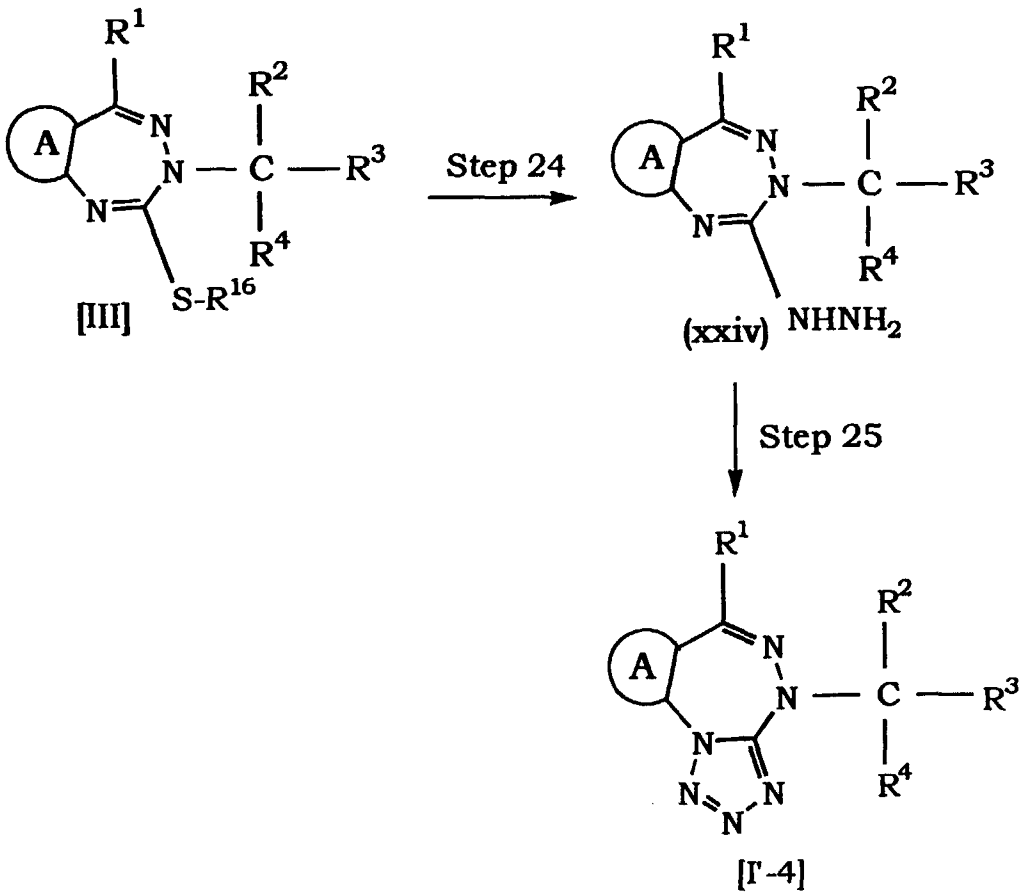


[III] (xxiv) [I'-4]

Step 24

The compound [III] obtained in Step 6 or a salt thereof is reacted with hydrazine or a hydrate thereof in a solvent such as methanol, ethanol, n-propanol and isopropanol from room temperature to under heat-refluxing to give compound (xxiv) wherein $R^1$, $R^2$, $R^3$, $R^4$ and A are respectively as defined above.

Step 25

The compound (xxiv) obtained in Step 24 or a salt thereof is reacted with sodium nitrite in a solvent such as water in the presence of an acid such as hydrochloric acid or acetic add under ice-cooling to room temperature, preferably from 0°C to 10°C to give the objective compound [I'-4] wherein $R^1$, $R^2$, $R^3$, $R^4$ and A are respectively as defined above.

Production 5

A production method of, of the compounds of the formula [I'], the objective compound [I'-5] wherein

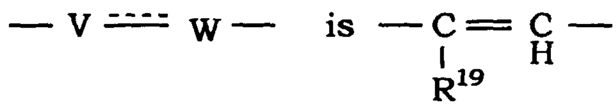

wherein $R^{19}$ is as defined above, is shown in the following.

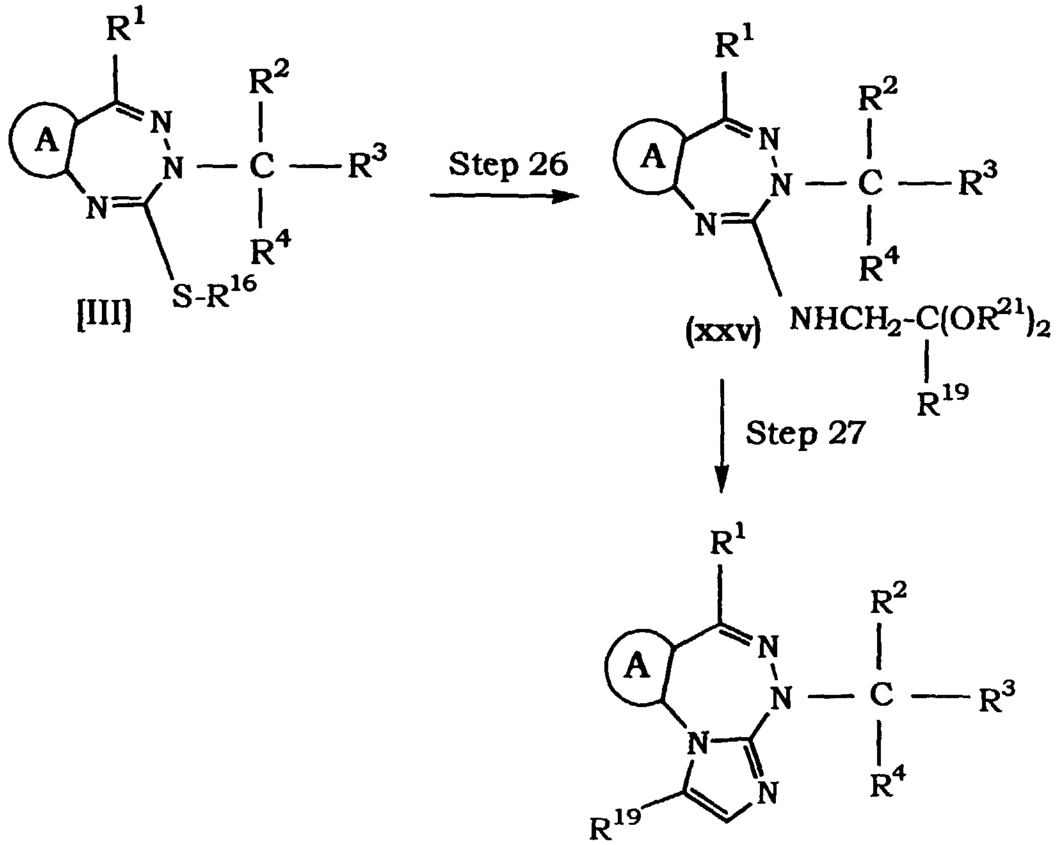


[III]

(xxv)

[I'-5]

Step 26

A compound [III] obtained in Step 6 or a salt thereof is reacted with aminoketone dialkyl acetal such as aminoacetaldehyde dimethyl acetal in a solvent such as diethylene glycol, ethylene glycol monomethyl ether and 2-ethoxyethanol under heating preferably with reflux under heating to give compound (xxv) wherein $R^{21}$ is lower alkyl and $R^1$, $R^2$, $R^3$, $R^4$, $R^{19}$ and A are respectively as defined above. In this reaction, addition of an acid such as acetic acid, hydrochloric acid, p-toluenesulfonic acid and trifluoroacetic acid is sometimes preferable.

Step 27

The compound (xxv) obtained in Step 26 or a salt thereof is reacted with a strong acid such as hydrochloric acid, sulfuric acid and hydrobromic acid in a solvent such as 1,4-dioxane, acetic acid and water, or a mixed solvent thereof, under heating, preferably with reflux under heating to give the objective compound [I'-5] wherein $R^1$, $R^2$, $R^3$ $R^4$, $R^{19}$ and A are respectively as defined above.

The diazepine compound of the formula [I''] of the present invention can be produced according to the method of WO 93/07129.

The compound of the present invention of the formula [I] thus obtained has superior cytokine production inhibitory action, and is useful as a cytokine production inhibitor, particularly, a production inhibitor of IL-6, TNF-α, IL-8, IFNγ,

IL-2 and GM-CSF. In particular, the compound of the formula [I] of the present invention exhibits superior antiinflammatory action and is useful as an antiinflammatory drug. When the compound of the present invention is used as a cytokine production inhibitor or antiinflammatory agent, it is generally administered systemically or topically by oral or parenteral administration.

In the present invention, cytokine includes IL-6, TNF-α, IL-8, IFNγ, IL-2, GM-CSF and the like. Examples of the diseases caused by abnormal production of cytokine include chronic inflammatory diseases, autoimmune diseases, viral diseases, cancer and the like. Specific examples include autoimmune diseases such as chronic rheumatoid arthritis and SLE, atrial myxoma, Castleman's disease, myeloma, Lennert's T lymphoma, mesangial proliferative nephritis, cachexia caused by terminal cancer or AIDS, adult respiratory distress syndrome (ARDS), viral infections such as viral hepatitis, acute myocardial infarction, gout, psoriasis, asthma, fulminant hepatitis, malignant tumor and the like. The cytokine production inhibitor of the present invention can be used for the prophylaxis and/or treatment of the above-mentioned diseases caused by abnormal production of cytokine.

While the dose varies depending on the age, body weight, symptom, therapeutic effect, administration route, treatment time and the like, it is generally from 0.01 mg to 100 mg for an adult, which is orally or parenterally administered in one to several doses per day.

When the compound of the present invention is formulated into a solid composition for oral administration, it can be prepared into a dosage form such as tablet, pill, powder, granule and the like. For such a solid composition, one or more active substances are admixed with at least one inert diluent, dispersing agent or adsorbent, such as lactose, mannitol, glucose, hydroxypropylcellulose, microcrystalline cellulose, starch, polyvinylpyrrolidone magnesium aluminometasilicate and silicic anhydride powder. Moreover, the composition may comprise additives other than the diluent by a conventional method.

When the compound is formulated into a tablet or pill, a gastric coating or an enteric coating of, for example, sucrose, gelatin, hydroxypropylcellulose or hydroxymethylcellulose phthalate, may be applied, or two more layers may be formed for coating. Moreover, a capsule made from a substance such as gelatin and ethylcellulose may be used.

When a liquid composition for oral administration is desired, the compound can be formulated into a dosage form of, for example, a pharmaceutically acceptable emulsion, solution, suspension, syrup, elixir and the like. Examples of the diluent to be used include purified water, ethanol, vegetable oil and emulsifier. This composition may also comprise, besides the diluent, auxiliary agents such as wetting agent, suspending agent, sweetener, flavor, aromatic and preservative.

When the compound is prepared into an injection for parenteral administration, a sterile aqueous or nonaqueous solvent, solubilizer, suspending agent or emulsifier is used. Examples of the aqueous solvent, solubilizer and suspending agent include distilled water for injection, physiological saline, cyclodextrin and its derivatives, organic amines such as triethanolamine, diethanolamine, monoethanolamine and triethylamine, and inorganic alkaline solution. Examples of nonaqueous solvent include propylene glycol, polyethylene glycol and vegetable oils such as olive oil, and alcohols such as ethanol. As the solubilizer, for example, polyoxethylene hydrogenated castor oil, surfactants such as sucrose fatty acid ester (forming mixed micelle), lecithin and hydrogenated lecithin (forming liposome) may be used. In addition, an emulsion preparation comprising a nonaqueous solvent such as vegetable oil, and lecithin, polyoxethylene hydrogenated castor oil or polyoxethylene polyoxypropylene glycol may be produced.

As other compositions for parenteral administration, an external liquid, liniment such as ointment, suppository or pessary comprising one or more active ingredients, which can be formulated by a method known *per se*, may be employed.

## Examples

The compounds of the present invention which are expressed by the formula [I] and methods for producing same are explained in detail by illustrative preparative examples, synthetic examples and examples in the following. The symbols used in the preparative examples, synthetic examples and examples and in Tables mean the following.

| | |
|---|---|
| Me | methyl |
| Et | ethyl |
| Pr | propyl |
| t-Bu | tert-butyl |
| Ac | acetyl |

Preparative Example 1 (Step 1')

3,4-Dimethoxybenzlhydrazine hydrochloride

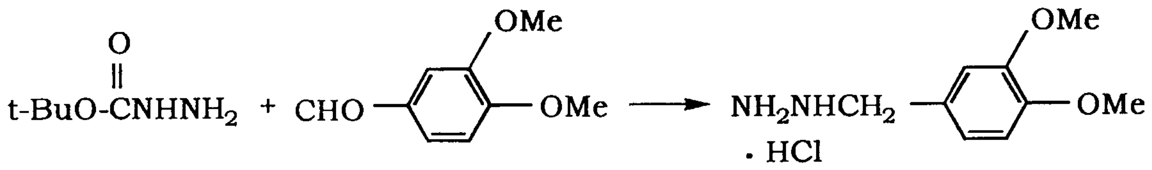

tert-Butyl carbazate (59.65 g) and 3,4-dimethoxybenzaldehyde (75 g) were dissolved in ethanol (1.3 L). To the solution were added acetic acid (77 ml) and 10% palladium-carbon (1.5 g), and the mixture was subjected to catalytic reduction under a hydrogen atmosphere at 1 atm for 2 days with vigorous stirring. The catalyst was removed by filtration, and the filtrate was concentrated under reduced pressure to give an oil. Anisole (54 ml) and 1,4-dioxane (100 ml) were added to the oil. The mixture was cooled in an ice bath, and a 4N hydrogen chloride-dioxane solution (360 ml) was added. The mixture was stirred under ice-cooling for one hour and at room temperature for 4 hours. After the completion of the reaction, diethyl ether (800 ml) was gradually added to the mixture, and the precipitated viscous solid was collected by filtration. The solid was dissolved in methanol (1.5 L) heated to 50°C, and the solution was concentrated to about a half volume to precipitate crystals. The crystals were collected by filtration, successively washed with cold methanol and diethyl ether, and dried to give 57.8 g of the title compound as, colorless needles.

Melting point : 178-180°C

Preparative Example 2 (Step 1')

3-Pyridylmethylhydrazine dihydrochloride

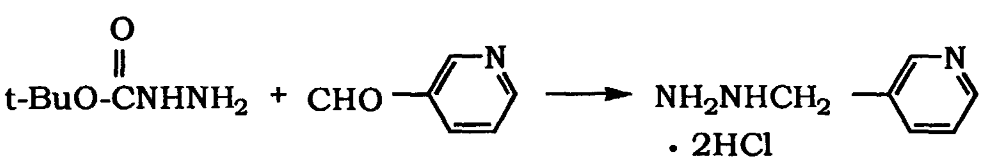

tert-Butyl carbazate (13.22 g), 3-pyridinecarboxaldehyde (9.44 ml) and acetic acid (11.5 ml) were dissolved in methanol (230 ml) under ice-cooling, and the solution was stirred at room temperature for one hour. The air in the reactor was replaced with an argon gas, and 10% palladium-carbon (1.0 g) suspended in an adequate amount of methanol was added under an argon atmosphere. The air inside the reactor was replaced with hydrogen, and the mixture was subjected to catalytic reduction for 2 days with vigorous stirring. The catalyst was removed by filtration, the filtrate (1.7 L) (about 0.93 mol inclusive) and anisole (101 ml, 0.93 mol) were mixed, and the mixture was cooled with ice. A 4N hydrochloric acid/dioxane solution (0.93 L) was added to the mixture with stirring under ice-cooling, and the mixture was stirred at 50°C for one hour and at 60°C for 3 hours. The reaction mixture was cooled to room temperature. The precipitated crystals were collected by filtration, washed with ethanol, and dried under reduced pressure to give 8.68 g of the title compound as pale-yellow needles.

Melting point : 189-191°C
$^1$H NMR (300MHz, $\delta$ ppm, DMSO-$d_6$)

4.27(2H,s), 8.03(1H,dd,J=7.8 and 5.7Hz), 8.55(1H,d,J=7.8Hz), 8.86(1H,d,J=5.7Hz), 8.91(1H,s)

Preparative Examples 3 and 4 (Step 1')

In the same manner as in Preparative Example 1, the compound of Preparative Example 3 was obtained from tert-butyl carbazate and 4-methoxybenzaldehyde, and in the same manner as in Preparative Example 2, the compound of Preparative Example 4 was obtained from tert-butyl carbazate and 4-pyridinecarboxaldehyde. The compounds are shown in Table 1.

**Table 1**

| Preparative Ex. | Structural formula | Melting point (°C) |
|---|---|---|
| 3 | 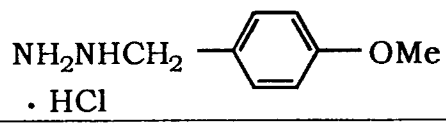 | 184-185 |
| 4 | 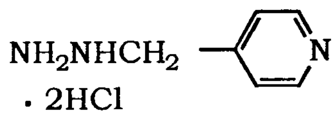 | 156-158 |

Preparative Example 5 (Step 1)

2-(4-Chlorobenzoyl)phenyl isothiocyanate

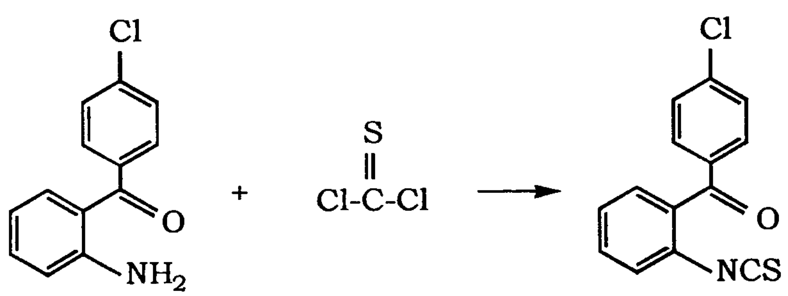

According to the method described in US Patent No. 4,144,233, the title compound (74.1 g) was obtained as yellow crystals from thiophosgene (22.6 ml) and 2-aminophenyl 4-chlorophenyl ketone (62.6 g) (Tokyo Kasei Kogyo).

Melting point : 80-82°C
Mass spectrum (low resolution): 274.1

Preparative Examples 6-15 (Step 1)

In the same manner as in Preparative Example 5, the compound of Preparative Example 6 was obtained from 2-aminophenyl phenyl ketone, the compound of Preparative Example 7 was obtained from 2-amino-5-chlorophenyl phenyl ketone, the compound of Preparative Example 8 was obtained from 2-amino-4-methylphenyl phenyl ketone, the compound of Preparative Example 9 was obtained from 2-amino-5-nitrophenyl phenyl ketone, the compound of Preparative Example 10 was obtained from 2-aminonophenyl 4-methylphenyl ketone, the compound of Preparative Example 11 was obtained from 2-amino-5-chlorophenyl 2-chlorophenyl ketone, the compound of Preparative Example 12 was

obtained from 2-amino-4-methylphenyl 4-chlorophenyl ketone, the compound of Preparative Example 13 was obtained from 2-aminophenyl 4-bromophenyl ketone, the compound of Preparative Example 14 was obtained from tert-butyl 4-(2-aminobenzoyl)benzoate, and the compound of Preparative Example 15 was obtained from 2-aminopyridin-3-yl 4-chlorophenyl ketone. The compounds are shown in Tables 2 and 3.

**Table 2**

| Prep. Ex. | Structural formula | Mass spectrography (low resolution) | $^1H$ NMR (300MHz, δppm, $CDCl_3$) | Melting point (°C) |
|---|---|---|---|---|
| 6 | 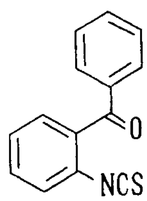 | 239.0 | 7.34-7.39(2H, m), 7.47-7.55(4H, m), 7.59-7.65(1H, m), 7.80-7.83(2H, m). | Oily substance |
| 7 | 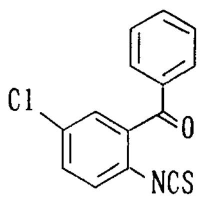 | 274.0 | | 44-46 |
| 8 | 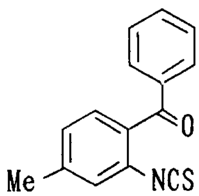 | 254.1 | | 45-48 |
| 9 | 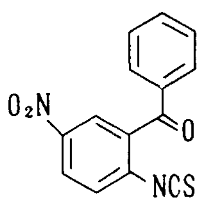 | 285.0 | | 82-85 |
| 10 | 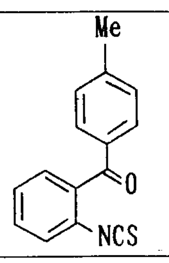 | 254.1 | | Oily substance |
| 11 | 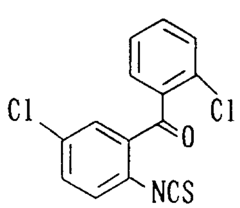 | 307.9 | 7.25-7.28(2H, m), 7.38-7.52(4H, m), 7.57 (1H, d, J=2.3Hz). | |

**Table 3**

| Prep. Ex. | Structural formula | Mass spectrography (low resolution) | $^1$H NMR (300MHz, δppm, $CDCl_3$) | Melting point (℃) |
|---|---|---|---|---|
| 12 | 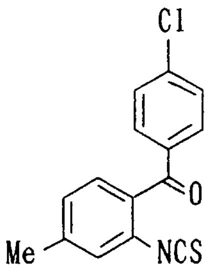 | 289 | | |
| 13 | 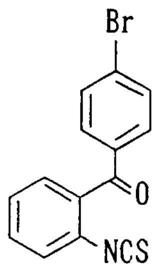 | 319 | | |
| 14 | 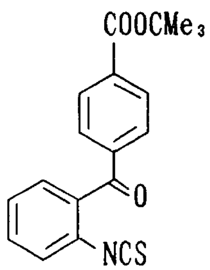 | | 1.62(9H, s), 7.35-7.40(2H, m), 7.48-7.55(2H, m), 7.81 (2H, d, J=8.5Hz), 8.09 (2H, d, J=8.5Hz) | Amorphous solid |
| 15 | 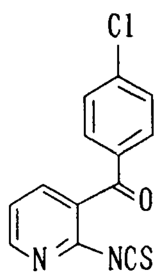 | 275 | | |

Preparative Example 16 (Step 1)

3-(4-Chlorobenzoyl)-4,5-dimethylthiophene-2-isothiocyanate

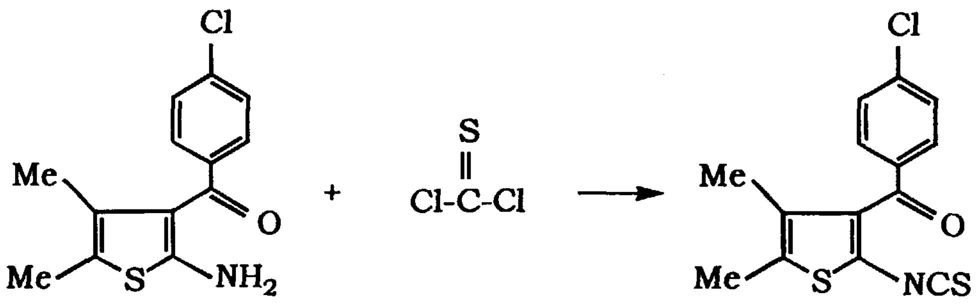

In the same maner as in Preparative Example 5, the tide compound (1.16 g) was obtained as a crude oil from 2-amino-3-(4-chlorobenzoyl)-4,5-dimethylthiophene (1 g) obtained by the method described in Japanese Patent Unexamined Publication No. 256681/1990 and thiophosgene (316 μl).

Preparative Example 17-22

In the same manner as in Preparative Example 16, the compound of Preparative Example 17 was obtained from 2-amino-3-(4-chlorobenzoyl)-5-ethylthiophere, the compound of Preparative Example 18 was obtained from 2-amino-3-benzoyl-5-methylthiophene, the compound of Preparative Example 19 was obtained from 2-amino-3-benzoyl-4,5-dimethylthiophene, the compound of Preparative Example 20 was obtained from 2-amino-3-benzoyl-5-ethylthiophene, the compound of Preparative Example 21 was obtained from 2-amino-3-(4-methoxybenzoyl)-4,5-dimethylthiophene, and the compound of Preparative Example 22 was obtained from 2-amino-3-(2-chlorobenzoyl)-4,5-dimethylthiophene. The compounds are shown in Tables 4 and 5.

**Table 4**

| Prep. Ex. | Structural formula | Mass spectrography (low resolution) | $^1$H NMR (300MHz, δppm, $CDCl_3$) |
|---|---|---|---|
| 17 | 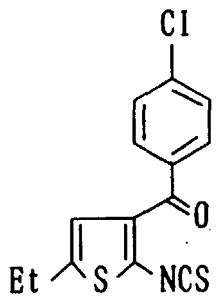 | 308.0 | 1.31 (3H, t, J=7.6Hz), 2.77 (2H, q, J=7.6Hz), 6.80(1H, s), 7.47 (2H, d, J=8.5Hz), 7.75 (2H, d, J=8.5Hz). |
| 18 | 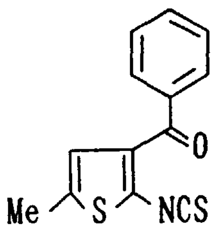 | 260.0 | 2.44 (3H, d, J=1.5Hz), 6.79 (1H, q, J=1.5Hz), 7.46-7.52(2H, m), 7.56-7.61(1H, m), 7.79-7.82(2H, m). |
| 19 | 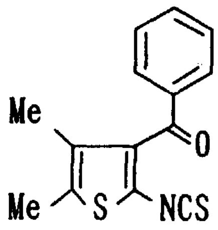 | 307.9 | 2.05(3H, s), 2.33(3H, s), 7.48-7.62(3H, m), 7.82-7.86(2H, m). |

**Table 5**

| Prep. Ex. | Structural formula | Mass spectrography (low resolution) | $^1$H NMR (300MHz, δppm, $CDCl_3$) | Melting point (°C) |
|---|---|---|---|---|
| 20 | 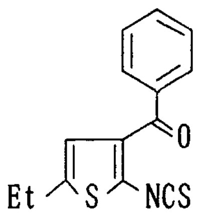 | 274.0 | 1.31(3H, t, J=6Hz), 2.78(2H, m), 6.82 (1H, t, J=1.0Hz), 7.47-7.62(3H, m), 7.79-7.83(2H, m). | |
| 21 | 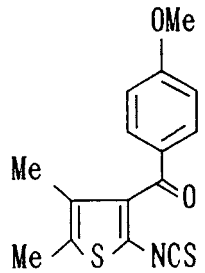 | 304.0 | | |
| 22 | 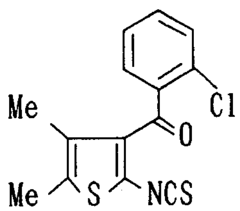 | | | 76-79 |

Preparative Example 23 (Step 2)

N-[2-(4-Chlorobenzoyl)phenyl]-1-(3,4-dimethoxybenzyl)hydrazinecarbothioamide

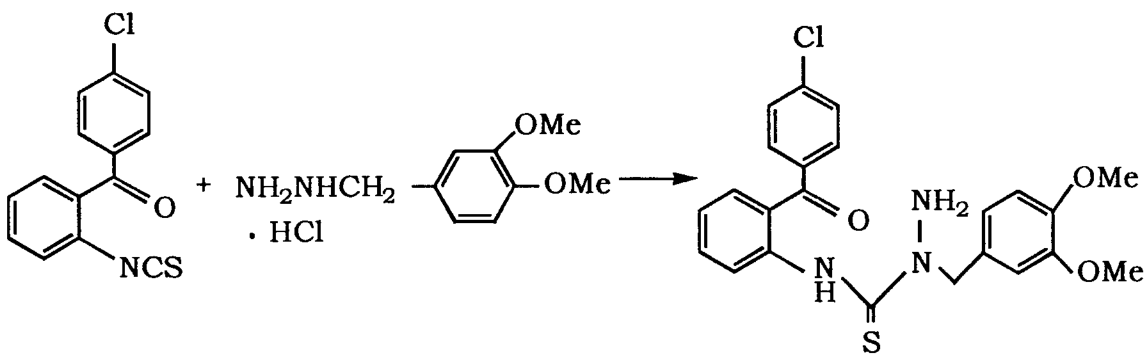

3,4-Dimethoxybenzlhydrazine hydrochloride (22.99 g) obtained in Preparative Example 1 was dissolved in methanol (460 ml), the solution was cooled with ice, and triethylamine (14.8 ml) was dropwise added over 5 minutes. A solution of 2-(4-chlorobenzoyl)phenyl isothiocyanate (24.2 g) obtained in Preparative Example 5 in tetrahydrofuran (240 ml) was added to the reaction mixture under ice-cooling. The mixture was stirred under ice-cooling for 15 minutes and at room temperature for 40 minutes. After the completion of the reaction, the solvent was distilled away under reduced pressure. The residue was dissolved in dichloromethane (500 ml) and washed twice with water. The organic layer was dried over anhydrous sodium sulfate, filtrated, and concentrated under reduced pressure. The residue was crystallized from diethyl ether to give 34.7 g of the title compound as pale-yellow needles.

Melting point : 162-166°C

Preparative Examples 24-33 (Step 2)

In the same manner as in Preparative Example 23, the compound of Preparative Example 24 was obtained from the compounds of Preparative Examples 3 and 6, the compound of Preparative Example 25 was obtained from the compounds of Preparative Examples 3 and 5, the compound of Preparative Example 26 was obtained from the compounds of Preparative Examples 3 and 7, the compound of Preparative Example 27 was obtained from the compounds of Preparative Examples 3 and 9, the compound of Preparative Example 28 was obtained from the compounds of Preparative Examples 3 and 10 , the compound of Preparative Example 29 was obtained from the compounds of Preparative Examples 3 and 11, the compound of Preparative Example 30 was obtained from the compounds of Preparative Examples 3 and 12, the compound of Preparative Example 31 was obtained from the compounds of Preparative Examples 3 and 13, the compound of Preparative Example 32 was obtained from the compounds of Preparative Examples 3 and 14, and the compound of Preparative Example 33 was obtained from the compounds of Preparative Examples 3 and 15. The compounds are shown in Tables 6 to 9.

**Table 6**

| Prep. Ex. | Structural formula | $^1$H NMR (300MHz, δppm, $CDCl_3$) | Melting point (°C) |
|---|---|---|---|
| 24 | 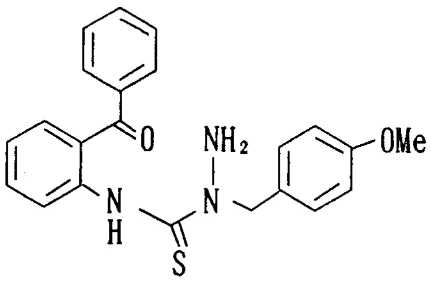 | 3.72(2H, s), 3.79(3H, s), 5.38(2H, s), 6.86(2H, s), 7.17(1H, m), 7.30(2H, m), 7.44-7.61(5H, m), 7.80(2H, m), 8.78 (1H, d, J=10.1Hz), 11.77(1H, s). | Oily substance |
| 25 | 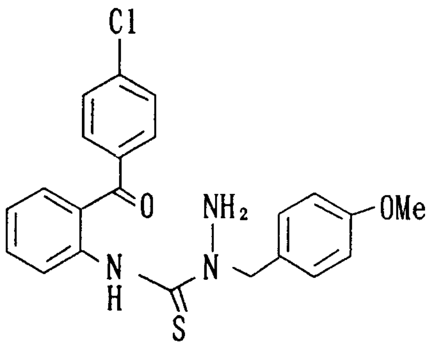 | | 180 |
| 26 | 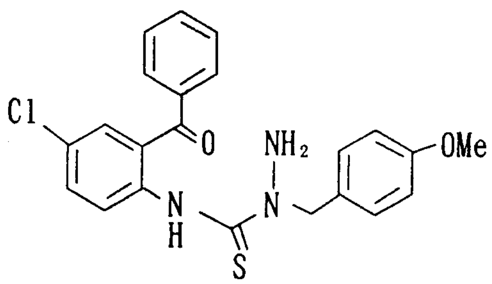 | | 132-134 (decomposition) |

**Table 7**

| Prep. Ex. | Structural formula | Melting point (°C) |
|---|---|---|
| 27 | 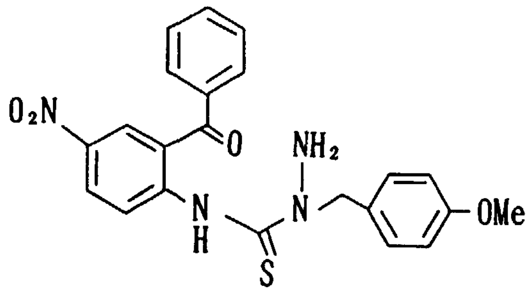 | 142-144 |
| 28 | 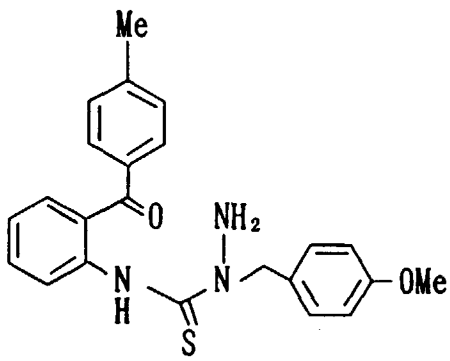 | 170-172 |
| 29 | 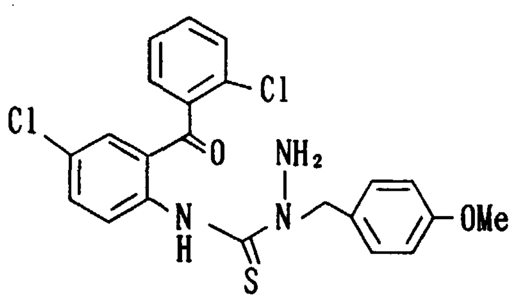 | 135-138 |

**Table 8**

| Prep. Ex. | Structural formula | $^1$H NMR (300MHz, δppm, $CDCl_3$) | Melting point (°C) |
|---|---|---|---|
| 30 | 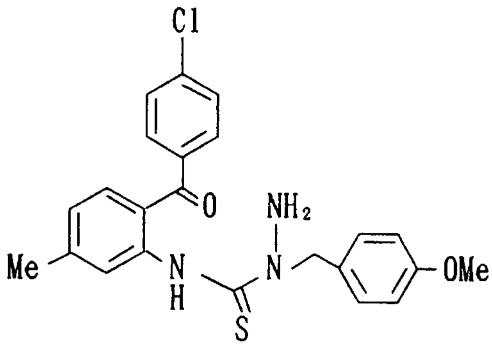 | 2.46(3H, s), 3.79(3H, s), 5.38(2H, s), 6.87 (2H, d, J=8.4Hz), 6.96 (1H, d, J=8.1Hz), 7.26-7.35(5H, m), 7.43 (2H, d, J=9.0Hz), 7.70 (2H, d, J=8.7Hz), 8.60(1H, s), 11.74(1H, s) | 171.0 - 172.5 |
| 31 | 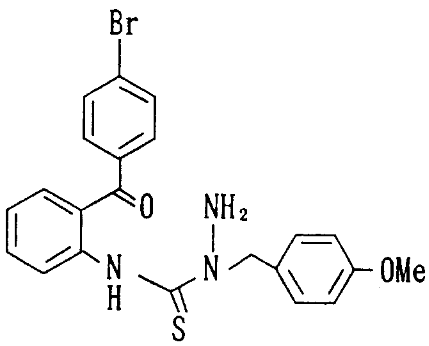 | 3.80(3H, s), 5.37(2H, s), 6.87 (2H, d, J=8.6Hz), 7.17-7.29(4H, m), 7.44 (2H, d, J=7.8Hz), 7.59-7.69(5H, m), 8.69 (1H, d, J=8.2Hz) | 173.0 - 174.5 |

**Table 9**

| Prep. Ex. | Structural formula | $^1$H NMR (300MHz, δppm, $CDCl_3$) |
|---|---|---|
| 32 | 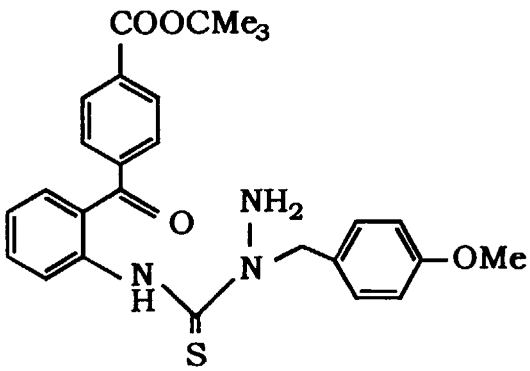 | 1.62(9H, s.), 3.75(2H, brs), 3.79(3H, s), 5.39(2H, s), 6.87 (2H, d, J=8.6Hz), 7.16 (1H, t, J=4.2Hz), 7.29 (2H, d, J=8.6Hz), 7.43 (1H, d, J=9.3Hz), 7.58-7.60(1H, m), 7.82 (2H, d, J=8.4Hz), 8.07 (2H, d, J=8.4Hz), 8.79 (1H, d, J=8.2Hz) |
| 33 | 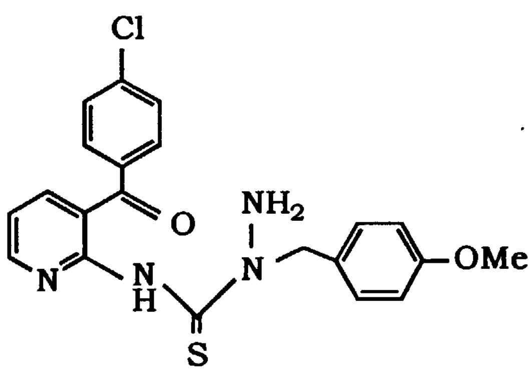 | 3.74(2H, brs), 3.80(3H, s), 5.24(2H, s), 6.87 (2H, d, J=8.7Hz), 7.17 (2H, d, J=8.4Hz), 7.21-7.25(1H, m), 7.43 (2H, d, J=8.4Hz), 7.81-7.85(3H, m), 8.59-8.60(1H, m), 10.6(1H, brs) |

Preparative Example 34 (Step 2)

N-[2-4-Chlorobenzoyl)phenyl]-1-(pyridin-3-ylmethyl)hydrazinecarbothioamide

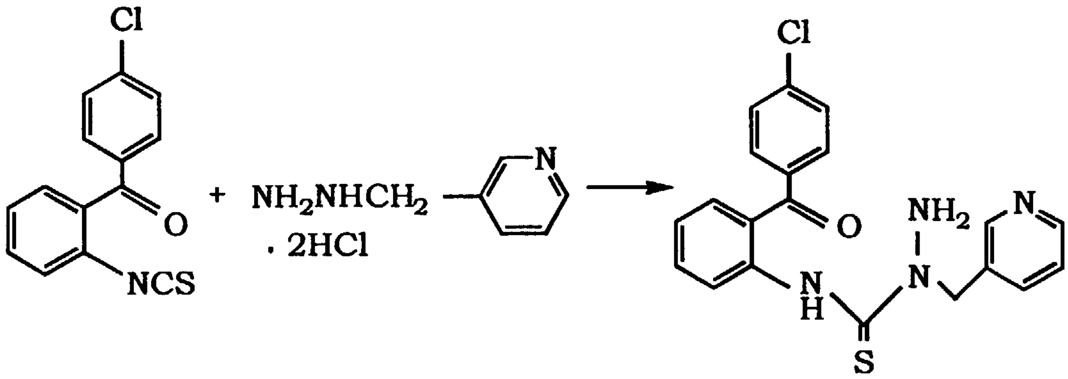

3-Pyridylmethylhydrazine dihydrochloride (11.8 g) obtained in Preparative Example 2 was dissolved in water (25 ml), and sodium hydrogencarbonate (10.08 g) was added. The solution was cooled with ice. A solution of 2-(4-chlorobenzoyl)phenyl isothiocyanate (16.4 g) obtained in Preparative Example 5 in tetrahydrofuran (30 ml) was cooled with ice and added to the solution previously prepared. The mixture was stirred under ice-cooling for 30 minutes. The temperature of the reaction mixture was raised to room temperature, and ethanol (30 ml) was added. The precipitated crystals were collected by filtration, washed with a mixed solvent of ethanol:water (80:20), and dried under reduced pressure to give 19.9 g of the title compound as colorless crystals.

Melting point : 165-166°C

Preparative Example 35 (Step 2)

N-[3-(4-Chlorobenzoyl)-4,5-dimethylthiophen-2-yl]-1-(4-methoxybenzyl)hydrazinecarbothioamide

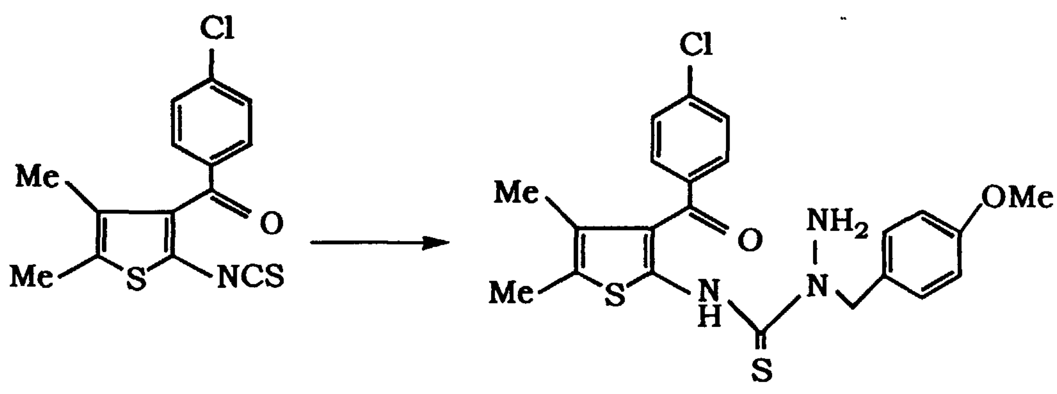

4-Methoxybenzylhydrazine hydrochloride (745 mg) obtained in Preparative Example 3 was dissolved in methanol (10 ml). The solution was cooled with ice, and triethylamine (550 μl) was dropwise added. A solution of 3-(4-chlorobenzoyl)-4,5-dimethylthiophene-2-isothiocyanate (1.16 g) obtained in Preparative Example 16 in tetrahydrofuran (10 ml) was cooled with ice, and added to the reaction mixture previously prepared. The mixture was stirred under ice-cooling for 30 minutes and at room temperature for 30 minutes. After the completion of the reaction, the solvent was distilled away under reduced pressure, and the residue was subjected to silica gel column chromatography. A product obtained by concentration of the fractions eluted with hexane:ethyl acetate=3:1 was crystallized from diethyl ether to give 830 mg of the title compound as yellow crystals.

Melting point : 157-160°C

Preparative Examples 36-41 (Step 2)

In the same manner as in Preparative Example 35, the compound of Preparative Example 36 was obtained from the compounds of Preparative Examples 3 and 17, the compound of Preparative Example 37 was obtained from the compounds of Preparative Examples 3 and 18, the compound of Preparative Example 38 was obtained from the compounds of Preparative Examples 3 and 19, the compound of Preparative Example 39 was obtained from the compounds of Preparative Examples 3 and 20, the compound of Preparative Example 40 was obtained from the compounds of Preparative Examples 3 and 21, and the compound of Preparative Example 41 was obtained from the compounds of Preparative Examples 3 and 22. The compounds are shown in Tables 10 and 11.

**Table 10**

| Prep. Ex. | Structural formula | $^1$H NMR (300MHz, δppm, $CDCl_3$) | Melting point (°C) |
|---|---|---|---|
| 36 | 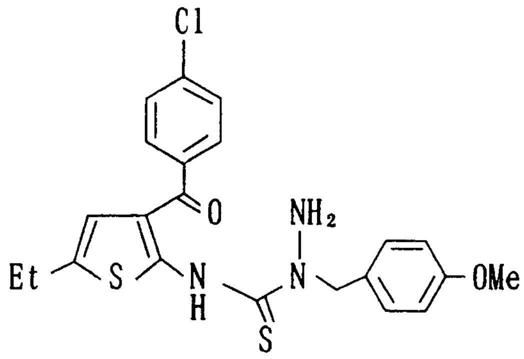 | 1.29 (3H, t, J=9.0Hz), 2.73 (2H, q, J=9.0Hz), 3.81(3H, s), 3.90(2H, s), 5.36(2H, s), 6.71(1H, m), 6.90 (2H, d, J=8.8Hz), 7.33 (2H, d, J=8.8Hz), 7.44 (2H, d, J=8.8Hz), 7.64 (2H, d, J=8.8Hz). | |
| 37 | 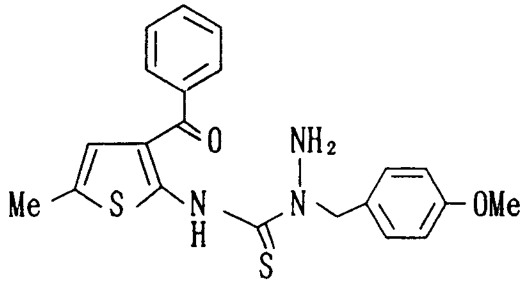 | 2.36(3H, s), 3.80(3H, s), 3.92(2H, s), 5.35(2H, s), 6.75 (1H, d, J=1.2Hz), 6.89 (2H, d, J=8.4Hz), 7.32 (2H, d, J=9.0Hz), 7.42-7.55(3H, m), 7.67-7.70(2H, m), 14.03(1H, s). | 166-169 |
| 38 | 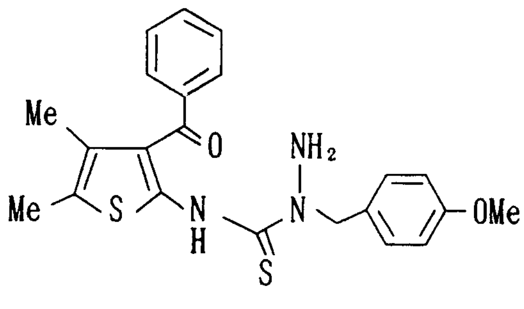 | | 140-143 |

**Table 11**

| Prep. Ex. | Structural formula | Melting point (°C) |
|---|---|---|
| 39 | 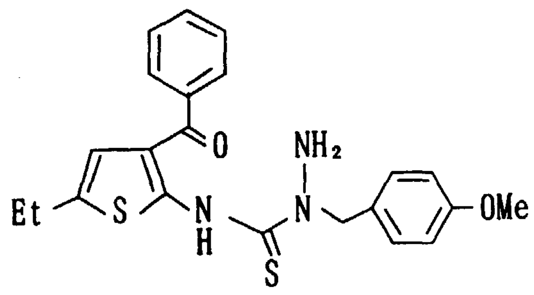 | 131-132 |
| 40 | 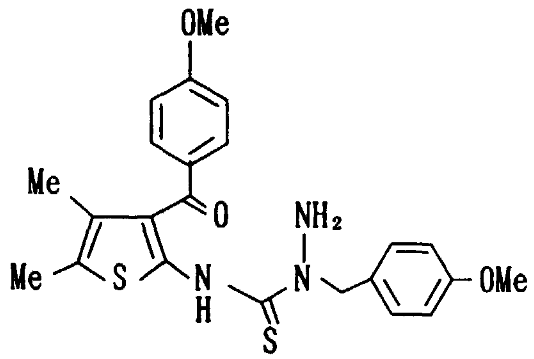 | 133-134 |
| 41 | 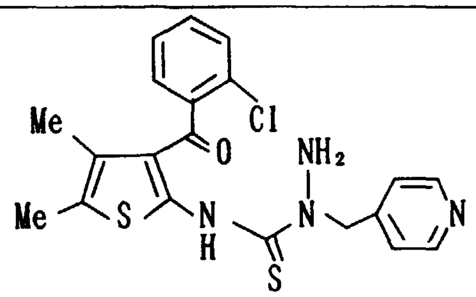 | 154-157 |

Preparative Example 42 (Step 3)

5-(4-Chlorophenyl)-3-(3,4-dimethoxybenzyl)-1,3-dihydrobenzo[e][1,2,4]triazepine-2-thione

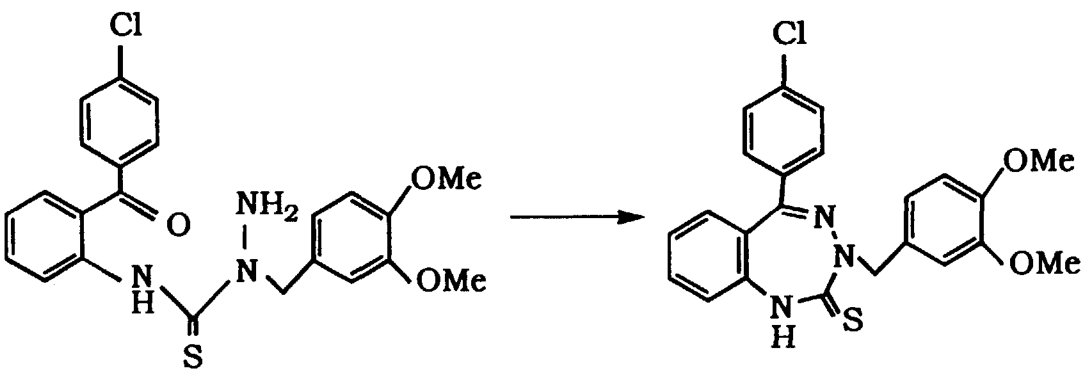

N-[2-(4-Chlorobenzoyl)phenyl]-1-(3,4-dimethoxybenzyl)hydrazinecarbothioamide (35.75 g) obtained in Preparative Example 23 was suspended in ethanol (360 ml). p-Toluenesulfonic acid monohydrate (0.45 g) was added, and the mixture was refluxed under heating for one hour. After the completion of the reaction, the reaction mixture was concentrated under reduced pressure. The residue was dissolved in diethyl ether (500 ml), and successively washed with water, a saturated aqueous sodium hydrogencarbonate solution and water. The organic layer was dried, filtrated and concentrated, and the precipitated crystals were collected by filtration to give 28.6 g of the title compound as yellow crystals.

Melting point : 136-137°C

Preparative Example 43 (Step 3)

3-(4-Methoxybenzyl)-5-phenyl-1,3-dihydrobenzo[e][1,2,4]triazepine-2-thione

Preparative Example 44 (Step 3)

5-(4-Chlorophenyl)-3-(4-methoxybenzyl)-1,3-dihydrobenzo[e][1,2,4]triazepine-2-thione

Preparative Example 45 (Step 3)

7-Chloro-3-(4-methoxybenzyl)-5-phenyl-1,3-dihydrobenzo[e][1,2,4]triazepine-2-thione

Preparative Example 46 (Step 3)

3-(4-Methoxybenzyl)-7-nitro-5-phenyl-1,3-dihydrobenzo[e][1,2,4]triazepine-2-thione

Preparative Example 47 (Step 3)

3-(4-Methoxybenzyl)-5-(4-methylphenyl)-1,3-dihydrobenzoe[e][1,2,4]triazepine-2-thione

Preparative Example 48 (Step 3)

7-Chloro-5-(2-chlorophenyl)-3-(4-methoxybenzyl)-1,3-dihydrobenzo[e][1,2,4]triazepine-2-thione

Preparative Example 49 (Step 3)

5-(4-Chlorophenyl)-3-(4-methoxybenzyl)-8-methyl-1,3-dihydrobenzo[e][1,2,4]triazepine-2-thione

Preparative Example 50 (Step 3)

5-(4-Bromophenyl)-3-(4-methoxybenzyl)-1,3-dihydrobenzo[e][1,2,4]triazpine-2-thione

Preparative Example 51 (Step 3)

tert-Butyl 4-[3-(4-methoxybenzyl)-2,3-dihydro-1H-benzo[e][1,2,4]triazepine-2-thioxo-5-yl]benzoate

Preparative Example 52 (Step 3)

5-(4-Chlorophenyl)-3-(4-methoxybenzyl)-1,3-dihydropyrido[2,3-e][1,2,4]triazepine-2-thione

In the same manner as in Preparative Example 42, the compounds of Preparative Examples 43, 44, 45, 46, 47, 48, 49, 50, 51 and 52 were obtained from the compounds of Preparative Examples 24, 25, 26, 27, 28, 29, 30, 31, 32 and 33, respectively. The compounds are shown in Tables 12-14.

**Table 12**

| Prep. Ex. | Structural formula | Melting point (°C) |
|---|---|---|
| 43 | 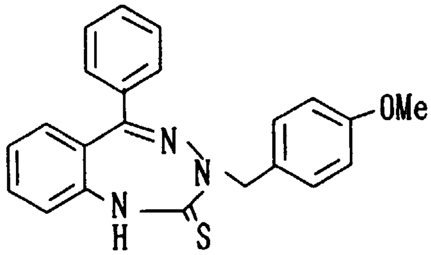 | 137-141 |
| 44 | 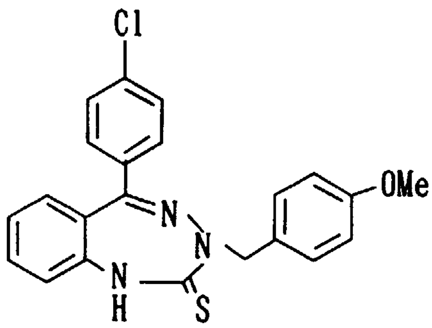 | 152 |
| 45 | 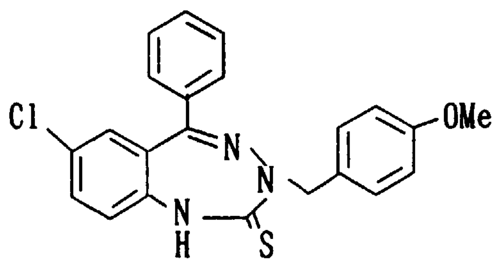 | 159-163 |

**Table 13**

| Prep. Ex. | Structural formula | $^1$H NMR (300MHz, δppm, $CDCl_3$) | Melting point (°C) |
|---|---|---|---|
| 46 | 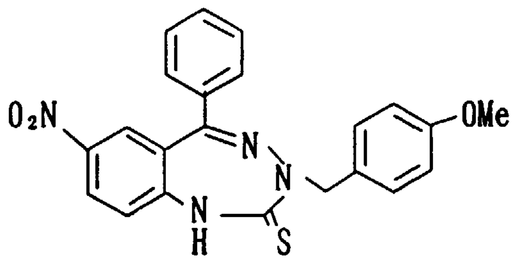 | 3.79(3H, s), 5.30(2H, s), 6.86 (2H, d, J=8.7Hz), 7.07 (1H, d, J=8.8Hz), 7.21-7.51(7H, m), 7.75(1H, s), 7.86 (1H, d, J=2.5Hz), 8.29 (1H, dd, J=8.8 and 2.6Hz). | Oily substance |
| 47 | 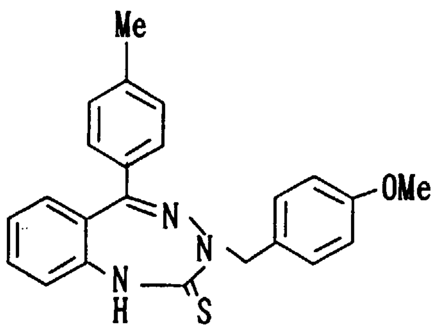 | | 135-136 |
| 48 | 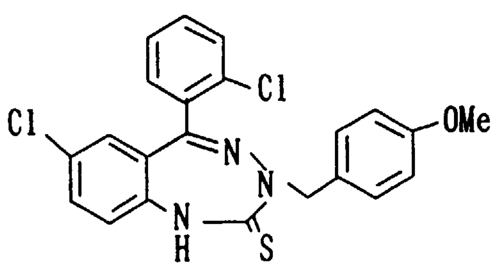 | | 155-157 |

**Table 14**

| Prep. Ex. | Structural formula | $^1$H NMR (300MHz, δppm, $CDCl_3$) | Melting point (°C) |
|---|---|---|---|
| 49 | 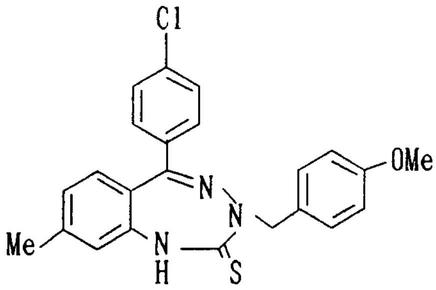 | 2.37(3H, s), 3.78(3H, s), 5.27(2H, s), 6.74-6.90(5H, m), 7.15-7.30(7H, m) | 192.5-194.0 |
| 50 | 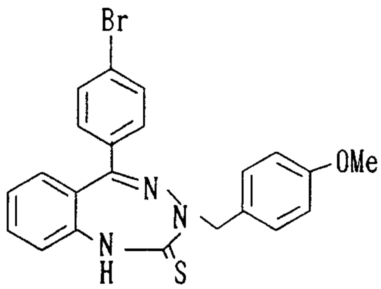 | 3.78(3H, s), 5.27(2H, s), 6.82 (2H, d, J=6.6Hz), 6.93 (2H, d, J=8.0Hz), 7.09-7.12(2H, m), 7.22-7.26(3H, m), 7.44-7.47(4H, m) | 158.0-159.5 |
| 51 | 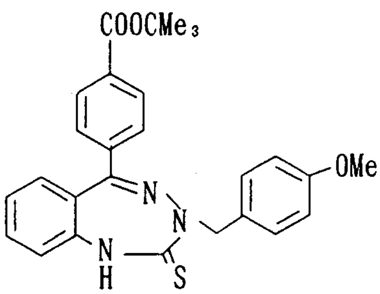 | 1.59(9H, s), 3.79(3H, s), 5.30(2H, s), 6.83 (2H, d, J=8.7Hz), 6.88-6.96(2H, m), 7.06-7.08(1H, m), 7.25-7.30(3H, m), 7.44-7.48(2H, m), 7.93 (2H, d, J=8.5Hz) | |
| 52 | 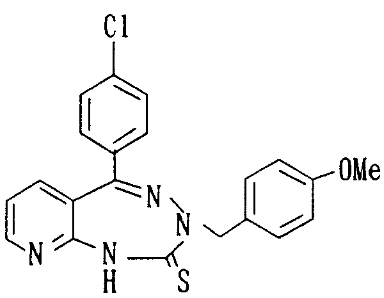 | 3.79(3H, s), 5.34(2H, s), 6.84 (2H, d, J=8.7Hz), 7.03-7.07(1H, m), 7.14 (2H, d, J=8.6Hz), 7.28-7.33(5H, m), 8.39(1H, s), 8.47-8.49(1H, m) | |

Preparative Example 53 (Step 3)

5-(4-Chlorophenyl)-3-(pyridin-3-ylmethyl)-1,3-dihydrobenzo[e][1,2,4]triazepine-2-thione hydrochloride

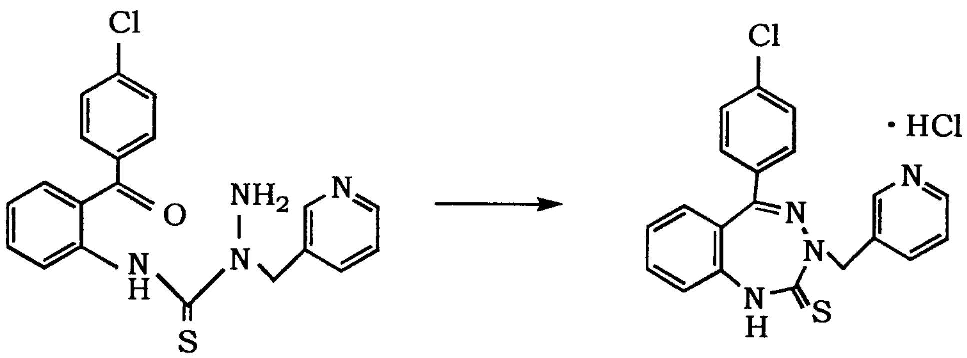

N-[2-(4-Chlorobenzoyl)phenyl]-1-(pyridin-3-ylmethyl)hydriazecarbothioamide (1.0 g) obtained in Preparative Example 34 was suspended in 2-propanol, 4N hydrochloric acid/ 1,4-dioxane (0.63 ml) was added, and the mixture was stirred at 60°C for one hour. The reaction mixture was cooled to room temperature, added with diethyl ether (20 ml), and allowed to stand for 2 hours. The resulting crystals were collected by filtration, and washed with diethyl ether to give 985 mg of the title compound as yellow crystals.

Melting point : 136-137°C

Preparative Example 54 (Step 3)

5-(4-Chlorophenyl)-3-(pyridin-3-ylmethyl)-1,3-dihydrobenzo[e][1,2,4]triazepine-2-thione p-toluenesulfonate

In the same manner as in Preparative Example 53, the title compound was obtained. The compound is shown in Table 15.

**Table 15**

| Preparative Ex. | Structural formula | Melting point (°C) |
|---|---|---|
| 54 | 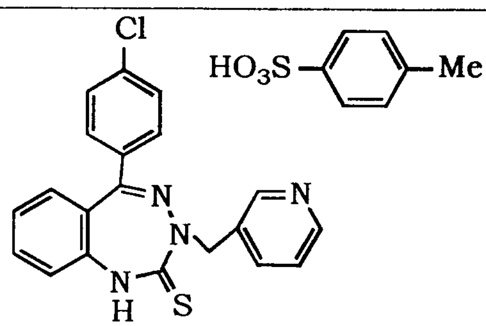 | 199-202 |

Preparative Example 55 (Step 3)

4-(4-Chlorophenyl)-6-(4-methoxybenzyl)-2,3-dimethyl-6,8-dihydro-1-thia-5,6,8-triazaazulene-7-thione

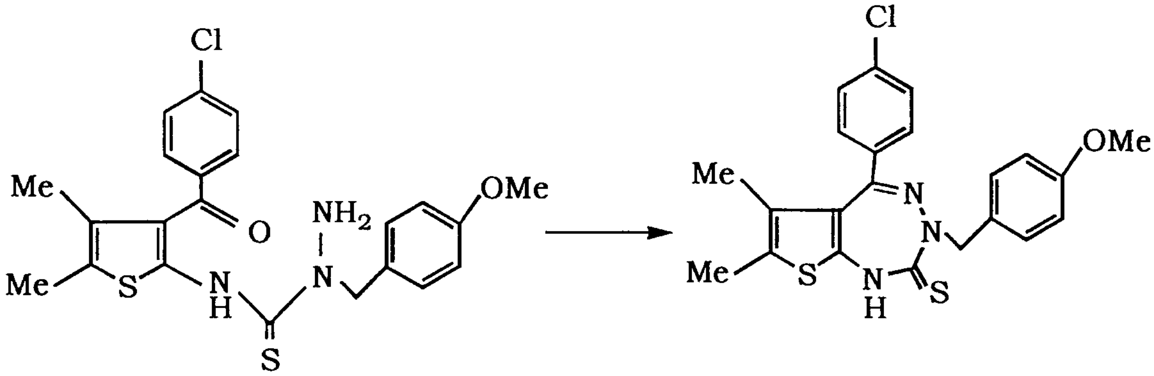

N-[3-(4-Chlorobenzoyl)-4,5-dimethylthiophen-2-yl]-1-(4-methoxybenzyl)hydrazinecarbothioamide (750 mg) obtained in Preparative Example 35 was suspended in ethanol (15 ml), and p-toluenesulfonic acid monohydrate (31 mg) was added. The mixture was refluxed under heating for one hour. After the completion of the reaction, the reaction mixture was concentrated under reduced pressure. The residue was dissolved in chloroform, and successively washed with water, a saturated aqueous sodium hydrogencarbonate solution and water. The organic layer was dried over anhydrous sodium sulfate, filtrated, and concentrated under reduced pressure. The residue was purified by silica gel column chromatography (hexane:ethyl acetate=3:1) to give 530 mg of the title compound as yellow crystals.

Melting point : 108-110°C

Preparative Example 56 (Step 3)

4-(4-Chlorophenyl)-2-ethyl-6-(4-methoxybenzyl)-6,8-dihydro-1-thia-5,6,8-triazaazulene-7-thione

Preparative Example 57 (Step 3)

6-(4-Methoxybenzyl)-2-methyl-4-phenyl-6,8-dihydro-1-thia-5,6,8-triaaszulene-7-thione

Preparative Example 58 (Step 3)

6-(4-Methoxybenzyl)-2,3-dimethyl-4-phenyl-6,8-dihydro-1-thia-5,6,8-triazaazulene-7-thione

Preparative Example 59 (Step 3)

2-Ethyl-6-(4-methoxybenzyl)-4-phenyl-6,8-dihydro-1-thia-5,6,8-triazaazulene-7-thione

Preparative Example 60 (Step 3)

6-(4-Methoxybenzyl)-4-(4-methoxyphenyl)-2,3-dimethyl-6,8-dihydro-1-thia-5,6,8-triazaazulene-7-thione

Preparative Example 61 (Step 3)

4-(2-Chlorophenyl)-2,3-dimethyl-6-(pyridin-4-ylmethyl)-6,8-dihydro-1-thia-5,6,8-triazaaulene-7-thione

In the same manner as in Preparative Example 55, the compounds of Preparative Examples 56, 57, 58, 59, 60 and 61 were obtained from the compounds of Preparative Examples 36, 37, 38, 39, 40 and 41, respectively. The

compounds are shown in Tables 16 and 17.

**Table 16**

| Prep. Ex. | Structural formula | $^1$H NMR (300MHz, δppm, $CDCl_3$) | Melting point (°C) |
|---|---|---|---|
| 56 | 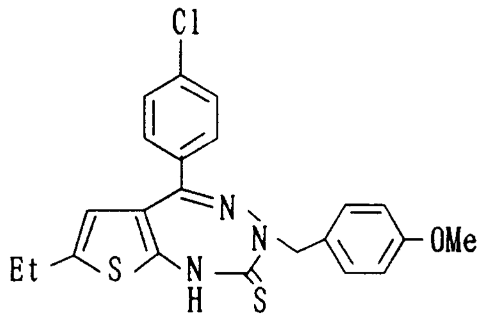 | 1.26 (3H, t, J=7.5Hz), 2.71 (2H, q, J=7.5Hz), 3.79(3H, s), 5.22(2H, s), 6.20(1H, s), 6.85 (2H, d, J=8.7Hz), 7.19 (2H, d, J=8.7Hz), 7.27-7.32(4H, m), 7.77(1H, m). | 135 |
| 57 | 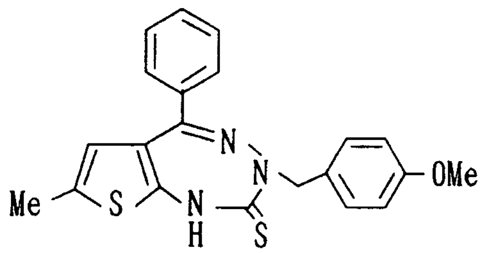 | | 179-181 |
| 58 | 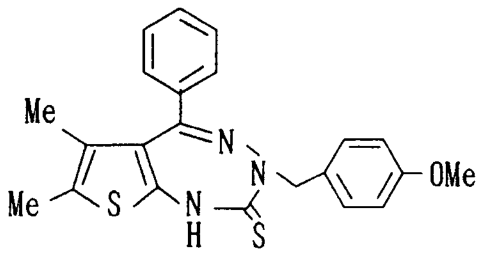 | 1.39(3H, s), 2.23(3H, s), 3.78(3H, s), 5.17(2H, bs), 6.83 (2H, d, J=8.7Hz), 7.11-7.15(2H, m), 7.24-7.42(4H, m), 7.59(1H, m). | 157-160 |

**Table 17**

| Prep. Ex. | Structural formula | Melting point (°C) |
|---|---|---|
| 59 | 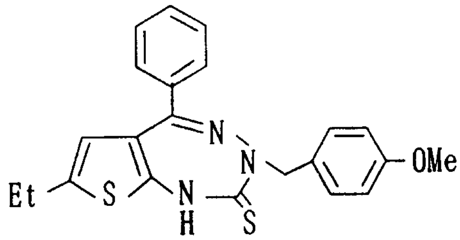 | 198-200 |
| 60 | 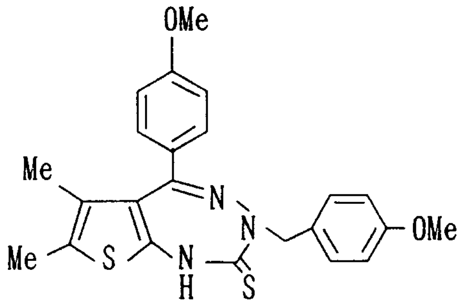 | 148-150 |
| 61 | 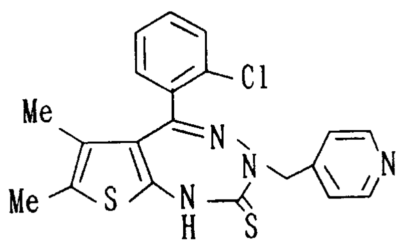 | 196-199 |

Preparative Example 62 (Step 2, Step3)

3-(4-Methoxybenzyl)-8-methyl-5-phenyl-1,3-dihydrobenzo[e][1,2,4]triazepine-2-thione

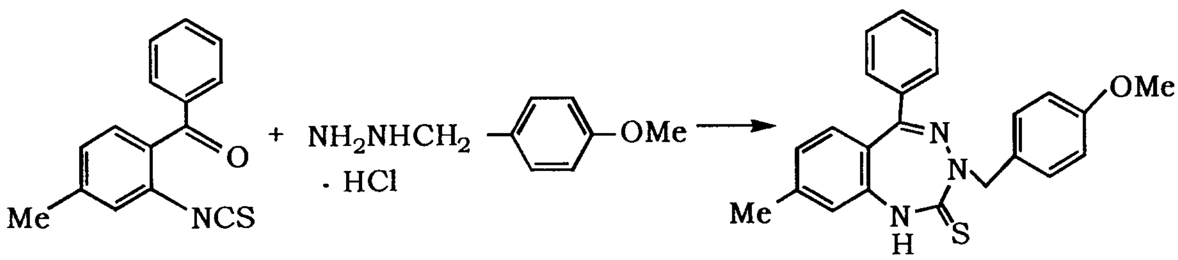

In the same manner as in Preparative Example 23, 2-benzoyl-5-methylphenyl isothiocyanate (4.2 g) obtained in Preparative Example 8, 4-methoxybenzylhydrazine hydrochloride (3.3 g) obtained in Preparative Example 3 and triethylamine (2.44 ml) were reacted. The reaction solvent was distilled away, and the oily residue was heated at 50°C for 2 hours. The mixture was extracted in the same manner as in Preparative Example 1, and crystallized from chloroform-diethyl ether to give 1.78 g of the title compound as yellow crystals.

Melting point : 190-191°C

Preparative Example 63 (Step 4)

5-(4-Chlorophenyl)-3-(4-methoxybenzyl)-3H-benzo[e][1,2,4]triazepin-2-ylhydrazine

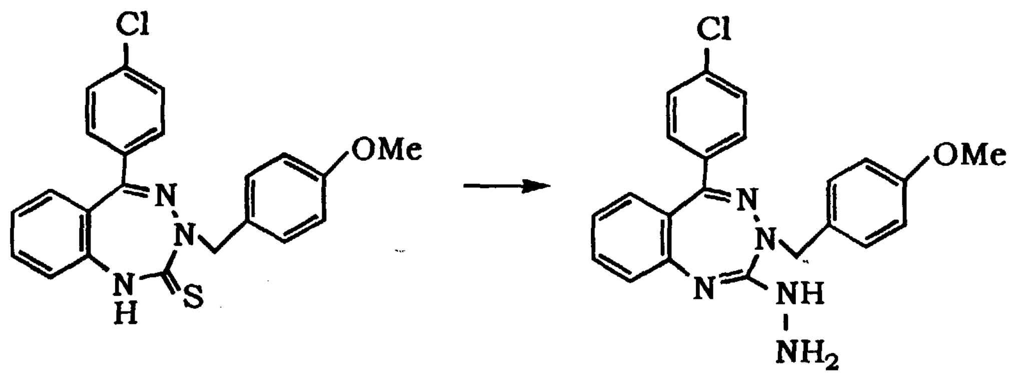

5-(4-Chlorophenyl)-3-(4-methoxybenzyl)-1,3-dihydrobenzo[e][1,2,4]triazepine-2-thione (41 mg) obtained in Preparative Example 44 was dissolved in tetrahydrofuran (0.4 ml), and hydrazine monohydrate (49 μl) was added. The mixture was stirred at room temperature for 18 hours. After the completion of the reaction, the reaction mixture was concentrated under reduced pressure. The residue was dissolved in ethyl acetate, and washed three times with water. The organic layer was dried, filtrated, and concentrated under reduced pressure. The residue was purified by preparative thin-layer chromatography (chloroform:methanol=20:1) to give 27 mg of the title compound as a pale-yellow powder.

$^1H$ NMR (300MHz, δ ppm, $CDC1_3$)

3.78(3H,s), 4.71(2H,s), 6.82(2H,d,J=8.8Hz), 7.00(3H,m), 7.15-7.28(6H,m), 7.41(1H,m)

Preparative Example 64 (Step 6)

5-(4-Chlorophenyl)-3-(3,4-dimethoxybenzyl)-2-methylthio-3H-benzo[e][1,2,4]triazepine

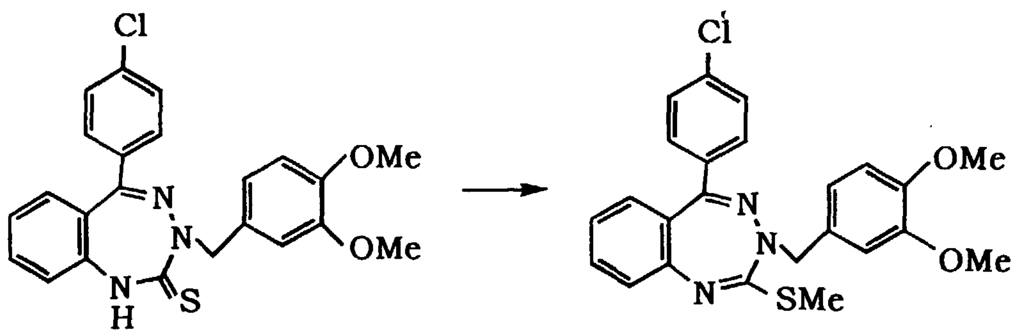

5-(4-Chlorophenyl)-3-(3,4-dimethoxybenzyl)-1,3-dihydrobenzo[e][1,2,4]triazepine-2-thione (41 g) obtained in Preparative Example 42 was dissolved in anhydrous N,N-dimethylformamide (200 ml), and sodium hydride (60% in oil, 3.51 g) was added. The mixture was stirred at room temperature for 30 minutes under an argon atmosphere, and cooled with ice. Methyl iodide (5.47 ml) was added, and the mixture was stirred under ice-cooling for 30 minutes and at room temperature for 1 hour. After the completion of the reaction, the reaction mixture was cooled with ice, added with acetic acid (1.67 ml), and concentrated under reduced pressure. The resulting residue was dissolved in ethyl acetate, and successively washed with a 5% aqueous citric acid solution, a saturated aqueous sodium hydrogencarbonate solution and water. The ethyl acetate layer was dried, filtrated, and concentrated under reduced pressure. The resulting oil was crystallized from diethyl ether-n-hexane to give 30.43 g of the title compound as pale-yellow crystals.

Melting point : 136-137°C

Preparative Example 65 (Step 6)

3-(4-Methoxybenzyl)-2-methylthio-5-phenyl-3H-benzo[e][1,2,4]triazepine

Preparative Example 66 (Step 6)

7-Chloro-3-(4-methoxybenzyl)-2-methylthio-5-phenyl-3H-benzo[e][1,2,4]triazepine

Preparative Example 67 (Step 6)

3-(4-Methoxybenzyl)-8-methyl-2-methylthio-5-phenyl-3H-benzo[e][1,2,4]triazepine

Preparative Example 68 (Step 6)

3-(4-Methoxybenzyl)-2-methylthio-7-nitro-5-phenyl-3H-benzo[e][1,2,4]triazepine

Preparative Example 69 (Step 6)

3-(4-Methoxybenzyl)-5-(4-methylphenyl)-2-methylthio-3H-benzo[e][1,2,4]triazepine

Preparative Example 70 (Step 6)

7-Chloro-5-(2-Chlorophenyl)-3-(4-methoxybenzyl)-2-methylthio-3H-benzo[e][1,2,4]triazepine

Preparative Example 71 (Step 6)

5-(4-Chlorophenyl)-3-(4-methoxybenzyl)-8-methyl-2-methylthio-3H-benzo[e][1,2,4]triazepine

Preparative Example 72 (Step 6)

5-(4-Bromophenyl)-3-(4-methoxybenzyl)-2-methylthio-3H-benzo[e][1,2,4]triazepine

Preparative Example 73 (Step 6)

tert-Butyl 4-[3-(4-methoxybenzyl)-2-methylthio-3H-benzo[e][1,2,4]triazepin-5-yl]benzoate

Preparative Example 74 (Step 6)

5-(4-Chlorophenyl)-3-(4-methoxybenzyl)-2-methylthio-3H-pyrido[2,3-e][1,2,4]triazepine

In the same manner as in Preparative Example 64, the compounds of Preparative Examples 65, 66, 67, 68, 69, 70, 71, 72, 73 and 74 were obtained from the compounds of Preparative Examples 43, 45, 62, 46, 47, 48, 49, 50, 51 and 52, respectively. The compounds are shown in Tables 18-20.

**Table 18**

| Prep. Ex. | Structural formula | $^1$H NMR (300MHz, δppm, $CDCl_3$) |
|---|---|---|
| 65 | 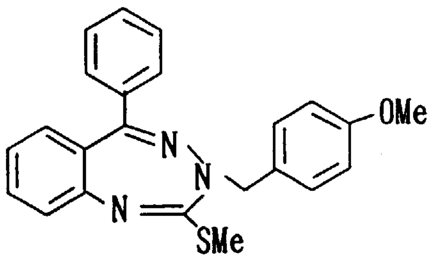 | 2.35(3H, s), 3.79(3H, s), 4.71(2H, s), 6.83 (2H, d, J=8.8Hz), 6.97 (2H, d, J=3.9Hz), 7.15 (1H, d, J=8.0Hz), 7.24-7.43(8H, m). |
| 66 | 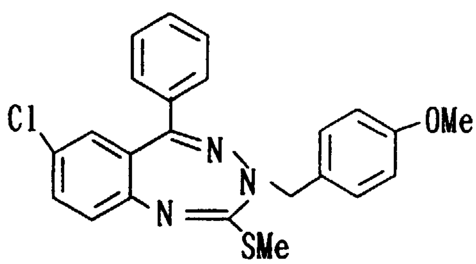 | 2.52(3H, s), 3.79(3H, s), 4.70(2H, s), 6.84(2H, m), 6.93 (1H, d, J=2.4Hz), 7.07 (1H, d, J=8.5Hz), 7.22-7.42(8H, m). |
| 67 | 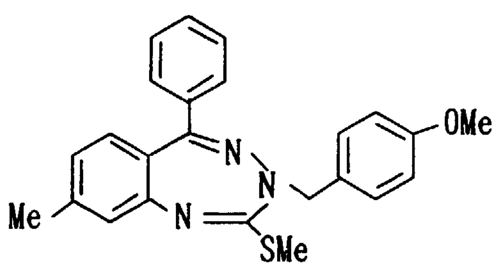 | 2.39(3H, s), 2.57 (3H, broad s), 3.79(3H, s), 4.73(2H, s), 6.79-6.87(4H, m), 7.03(1H, m), 7.24-7.38(7H, m). |

Table 19

| Prep. Ex. | Structural formula | $^1$H NMR (300MHz, δppm, $CDCl_3$) | Melting point (°C) |
|---|---|---|---|
| 68 | 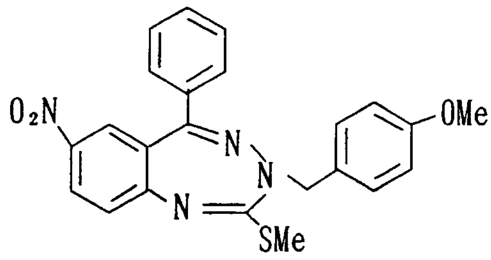 | 2.56(3H, s), 3.80(3H, s), 4.71(2H, s), 6.85 (2H, d, J=9.0Hz), 7.20-7.46(8H, m), 7.86 (1H, d, J=3.0Hz), 8.23 (1H, dd, J=7.2 and 2.6Hz). | 148-151 |
| 69 | 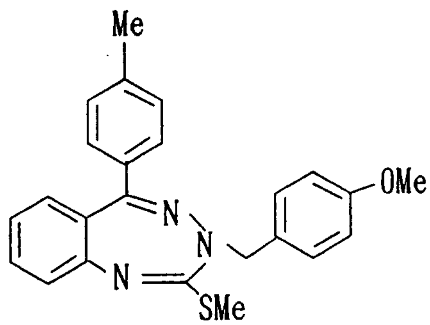 | 2.34(3H, s), 2.53(3H, s), 3.78(3H, s), 4.70(2H, s), 6.82 (2H, d, J=8.4Hz), 6.96-6.98(2H, m), 7.08-7.23(7H, m), 7.32-7.42(1H, m). | Amorphous solid |
| 70 | 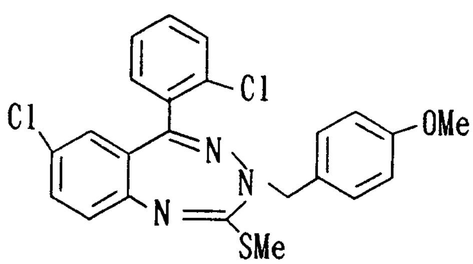 | | 139-140 |

**Table 20**

| Prep. Ex. | Structural formula | $^1$H NMR (300MHz, δppm, $CDCl_3$) | Melting point (℃) |
|---|---|---|---|
| 71 | 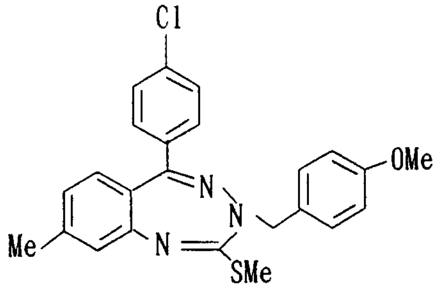 | 2.38(3H, s), 2.52(3H, s), 3.78(3H, s), 4.69(2H, s), 6.77-6.84(4H, m), 6.98(1H, s), 7.21-7.30(6H, m) | Amorphous solid |
| 72 | 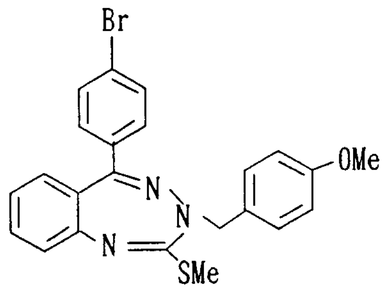 | 2.53(3H, s), 3.78(3H, s), 4.69(2H, s), 6.82 (2H, d, J=8.7Hz), 6.93-6.97(2H, m), 7.13-7.25(5H, m), 7.41-7.44(3H, m) | Amorphous solid |
| 73 | 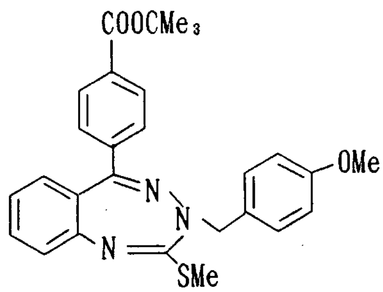 | 1.58(9H, s), 2.54(3H, s), 3.79(3H, s), 4.72(2H, s), 6.83 (2H, d, J=8.7Hz), 6.85-6.96(2H, m), 7.14 (1H, d, J=8.1Hz), 7.25 (2H, d, J=8.6Hz), 7.35 (2H, d, J=8.4Hz), 7.36-7.41(1H, m), 7.91 (2H, d, J=8.5Hz) | |
| 74 | 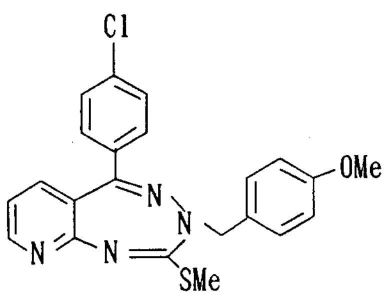 | 2.62(3H, s), 3.79(3H, s), 4.71(2H, s), 6.83 (2H, d, J=8.8Hz), 6.92-6.96(1H, m), 7.21-7.33(7H, m), 8.57-8.60(1H, m) | |

Preparative Example 75 (Step 6)

5-(4-Chlorophenyl)-2-methylthio-3-(pyridin-3-ylmethyl)-3H-benzo[e][1,2,4]triazepine

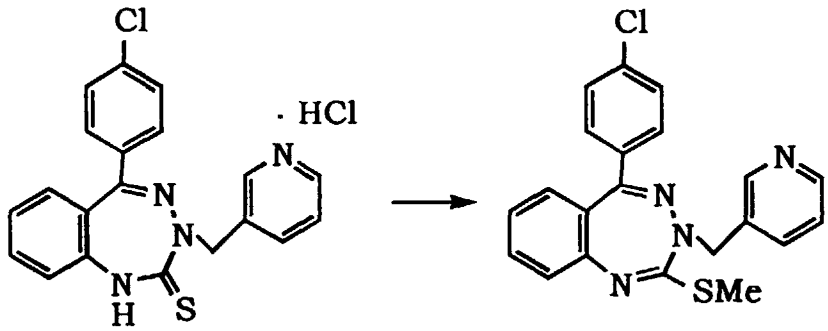

5-(4-Chlorophenyl)-3-(pyridin-3-ylmethyl)-1,3-dihydrobenzo[e][1,2,4]triazepine-2-thione hydrochloride (1.97 g) obtained in Preparative Example 53 was dissolved in anhydrous N,N-dimethylformamide (20 ml), and sodium hydride (380 mg) was added under ice-cooling with stirring. The mixture was allowed to warm up to room temperature, and stirred for 30 minutes. The mixture was cooled again with ice, and methyl iodide (296 μl) was added to the mixture. The mixture was allowed to warm up to room temperature, and stirred for 30 minutes. The reaction mixture was poured into water (50 ml), and neutralized with citric acid. The reaction mixture was extracted with ethyl acetate (50 ml), washed with water five times, dried over anhydrous sodium sulfate, and filtrated. The filtrate was concentrated under reduced pressure to give 1.79 g of the title compound as a yellow oil.

$^1$H NMR (300MHz, δ ppm, $CDC1_3$)
2.56(3H,s), 4.77(2H,s), 6.92-7.02(2H,m), 7.15-7.32(6H,m), 7.43(1H,m), 7.61(1H,m), 8.50(1H,dd,J=4.8 and 1.7Hz), 8.59(1H,d,J=1 .7Hz)

Preparative Example 76 (Step 6)

5-(4-Chlorophenyl)-2-methylthio-3-(pyridin-3-ylmethyl)-3H-benzo[e][1,2,4]triazepine

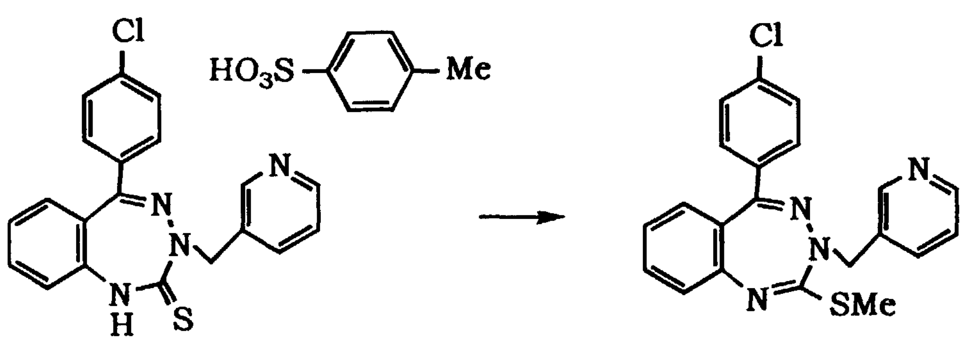

In the same manner as in Preparative Example 75, the title compound was obtained as an amorphous compound from 5-(4-chlorophenyl)-3-(pyridin-3-ylmethyl)-1,3-dihydrobenzo[e][1,2,4]triazepine-2-thione p-toluenesulfonate obtained in Preparative Example 54. The NMR data of the compound is consistent with that of Preparative Example 75.

Preparative Example 77 (Step 6)

4-(4-Chlorophenyl)-6-(4-methoxybenzyl)-2,3-dimethyl-7-methylthio-6H-1-thia-5,6,8-triazaazulene

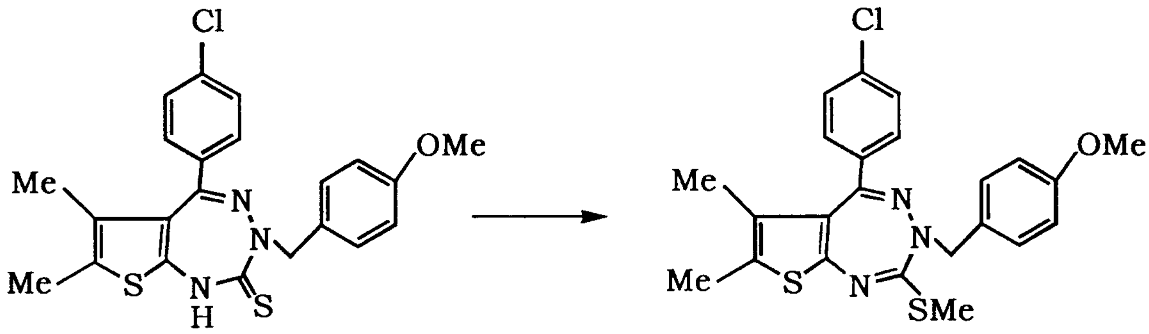

4-(4-Chlorophenyl)-6-(4-methoxybenzyl)-2,3-dimethyl-6,8-dihydro-1-thia-5,6,8-triazaazulene-7-thione (442 mg) obtained in Preparative Example 55 was dissolved in acetone (5 ml), and anhydrous potassium carbonate (1.38 g) and methyl iodide (74.7 μl) were added. The mixture was stirred at room temperature for one hour. Potassium carbonate was removed by filtration, and the filtrate was concentrated under reduced pressure. The residue was purified by silica gel column chromatography (hexane:ethyl acetate=4:1) to give 430 mg of the title compound as an amorphous compound.

Preparative Example 78 (Step 6)

4-(4-Chlorophenyl)-2-ethyl-6-(4-methoxybenzyl)-7-methylthio-6H-1-thia-5,6,8-triazaazulene

Preparative Example 79 (Step 6)

6-(4-Methoxybenzyl)-2-methyl-7-methylthio-4-phenyl-6H-1-thia-5,6,8-triazaazulene

Preparative Example 80 (Step 6)

6-(4-Methoxybenzyl)-2,3-dimethyl-7-methylthio-4-phenyl-6H-1-thia-5,6,8-triazaazulene

Preparative Example 81 (Step 6)

2-Ethyl-6-(4-methoxybenzyl)-7-methylthio-4-phenyl-6H-1-thia-5,6,8-triazaazulene

Preparative Example 82 (Step 6)

6-(4-Methoxybenzyl)-4-(4-methoxyphenyl)-2,3-dimethyl-7-methylthio-6H-1-thia-5,6,8-triazaazulene

Preparative Example 83 (Step 6)

4-(2-Chlorophenyl)-2,3-dimethyl-7-methylthio-6-(pyridin-4-ylmethyl)-6H-1-thia-5,6,8-triazaazulene

In the same manner as in Preparative Example 77, the compounds of Preparative Examples 78, 79, 80, 81, 82 and 83 were obtained from the compounds of Preparative Examples 56, 57, 58, 59, 60 and 61, respectively. The obtained compounds are shown in Tables 21 and 22.

**Table 21**

| Prep. Ex. | Structural formula | $^1$H NMR (300MHz, δppm, $CDCl_3$) | Melting point (°C) |
|---|---|---|---|
| 78 | 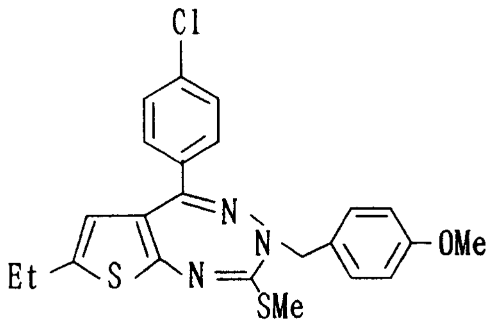 | 1.26 (3H, t, J=7.5Hz), 2.51(3H, s), 2.72(2H, m), 3.80(3H, s), 4.72(2H, s), 6.26(1H, s), 6.86 (2H, d, J=8.7Hz), 7.20-7.36(6H, m). | |
| 79 | 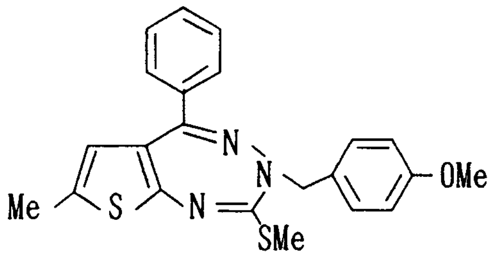 | 2.36 (3H, d, J=1.1Hz), 2.51(3H, s), 3.80(3H, s), 4.73(2H, s), 6.26 (1H, q, J=1.1Hz), 6.84-6.88(2H, m), 7.28-7.42(7H, m). | Amorphous solid |
| 80 | 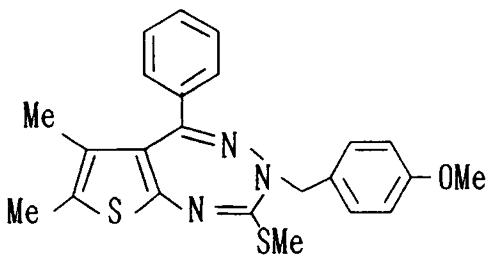 | | 127-128 |

**Table 22**

| Prep. Ex. | Structural formula | $^1$H NMR (300MHz, δppm, $CDCl_3$) | Melting point (℃) |
|---|---|---|---|
| 81 | 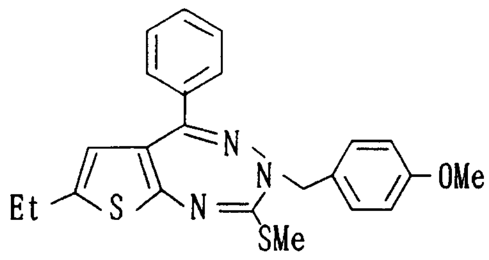 | 1.26 (3H, t, J=7.5Hz), 2.51(3H, s), 2.72(2H, m), 3.80(3H, s), 4.74(2H, s), 6.29 (1H, t, J=1.0Hz), 6.87 (2H, d, J=8.7Hz), 7.28-7.38(7H, m). | Amorphous solid |
| 82 | 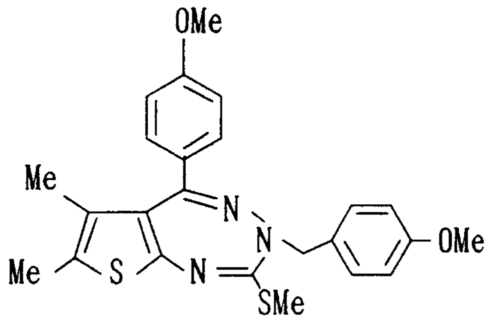 | 1.45(3H, s), 2.25(3H, s), 2.49(3H, s), 3.79(3H, s), 3.80(3H, s), 4.71(2H, broad), 6.79 (2H, d, J=8.9Hz), 6.85 (2H, d, J=8.7Hz), 7.14 (2H, d, J=8.8Hz), 7.32 (2H, d, J=8.6Hz). | Amorphous solid |
| 83 | 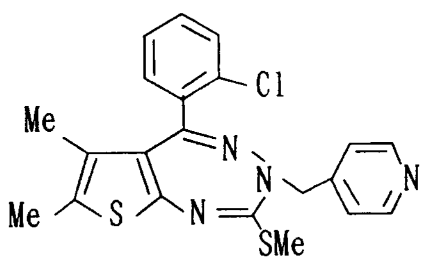 | | 156-159 |

Preparative Example 84 (Step 6)

5-(4-Chlorophenyl)-3-(4-methoxybenzyl)-2-methylthio-3H-benzo[e][1,2,4]triazepine

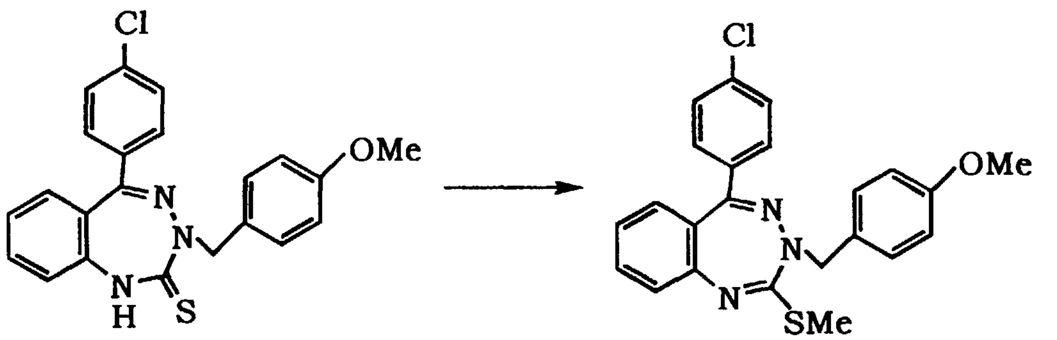

5-(4-Chlorophenyl)-3-(4-methoxybenzyl)-1,3-dihydrobenzo[e][1,2,4]triazepine-2-thione (100 mg) obtained in Preparative Example 44 was dissolved in acetone (1 ml), and anhydrous potassium carbonate (339 mg) and methyl iodide (18 μl) were added. The mixture was stirred at room temperature for 2 hours. Potassium carbonate was removed by filtration, and the filtrate was concentrated under reduced pressure. The residue was dissolved in diethyl ether, and successively washed with water, a 5% aqueous citric acid solution and water. The organic layer was dried, filtrated, and concentrated under reduced pressure. The residue was purified by preparative thin-layer chromatography (n-hexane:ethyl acetate=3:1) to give 97 mg of the title compound.

$^1H$ NMR (300MHz δ ppm, CDC1$_3$)
2.53(3H,s), 3.78(3H,s), 4.70(2H,s), 6.82(2H,d,J=8.7Hz), 6.96(2H,m), 7.15(1H,d,J=7.9Hz), 7.21-7.28(6H,m), 7.40(1H,m)

Preparative Example 85 (Step 7")

5-(4-Chlorophenyl)-3-(4-methoxybenzyl)-3H-benzo[e][1,2,4]triazepin-2-ylhydrazine

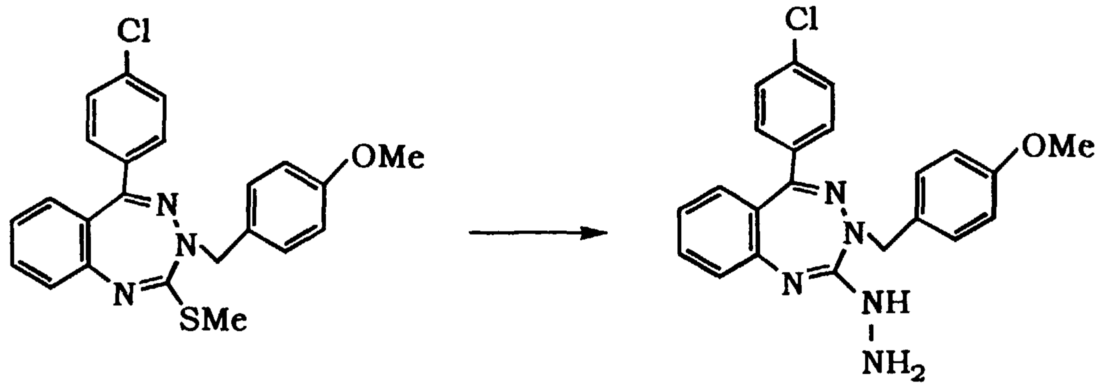

5-(4-Chlorophenyl)-3-(4-methoxybenzyl)-2-methylthio-3H-benzo[e][1,2,4]triazepine (42.2 mg) obtained in Preparative Example 84 and hydrazine monohydrate (10 μl) were dissolved in ethanol (0.4 ml), and the solution was stirred under heating at 70°C for 24 hours. After the completion of the reaction, the mixture was concentrated under reduced

pressure. The resulting residue was dissolved in ethyl acetate, and washed three times with water. The organic layer was dried, filtrated, and concentrated under reduced pressure. The residue was purified by preparative thin-layer chromatography (chloroform:methanol=20: 1) to give 10.8 mg of the title compound. The spectrum data of the compound was consistent with that of Preparative Example 63.

Preparative Example 86 (Step 7)

Acetic acid N'-[5-(4-chlorophenyl)-3-(3,4-dimethoxybenzyl)-3H-benzo[e][1,2,4]triazepin-2-yl]hydrazide

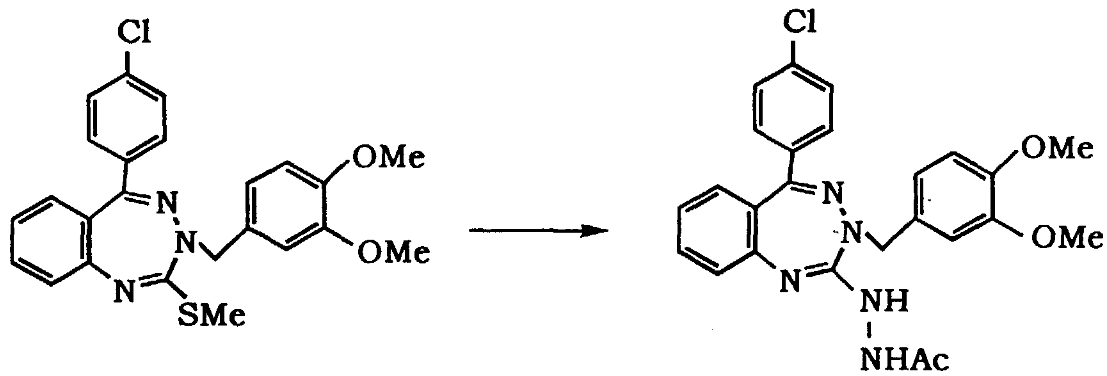

5-(4-Chlorophenyl)-3-(3,4-dimethoxybenzyl)-2-methylthio-3H-benzo[e][1,2,4]triazepine (51.5 g) obtained in Preparative Example 64 was dissolved in n-butanol (110 ml), and acetylhydrazine (16.89 g) was added. The mixture was stirred at 110°C for 3 hours. The reaction mixture was cooled and the precipitated solid was collected by filtration, and successively washed with diethyl ether and water. The solid was dissolved in dichloromethane (1 L), and dried over anhydrous sodium sulfate. The drying agent was removed by filtration, and the filtrate was concentrated. Diethyl ether was added to the concentrated filtrate to give 48.1 g of the title compound as crystals.

Melting point : 145-146°C

Preparative Example 87 (Step 7)

Acetic acid N'-[3-(4-methoxybenzyl)-5-phenyl-3H-benzo[e][1,2,4]triazepin-2-yl]hydrazide

Preparative Example 88 (Step 7)

Acetic acid N'-[3-(4-methxybenzyl)-8-methyl-5-phenyl-3H-benzo[e][1,2,4]triazepin-2-yl]hydrazide

In the same manner as in Preparative Example 86, the compounds of Preparative Examples 87 and 88 were obtained from the compounds of Preparative Examples 65 and 67, respectively. The compounds are shown in Table 23.

**Table 23**

| Prep. Ex. | Structural formula | Melting point (°C) |
|---|---|---|
| 87 | 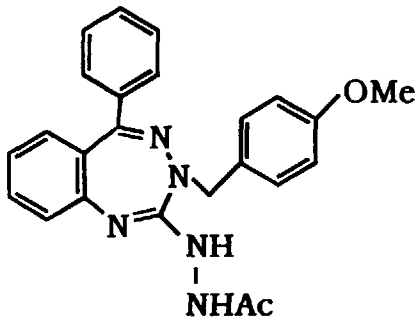 | 213-216 |
| 88 | 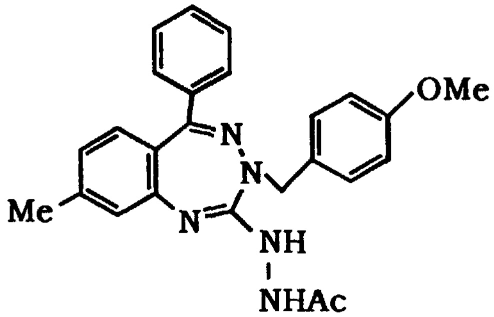 | 235-238 |

Synthetic Example 1 (Step 5)

6-(4-Chlorophenyl)-4-(4-methoxybenzyl)-1-methyl-4H-2,3,4,5,10b-pentaazabenz[e]azulene

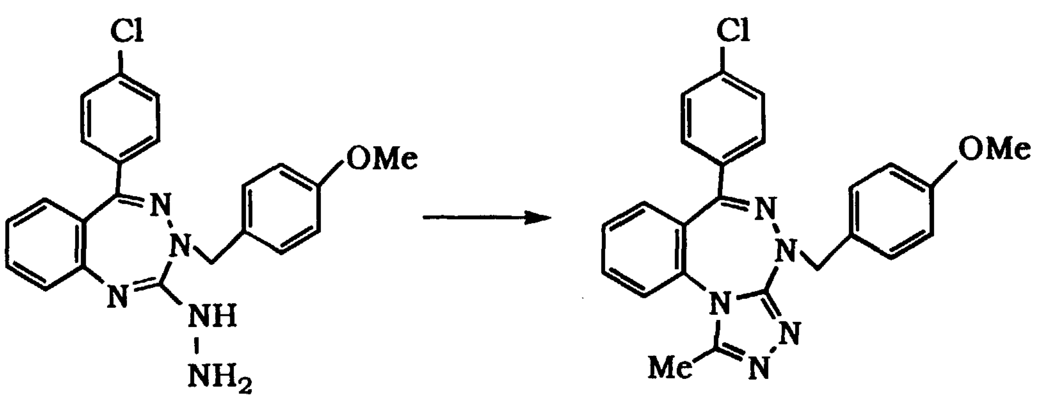

5-(4-Chlorophenyl)-3-(4-methoxybenzyl)-3H-benzo[e][1,2,4]triazepin-2-ylhydrazine (23 mg) obtained in Preparative Example 63 or Preparative Example 85, triethyl orthoacetate (15 μl) and p-toluenesulfonic acid monohydrate (2.4 mg) were suspended in toluene (0.5 ml). The suspension was refluxed under heating for 3 hours and cooled. Ethyl acetate was added, and the organic layer was successively washed with an aqueous sodium hydrogencarbonate solution and water. The organic layer was dried, filtrated, and concentrated under reduced pressure. The residue was crystallized from ethyl acetate-diethyl ether to give 16 mg of the title compound as colorless needles.

Melting point : 192-193°C
$^1$H NMR (300MHz δ ppm, $CDC1_3$)
2.60(3H,s), 3.79(3H,s), 4.89-5.04(2H,m), 6.83-6.85(2H,m), 6.96(2H,m), 7.18-7.37(9H,m), 7.59-7.64(1H,m)

Synthetic Example 2 (Step 8)

6-(4-Chlorophenyl)-4-(3,4-dimethoxybenzyl)-1-methyl-4H-2,3,4,5,10b-pentaazabenz[e]azulene

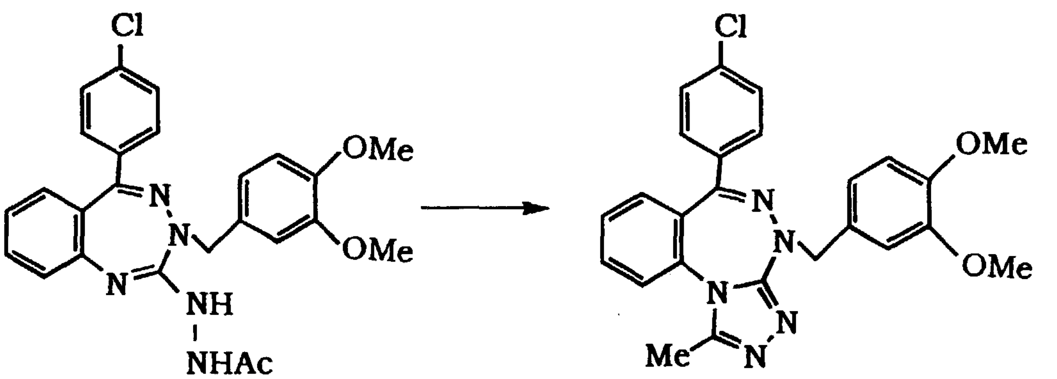

Acetic acid N'-[5-(4-chlorophenyl)-3-(3,4-dimethoxybenzyl)-3H-benzo[e][1,2,4]triazepin-2-yl]hydrazide (48 g) obtained in Preparative Example 86 and p-toluenesulfonic acid monohydrate (2.1 g) were suspended in toluene, and the suspension was stirred under heating at 110°C for 30 minutes. After the completion of the reaction, the solvent was distilled away under reduced pressure. The residue was dissolved in dichloromethane, and successively washed with a saturated aqueous sodium hydrogencarbonate and water. The dichloromethane layer was dried, filtrated, and concentrated. The residue was crystallized from diethyl ether to give 42.4 g of the title compound as colorless crystals.

Melting point : 235-237°C

Synthetic Example 3 (Step 8)

4-(4-Methoxybenzyl)-1-methyl-6-phenyl-4H-2,3,4,5,10b-pentaazabenz[e]azulene

Synthetic Example 4 (Step 8)

4-(4-Methoxybenzyl)-1,9-dimethyl-6-phenyl-4H-2,3,4,5,10b-pentaazabenz[e]azulene

In the same manner as in Synthetic Example 2, the compounds of Synthetic Examples 3 and 4 were obtained from the compounds of Preparative Examples 87 and 88, respectively. The compounds are shown in Table 24.

**Table 24**

| Synthetic Ex. | Structural formula | Melting point (°C) |
|---|---|---|
| 3 | 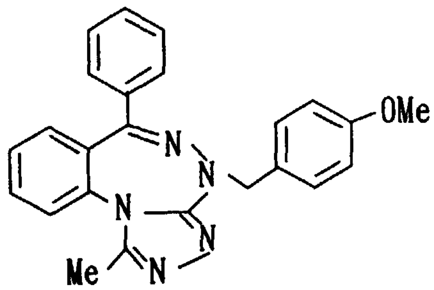 | 139-140 |
| 4 | 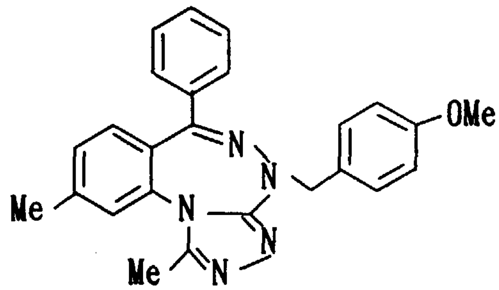 | 101-105 |

Synthetic Example 5 (Step 7')

8-Chloro-4-(4-methoxybenzyl)-1-methyl-6-phenyl-4H-2,3,4,5,10b-pentaazabenz[e]azulene

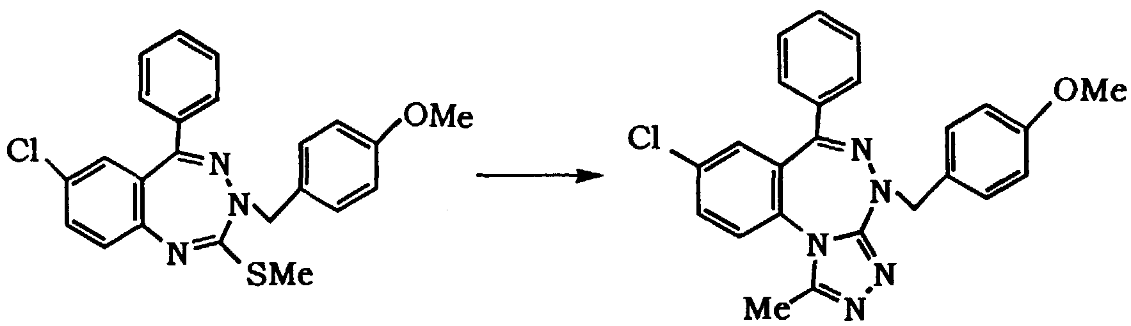

7-Chloro-3-(4-methoxybenzyl)-2-methylthio-5-phenyl-3H-benzo[e][1,2,4]triazepine (600 mg) obtained in Preparative Example 66, acetylhydrazine (220 mg) and p-toluenesulfonic acid monohydrate (80 mg) were dissolved in n-butanol (6 ml), and the solution was stirred under heating at 90°C for 2 hours and 110°C for 1.5 hours. After the completion of the reaction, the solvent was distilled away under reduced pressure, and the residue was dissolved in ethyl acetate, and successively washed with water, a saturated aqueous sodium hydrogencarbonate solution and water. The organic layer was dried, filtrated, and concentrated under reduced pressure. The residue was applied to silica gel column chromatography, and a product obtained from the fraction eluted with chloroform: acetone=5:1 was crystallized

from diethyl ether to give 100.7 mg of the title compound as crystals.

Melting point : 116-118°C

Synthetic Example 6 (Step 7')

4-(4-Methoxybenzyl)-1-methyl-8-nitro-6-phenyl-4H-2,3,4,5,10b-pentaazabenz[e]azulene

Synthetic Example 7 (Step 7')

4-(4-Methoxybenzyl)-1-methyl-6-(4-methylphenyl)-4H-2,3,4,5,10b-pentaazabenz[e)azulene

Synthetic Example 8 (Step 7')

8-Chloro-6-(2-chlorophenyl)-4-(4-methoxybenzyl)-1-methyl-4H-2,3,4,5,10b-pentaazabenz[e]azulene

Synthetic Example 9 (Step 7')

6-(4-Chlorophenyl)-4-(4-methoxybenzyl)-1,9-dimethyl-4H-2,3,4,5,10b-pentaazabenz[e]azulene

Synthetic Example 10 (Step 7')

6-(4-Bromophenyl)-4(4-methoxybenzyl)-1-methyl-4H-2,3,4,5,10b-pentaazabenz[e]azulene

Synthetic Example 11 (Step 7')

tert-Butyl 4-[4-(4-methoxybenzyl)-1-methyl-4H-2,3,4,5,10b-pentaazabenz[e]azulene-6-yl]benzoate

Synthetic Example 12 (Step 7')

6-(4-Chlorophenyl)-4-(4-methoxybenzyl)-1-methyl-4H-2,3,4,5,10,10b-hexaazabenz[e]azulene

In the same manner as in Synthetic Example 5, the compounds of Synthetic Examples 6, 7, 8, 9, 10, 11 and 12 were obtained from the compounds of Preparative Examples 68, 69, 70, 71, 72, 73 and 74, respectively. The compounds are shown in Tables 25 and 26.

**Table 25**

| Synth. Ex. | Structural formula | $^1$H NMR (300MHz, δppm, $CDCl_3$) | Melting point (°C) |
|---|---|---|---|
| 6 | 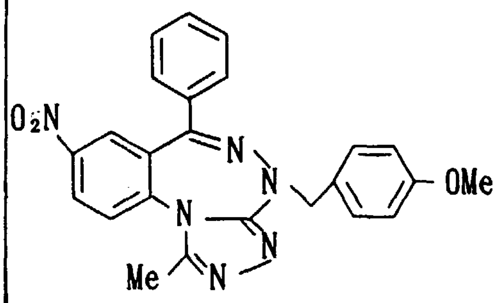 | 2.67(3H, s), 3.79(3H, s), 4.97(2H, broad), 6.85 (2H, d, J=8.8Hz), 7.32 (2H, d, J=8.7Hz), 7.37-7.51(6H, m), 8.09 (1H, d, J=2.5Hz), 8.45 (1H, dd, J=8.6 and 2.6Hz). | 189 (decompo-sition) |
| 7 | 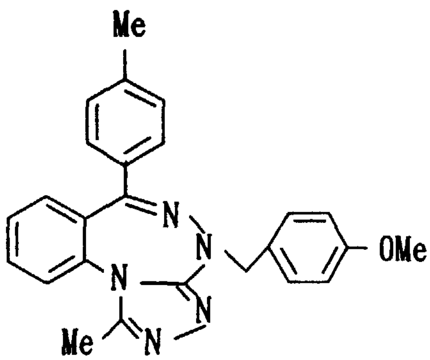 | | 194-196 |
| 8 | 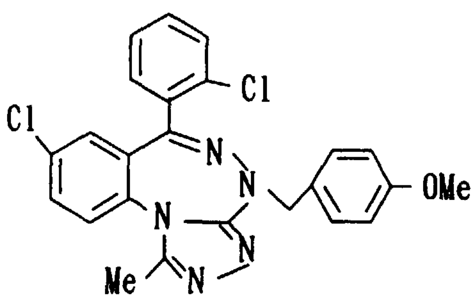 | | 188-191 |

**Table 26**

| Synth. Ex. | Structural formula | $^1H$ NMR (300MHz, δppm, $CDCl_3$) | Melting point (°C) |
|---|---|---|---|
| 9 | 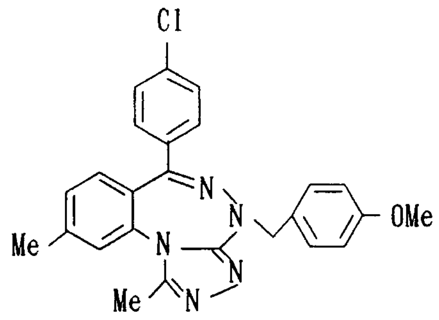 | 2.48(3H, s), 2.60(3H, s), 3.79(3H, s), 4.87(1H, m), 5.03(1H, m), 6.83 (2H, d, J=8.6Hz), 7.05-7.16(3H, m), 7.26-7.35(9H, m) | 213.5-216.0 |
| 10 | 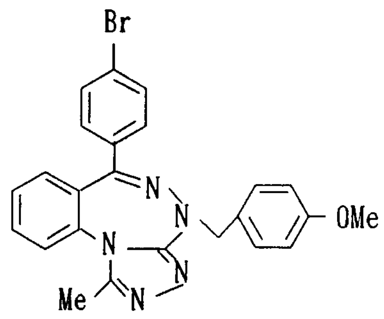 | 2.60(3H, s), 3.79(3H, s), 4.88(1H, m), 5.04(1H, m), 6.84 (2H, d, J=6.7Hz), 7.17-7.64(10H, m) | 204.5-205.5 |
| 11 | 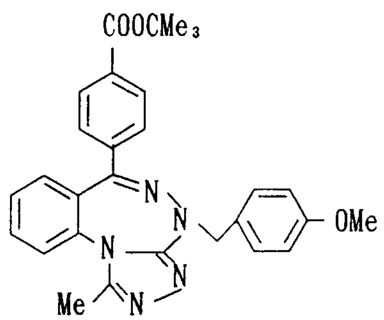 | 1.59(9H, s), 2.61(3H, s), 3.80(3H, s), 4.85-5.12(2H, m), 6.85 (2H, d, J=8.7Hz), 7.14 (1H, d, J=8.0Hz), 7.31-7.36(4H, m), 7.44 (2H, d, J=8.5Hz), 7.60-7.63(1H, m), 7.94 (2H, d, J=8.5Hz) | Amorphous solid |
| 12 | 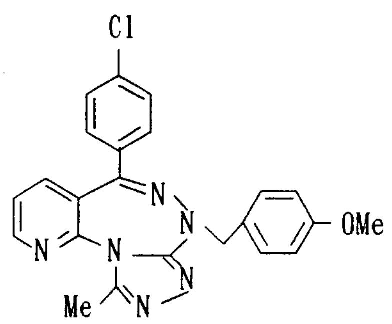 | 2.72(3H, s), 3.80(3H, s), 4.99(2H, s), 6.86 (2H, d, J=8.7Hz), 7.27-7.35(7H, m), 7.50-7.53(1H, m), 8.61-8.63(1H, m) | |

Synthetic Example 13 (Step 7')

4-(4-Chlorophenyl)-6-(4-methoxbenzyl)-2,3,9-trimethyl-6H-5,6,7,8,9a-pentaazathieno[2,3-e]azulene

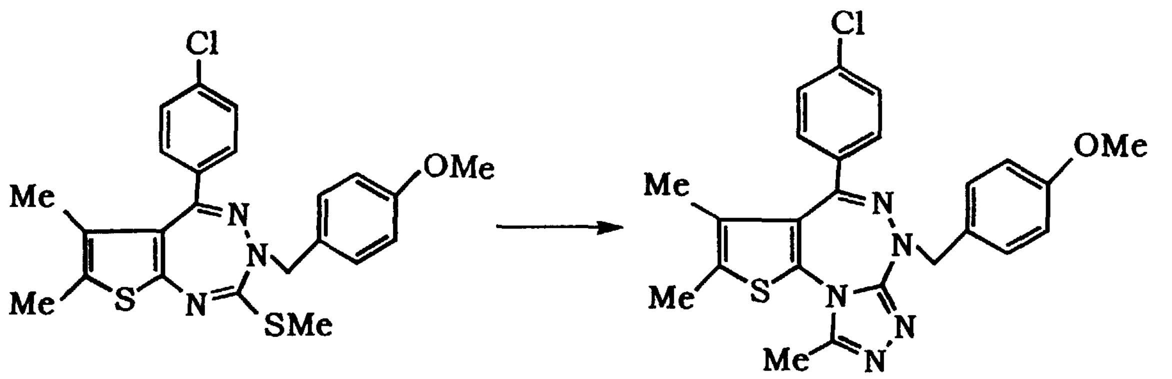

4-(4-Chlorophenyl)-6-(4-methoxybenzyl)-2,3-dimethyl-7-methylthio-6H-1-thia-5,6,8-triazaazulene (350 mg) obtained in Preparative Example 77, acetylhydrazine (114 mg) and p-toluenesulfonic acid monohydrate (14.6 mg) were dissolved in n-butanol (13.5 ml),and the solution was stirred under heating at 110°C for 12 hours. After the completion of the reaction, the solvent was distilled away under reduced pressure. The residue was dissolved in ethyl acetate, and successively washed with water, a saturated aqueous sodium hydrogencarbonate solution and water. The organic layer was dried, filtrated, and concentrated under reduced pressure. The residue was applied to silica gel column chromatography, and a product obtained from the fraction eluted with chloroform:methanol=5:1 was crystallized from a mixed solvent of ethyl acetate and diethyl ether (1:3) to give 160 mg of the title compound as colorless crystals.

Melting point : 214-216°C
$^1$H NMR (300MHz, $\delta$ ppm, $CDCl_3$)
1.26(3H,s), 2.36(3H,s), 2.65(3H,s), 3.81 (3H,s), 4.95(2H,m), 6.86(2H,d,J=8.7Hz), 7.23-7.31 (4H,m), 7.35(2H,d,J=8.7Hz)

Synthetic Example 14 (Step 7')

4-(4-Chlorophenyl)-2-ethyl-6-(4-methoxybenzyl)-9-methyl-6H-5,6,7,8,9a-pentaazathieno[2,3-e]azulene

Synthetic Example 15 (Step 7')

6-(4-Methoxybenzyl)-2,9-dimethyl-4-phenyl-6H-5,6,7,8,9a-pentaazathieno[2,3-e]azulene

Synthetic Example 16 (Step 7')

6-(4-Methoxybenzyl)-2,3,9-trimethyl-4-phenyl-6H-5,6,7,8,9a-pentaazathieno[2,3-e]azulene

Synthetic Example 17 (Step 7')

2-Ethyl-6-(4-methoxybenzyl)-9-methyl-4-phenyl-6H-5,6,7,8,9a-pentaazathieno[2,3-e]azulene

Synthetic Example 18 (Step 7')

6-(4-Methoxybenzyl)-4-(4-methoxyphenyl)-2,3,9-trimethyl-6H-5,6,7,8,9a-pentaazathieno[2,3-e]azulene

In the same manner as in Synthetic Example 13, the compounds of Synthetic Examples 14, 15, 16, 17 and 18 were obtained from the compounds of Preparative Examples 78, 79, 80, 81 and 82, respectively. The compounds are

shown in Tables 27 and 28.

**Table 27**

| Synth. Ex. | Structural formula | $^1$H NMR (300MHz, δppm, $CDCl_3$) | Melting point (°C) |
|---|---|---|---|
| 14 | 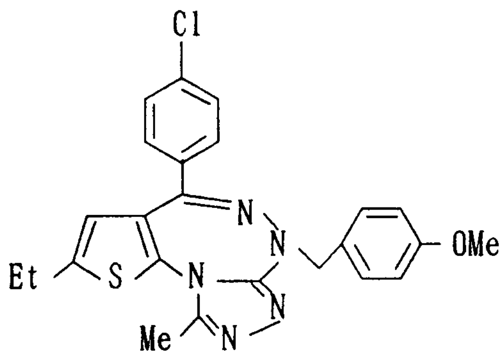 | | 141-143 |
| 15 | 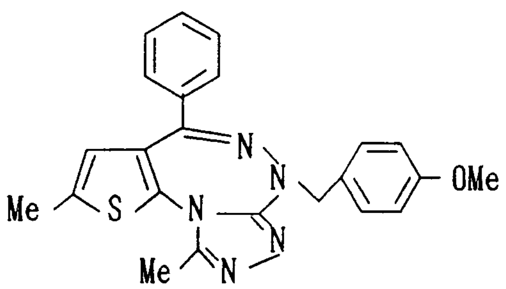 | 2.47 (3H, d, J=1.1Hz), 2.61(3H, s), 3.81(3H, s), 4.97(2H, s), 6.42 (1H, q, J=1.1Hz), 6.85-6.90(2H, m), 7.32-7.40(7H, m). | Amorphous solid |
| 16 | 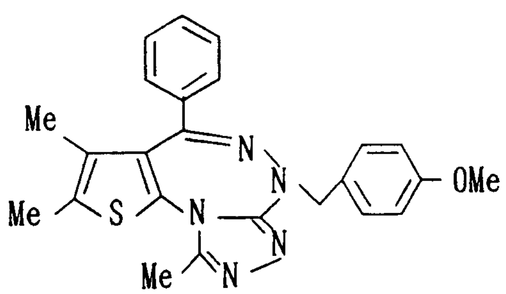 | | 169-171 |

**Table 28**

| Synth. Ex. | Structural formula | Melting point (°C) |
|---|---|---|
| 17 | 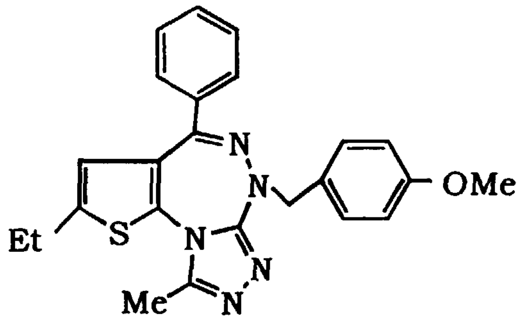 | 118-119 |
| 18 | 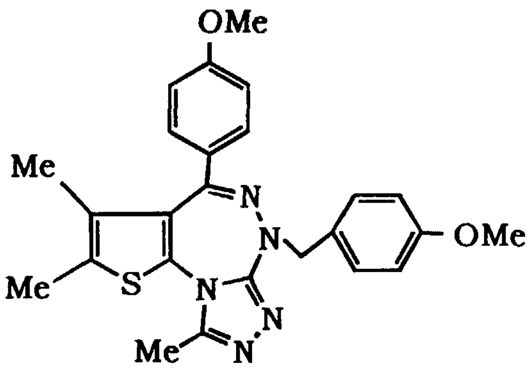 | 221-225 |

Synthetic Example 19 (Step 7')

6-(4-Chlorophenyl)-1-methyl-4-(pyridin-3-ylmethyl)-4H-2,3,4,5,10b-pentaazabenz[e]azulene

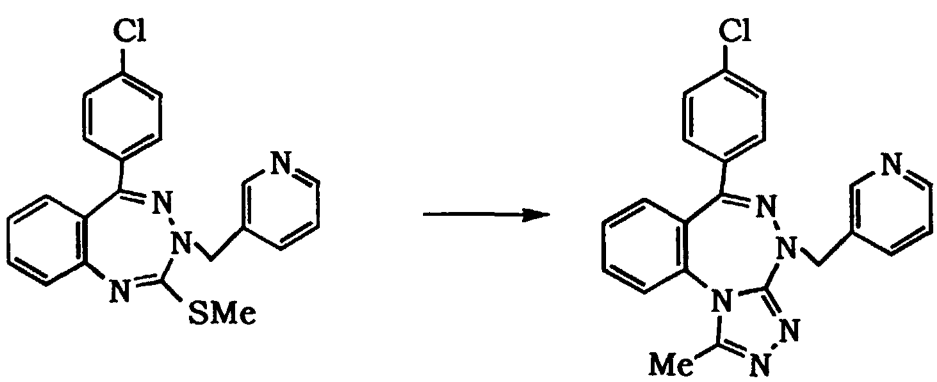

5-(4-Chlorophenyl)-2-methylthio-3-(pyridin-3-ylmethyl)-3H-benzo[e][1,2,4]triazepine (800 mg) obtained in Preparative Example 75 was dissolved in 1-butanol (4 ml), and acetylhydrazine (151 mg) and p-toluenesulfonic acid monohydrate (19.4 mg) were added. The mixture was stirred at 100°C for 3 hours in a water bath. The reaction mixture was cooled to room temperature, gradually added with diethyl ether (10 ml), and allowed to stand for one hour. The precipitated crystals were collected by filtration, and washed with diethyl ether to give 488 mg of the title compound as colorless crystals.

Melting point : 240-243°C
$^1H$ NMR (300MHz, δ ppm, $CDC1_3$)
2.62(3H,s), 4.98(1H,m), 5.11(1H,m), 7.18-7.40(8H,m), 7.64(1H,dt,J=7.8 and 1.4Hz), 7.72(1H,dt,J=7.8 and 1.9Hz), 8.51(1H,dd,J=4.8 and 1.6Hz), 8.65(1H,d,J=1.6Hz)

The title compound can be also prepared according to a method similar to that in Synthetic Example 21 (see Synthetic Example 63).

Synthetic Example 20 (Step 7')

4-(2-Chlorophenyl)-2,3,9-trimethyl-6-(pyridin-4-ylmethyl)-6H-5,6,7,8,9a-pentaazathieno[2,3-e]azulene

In the same manner as in Synthetic Example 19, the title compound was obtained from the compound of Preparative Example 83. The compound is shown in Table 29.

**Table 29**

| Synth. Ex. | Structural formula | Melting point (°C) |
|---|---|---|
| 20 | 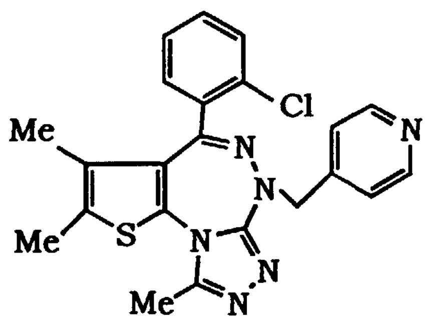 | 199-202 |

Preparative Example 89 ( Step 9)

6-(4-Chlorophenyl)-1-methyl-4H-2,3,4,5,10b-pentaazabenz[e]azulene

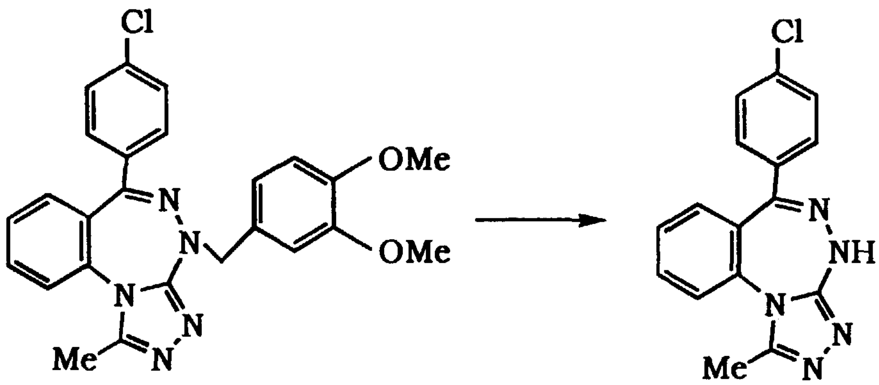

6-(4-Chlorophenyl)-4-(3,4-dimethoxybenzyl)-1-methyl-4H-2,3,4,5,10b-pentaazabenz[e]azulene (2.02 g) obtained in Synthetic Example 2 and anisole (1.4 ml) were dissolved in trifluoroacetic acid (8 ml), and concentrated sulfuric acid (1.3 ml) was added. The mixture was stirred for 10 minutes. Concentrated sulfuric acid (3.9 ml) was added, and the mixture was stirred for 45 minutes. The mixture was added with concentrated sulfuric acid (1.3 ml), and stirred for 1.5 hours. The reaction mixture was poured into ice-water (300 ml), and exacted with ethyl acetate. The organic layer was successively washed with an aqueous sodium hydrogencarbonate solution and water, dried, filtrated, and concentrated. The residue was crystallized from diethyl ether to give 748 mg of the title compound as colorless crystals.

Melting point : 231-233°C

Preparative Example 90 (Step 9)

1-Methyl-6-(4-methylphenyl)-4H-2,3,4,5,10b-pentaazabenz[e]azulene

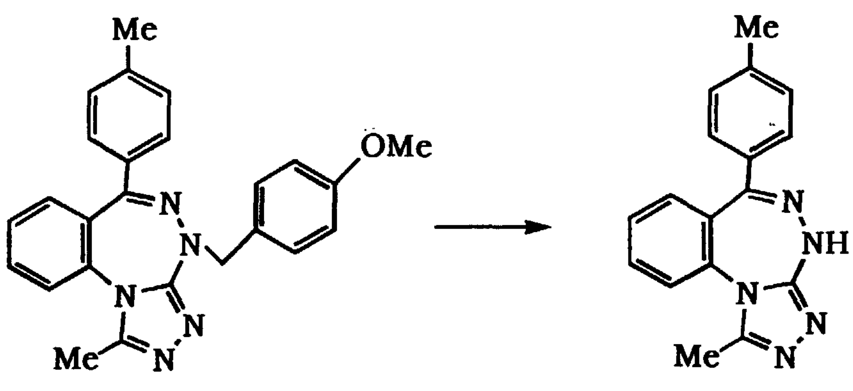

4-(4-Methoxybenzyl)-1-methyl-6-(4-methylphenyl)-4H-2,3,4,5,10b-pentaazabenz[e]azulene (800 mg) obtained in Synthetic Example 7 was dissolved in a 25% hydrobromic acid/acetic acid solution (8 ml), and anisole (0.22 ml) was added. The mixture was stirred under heating at 40°C for 3 hours. Diisopropyl ether (200 ml) was added to the reaction mixture, and the resulting crystals were collected by filtration. The crystals were dissolved in water (10 ml), and

sodium hydrogen-carbonate was added to neutralize the solution. The precipitated crystals were collected by filtration, dissolved in methylene chloride (30 ml), washed with water, and dried over anhydrous sodium sulfate. After filtration, the filtrate was concentrated, and the residue was crystallized from ethyl acetate to give 345 mg of the title compound.

Melting point : 259-261°C

Preparative Example 91 (Step 9)

7-Nitro-1-methyl-6-phenyl-4H-2,3,4,5,10b-pentaazabenz[e]azulene

Preparative Example 92 (Step 9)

8-Chloro-6-(2-chlorophenyl)-1-methyl-4H-2,3,4,5,10b-pentaazabenz[e]azulene

In the same manner as in Preparative Example 89, the compounds of Preparative Examples 91 and 92 were obtained from the compounds of Synthetic Examples 6 and 8, respectively. The compounds are shown in Table 30.

**Table 30**

| Prep. Ex. | Structural formula | Melting point (°C) |
|---|---|---|
| 91 | 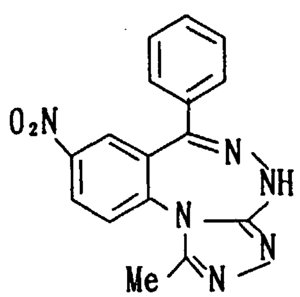 | 258-260 |
| 92 | 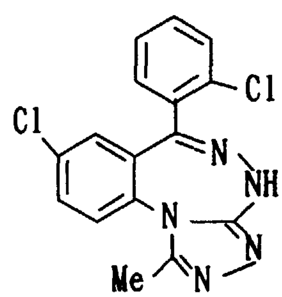 | 282-284 |

Preparative Example 93 (Step 9)

1-Methyl-6-phenyl-4H-2,3,4,5,10b-pentaazabenz[e]azulene

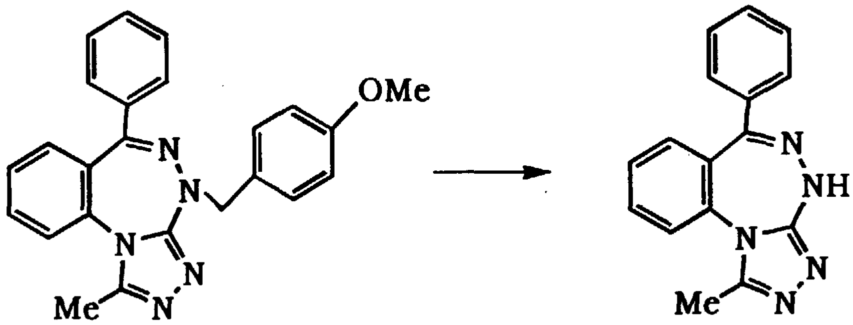

4-(4-Methoxybenzyl)-1-methyl-6-phenyl-4H-2,3,4,5,10b-pentaazabenz[e]azulene (94 mg) obtained in Synthetic Example 3 and anisole (20 μl) were dissolved in a 25% hydrobromic acid/acetic acid solution (6.9 ml), and the solution was stirred at room temperature for 32 hours. The reaction mixture was concentrated under reduced pressure, and ethyl acetate and a saturated aqueous sodium hydrogen-carbonate solution were added to the residue. The organic layer was saparated. The organic layer was washed with water, dried, filtrated, and concentrated under reduced pressure. The residue was purified by preparative thin-layer chromatography (chloroform:methanol=20:1), and crystallized from diethyl ether to give 48.5 mg of the title compound as colorless needles.

Melting point : 227-230°C

Preparative Example 94 (Step 9)

1,9-Dimethyl-6-phenyl-4H-2,3,4,5,10b-pentaazabenz[e]azulene

Preparative Example 95 (Step 9)

6-(4-Chlorophenyl)-1,9-dimethyl-4H-2,3,4,5,10b-pentaazabenz[e]azulene

Preparative Example 96 (Step 9)

6-(4-Bromophenyl)-1-methyl-4H-2,3,4,5,10b-pentaazabenz[e]azulene

Preparative Example 97 (Step 9)

6-(4-Chlorophenyl)-1-methyl-4H-2,3,4,5,10,10b-hexaazabenz[e]azulene

In the same manner as in Preparative Example 93, the compounds of Preparative Examples 94, 95, 96 and 97 were obtained from the compounds of Synthetic Examples 4, 9, 10 and 12, respectively. The compounds are shown in Table 31.

**Table 31**

| Prep. Ex. | Structural formula | $^1H$ NMR (300MHz, δppm, $CDCl_3$) | Melting point (℃) |
|---|---|---|---|
| 94 | 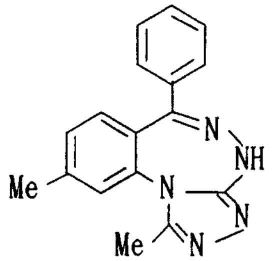 | | 248-251 |
| 95 | 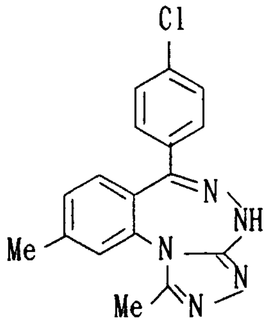 | 2.33(3H, s), 2.48(3H, s), 6.78(2H, brs), 6.97(1H, s), 7.26(1H, s), 7.39-7.53(4H, m), 8.94(1H, s) | |
| 96 | 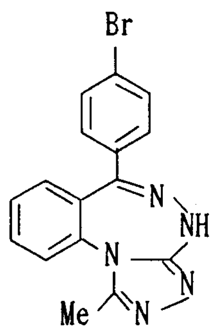 | 2.62(3H, s), 7.24-7.63(8H, m), 7.78(1H, s) | 256.5-257.5 |
| 97 | 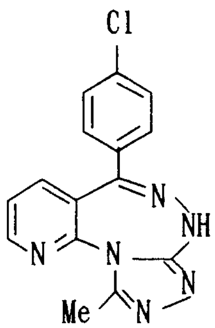 | 2.73(3H, s), 7.28-7.47(5H, m), 7.56-7.59(1H, m), 8.31(1H, s), 8.61-8.63(1H, m) | |

Preparative Example 98 (Step 9)

8-Chloro-1-methyl-6-phenyl-4H-2,3,4,5,10b-pentaazabenz[e]azulene hydrobromide

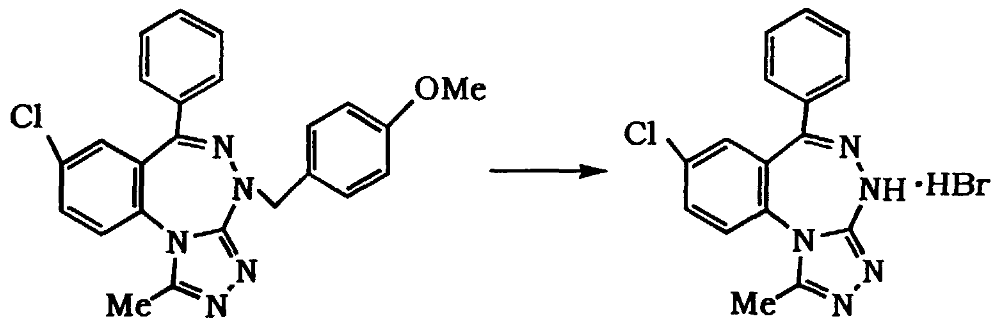

8-Chloro-4-(4-methoxybenzyl)-1-methyl-6-phenyl-4H-2,3,4,5,10b-pentaazabenz[e]azulene (105 mg) obtained in Synthetic Example 5 was reacted according to a method similar to that in Preparative Example 93 and the solvent was distilled away under reduced pressure to give crystals. The crystals were washed with diethyl ether and dried to give 108.2 mg of the title compound.

Melting point : 191-192°C (dec.)

Preparative Example 99 (Step 9)

8-Chloro-1-methyl-6-phenyl-4H-2,3,4,5,10b-pentaazabenz[e]azulene

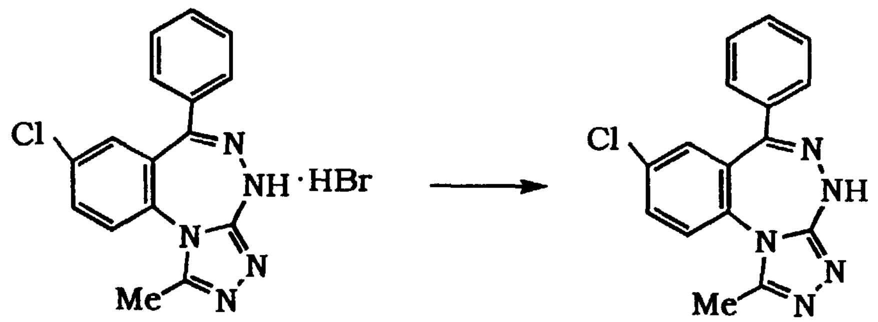

8-Chloro-1-methyl-6-phenyl-4H-2,3,4,5,10b-pentaazabenz[e]azulene hydrobromide (102 mg) obtained in Preparative Example 98 was extracted according to the method described in Preparative Example 98, and the extract was crystallized from diethyl ether to give the title compound as crystals.

Melting point : 140-142°C

Preparative Example 100 (Step 9)

4-(4-Chlorophenyl)-2,3,9-trimethyl-6H-5,6,7,8,9a-pentaazathieno[2,3-e]azulene

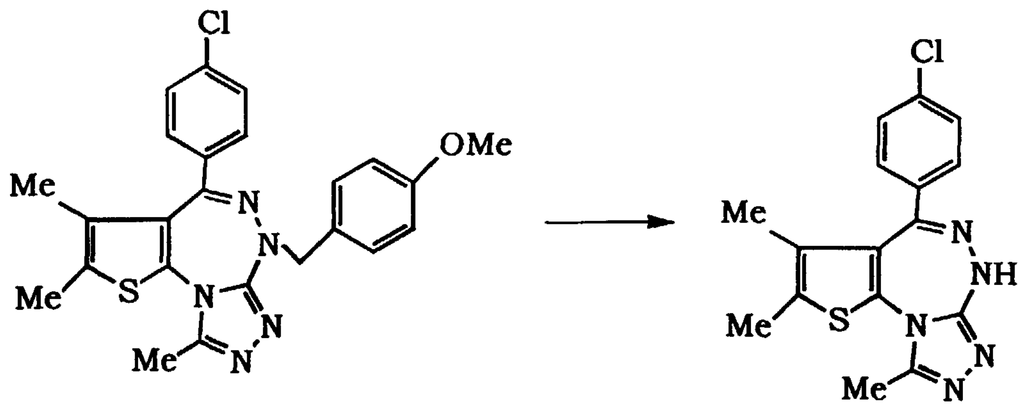

4-(4-Chlorophenyl)-6-(4-methoxybenzyl)-2,3,9-trimethyl-6H-5,6,7,8,9a-pentaazathieno[2,3-e]azulene (100 mg) obtained in Synthetic Example 13 and anisole (24 μl) were dissolved in a 25% hydrobromic acid/acetic acid solution (1 ml), and the solution was stirred at 40°C for 5 hours. The reaction mixture was cooled to room temperature, and diisopropyl ether (30 ml) was added. The precipitated oil on the reactor wall was separated from the organic solvent. The oil was dissolved in chloroform, successively washed with water, a saturated aqueous sodium hydrogencarbonate solution and water, and dried over anhydrous sodium sulfate. After filtration, the filtrate was concentrated under reduced pressure, and the rssidue was crystallized from diethyl ether to give 68 mg of the title compound as yellow crystals.

Melting point : 247°C
$^1$H NMR (300MHz, δ ppm $CDC1_3$)
1.58(3H,s), 2.36(3H,s), 2.62(3H,s), 7.35(2H,d,J=8.9Hz), 7.43(2H,d,J=8.9Hz), 7.83(1H,s)

Preparation Example 101 (Step 9)

4-(4-Chlorophenyl)-2-ethyl-9-methyl-6H-5,6,7,8,9a-pentaazathieno[2,3-e]azulene

Preparation Example 102 (Step 9)

2,9-Dimethyl-4-phenyl-6H-5,6,7,8,9a-pentaazathieno[2,3-e]azulene

Preparative Example 103 (Step 9)

2,3,9-Trimethyl-4-phenyl-6H-5,6,7,8,9a-pentaazathieno[2,3-e]azulene

Preparative Example 104 (Step 9)

2-Ethyl-9-methyl-4-phenyl-6H-5,6,7,8,9a-pentaazathieno[2,3-e]azulene

Preparative Example 105 (Step 9)

4-(4-Methoxyphenyl)-2,3,9-trimethyl-6H-5,6,7,8,9a-pentaazathieno[2,3-e]azulene

In the same manner as in Preparative Example 100, the compounds of Preparative Examples 101, 102, 103, 104 and 105 were obtained from the compounds of Synthetic Examples 14, 15, 16, 17 and 18, respectively. The compounds are shown in Tables 32 and 33.

**Table 32**

| Prep. Ex. | Structural formula | $^1$H NMR (300MHz, δppm, $CDCl_3$) | Melting point (°C) |
|---|---|---|---|
| 101 | 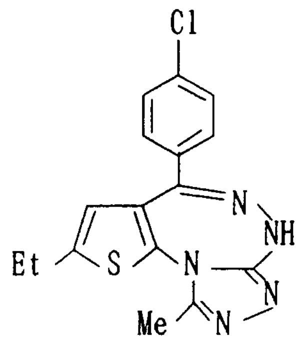 | 1.32(3H, t, J=7.5Hz), 2.62(3H, s), 2.82(2H, t, J=7.5Hz), 6.43(1H, s), 7.37(2H, d, J=8.4Hz), 7.49(2H, d, J=8.4Hz), 7.63(1H, m). | |
| 102 | 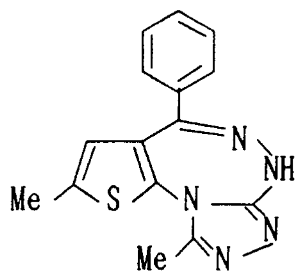 | 2.47(3H, d, J=1.5Hz), 2.61(3H, s), 6.44(1H, q, J=1.5Hz), 7.36-7.45(3H, m), 7.52-7.55(2H, m), 7.68(1H, brs). | 226-232 |
| 103 | 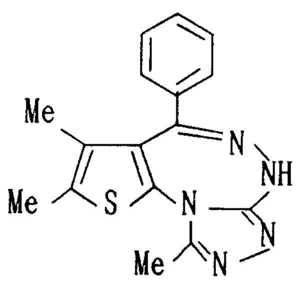 | 1.63(3H, s), 2.36(3H, s), 2.62(3H, s), 7.34-7.48(5H, m), 7.62(1H, brs). | |

**Table 33**

| Prep. Ex. | Structural formula | Melting point (°C) |
|---|---|---|
| 104 | 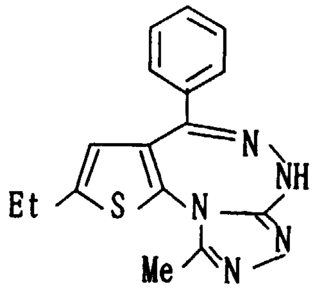 | 183-184 |
| 105 | 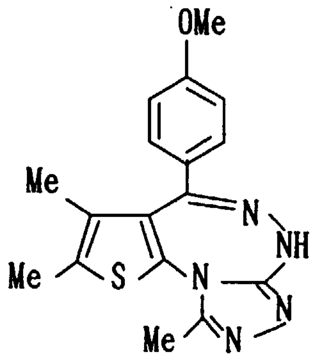 | 254-257 |

Synthetic Example 21 (Step 10-1)

6-(4-Chlorophenyl)-4-(3-cyanobenzyl)-1-methyl-4H-2,3,4,5,10b-pentaazabenz[e]azulene

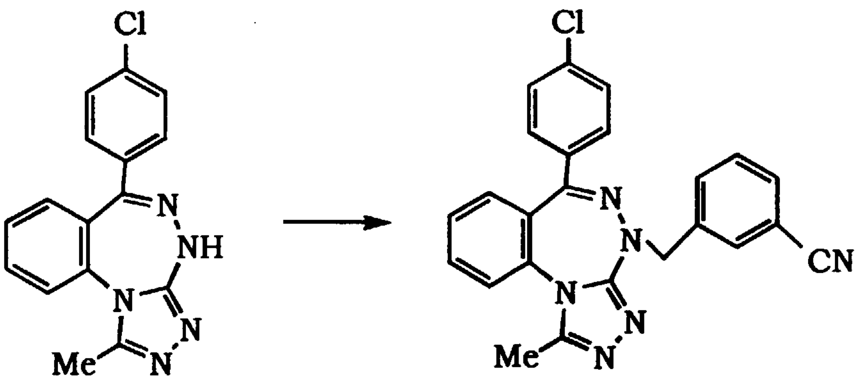

6-(4-Chlorophenyl)-1-methyl-4H-2,3,4,5,10b-pentaazabenz[e]azulene (20 mg) obtained in Preparative Example 89 was dissolved in anhydrous N,N-dimethylformamide (0.4 ml) under an argon atmosphere, and the solution was ice-cooled. Potassium hydroxide (13 mg) pulverized in a mortar was added to the solution all at once. 3-(Chloromethyl)benzonitrile (15.2 mg) was added, and the mixture was stirred under ice-cooling for 10 minutes and at room temperature for one hour. After the completion of the reaction, the reaction mixture was cooled with ice, and a 5% aqueous citric acid solution and ethyl acetate were added. The organic layer was separated, and successively washed with a saturated aqueous sodium hydrogencarbonate solution and water. The organic layer was dried, filtrated, and concentrated under reduced pressure. The resulting residue was crystallized from diethyl ether to give 8.4 mg of the title compound.

Melting point : 140-141°C
$^1$H NMR (300MHz, δ ppm, $CDC1_3$)
2.62(3H,s), 4.92-5.16(2H,m), 7.24-7.69(12H,m)

Synthetic Example 22-85 (Step 10-1)

In the same manner as in Synthetic Example 21, the following compounds of Examples 22-85 were obtained from the compound of Preparative Example 89. The compounds are shown in Tables 34-38.

Synthetic Example 22

6-(4-Chlorophenyl)-4-(2-fluorobenzyl)-1-methyl-4H-2,3,4,5,10b-pentaazabenz[e]azulene

Synthetic Example 23

6-(4-Chlorophenyl)-4-(3-fluorobenzyl)-1-methyl-4H-2,3,4,5,10b-pentaazabenz[e]azulene

Synthetic Example 24

6-(4-Chlorophenyl)-4-(4-fluorobenzyl)-1-methyl-4H-2,3,4,5,10b-pentaazabenz[e]azulene

Synthetic Example 25

6-(4-Chlorophenyl)-4-(2,4-difluorobenzyl)-1-methyl-4H-2,3,4,5,10b-pentaazabenz[e]azulene

Synthetic Example 26

6-(4-Chlorophenyl)-4-(2,5-difluorobenzyl)-1-methyl-4H-2,3,4,5,10b-pentaazabenz[e]azulene

Synthetic Example 27

6-(4-Chlorophenyl)-4-(3,5-difluorobenzyl)-1-methyl-4H-2,3,4,5,10b-pentaazabenz[e]azulene

Synthetic Example 28

6-(4-Chlorophenyl)-4-(3,4-difluorobenzyl)- 1-methyl-4H-2,3,4,5,10b-pentaazabenz[e]azulene

Synthetic Example 29

6-(4-Chlorophenyl)-1-methyl-4-(2-trifuoromethylbenzyl)-4H-2,3,4,5,10b-pentaazabenz[e]azulene

Synthetic Example 30

6-(4-Chlorophenyl)-1-methyl-4-(4-trifluomethylbenzyl)-4H-2,3,4,5,10b-pentaazabenz[e]azulene

Synthetic Example 31

6-(4-Chlorophenyl)-1-methyl-4-(3-trifluomethylbenzyl)-4H-2,3,4,5,10b-pentaazabenz[e]azulene

Synthetic Example 32

6-(4-Chlorophenyl)- 1-methyl-4-(4-trifluoromethoxybenzyl)-4H-2,3,4,5,10b-pentaazabenz[e]azulene

Synthetic Example 33

6-(4-Chlorophenyl)-1-methyl-4-(3-nitrobenzyl)-4H-2,3,4,5,10b-pentaazabenz[e]azulene

Synthetic Example 34

4-(2-Chlorobenzyl)-6-(4-chlorophenyl)-1-methyl-4H-2,3,4,5,10b-pentaazabenz[e]azulene

Synthetic Example 35

4-(3-Chlorobenzyl)-6-(4-chlorophenyl)-1-methyl-4H-2,3,4,5,10b-pentaazabenz[e]azulene

Synthetic Example 36

4-(4-Chlorobenzyl)-6-(4-chlorophenyl)-1-methyl-4H-2,3,4,5,10b-pentaazabenz[e]azulene

Synthetic Example 37

6-(4-Chlorophenyl)-4-(2-cyanobenzyl)-1-methyl-4H-2,3,4,5,10b-pentaazabenz[e]azulene

Synthetic Example 38

6-(4-Chlorophenyl)-4-(3-cyanbenzyl)-1-methyl-4H-2,3,4,5,10b-pentaazabenz[e]azulene

Synthetic Example 39

6-(4-Chlorophenyl)-4-(4-cyanobenzyl)- 1-methyl-4H-2,3,4,5,10b-pentaazabenz[e]azulene

Synthetic Example 40

6-(4-Chlorophenyl)-4-(2-methoxybenzyl)-1-methyl-4H-2,3,4,5,10b-pentaazabenz[e]azulene

Synthetic Example 41

6-(4-Chlorophenyl)-4-(3-methoxybenzyl)-1-methyl-4H-2,3,4,5,10b-pentaazabenz[e]azulene

Synthetic Example 42

6-(4-Chlorophenyl)-4-(4-methoxybenzyl)-1-methyl-4H-2,3,4,5,10b-pentaazabenz[e]azulene

Synthetic Example 43

6-(4-Chlorophenyl)-4-(2,5-dimethoxybenzyl)-1-methyl-4H-2,3,4,5,10b-pentaazabenz[e]azulene

Synthetic Example 44

6-(4-Chlorophenyl)-1-methyl-4-(3,4,5-trimethoxybenzyl)-4H-2,3,4,5,10b-pentaazabenz[e]azulene

Synthetic Example 45

4-(5-Acetyl-2-methoxybenzyl)-6-(4-chlorophenyl)-1-methyl-4H-2,3,4,5,10b-pentaazabenz[e]azulene

Synthetic Example 46

6-(4-Chlorophenyl)-1-methyl-4-(3,4-methylenedioxybenzyl)-4H-2,3,4,5,10b-pentaazabenz[e]azulene

Synthetic Example 47

4-(2-Chloro-4,5-methylendioxybenzyl)-6-(4-chlorophenyl)-1-methyl-4H-2,3,4,5,10b-pentaazabenz[e]azulene

Synthetic Example 48

6-(4-Chlorophenyl)-4-(2-methoxy-5-nitrobenzyl)-1-methyl-4H-2,3,4,5,10b-pentaazabenz[e]azulene

Synthetic Example 49

6-(4-Chlorophenyl)-4-(4-methoxy-3-nitrobenzyl)-1-methyl-4H-2,3,4,5,10b-pentaazabenz[e]azulene

Synthetic Example 50

4-(3-Chloro-4-methoxybenzyl)-6-(4-chlorophenyl)-1-methyl-4H-2,3,4,5,10b-pentaazabenz[e]azulene

Synthetic Example 51

6-(4-Chlorophenyl)-4-(3,5-dichloro-4-methoxybenzyl)-1-methyl-4H-2,3,4,5,10b-pentaazabenz[e]azulene

Synthetic Example 52

6-(4-Chlorophenyl)-1-methyl-4-(2-methylbenzyl)-4H-2,3,4,5,10b-pentaazabenz[e]azulene

Synthetic Example 53

6-(4-Chlorophenyl)-1-methyl-4-(3-methylbenzyl)-4H-2,3,4,5,10b-pentaazabenz[e]azulene

Synthetic Example 54

6-(4-Chlorophenyl)-1-methyl-4-(4-methylbenzyl)-4H-2,3,4,5,10b-pentaazabenz[e]azulene

Synthetic Example 55

4-(4-tert-Butylbenzyl)-6-(4-chlorophenyl)- 1-methyl-4H-2,3,4,5,10b-pentaazabenz[e]azulene

Synthetic Example 56

6-(4-Chlorophenyl)-1-methyl-4-(naphthalen-1-ylmethyl)-4H-2,3,4,5,10b-pentaazabenz[e]azulene

Synthetic Example 57

6-(4-Chlorophenyl)-1-methyl-4-(naphthalen-2-ylmethyl)-4H-2,3,4,5,10b-pentaazabenz[e]azulene

Synthetic Example 58

4-(4-Benzyloxybenzyl)-6-(4-chlorophenyl)-1-methyl-4H-2,3,4,5,10b-pentaazabenz[e]azulene

Synthetic Example 59

4-Benzyl-6-(4-chlorophenyl)-1-methyl-4H-2,3,4,5,10b-pentaazabenz[e]azulene

Synthetic Example 60

6-(4-Chlorophenyl)-1-methyl-4-(4-phenylbenzyl)-4H-2,3,4,5,10b-pentaazabenz[e]azulene

Synthetic Example 61

4-(4-Chlorophenoxymethyl)-6-(4-chlorophenyl)- 1-methyl-4H-2,3,4,5,10b-pentaazabenz[e]azulene

Synthetic Example 62

6-(4-Chlorophenyl)-1-methy-4-(pyridin-2-ylmethyl)-4H-2,3,4,5,10b-pentaazabenz[e]azulene

Synthetic Example 63

6-(4-Chlorophenyl)- 1-methyl-4-(pyridin-3-ylmethyl)-4H-2,3,4,5,10b-pentaazabenz[e]azulene

Synthetic Example 64

6-(4-Chlorophenyl)-4-[2-(indol-3-yl)ethyl]-1-methyl-4H-2,3,4,5,10b-pentaazabenz[e]azulene

Synthetic Example 65

6-(4-Chlorophenyl)-4-(2-methyl-1,3-thiazol-4-ylmethyl)-1-methyl-4H-2,3,4,5,10b-pentaazabenz[e]azulene

Synthetic Example 66

6-(4-Chlorophenyl)-4-(5-chlorothiophen-2-ylmethyl)-1-methyl-4H-2,3,4,5,10b-pentaazabenz[e]azulene

Synthetic Example 67

6-(4-Chlorophenyl)-1-methyl-4-(3,5-dimethylisoxazol-4-ylmethyl)-4H-2,3,4,5,10b-pentaazabenz[e]azulene

Synthetic Example 68

6-(4-Chlorophenyl)-1-methyl-4-phenethyl-4H-2,3,4,5,10b-pentaazabenz[e]azulene

Synthetic Example 69

6-(4-Chlorophenyl)-1-methyl-4-(3-phenylpropyl)-4H-2,3,4,5,10b-pentaazabenz[e]azulene

Synthetic Example 70

6-(4-Chlorophenyl)-4-(3,3-diphenylpropyl)-1-methyl-4H-2,3,4,5,10b-pentaazabenz[e]azulene

Synthetic Example 71

6-(4-Chlorophenyl)-4-cyclopropylmethyl-1-methyl-4H-2,3,4,5,10b-pentaazabenz[e]azulene

Synthetic Example 72

6-(4-Chlorophenyl)-4-cyclohexylmethyl-1-methyl-4H-2,3,4,5,10b-pentaazabenz[e]azulene

Synthetic Example 73

6-(4-Chlorophenyl)-4-(2-cyclohexylethyl)-1-methyl-4H-2,3,4,5,10b-pentaazabenz[e]azulene

Synthetic Example 74

6-(4-Chlorophenyl)-1-methyl-4-(3-phenyl-2-propenyl)-4H-2,3,4,5,10b-pentaazabenz[e]azulene

Synthetic Example 75

4-Allyl-6-(4-chlorophenyl)-1-methyl-4H-2,3,4,5,10b-pentaazabenz[e]azulene

Synthetic Example 76

6-(4-Chlorophenyl)-1-methyl-4-(2-methyl-2-propenyl)-4H-2,3,4,5,10b-pentaazabenz[e]azulene

Synthetic Example 77

6-(4-Chlorophenyl)-4-(2-chloro-2-propenyl)-1-methyl-4H-2,3,4,5,10b-pentaazabenz[e]azulene

Synthetic Example 78

4-(2-Bromo-2-propenyl)-6-(4-chlorophenyl)-1-methyl-4H-2,3,4,5,10b-pentaazabenz[e]azulene

Synthetic Example 79

6-(4-Chlorophenyl)-4-(2,3-dichloro-2-propenyl)-1-methyl-4H-2,3,4,5,10b-pentaazabenz[e]azulene

Synthetic Example 80

6-(4-Chlorophenyl)-4-(3,4-dibenzyloxybenzyl)-1-methyl-4H-2,3,4,5,10b-pentaazabenz[e]azulene

Synthetic Example 81

4-Benzyloxymethyl-6-(4-chlorophenyl)-1-methyl-4H-2,3,4,5,10b-pentaazabenz[e]azulene,

Synthetic Example 82

6-(4-Chlorophenyl)-1-methyl-4-(3-phenoxypropyl)-4H-2,3,4,5,10b-pentaazabenz[e]azulene

Synthetic Example 83

6-(4-Chlorophenyl)-4-(3,3-dichloro-2-propenyl)-1-methyl-4H-2,3,4,5,10b-pentaazabenz[e]azulene

Synthetic Example 84

6-(4-Chlorophenyl)-4-(4-methoxy-3-methylbenzyl)-1-methyl-4H-2,3,4,5,10b-pentaazabenz[e]azulene

Synthetic Example 85

6-(4-Chlorophenyl)-4-(3,4-dichlorobenzyl)-1-methyl-4H-2,3,4,5,10b-pentaazabenz[e]azulene

**Table 34**

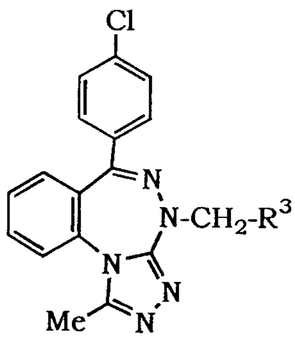

| Syn.Ex. | $R^3$ | Melting point (℃) | Syn.Ex. | $R^3$ | Melting point (℃) |
|---|---|---|---|---|---|
| 22 | 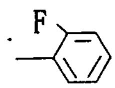 | 228-229 | 30 | 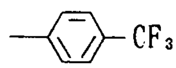 | 205-209 |
| 23 | 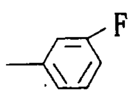 | 193-196 | 31 | 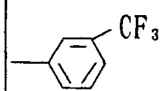 | 147-148 |
| 24 | 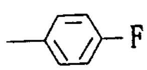 | 256-257 | 32 | 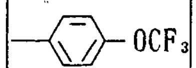 | 122 |
| 25 | 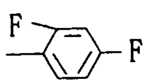 | 209-210 | 33 | 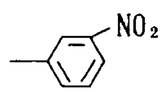 | 172-175 |
| 26 | 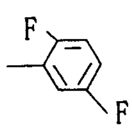 | 205 | 34 | 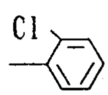 | 234-235 |
| 27 | 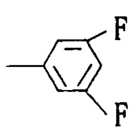 | 206-211 | 35 | 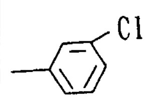 | 194-195 |
| 28 | 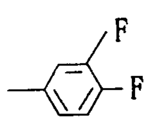 | 195-196 | 36 | 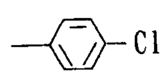 | 252-253 |
| 29 | 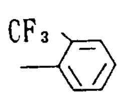 | 170-171 | 37 | 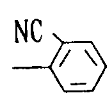 | 223-224 |

**Table 35**

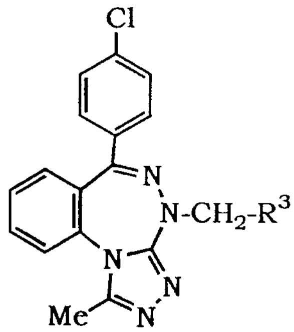

| Syn.Ex. | $R^3$ | Melting point (℃) | Syn.Ex. | $R^3$ | Melting point (℃) |
|---|---|---|---|---|---|
| 38 | 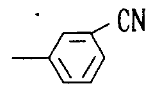 | 140-141 | 46 | 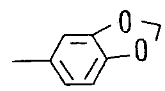 | 206-208 |
| 39 | 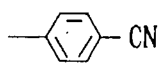 | 287-289 (decomposition) | 47 | 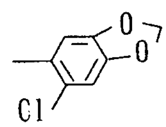 | 273-274 |
| 40 | 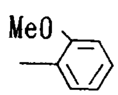 | 85-89 | 48 | 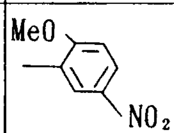 | 246 |
| 41 | 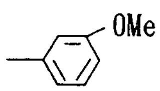 | 202-204 | 49 | 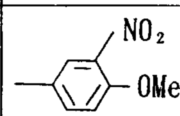 | 218-221 (decomposition) |
| 42 | 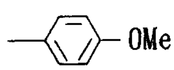 | 191-193 | 50 | 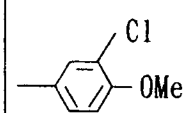 | 191-193 |
| 43 | 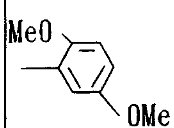 | 163-165 | 51 | 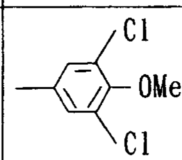 | 189 |
| 44 | 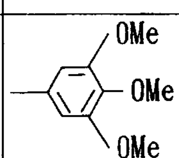 | 182-183 | 52 | 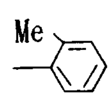 | 248-249 |
| 45 | 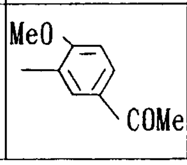 | 144-148 | 53 | 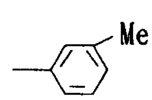 | 207-208 |

**Table 36**

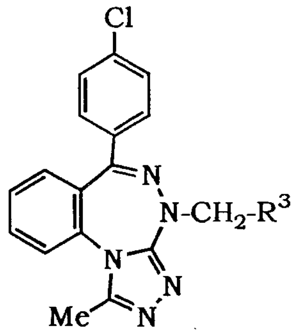

| Syn.Ex. | $R^3$ | Melting point (℃) | Syn.Ex. | $R^3$ | Melting point (℃) |
|---|---|---|---|---|---|
| 54 | 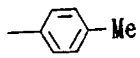 | 194-197 | 62 | 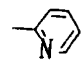 | 187-188 |
| 55 | 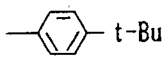 | 154-156 | 63 | 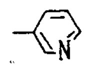 | 233-239 |
| 56 | 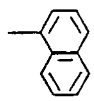 | 129-134 | 64 | 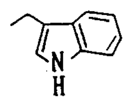 | 198-202 |
| 57 | 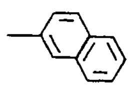 | 220-223 | 65 | 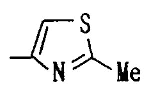 | 223-226 |
| 58 | 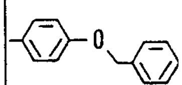 | 175-176 | 66 | 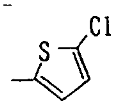 | 203-205 |
| 59 | 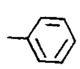 | 235-238 | 67 | 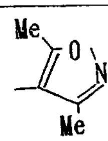 | 115-118 |
| 60 | 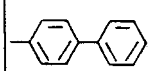 | 171 | 68 | 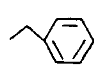 | 202 |
| 61 | 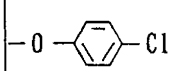 | 180-181 | 69 | 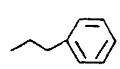 | 156-157 |

**Table 37**

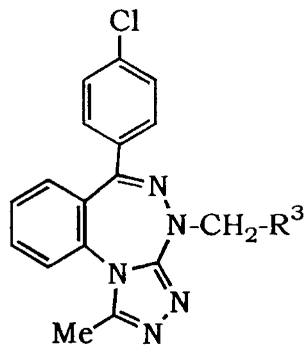

| Syn.Ex. | $R^3$ | Melting point (℃) | Syn.Ex. | $R^3$ | Melting point (℃) |
|---|---|---|---|---|---|
| 70 | 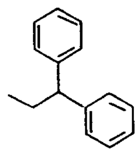 | 202-203 | 75 | 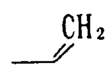 | 170-171 |
| 71 | 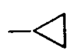 | 177-178 | 76 | 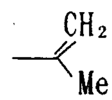 | 153-154 |
| 72 | 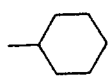 | 179-181 | 77 | 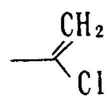 | 162-164 |
| 73 | 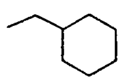 | 249-251 | 78 | 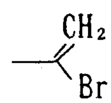 | 180-181 |
| 74 | 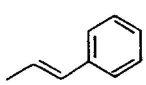 | 204-205 | 79 | 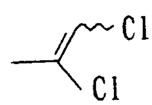 | 168-170 (decomposition) |

**Table 38**

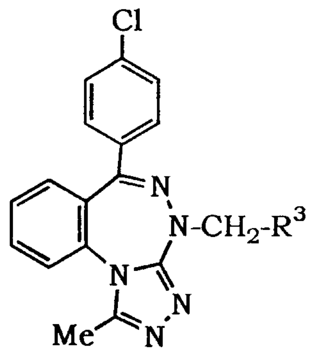

| Syn.Ex. | $R^3$ | $^1$H NMR (300MHz, δppm, $CDCl_3$) | Melting point (°C) |
|---|---|---|---|
| 80 | 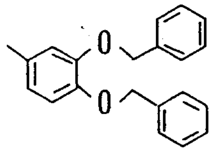 | 2.60(3H, s), 4.81-4.85(1H, m), 5.02-5.05(1H, m), 5.06(2H, s), 5.13(2H, s), 6.72-7.00(3H, m), 7.17-7.44(17H, m), 7.60-7.66(1H, m). | |
| 81 | 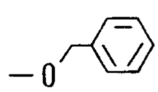 | 2.60(3H, s), 4.65(2H, s), 5.38(1H, m), 5,49(1H, m), 7.18-7.41(10H, m), 7.52(2H, d, J=8.6Hz), 7.63(1H, dt, J=7.6 and 1.4Hz). | |
| 82 | 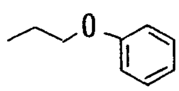 | 2.28(2H, m), 2.59(3H, s), 4.04(4H, m), 6.81-6.93(3H, m), 7.19-7.64(10H, m). | 138-142 |
| 83 | 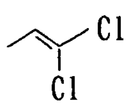 | 2.60(3H, s), 4.54(2H, d, J=6.6Hz), 6.27(1H, t, J=6.6Hz), 7.25-7.41(5H, m), 7.47-7.52(2H, m), 7.60-7.67(1H, m). | |
| 84 | 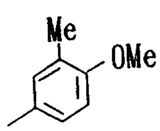 | | 187-188 |
| 85 | 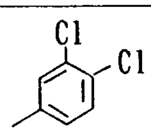 | | 181-183 |

Synthetic Example 86 (Step 10-1)

6-(4-Chlorophenyl)-1-methyl-4-(pyridin-4-ylmethyl)-4H-2,3,4,5,10b-pentaazabenz[e]azulene

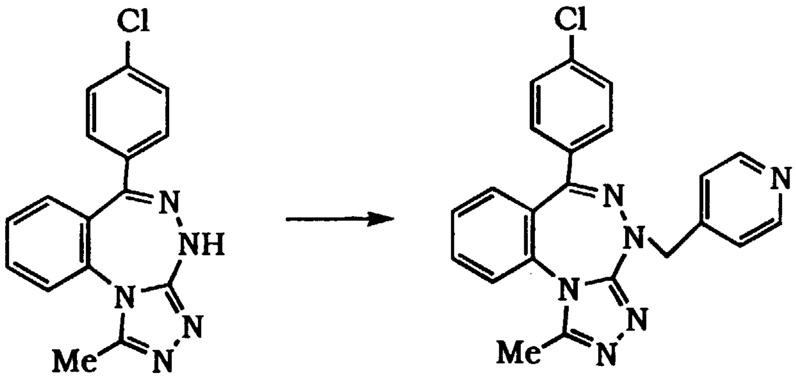

6-(4-Chlorophenyl)-1-methyl-4H-2,3,4,5,10b-pentaazabenz[e]azulene (783 mg) obtained in Preparative Example 89 was dissolved in anhydrous N,N-dimethylformamide (8 ml) under an argon gas atmosphere and cooled in an ice-bath. Sodium hydride (60% in oil, 120 mg) was added thereto and the mixture was stirred under ice-cooling for 5 minutes and at room temperature for 10 minutes. The reaction mixture was cooled in an ice-bath again and 4-picolyl chloride hydrochloride (415 mg) was added in a solid state to the reaction mixture. The mixture was stirred under ice-cooling for 25 minutes. After the completion of the reaction, ice-water (50 ml) and ethyl acetate (40 ml) were added and the organic layer was separated. The organic layer was washed with water three times, dried over anhydrous sodium sulfate and filtered. The filtrate was concentrated under reduced pressure and the obtained oil was crystallized from diethyl ether to give 760 mg of the title compound.

Melting point : 223-225°C
$^1$H NMR (300MHz, $\delta$ ppm, $CDCl_3$)
2.63(3H,s), 4.98-5.14(2H,m), 7.25-7.70(10H,m), 8.54(2H,dd,J=4.5 and 3.0Hz)

Synthetic Examples 87 to 108 (Step 10-1)

In the same manner as in Synthetic Example 86, the compounds of Synthetic Examples 87-91 and 107 were obtained from the compound of Preparative Example 89, the compounds of Synthetic Examples 92 and 93 were obtained from the compound of Preparative Example 99, the compounds of Synthetic Examples 94 and 95 were obtained from the compound of Preparative Example 93, the compounds of Synthetic Examples 96, 97, 98, 99 and 100 were obtained from the compound of Preparative Example 94, the compounds of Synthetic Examples 101 and 102 were obtained from the compound of Preparative Example 91, the compounds of Synthetic Examples 103 and 104 were obtained from the compound of Preparative Example 90, the compounds of Synthetic Examples 105 and 106 were obtained from the compound of Preparative Example 92, and the compound of Synthetic Example 108 was obtained from the compound of Preparative Example 95. These compounds are shown in Tables 39 to 43.

Synthetic Example 87

6-(4-Chlorophenyl)-1-methyl-4-(4-methylsulfonylbenzyl)-4H-2,3,4,5,10b-pentaazabenz[e]azulene

Synthetic Example 88

6-(4-Chlorophenyl)-1-methyl-4-(4-nitrobenzyl)-4H-2,3,4,5,10b-pentaazabenz[e]azulene

Synthetic Example 89

6-(4-Chlorophenyl)-4-(2,6-dichloropyridin-4-ylmethyl)-1-methyl-4H-2,3,4,5,10b-pentaazabenz[e]azulene

Synthetic Example 90

6-(4-Chlorophenyl)-4-(2,2,2-trifluoroethyl)-1-methyl-4H-2,3,4,5,10b-pentaazabenz[e]azulene

Synthetic Example 91

6-(4-Chlorophenyl)-4-(3,5-dinitrobenzyl)-1-methyl-4H-2,3,4,5,10b-pentaazabenz[e]azulene

Synthetic Example 92

8-Chloro-1-methyl-6-phenyl-4-(pyridin-4-ylmethyl)-4H-2,3,4,5,10b-pentaazabenz[e]azulene

Synthetic Example 93

8-Chloro-1-methyl-6-phenyl-4-(pyridin-3-ylmethyl)-4H-2,3,4,5,10b-pentaazabenz[e]azulene

Synthetic Example 94

1-Methyl-6-phenyl-4-(pyridin-3-ylmethyl)-4H-2,3,4,5,10b-pentaazabenz[e]azulene

Synthetic Example 95

1-Methyl-6-phenyl-4-(pyridin-4-ylmethyl)-4H-2,3,4,5,10b-pentaazabenz[e]azulene

Synthetic Example 96

1,9-Dimethyl-6-phenyl-4-(pyridin-3-ylmethyl)-4H-2,3,4,5,10b-pentaazabenz[e]azulene

Synthetic Example 97

1,9-Dimethyl-6-phenyl-4-(pyridin-4-ylmethyl)-4H-2,3,4,5,10b-pentaazabenz[e]azulene

Synthetic Example 98

4-(3-Cyanobenzyl)-1,9-dimethyl-6-phenyl-4H-2,3,4,5,10b-pentaazabenz[e]azulene

Synthetic Example 99

4-(4-Cyanbenzyl)-1,9-dimethyl-6-phenyl-4H-2,3,4,5,10b-pentaazabenz[e]azulene

Synthetic Example 100

4-(3,4-Dichlorobenzyl)-1,9-dimethyl-6-phenyl-4H-2,3,4,5,10b-pentaazabenz[e]azulene

Synthetic Example 101

1-Methyl-8-nitro-6-phenyl-4-(pyridin-3-ylmethyl)-4H-2,3,4,5,10b-pentaazabenz[e]azulene

Synthetic Example 102

1-Methyl-8-nitro-6-phenyl-4-(pyridin-4-ymethyl)-4H-2,3,4,5,10b-pentaazabenz[e]azulene

Synthetic Example 103

1-Methyl-6-(4-methylphenyl)-4-(pyridin-4-ylmethyl)-4H-2,3,4,5,10b-pentaazabenz[e]azulene

Synthetic Example 104

4-(3-Cyanobenzyl)-1-methyl-6-(4-methylphenyl)-4H-2,3,4,5,10b-pentaazabenz[e]azulene

Synthetic Example 105

8-Chloro-6-(2-chlorophenyl)-1-methyl-4-(pyridin-3-ylmethyl)-4H-2,3,4,5,10b-pentaazabenz[e]azulene

Synthetic Example 106

8-Chloro-6-(2-chlorophenyl)-1-methyl-4-(pyridin-4-ylmethyl)-4H-2,3,4,5,10b-pentaazabenz[e]azulene

Synthetic Example 107

Methyl 4-[6-(4-chlorophenyl)-1-methyl-4H-2,3,4,5,10b-pentaazabenz[e]azulen-4-ylmethyl]benzoate

Synthetic Example 108

6-(4-Chlorophenyl)-4-(4-cyanbenzyl)-1,9-dimethyl-4H-2,3,4,5,10b-pentaazabenz[e]azulene

## Table 39

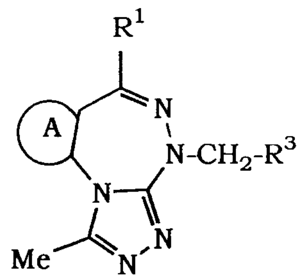

| Synth. Ex. | A | $R^1$ | $R^3$ | $^1$H NMR (300MHz, δppm, $CDCl_3$) | Melting point (℃) |
|---|---|---|---|---|---|
| 87 | 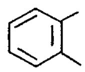 | 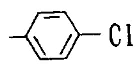 | 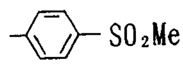 | 2.62(3H, s), 3.03(3H, s), 5.03(1H, m), 5.19(1H, m), 7.23-7.43(7H, m), 7.56-7.69(3H, m), 7.87 (2H, d, J=8.4Hz). | |
| 88 | 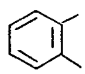 | 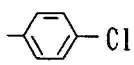 | 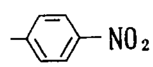 | 2.62(3H, s), 5.03(1H, m), 5.11(1H, m), 7.24-7.43(7H, m), 7.54 (2H, d, J=8.8Hz), 7.68(1H, m), 8.17 (2H, d, J=8.8Hz). | |
| 89 | 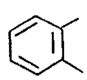 | 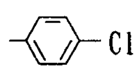 | 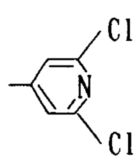 | 2.63(3H, s), 4.92(1H, m), 5.09(1H, m), 7.26-7.47(9H, m), 7.70 (1H, dt, J=7.8 and 1.6Hz). | |
| 90 | 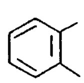 | 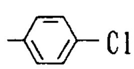 | 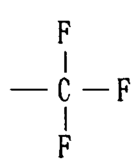 | | 177-180 (decom-position) |

**Table 40**

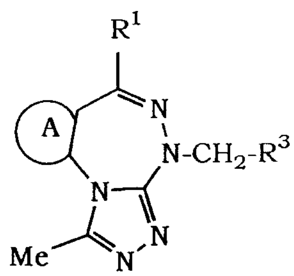

| Synth. Ex. | A | $R^1$ | $R^3$ | $^1H$ NMR (300MHz, δppm, $CDCl_3$) | Melting point (°C) |
|---|---|---|---|---|---|
| 91 | 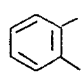 | 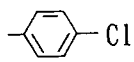 | 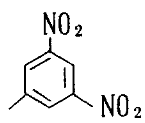 | 2.63(3H, s), 5.15(1H, m), 5.28(1H, m), 7.20-7.47(6H, m), 7.68(1H, m), 8.63 (1H, d, J=2.2Hz), 8.95 (1H, t, J=2.2Hz). | Amorphous |
| 92 | 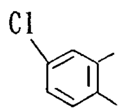 | 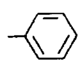 | 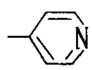 | 2.60(3H, s), 4.98(1H, m), 5.12(1H, m), 7.26-7.47(9H, m), 7.62 (1H, dd, J=8.8 and 2.4Hz), 8.55(1H, m), 8.56(1H, m). | Amorphous |
| 93 | 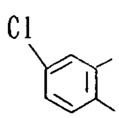 | 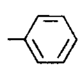 | 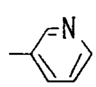 | 2.60(3H, s), 4.98(1H, m), 5.11(1H, m), 7.21 (1H, d, J=2.4Hz), 7.25-7.31(2H, m), 7.35-7.38(3H, m), 7.42-7.48(1H, m), 7.59 (1H, dd, J=8.7 and 2.4Hz), 7.74 (1H, dt, J=8.0 and 1.8Hz), 8.53 (1H, dd, J=4.8 and 1.6Hz), 8.67 (1H, d, J=1.8Hz). | Amorphous |

**Table 41**

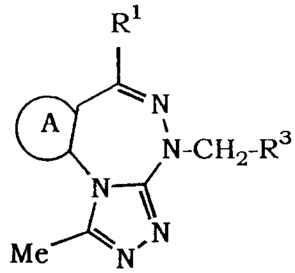

| Synth. Ex. | A | $R^1$ | $R^3$ | Melting point (°C) |
|---|---|---|---|---|
| 94 | 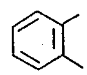 | 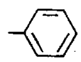 | 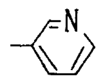 | 179-181 |
| 95 | 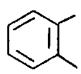 | 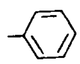 | 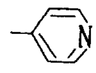 | 193-194 |
| 96 | 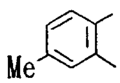 | 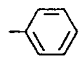 | 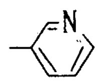 | 196-197 |
| 97 | 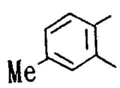 | 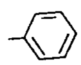 | 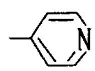 | 240-241 |
| 98 | 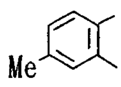 | 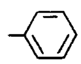 | 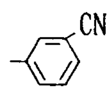 | 165-166 |
| 99 | 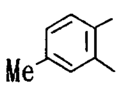 | 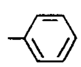 | 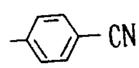 | 214-216 |
| 100 | 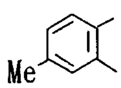 | 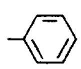 | 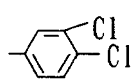 | 158-159 |
| 101 | 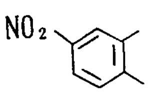 | 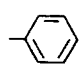 | 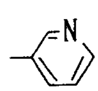 | 212-213 |
| 102 | 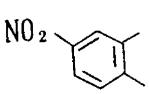 | 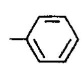 | 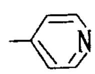 | 202-203 |

**Table 42**

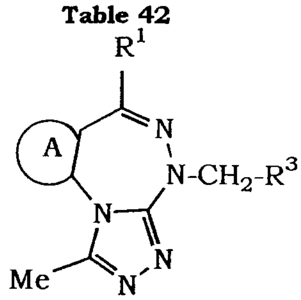

| Synth. Ex. | A | $R^1$ | $R^3$ | $^1$H NMR (300MHz, δppm, $CDCl_3$) | Melting point (℃) |
|---|---|---|---|---|---|
| 103 | 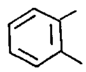 | 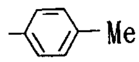 | 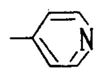 | | 205-207 |
| 104 | 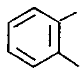 | 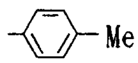 | 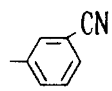 | 2.36(3H, s)<br>2.62(3H, s),<br>5.07(2H, m),<br>7.16<br>(2H, d, J=9Hz),<br>7.25-7.68<br>(10H, m). | Amorphous |
| 105 | 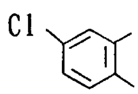 | 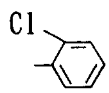 | 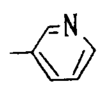 | | 162-165 |
| 106 | 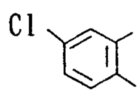 | 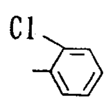 | 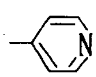 | 2.61(3H, s),<br>4.98(1H, m),<br>5.15(1H, m),<br>7.27-7.46(9H, m),<br>7.63<br>(1H, dd, J=8.9 and 4.0Hz),<br>8.55<br>(2H, dd, J=4.5 and 2.0Hz). | Amorphous |
| 107 | 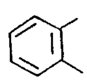 | 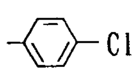 | 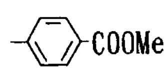 | | 137-139 |

**Table 43**

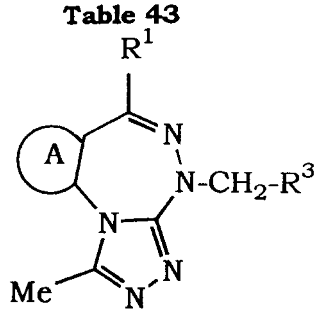

| Synth. Ex. | A | $R^1$ | $R^3$ | $^1$H NMR (300MHz, δppm, $CDCl_3$) | Melting point (°C) |
|---|---|---|---|---|---|
| 108 | 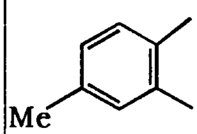 | 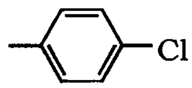 | 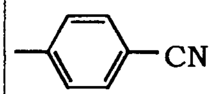 | 2.51(3H, s), 2.61(3H, s), 4.99(1H, m), 5.15(1H, m), 7.10-7.29(7H, m), 7.46-7.60(4H, m) | 206.5-208.0 |

Synthetic Example 109 (Step 10-1)

4-(4-Bromobenzyl)-6-(4-bromophenyl)-1-methyl-4H-2,3,4,5,10b-pentaaabenz[e]azulene

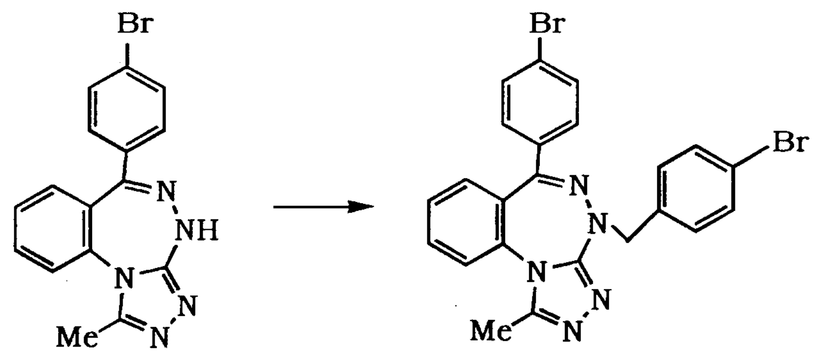

6-(4-Bromophenyl)-1-methyl-4H-2,3,4,5,10b-pentaazabenzo[e]azulene (2.13 g) obtained in Preparative Example 96 was dissolved in anhydrous N,N-dimethyl-formamide (37 ml) under an argon gas atmosphere and ice-cooled. Sodium hydride (60% in oil, 258 mg) was added thereto and the mixture was stirred under ice-cooling for 5 minutes and at room temperature for 30 minutes. The reaction mixture was again ice-cooled and 4-bromobenzyl bromide (1.57 g) was added in solid to the reaction mixture. The mixture was stirred under ice-cooling for 15 minutes. After the completion of the reaction, ice water and chloroform were added and the organic layer was separated. The organic layer was washed four times with water, dried over anhydrous sodium sulfate and filtered. The filtrate was concentrated and the resulting crystals (2.96 g) were recrystallized from a mixed solvent of chloroform : methanol (10:1) to give 2.62 g of the title compound as colorless crystals.

Melting point : 248.5-249.5°C

$^1H$ NMR (300MHz, δ ppm, $CDC1_3$)
2.60(3H,s), 4.89(1H,m), 5.05(1H,m), 7.20-7.48(11H,m), 7.61-7.66(1H,m)

Synthetic Example 111 (Step 10-1)

4-(4-Cyanobenzyl)-1-methyl-6-phenyl-4H-2,3,4,5,10b-pentaazabenz[e]azulene

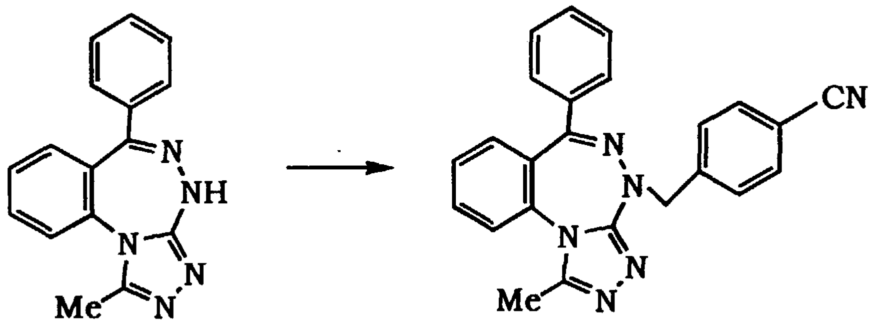

1-Methyl-6-phenyl-4H-2,3,4,5,10b-pentaazabenz[e]azulene (200 mg) obtained in Preparative Example 93 was dissolved in anhydrous N,N-dimethylformamide (3.4 ml) under an argon gas atmosphere and ice-cooled. Sodium hydride (60% in oil, 47 mg) was added thereto and the mixture was stirred under ice-cooling for 5 minutes and at room temperature for 1 hour. The reaction mixture was again ice-cooled and α-bromo-p-tolunitrile (228 mg) was added in solid to the reaction mixture. The mixture was stirred at room temperature for 2 hours. After the completion of the reaction, ethyl acetate and ice water were added and the organic layer was separated. The organic layer was washed three times with water, dried over anhydrous sodium sulfate and filtered. The filtrate was concentrated under reduced pressure and the resulting residue was subjected to silica gel column chromatography (chloroform:methanol=97:3). The pure fractions were collected and concentrated. The obtained residue was crystallized from a mixed solvent of chloroform : diethyl ether to give 145 mg of the title compound as colorless crystals.

Melting point : 215-216.5°C
$^1H$ NMR (300MHz, δ ppm, $CDC1_3$)
2.62(3H,s), 5.02(1H,m), 5.17(1H,m), 7.26-7.68(13H,m)

Synthetic Example 112 (Step 10-24)

4-[6-(4-Chlorophenyl)-1-methyl-4H-2,3,4,5,10b-pentaazabenz[e]azulen-4-ylmethyl]benzoic acid

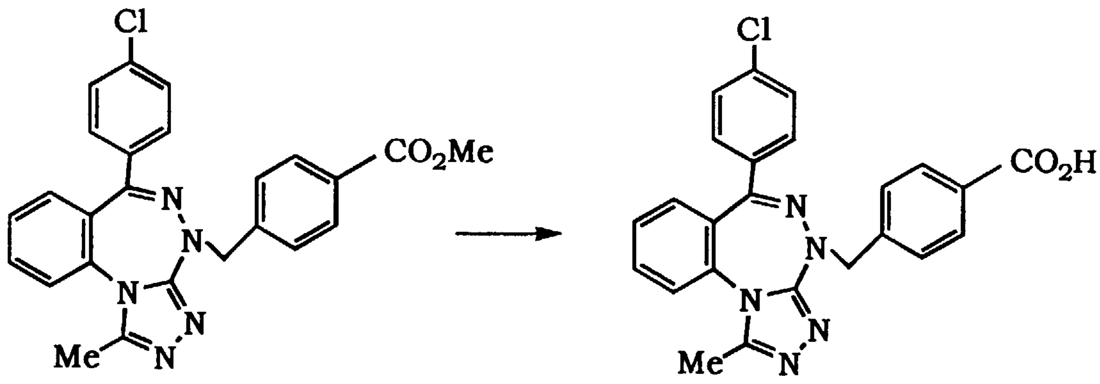

To a solution (1 ml) of methyl 4-[6-(4-chlorophenyl)-1-methyl-4H-2,3,4,5,10b-pentaazabenz[e]azulen-4-ylmethyl]benzoate (171 mg) obtained in Synthetic Example 107 in ethanol was added a 1N aqueous sodium hydroxide solution (0.37 ml) and the mixture was stirred at room temperature for 5 hours and at 45°C for 4 hours. After the completion of the reaction, the solvent was evaporated under reduced pressure and ethyl acetate and water were added to the residue. The aqueous layer was separated and adjusted to pH 3 with 1N hydrochloric acid under ice-cooling. The resulting residue was extracted with ethyl acetate, and the organic layer was washed three times with water, dried over anhydrous sodium sulfate and concentrated under reduced pressure. The resulting residue was crystallized from a mixture of chloroform : ethyl acetate to give 105.6 mg of the title compound as colorless crystals.

Melting point : 190-193°C

Synthetic Example 113 (Step 10-1)

4-(4-Chlorophenyl)-2,3,9-trimethyl-6-(pyridin-3-ylmethyl)-6H-5,6,7,8,9a-pentaazathieno[2,3-e]azulene

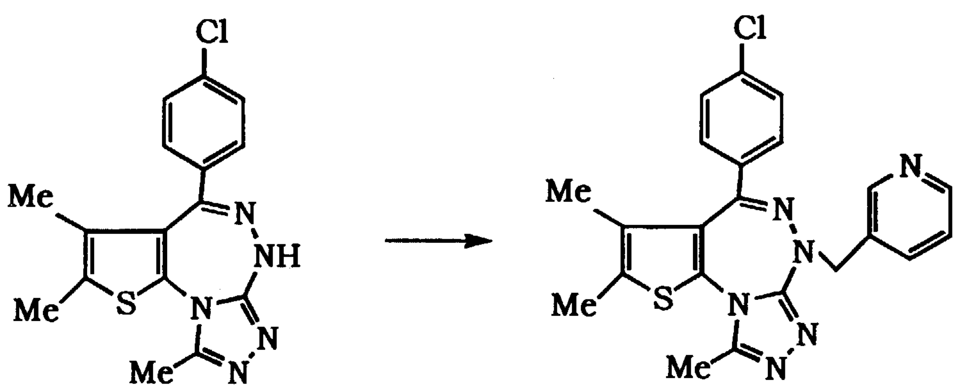

4-(4-Chlorophenyl)-2,3,9-trimethyl-6H-5,6,7,8,9a-pentaazathieno[2,3-e]azulene (1 g) obtained in Preparative Example 100 was dissolved in anhydrous N,N-dimethylformamide (10 ml) under an argon gas atmosphere and cooled

in an ice-bath. Sodium hydride (60% in oil, 244 mg) was added thereto and the mixture was stirred under ice-cooling for 5 minutes and at room temperature for 30 minutes. The reaction mixture was cooled in an ice-bath again and 3-picolyl chloride hydrochloride (525 mg) was added to the reaction mixture. The mixture was stirred under ice-cooling for 4 hours. After completion of the reaction, a 1% aqueous solution of citric acid (50 ml) and ethyl acetate (50 ml) were added and the organic layer was separated. The organic layer was washed with water, dried over anhydrous sodium sulfate and filtered. The filtrate was concentrated under reduced pressure and the obtained residue was subjected to silica gel column chromatography. The solid obtained by concentration of the fractions eluted with ethyl acetate : methanol (95:5) was crystallized from ethyl acetate : diethyl ether (1:3) to give 936 mg of the title compound as colorless crystals.

Melting point : 195-196°C
$^1$H NMR (300MHz, $\delta$ ppm, $CDC1_3$)
1.53(3H,s), 2.37(3H,s), 2.62(3H,s), 5.03(2H,s), 7.23-7.32(5H,m), 7.77(1H,m), 8.54(1H,dd,J=5.0 and 1.7Hz), 8.70(1H,d,J=2.0Hz)

Synthetic Examples 114 to 121, 123 to 130 (Step 10-1)

In the same manner as in Synthetic Example 113, the compounds of Synthetic Examples 114, 115 and 116 were obtained from the compound of Preparative Example 100, the compounds of Synthetic Examples 117, 118 and 119 were obtained from the compound of Preparative Example 101, the compounds of Synthetic Examples 120, 121 and 123 were obtained from the compound of Preparative Example 102, the compounds of Synthetic Examples 124 and 125 were obtained from the compound of Preparative Example 103, the compounds of Synthetic Examples 126 and 127 were obtained from the compound of Preparative Example 104, and the compounds of Synthetic Examples 128, 129 and 130 were obtained from the compound of Preparative Example 105. These compounds are shown in Tables 44-46.

Synthetic Example 114

4-(4-Chlorophenyl)-2,3,9-trimethyl-6-(pyridin-4-ylmethyl)-6H-5,6,7,8,9a-pentaazathieno[2,3-e]azulene

Synthetic Example 115

4-(4-Chlorophenyl)-6-(4-cyanobenzyl)-2,3,9-trimethyl-6H-5,6,7,8,9a-pentaazathieno[2,3-e]azulene

Synthetic Example 116

4-(4-Chlorophenyl)-6-(3,4-difluorobenzyl)-2,3,9-trimethyl-6H-5,6,7,8,9a-pentaazathieno[2,3-e]azulene

Synthetic Example 117

4-(4-Chlorophenyl)-2-ethyl-9-methyl-6-(pyridin-3-ylmethyl)-6H-5,6,7,8,9a-pentaazathieno[2,3-e]azulene

Synthetic Example 118

4-(4-Chlorophenyl)-2-ethyl-9-methyl-6-(pyridin-4-ylmethyl)-6H-5,6,7,8,9a-pentaazathieno[2,3-e]azulene

Synthetic Example 119

4-(4-Chlorophenyl)-6-(4-cyanobenzyl)-2-ethyl-9-methyl-6H-5,6,7,8,9a-pentaazathieno[2,3-e]azulene

Synthetic Example 120

2,9-Dimethyl-4-phenyl-6-(pyridin-3-ylmethyl)-6H-5,6,7,8,9a-pentaazathieno[2,3-e]azulene

Synthetic Example 121

2,9-Dimethyl-4-phenyl-6-(pyridin-4-ylmethyl)-6H-5,6,7,8,9a-pentaazathieno[2,3-e]azulene

Synthetic Example 123

6-(4-Chlorobenzyl)-2,9-dimethyl-4-phenyl-6H-5,6,7,8,9a-pentaazathieno[2,3-e]azulene

Synthetic Example 124

2,3,9-Trimethyl-4-phenyl-6-(pyridin-3-ylmethyl)-6H-5,6,7,8,9a-pentaazathieno[2,3-e]azulene

Synthetic Example 125

2,3,9-Trimethyl-4-phenyl-6-(pyridin-4-ylmethyl)-6H-5,6,7,8,9a-pentaazathieno[2,3-e]azulene

Synthetic Example 126

2-Ethyl-9-methyl-4-phenyl-6-(pyridin-3-ylmethyl)-6H-5,6,7,8,9a-pentaazathieno[2,3-e]azulene

Synthetic Example 127

2-Ethyl-9-methyl-4-phenyl-6-(pypidin-4-ylmethyl)-6H-5,6,7,8,9a-pentaazathieno[2,3-e]azulene

Synthetic Example 128

4-(4-Methoxyphenyl)-2,3,9-trimethyl-6-(pyridin-3-ylmethyl)-6H-5,6,7,8,9a-pentaazathieno[2,3-e]azulene

Synthetic Example 129

4-(4-Methoxyphenyl)-2,3,9-trimethyl-6-(pyridin-4-ylmethyl)-6H-5,6,7,8,9a-pentaazathieno[2,3-e]azulene

Synthetic Example 130

6-(4-Cyanobenzyl)-4-(4-methoxyphenyl)-2,3,9-trimethyl-6H-5,6,7,8,9a-pentaazathieno[2,3-e]azulene

**Table 44**

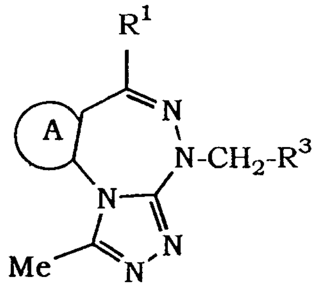

| Synth. Ex. | A | $R^1$ | $R^3$ | $^1$H NMR (300MHz, δppm, $CDCl_3$) | Melting point (°C) |
|---|---|---|---|---|---|
| 114 | 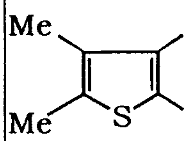 | 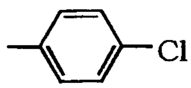 | 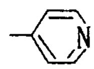 | 1.58(3H, s),<br>2.40(3H, s),<br>2.63(3H, s),<br>5.04(2H, m),<br>7.21-7.33(6H, m),<br>8.55-8.57(2H, m). | 195-196 |
| 115 | 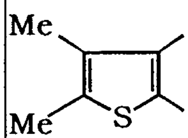 | 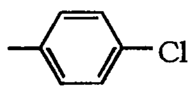 | 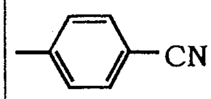 | | 210-211 |
| 116 | 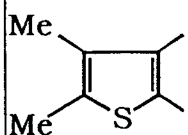 | 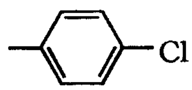 | 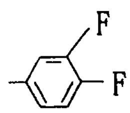 | | 189-191 |
| 117 | 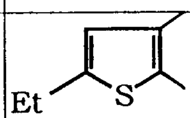 | 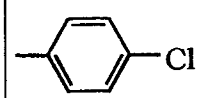 | 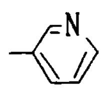 | | 182-183 |
| 118 | 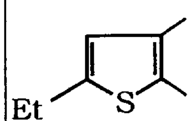 | 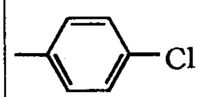 | 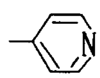 | | 107-108 |
| 119 | 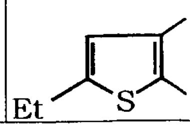 | 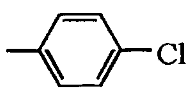 | 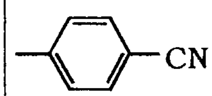 | | 199-200 |

**Table 45**

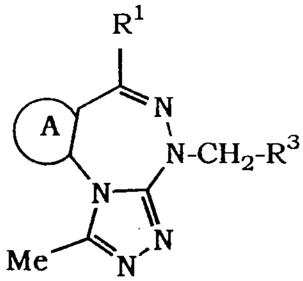

| Synth. Ex. | A | $R^1$ | $R^3$ | $^1H$ NMR (300MHz, δppm, $CDCl_3$) | Melting point (℃) |
|---|---|---|---|---|---|
| 120 | 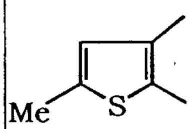 | 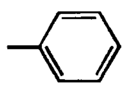 | 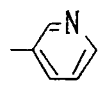 | 2.48 (3H, d, J=1.5Hz), 2.63(3H, s), 5.05(2H, s), 6.43 (1H, q, J=1.5Hz), 7.25-7.45(6H, m), 7.79 (1H, dt, J=7.5 and 2.0Hz), 8.53 (1H, dd, J=5.0 and 2.0Hz), 8.72 (1H, d, J=2.0Hz). | |
| 121 | 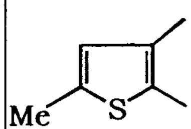 | 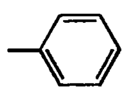 | 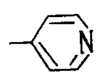 | | 188-190 |
| 123 | 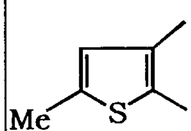 | 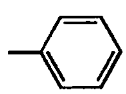 | 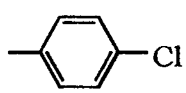 | | 128-130 |

**Table 46**

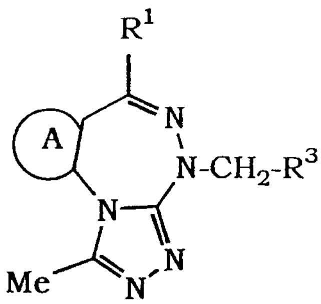

| Synth. Ex. | A | $R^1$ | $R^3$ | Melting point (°C) |
|---|---|---|---|---|
| 124 | 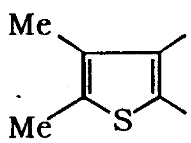 | 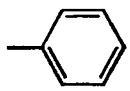 | 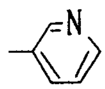 | 168-169 |
| 125 | 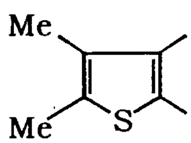 | 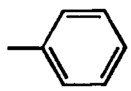 | 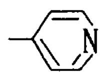 | 182-183 |
| 126 | 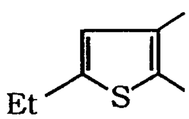 | 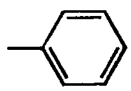 | 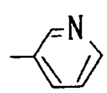 | 147-148 |
| 127 | 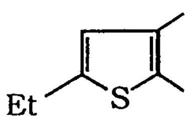 | 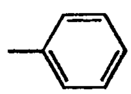 | 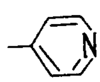 | 187-189 |
| 128 | 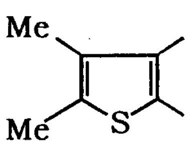 | 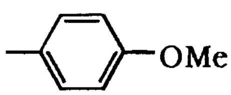 | 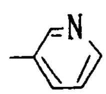 | 191-192 |
| 129 | 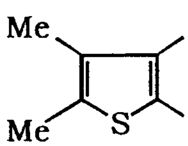 | 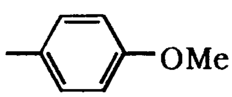 | 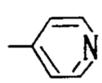 | 154-156 |
| 130 | 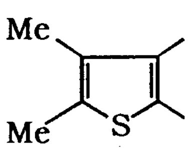 | 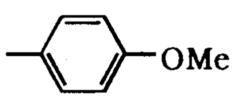 | 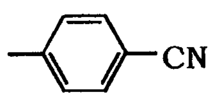 | 171-172 |

Synthetic Example 122 (Step 10-1)

6-(4-Cyanobenzyl)-2,9-dimethyl-4-phenyl-6H-5,6,7,8,9a-pentaazathieno[2,3-e]azulene

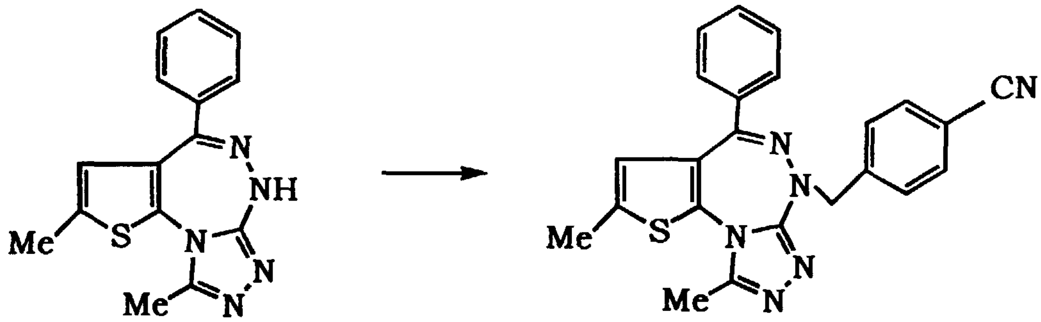

2,9-Dimethyl-4-phenyl-6H-5,6,7,8,9a-pentaazathieno[2,3-e]azulene (1.26 g) obtained in Preparative Example 102 was dissolved in anhydrous N,N-dimethylformamide (20 ml) under an argon gas atmosphere and ice-cooled. Sodium hydride (60% in oil, 176 mg) was added thereto and the mixture was stirred under ice-cooling for 15 minutes and at room temperature for 30 minutes. The reaction mixture was again ice-cooled and α-bromo-p-tolunitrile (823 mg) was added to the reaction mixture. The mixture was stirred at the same temperature for 30 minutes. Sodium hydride (14 mg) and α-bromo-p-tolunitrile (30 mg) were further added and the mixture was stirred at the same temperature for 15 minutes. Ethyl acetate and ice water were added and the organic layer was separated. The organic layer was washed four times with water, dried over anhydrous sodium sulfate and filtered. The filtrate was concentrated under reduced pressure to give a thick solution. Thereto was added diethyl ether to give 1.28 g of the title compound as yellow crystals.

Melting point : 192-195°C
$^1$H NMR (300MHz, δ ppm, $CDC1_3$)
2.49(3H,s), 2.62(3H,s), 5.09(2H,s), 6.45(1H,m), 7.31-7.46(5H,m), 7.54(2H,d,J=8.3Hz), 7.62(2H,d,J=8.3Hz)

Synthetic Example 131(Step 10-1)

4-(4-Chlorophenyl)-6-(4-fluorobenzyl)-2,3,9-trimethyl-6H-5,6,1,8,9a-pentaazathieno[2,3-e]azulene

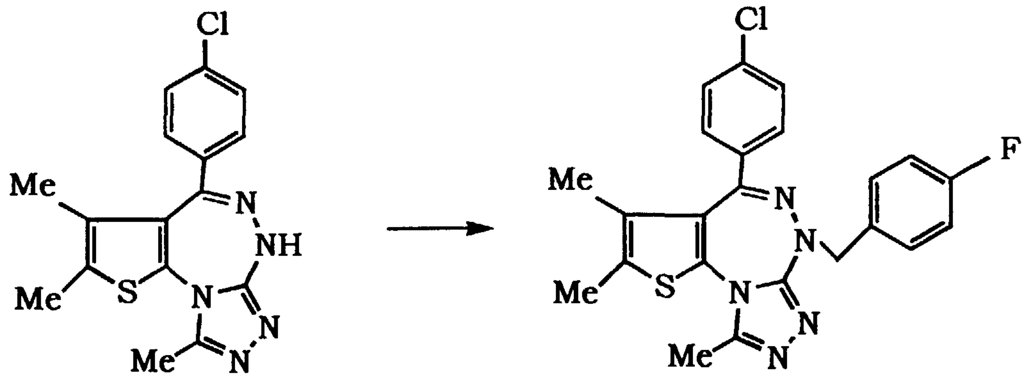

To a solution of 4-(4-chlorophenyl)-2,3,9-trimethyl-6H-5,6,7,8,9a-pentaazathieno[2,3-e]azulene (68.8 mg) obtained in Preparative Example 100 in anhydrous N,N-dimethylformamide (1 ml) was added, under an argon gas atmosphere, a powder (34 mg) of potassium hydroxide pulverized in a mortar. The resulting red mixture was ice-cooled and 4-fluorobenzyl bromide (20 μl) was added, which was followed by stirring for 20 minutes under the same conditions. Ethyl acetate and water were added to the mixture and the organic layer was washed 5 times with water. The organic layer was dried and concentrated. The obtained residue was crystallized from ether to give 78 mg of the title compound as pale-yellow crystals.

Melting point : 242-243°C

Synthetic Examples 132-134 (Step 10-1)

In the same manner as in Synthetic Example 131, the compounds of Synthetic Examples 132 and 133 were obtained from the compound of Preparative Example 100 and the compound of Synthetic Example 134 was obtained from the compound of Preparative Example 101. These compounds are shown in Table 47.

Synthetic Example 132

6-(4-Chlorobenzyl)-4-(4-chlorophenyl)-2,3,9-trimethyl-6H-5,6,7,8,9a-pentaazathieno[2,3-e]azulene

Synthetic Example 133

4-(4-Chlorophenyl)-6-(3,4-dichlorobenzyl)-2,3,9-trimethyl-6H-5,6,7,8,9a-pentaazathieno[2,3-e]azulene

Synthetic Example 134

4-(4-Chlorophenyl)-6-(3,4-dichlorobenzyl)-2-ethyl-9-methyl-6H-5,6,7,8,9a-pentaazathieno[2,3-e]azulene

**Table 47**

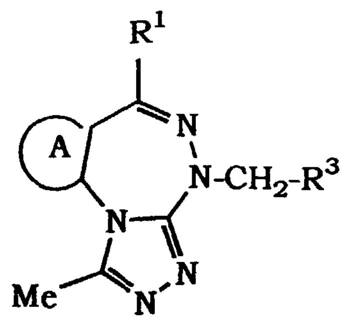

| Synth. Ex. | A | $R^1$ | $R^3$ | Melting point (°C) |
|---|---|---|---|---|
| 132 | 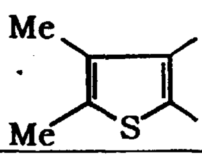 | 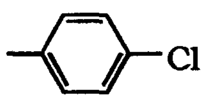 | 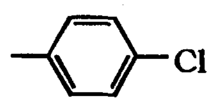 | 230-231 |
| 133 | 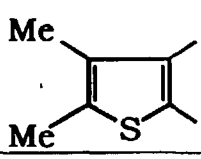 | 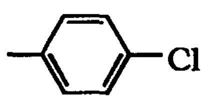 | 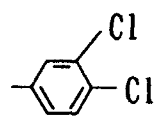 | 191-193 |
| 134 | 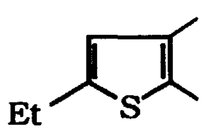 | 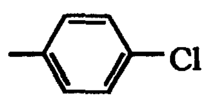 | 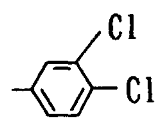 | 151-153 |

Synthetic Example 135

6-(4-Chlorophenyl)-1-methyl -4-(pyridin-3-ylmethyl)-4H-2,3,4,5,10b-pentaazabenz[e]azulene, monohydrochloride monohydrate

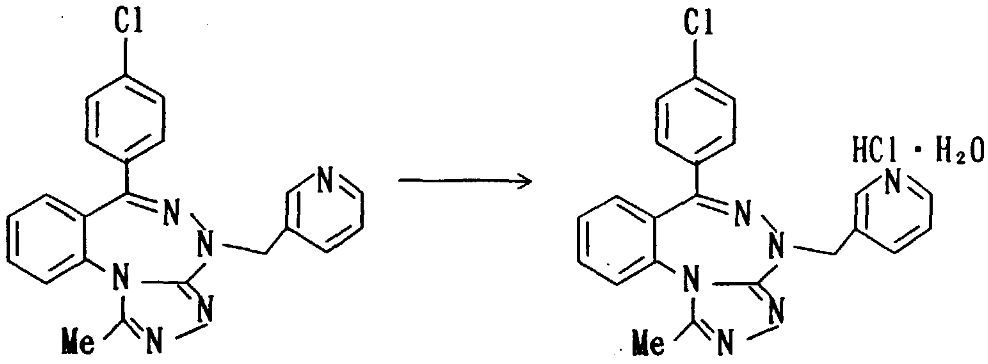

6-(4-Chlorophenyl)-1-methyl-4-(pyridin-3-ylmethyl)-4H-2,3,4,5,10b-pentaazabenz[e]azulene (501 mg) obtained in Synthetic Example 19 or 63 was suspended in ethanol (10 ml) and a 4N hydrochloric acid/ethyl acetate solution (0.4 ml) was added thereto. The solution was concentrated to 3 ml and the precipitated crystals were collected by filtration. The crystals were suspended in ethanol (10 ml). The suspension was heated and allowed to stand at room temperature to give 395 mg of the title compound as colorless needles.

Melting point : 238-240°C
$^1$H NMR (300MHz, $\delta$ ppm, DMSO-$d_6$)
2.49(3H,s), 4.98(1H,m), 5.08(1H,m), 7.15(1H,d,J=7.2Hz), 7.33-7.47(5H,m), 7.69(2H,m), 7.84(1H,dd,J=7.2 and 4.8Hz), 8.38(1H,d,J=7.5Hz), 8.72(1H,d,J=4.8Hz), 8.87(1H,s)

Synthetic Examples 136 to 141

In the same manner as in Synthetic Example 135, the compound of Synthetic Example 136 was obtained from the compound of Synthetic Example 19 or 63 and p-toluenesulfonic acid monohydrate, the compound of Synthetic Example 137 was obtained from the compound of Synthetic Example 19 or 63 and methanesulfonic acid, the compound of Synthetic Example 138 was obtained from the compound of Synthetic Example 19 or 63 and benzenesulfonic acid, the compound of Synthetic Example 139 was obtained from the compound of Synthetic Example 92 and a 4N hydrochloric acid/ethyl acetate solution, the compound of Synthetic Example 140 was obtained from the compound of Synthetic Example 120 and p-toluenesulfonic acid monohydrate, and the compound of Synthetic Example 141 was obtained from the compound of Synthetic Example 92 and p-toluenesulfonlc acid monohydrate. These compounds are shown in Tables 48 and 49.

Synthetic Example 136

6-(4-Chlorophenyl)-1-methyl-4-(pyridin-3-ylmethyl)-4H-2,3,4,5,10b-pentaazabenz[e]azulene p-toluenesulfonate

Synthetic Example 137

6-(4-Chlorophenyl)-1-methyl-4-(pyridin-3-ylmethyl)-4H-2,3,4,5,10b-pentaazabenz[e]azulene 2 methanesulfonate

Synthetic Example 138

6-(4-Chlorophenyl)-1-methyl-4-(pyridin-3-ylmethyl)-4H-2,3,4,5,10b-pentaazabenz[e]azulene, 1.5 benzensulfonate

Synthetic Example 139

8-Chloro-1-methyl-6-phenyl-4-(pyridin-4-ylmethyl)-4H-2,3,4,5,10b-pentaazabenz[e]azulene hydrochloride

Synthetic Example 140

2,9-Dimethyl-4-phenyl-6-(pyridin-3-ylmethyl)-6H-5,6,7,8,9a-pentaazathieno[2,3-e]azulene p-toluenesulfonate

Synthetic Example 141

8-Chloro-1-methyl-6-phenyl-4-(pyridin-4-ylmethyl)-4H-2,3,4,5,10b-pentaazabenz[e]azulene p-toluenesulfonate

**Table 48**

| Syn. Ex. | Structural formula | $^1$H NMR (300MHz, δppm, $CDCl_3$) | Melting point (°C) |
|---|---|---|---|
| 136 | 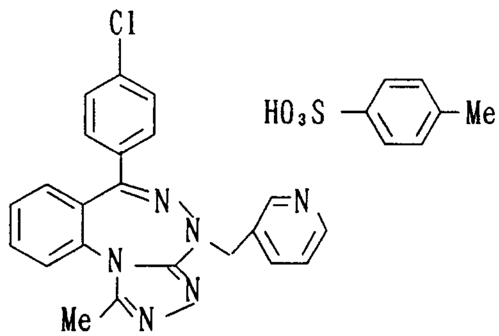 | 2.28(3H,s), 2.55 (3H,s), 5.04(1H,m), 5.14(1H,m), 7.10(2H,d,J=8.0Hz), 7.20(1H,d,J=8.0Hz), 7.41-7.51(7H,m), 7.74 (2H,m), 7.93(1H,dd, J=8.3 and 5.6Hz), 8.48 (1H,d,J=8.3Hz), 8.78(1H, d,J=5.6Hz), 8.94(1H,s). | 248-250 |
| 137 | 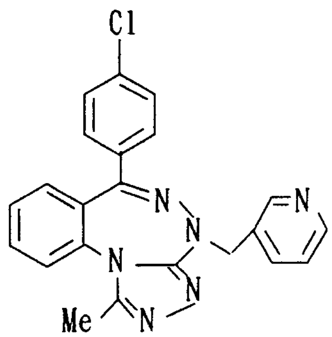 $(MeSO_3H)_2$ | | 213-215 |
| 138 | 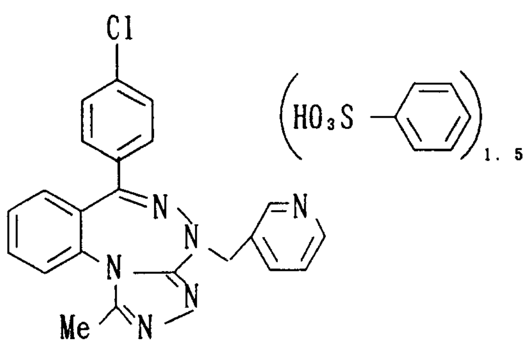 | | 247-250 |

## Table 49

| Syn. Ex. | Structural formula | $^1H$ NMR (300MHz, δppm, $CDCl_3$) | Melting point (℃) |
|---|---|---|---|
| 139 | 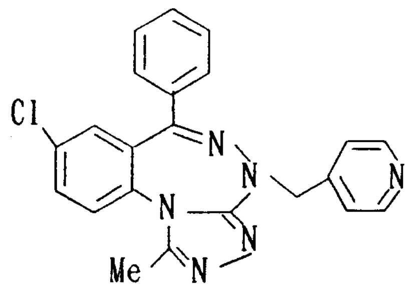 HCl | 2.57(3H, s), 5.12(1H, m), 5.28(1H, m), 7.22 (1H, d, J=2.0Hz), 7.38-7.54(5H, m), 7.80-7.89(2H, m), 7.94 (2H, d, J=6.5Hz), 8.82 (2H, d, J=5.6Hz). | |
| 140 | 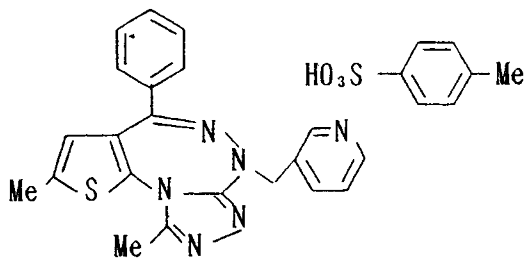 | | 206-209 |
| 141 | 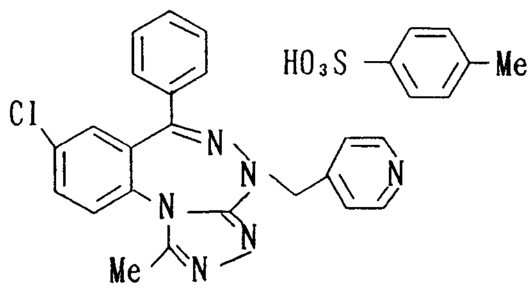 | 2.27(3H, s), 2.55(3H, s), 5.15(1H, m), 5.30(1H, m), 7.10 (2H, d, J=8.0Hz), 7.21 (1H, d, J=2.5Hz), 7.38-7.52(7H, m), 7.79-7.88(2H, m), 7.95 (2H, d, J=6.5Hz), 8.82 (2H, d, J=6.5Hz). | |

Synthetic Example 142

6-(4-Chlorophenyl)-1-methyl-4-(pyridin-3-ylmethyl)-4H-2,3,4,5,10b-pentaazabenz[e]azulene citrate

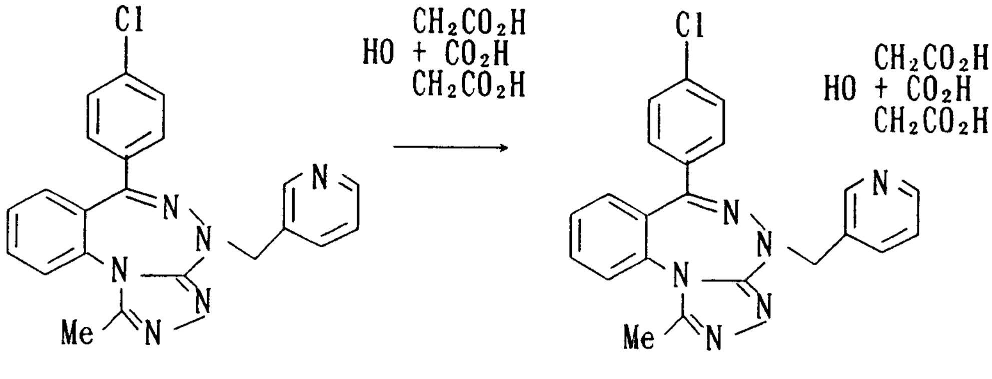

6-(4-Chlorophenyl)-1-methyl-4-(pyridin-3-ylmethyl)-4H-2,3,4,5,10b-pentaazabenz[e]azulene(401 mg) obtained in Synthetic Example 19 or 63 was suspended in ethanol (20 ml). Anhydrous citric acid (192 mg) was added to the suspension and the mixture was heated. The resulting solution was concentrated to 10 ml and diethyl ether (7 ml) was added. The mixture was allowed to stand at room temperature and the precipitated crystals were collected by filtration. The crystals were suspended in ethanol (20 ml). The suspension was heated and allowed to stand at room temperature to give 395 mg of the title compound as colorless needles.

Melting point : 208-209°C
$^1$H NMR (300MHz, δ ppm, DMSO-$d_6$)
2.53(3H,s), 2.66(2H,d,J=15.3Hz), 2.75(2H,d,J=15.3Hz), 4.86(1H,m), 5.01(1H,m), 7.20(1H,d,J=7.8Hz), 7.35-7.53(6H,m), 7.69-7.81(3H,m), 8.48(1H,dd,J=4.7 and 1.4Hz), 8.60(1H,d,J=1.4Hz)

Synthetic Example 143 (Step 10-1)

6-(4-Chlorophenyl)-1-methyl-4-(2-nitrobenzyl)-4H-2,3,4,5,10b-pentaazabenz[e]azulene

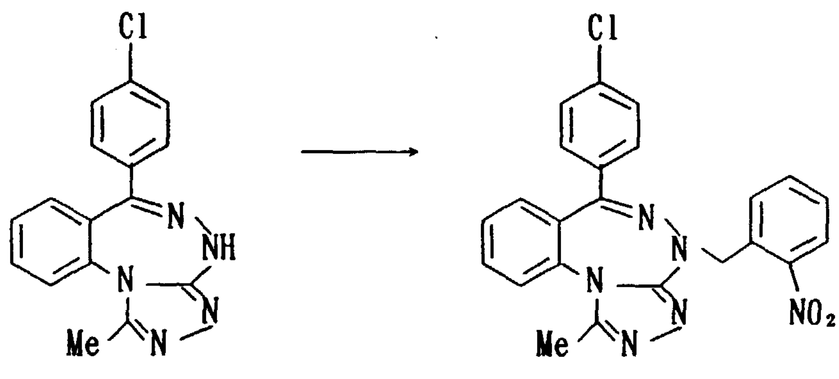

6-(4-Chlorophenyl)-1-methyl-4H-2,3,4,5,10b-pentaazabenz[e]azulene (217 mg) obtained in Preparative Example 89 was dissolved in anhydrous N,N-dimethylformamide (3 ml) under an argon gas atmosphere. 2-Nitrobenzyl bromide (756 mg) and potassium carbonate (967 mg) were added thereto and the mixture was stirred at room temperature for 3 hours. After completion of the reaction, the insoluble substance was filtered off. Ethyl acetate (50 ml) and water (40 ml) were added to the filtrate and organic layer was separate. The organic layer was successively washed with a 5% aqueous solution of citric acid, a saturated aqueous sodium hydrogencarbonate solution and water, dried, filtered and concentrated under reduced pressure. Then, the obtained residue was subjected to silica gel column chromatography. The residue obtained by concentration of the fractions eluted with chloroform : acetone (10:1) was crystallized from diethyl ether to give 46 mg of the title compound as yellow crystals.

Melting point : 204-205°C

Synthetic Example 144 (Step 10-1)

6-(4-Chlorophenyl)-4-ethoxycarbonylmethyl-1-methyl-4H-2,3,4,5,10b-pentaazabenz[e]azulene

In the same manner as in Synthetic Example 143, the compound of Synthetic Example 144 was obtained from the compound of Preparative Example 89. The compound is shown in Table 50.

**Table 50**

| Syn. Ex. | Structural formula | Melting point (°C) |
|---|---|---|
| 144 | 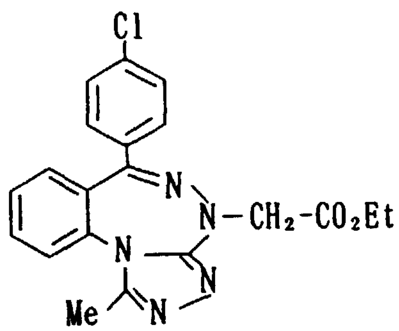 | 111-112 |

Synthetic Example 145 (Step 10-9)

[6-(4-Chlorophenyl)-1-methyl-4H-2,3,4,5,10b-pentaazabenz[e]azulen-4-yl]acetic acid

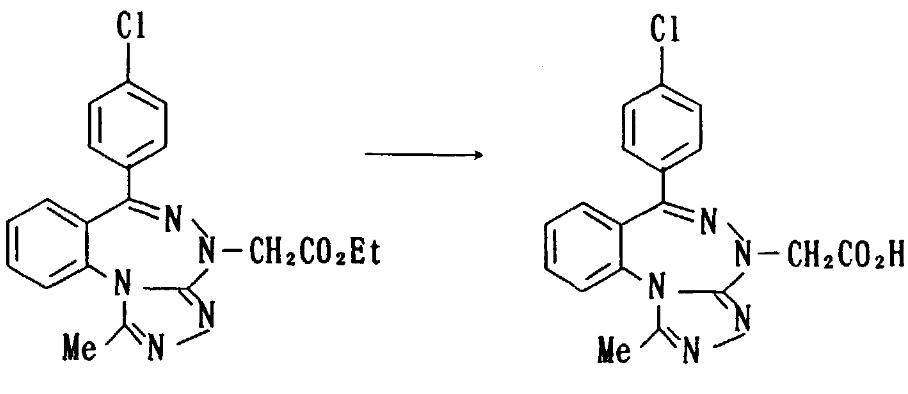

6-(4-Chlorophenyl)-4-ethoxycarbonylmethyl-1-methyl-4H-2,3,4,5,10b-pentaazabenz[e]azulene (151 mg) obtained in Synthetic Example 144 was dissolved in ethanol (1.5 ml) and cooled in an ice-bath. An aqueous solution of 1N sodium hydroxide (0.37 ml) was added thereto and the mixture was stirred under ice-cooling for 1 hour and at room temperature for 2 hours. The reaction mixture was cooled in an ice-bath again and 1N hydrochloric acid was added to the reaction mixture. The mixture was stirred for a while. Ethyl acetate (10 ml) and a saturated aqueous sodium chloride solution (5 ml) was added thereto and the organic layer was separated. The organic layer was further washed thee times with a saturated aqueous sodium chloride solution, dried and filtered. The filtrate was concentrated under reduced pressure and the obtained residue was crystallized from diethyl ether to give 129 mg of the title compound as crystals.

Melting point : 274-275°C

Synthetic Example 146 (Step 10-10)

6-(4-Chlorophenyl)-1-methyl-4-phenylcarbamoylmethyl-4H-2,3,4,5,10b-pentaazabenz[e]azulene

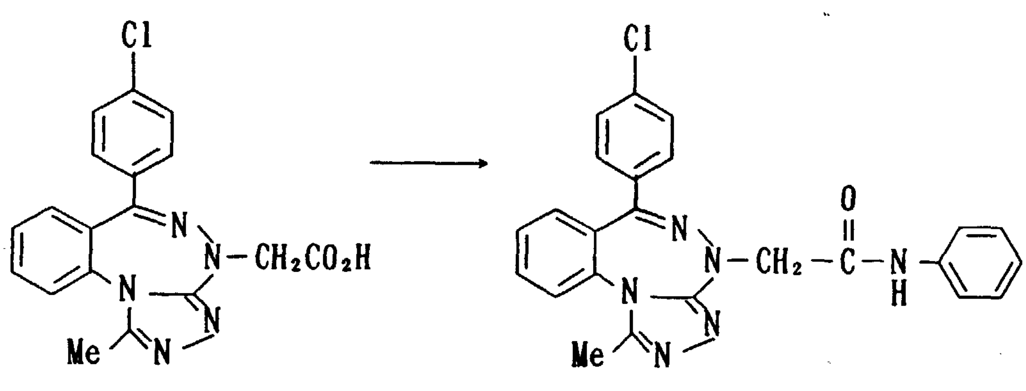

[6-(4-Chlorophenyl)-1-methyl-4H-2,3,4,5,10b-pentaazabenz[e]azulene]acetic acid (114 mg) obtained in Synthetic Example 145 was dissolved in anhydrous N,N-dimethylformamide (2.2 ml) and triethylamine (0.05 ml) was added. The mixture was cooled to-15°C in a dry ice-acetone bath and isobutyl chlorocarbonate (0.4 ml) was added for 1 minute. The mixture was stirred for 40 minutes maintaining the internal temperature at-15°C to-10°C and then aniline (0.34 ml) was added. The internal temperature was raised to 0°C for 30 minutes and the reaction mixture was stirred for another 30 minutes Ethyl acetate (15 ml) and water (10 ml) were added to the reaction mixture and the organic layer was separated. The organic layer was washed with water, dried and filtered. The filtrate was concentrated under reduced pressure. The obtained residue was dissolved in a small amount of chloroform and diethyl ether was added for crystallization to give 111 mg of the title compound as crystals.

Melting point : 249-252°C

Synthetic Examples 147 to 154 (Step 10-10)

In the same manner as in Synthetic Example 146, the compounds of Synthetic Examples 147 to 154 were obtained from the compound of Synthetic Example 145. These compounds are shown in Table 51.

Synthetic Example 147

6-(4-Chlorophenyl)-1-methyl-4-(4-methylphenylcarbamoylmethyl)-4H-2,3,4,5,10b-pentaazabenz[e]azulene

Synthetic Example 148

6-(4-Chlorophenyl)-4-(2-methoxyphenylcarbamoylmethyl)-1-methyl-4H-2,3,4,5,10b-pentaazabenz[e]azulene,

Synthetic Example 149

6-(4-Chlorophenyl)-4-(2,5-dimethoxyphenylcarbamoylmethyl)-1-methyl-4H-2,3,4,5,10b-pentaazabenz[e]azulene

Synthetic Example 150

4-(4-Chloro-2,5-dimethoxyphenylcarbamoylmethyl)-6-(4-chloro-phenyl)-1-methyl-4H-2,3,4,5,10b-pentaazabenz[e]azulene

Synthetic Example 151

6-(4-Chlorophenyl)-1-methyl-4-(naphthalen-1-ylcarbamoylmethyl)-4H-2,3,4,5,10b-pentaazabenz[e]azulene

Synthetic Example 152

6-(4-Chlorophenyl)-1-methyl-4-(pyridin-3-ylcarbamoylmethyl)-4H-2,3,4,5,10b-pentaazabenz[e]azulene

Synthetic Example 153

6-(4-Chlorophenyl)-4-(cyclohexylcarbamoylmethyl)-1-methyl-4H-2,3,4,5,10b-pentaazabenz[e]azulene

Synthetic Example 154

6-(4-Chlorophenyl)-1-methyl-4-n-propylcarbamoylmethyl-4H-2,3,4,5,10b-pentaazabenz[e]azulene

**Table 51**

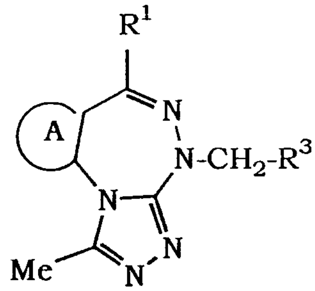

| Syn.Ex. | $R^3$ | Melting point (℃) | Syn.Ex. | $R^3$ | Melting point (℃) |
|---|---|---|---|---|---|
| 147 | 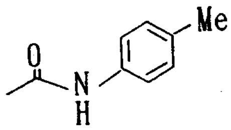 | 316-317 | 151 | 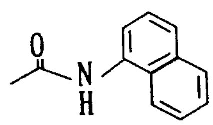 | 257-258 |
| 148 | 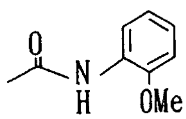 | 195-196 | 152 | 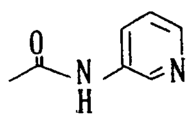 | 241-245 |
| 149 | 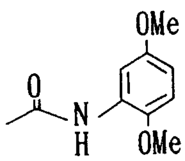 | 203-204 | 153 | 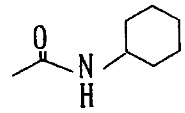 | 242 |
| 150 | 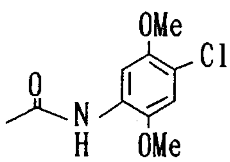 | 147-151 (decomposition) | 154 | 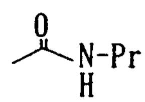 | 246-247 |

Synthetic Example 155 (Step 10-1)

4-Bromoacetyl-6-(4-(4-chlorophenyl)-1-methyl-4H-2,3,4,5,10b-pentaazabenz[e]azulene

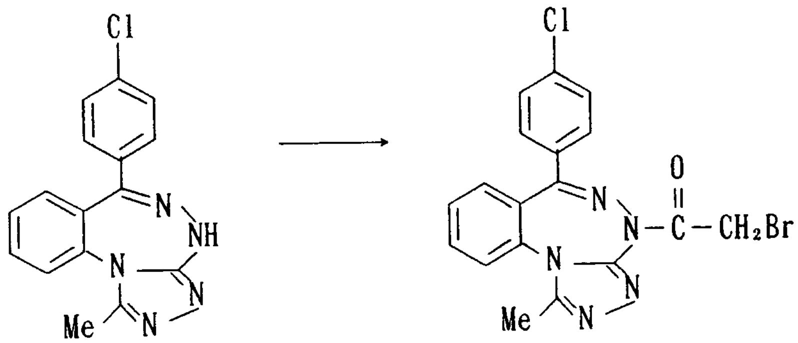

6-(4-Chlorophenyl)-1-methyl-4H-2,3,4,5,10b-pentaazabenz[e]azulene (100 mg) obtained in Preparative Example 89 was dissolved in anhydrous dichloromethane (1 ml) and anhydrous pyridine (52 μl) was added. The mixture was cooled in an ice-bath and bromoacetyl bromide (42 μl) was added. The mixture was stirred under ice-cooling for 30 minutes and at room temperature for 1.5 hours. Water was poured into the reaction mixture and the mixture was extracted with ethyl acetate. The organic layer was washed successively with a 5% aqueous citric acid solution and water, dried over anhydrous sodium sulfate and filtered. The filtrate was concentrated under reduced pressure and the residue was subjected to silica gel column chromatography to give the title compound (70 mg) as white crystals.

$^1$H NMR (300MHz, δ ppm, $CDC1_3$)
2.70(3H,s), 4.23-4.42(2H,m), 7.36-7.52(5H,m), 7.62-7.65(2H,m), 7.72-7.78(1H,m)

Synthetic Example 156 (Step 10-11)

6-(4-Chlorophenyl)-4-(2-methoxyphenylaminoacetyl)-1-methyl-4H-2,3,4,5,10b-pentaazabenz[e]azulene

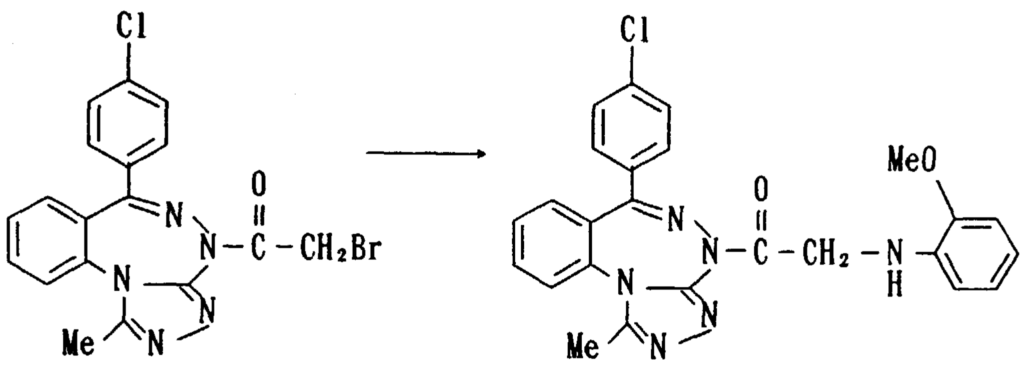

4-Bromoacetyl-6-(4-chlorophenyl)-1-methyl-4H-2,3,4,5,10b-pentaazabenz[e]azulene (8 mg) obtained in Synthetic Example 155 was suspended in ethanol and 2-anisidine (12.5 μl) was added. The mixture was stirred at 80°C for 3 hours. The reaction mixture was cooled to room temperature and concentrated under reduced pressure. Water (20 ml) was added to the residue and the mixture was extracted with ethyl acetate (20 ml). The organic layer was further

washed with water, dried over anhydrous sodium sulfate and filtered. The filtrate was concentrated under reduced pressure and the residue was purified by preparative thin-layer chromatography to give 2.4 mg of the title compound as an oil.

$^1$H NMR(300MHz, δ ppm,CDC1$_3$)
2.67(3H,s), 3.82(3H,s), 4.35(2H,m), 6.37(1H,m), 6.60-6.72(3H,m), 7.29-7.43(6H,m), 7.61-7.64(3H,m)

Synthetic Example 157 to 160 (Step 10-11)

In the same manner as in Synthetic Example 156, the compounds of Synthetic Examples 157 to 160 were obtained from the compound of Synthetic Example 155. These compounds are shown in Table 52.

Synthetic Example 157

6-(4-Chlorophenyl)-1-methyl-4-phenylaminoacetyl-4H-2,3,4,5,10b-pentaazabenz[e]azulene

Synthetic Example 158

6-(4-Chlorophenyl)-1-methyl-4-(4-methylphenylaminoacetyl)-4H-2,3,4,5,10b-pentaazabenz[e]azulene

Synthetic Example 159

6-(4-Chlorophenyl)-4-(3-fluorophenylaminoacetyl)-1-methyl-4H-2,3,4,5,10b-pentaazabenz[e]azulene

Synthetic Example 160

6-(4-Chlorophenyl)-4-(2,5-dimethoxyphenylaminoacetyl)-1-methyl-4H-2,3,4,5,10b-pentaazabenz[e]azulene

Table 52

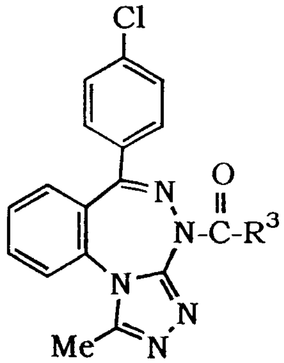

| Syn. Ex. | $R^3$ | $^1$H NMR (300MHz, δppm, $CDCl_3$) | Melting point (℃) |
|---|---|---|---|
| 157 | 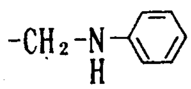 | | 130-133 |
| 158 | 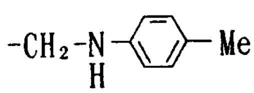 | 2. 19(3H, s), 2. 67(3H, s), 4. 25-4. 39(2H, m), 6. 42(2H, d, J=8. 2Hz), 6. 71(1H, s), 6. 87(2H, d, J=8. 2Hz), 7. 31-7. 44(5H, m), 7. 61-7. 66(3H, m). | |
| 159 | 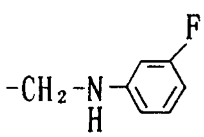 | 2. 68(3H, s), 4. 25-4. 40(2H, m), 6. 16-6. 20(1H, m), 6. 28-6. 37(2H, m), 6. 97-7. 05(1H, m), 7. 32-7. 47(5H, m), 7. 61-7. 70(3H, m). | |
| 160 | 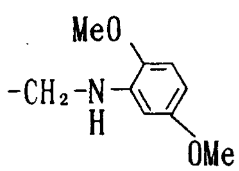 | 2. 67(3H, s), 3. 63(3H, s), 3. 77(3H, s), 4. 31(2H, m), 5. 98(1H, m), 6. 07-6. 11(1H, m), 6. 60-6. 62(1H, d, J=6. 0Hz), 7. 31-7. 43(6H, m), 7. 60-7. 64(3H, m). | |

Synthetic Example 161 (Step 10-11)

6-(4-Chlorophenyl)-1-methyl-4-phenylthioacetyl-4H-2,3,4,5,10b-pentaazabenz[e]azulene

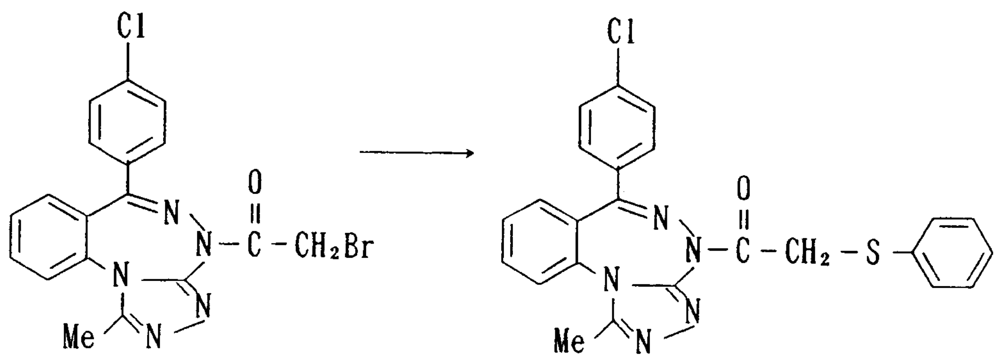

4-Bromoacetyl-6-(4-chlorophenyl)-1-methyl-4H-2,3,4,5,10b-pentaazabenz[e]azulene (8 mg) obtained in Synthetic Example 155 was dissolved in anhydrous dichloromethane (0.8 ml), and thiophenol (2 μl) and triethylamine (2.8 μl) were added. The mixture was stirred at room temperature for 2 hours. After the completion of the reaction, water (20 ml) was added to the reaction mixture and the mixture was extracted with ethyl acetate (20 ml). The organic layer was washed successively with a 5% aqueous citric acid solution and water, dried over anhydrous sodium sulfate and filtered. The filtrate was concentrated under reduced pressure and the residue was purified by preparative thin-layer chromatography to give 2.1 mg of the title compound as an amorphous solid.

$^1$H NMR (300MHz, δ ppm, $CDC1_3$)
2.69(3H,s), 3.96-4.12(2H,m), 7.03-7.76(13H,m)

Synthetic Example 162 (Step 10-1)

6-(4-Chlorophenyl)-1-methyl-4-phenylacetyl-4H-2,3,4,5,10b-pentaazabenz[e]azulene

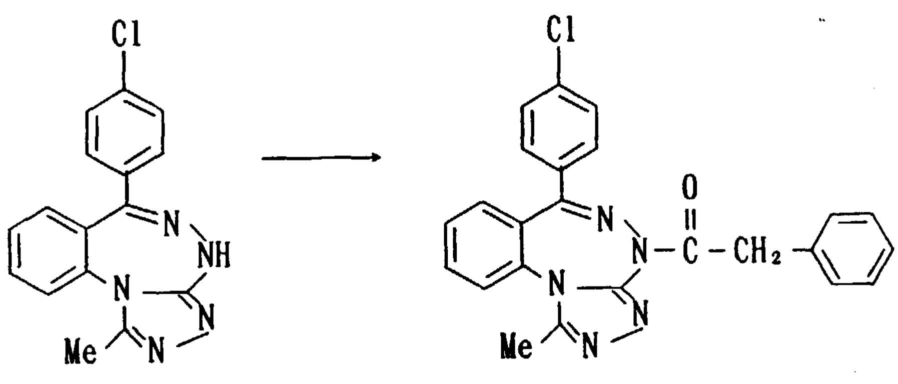

6-(4-Chlorophenyl)-1-methyl-4H-2,3,4,5,10b-pentaazabenz[e]azulene (20 mg) obtained in Preparative Example 89 was dissolved in anhydrous dichloromethane (0.2 ml). Pyridine (21 μl), 4-dimethylaminopyridine (1.5 mg) and phenylacetyl chloride (25.6 μl) were added and the mixture was stirred under ice-cooling for 30 minutes and at room

temperature for 16 hours. After the completion of the reaction, water (30 ml) was added to the reaction mixture and the mixture was extracted with dichloromethane (30 ml). The organic layer was washed successively with a 5% aqueous citric acid solution and water, dried over anhydrous sodium sulfate and filtered. The filtrate was concentrated under reduced pressure and the residue was purified by preparative thin-layer chromatography and crystallized from diethyl ether to give 6 mg of the title compound as colorless crystals.

Melting point : 89-90°C

Synthetic Example 163 (Step 10-1)

6-(4chlorophenyl)-1-methyl-4-phenyloxalyl-4H-2,3,4,5,10b-pentaazabenz[e]azulene

In the same manner as in Synthetic Example 162, the compound of Synthetic Example 163 was obtained from the compound of Preparative Example 89. The compound is shown in Table 53.

**Table 53**

| Syn. Ex. | Structural formula | Melting point (°C) |
|---|---|---|
| 163 | 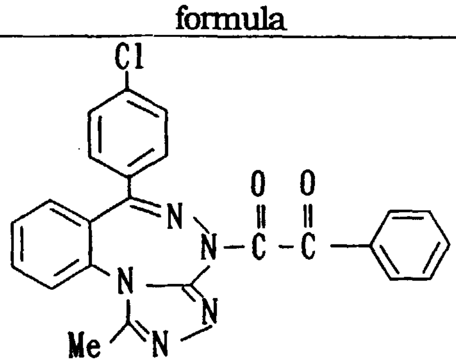 | 218-219 |

Synthetic Example 164 (Step 10-4)

6-(4-Chlorophenyl)-4-ethoxymethyl-1-methyl-4H-2,3,4,5,10b-pentaazabenz[e]azulene

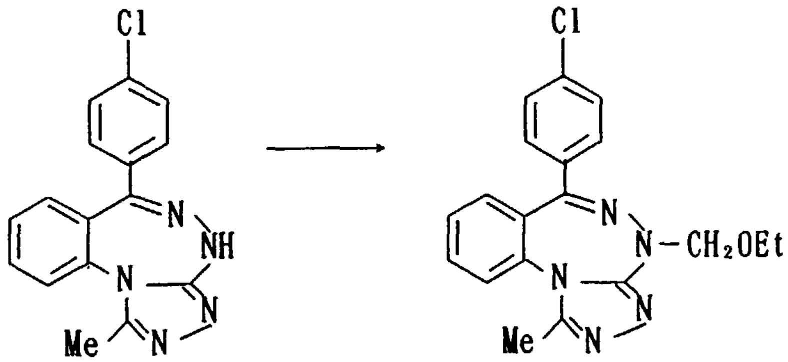

6-(4-Chlorophenyl)-1-methyl-4H-2,3,4,5,10b-pentaazabenz[e]azulene (84 mg) obtained in Preparative Example 89 was dissolved in ethanol (1 ml) and a 37% aqueous formaldehyde solution (0.12 ml) was added. The mixture was refluxed under heating for 3 days. The reaction solvent was distilled away under reduced pressure. The residue was purified by preparative thin-layer chromatography and crystallized from ethyl acetate/diethyl ether to give 70 mg of the title compound as colorless crystals.

Melting point : 98-100°C

Synthetic Example 165 (Step 10-4)

N-[6-(4-Chloromphenyl)-1-methyl-4H-2,3,4,5,10b-pentaazabenz[e]azulen-4-ylmethyl-N-phenyl-amine

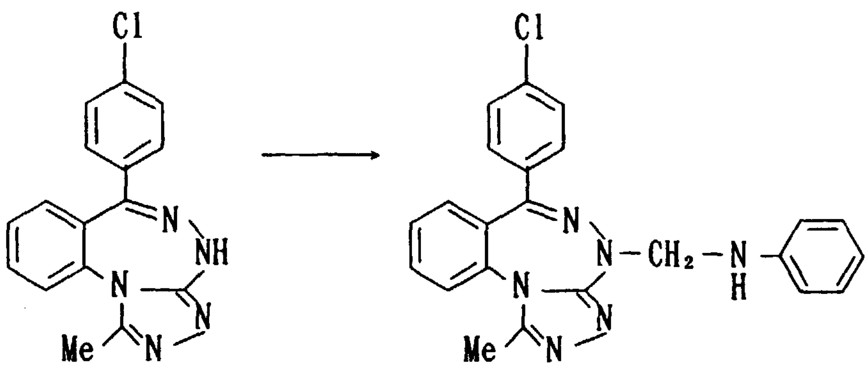

6-(4-Chlorophenyl)-1-methyl-4H-2,3,4,5,10b-pentaazabenz[e]azulene (20 mg) obtained in Preparative Example 89 was dissolved in ethanol (0.1 ml), and a 37% aqueous formaldehyde solution (6 µl) and aniline (7.6 µl) were added. The mixture was stirred under heating for 2 hours. The reaction mixture was cooled and the precipitated crystals were collected by filtration to give 13.8 mg of the title compound as colourless crystals.

Melting point : 223-224°C

Synthetic Example 166 (Step 10-5)

4-Benzlcarbamoyl-6-(4-chlorophenyl)-1-methyl-4H-2,3,4,5,10b-pentaazabenz[e]azulene

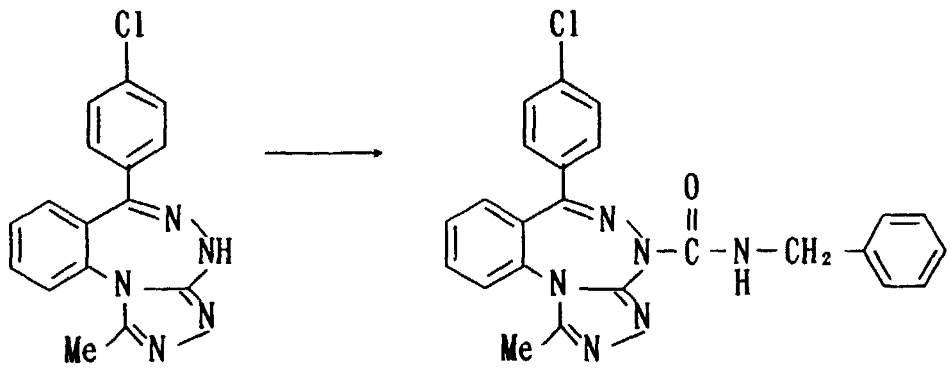

6-(4-Chlorophenyl)-1-methyl-4H-2,3,4,5,10b-pentaazabenz[e]azulene (206 mg) obtained in Preparative Example 89 was suspended in anhydrous acetonitrile. Sodium hydroxide (52 mg) pulverized in a mortar and benzyl isocyanate (100 μl) were added to the suspension and the mixture was stirred at room temperature for 2 hours and at 50°C for 2 hours. After the completion of the reaction, the reaction mixture was concentrated under reduced pressure. Water (30 ml) was added to the residue and the mixture was extracted with ethyl acetate. The organic layer was dried over anhydrous magnesium sulfate and filtered. The filtrate was concentrated under reduced pressure. The residue was purified by silica gel column chromatography and crystallized from ethyl acetate-diethyl ether to give 80 mg of the title compound.

Melting point : 146-149°C

Synthetic Example 167 (Step 10-5)

6-(4-Chlorophenyl)-1-methyl-4-(3-methylphenylcarbamoyl)-4H-2,3,4,5,10b-pentaazabenz[e]azulene

In the same manner as in Synthetic Example 166, the compound of Synthetic Example 167 was obtained from the compound of Preparative Example 89. The compound is shown in Table 54.

**Table 54**

| Syn. Ex. | Structural formula | Melting point (°C) |
|---|---|---|
| 167 | 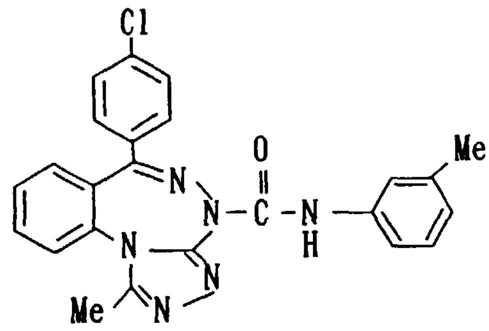 | 204-205 |

Synthetic Example 168 (Step 10-15)

6-(4-Chlorophenyl)-4-(4-hydroxybenzyl)-1-methyl-4H-2,3,4,5,10b-pentaazabenz[e]azulene

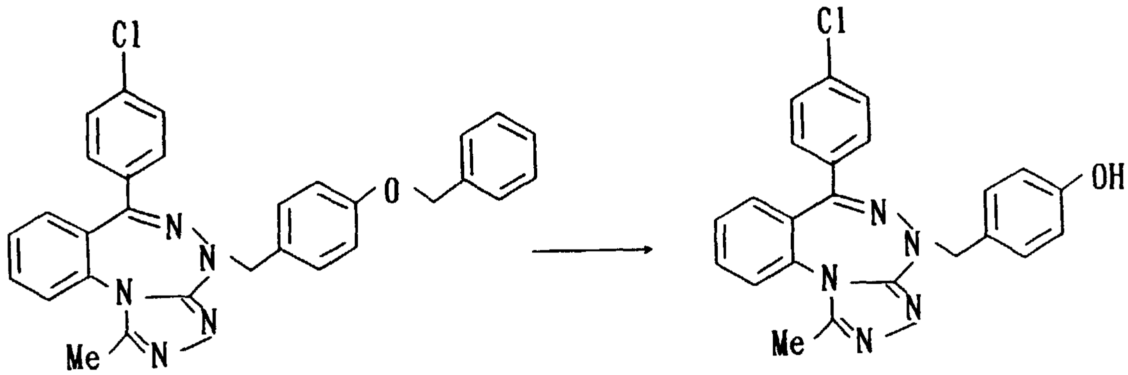

4-(4-Benzyloxybenzyl)-6-(4-chlorophenyl)-1-methyl-4H-2,3,4,5,10b-pentaazabenz[e]azulene (80 mg) obtained in Synthetic Example 58 was dissolved in ethanol (1 ml). Palladium black (4 mg) was added and the mixture was stirred under a hydrogen atmosphere at 3 atm for 2 days. The catalyst was filtered off and the filtrate was concentrated under reduced pressure. The residue was purified by preparative thin-layer chromatography (chloroform:methanol=20: 1) and crystallized from ethyl acetate-diethyl ether to give 29 mg of the title compound.

Melting point : 254-257°C

Synthetic Example 169 (Step 10-15)

6-(4-Chlorophenyl)-4-(3,4-dihydroxybenzyl)-1-methyl-4H-2,3,4,5,10b-pentaazabenz[e]azulene

In the same manner as in Synthetic Example 168, the compound of Synthetic Example 169 was obtained from

the compound of Synthetic Example 80. The compound is shown in Table 55.

**Table 55**

| Syn. Ex. | Structural formula | $^1$H NMR (300MHz, δppm, $CDCl_3$) |
|---|---|---|
| 169 | 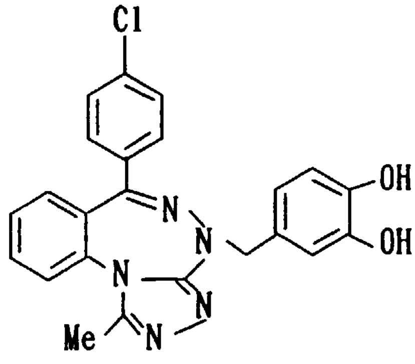 | 2.60(3H, s), 4.56-4.60(1H, m), 4.81-4.85(1H, m), 6.59-6.66(2H, m), 6.74-6.75(1H, m), 7.18-7.20 (1H, d, J=7.4Hz), 7.42-7.51(5H, m), 7.68-7.75(2H, m). |

Synthetic Example 170 (Step 10-16)

6-(4-Chlorophenyl)-4-(4-ethoxybenzyl)-1-methyl-4H-2,3,4,5,10b-pentaazabenz[e]azulene

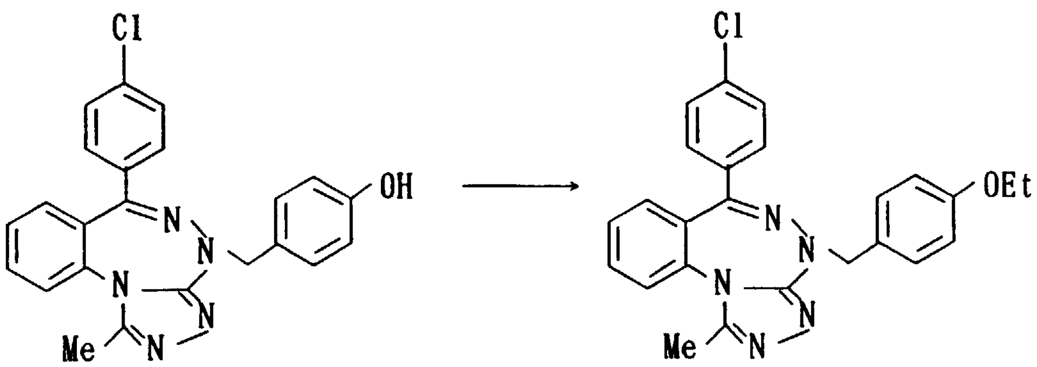

6-(4-Chlorophenyl)-4-(4-hydroxybenzyl)-1-methyl-4H-2,3,4,5,10b-petaazabenz[e]azulene (15 mg) obtained in Synthetic Example 168 was dissolved in tetrahydrofuran (2 ml) and the mixture was cooled in an ice-bath. A solution of diazoethane was added thereto and the mixture was stirred at 0°C overnight. The reaction mixture was concentrated and the residue was purified by preparative thin-layer chromatography to give 12 mg of the title compound.

$^1$H NMR (300 MHz, δ ppm, $CDC1_3$)
1.40(3H,t,J=7.2Hz), 2.60(3H,s), 4.02(2H,q,J=7.2Hz), 4.88-5.03(2H,m), 6.83(2H,d,J=9.0Hz), 7.18-7.64(10H,m)

Synthetic Example 171 (Step 10-2)

6-(4-Chlorophenyl)-1-methyl-4-(4-methylsulfonylphenyl) hydroxymethyl-4H-2,3,4,5,10b-pentaazabenz[e]azulene

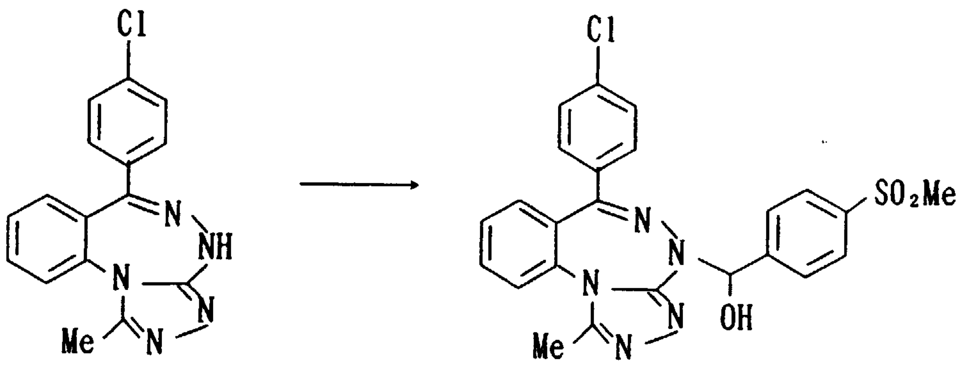

6-(4-Chlorophenyl)-1-methyl-4H-2,3,4,5,10b-pentaazabenz[e]azulene (93 mg) obtained in Preparative Example 89 was dissolved in N,N-dimethylformamide (1 ml). Pulverized sodium hydroxide (100 mg) was added to the solution and the mixture was stirred at room temperature for 5 minutes under dry air. Then, p-methylsulfonylbenzyl chloride (68 mg) was added and the mixture was stirred at room temperature for 5 minutes. After the completion of the reaction, the reaction mixture was cooled in an ice-bath and a 5% aqueous citric add solution and ethyl acetate were added. The organic layer was separated and successively washed with a saturated aqueous sodium hydrogencarbonate solution and water, dried and filtered. The filtrate was concentrated under reduced pressure. The residue was purified by preparative thin-layer chromatography and crystallized from ethyl acetate-diethyl ether to give 34 mg of the title compound.

Melting point : 142-144°C

Synthetic Example 172 (Step 10-12)

4-(4-Aminobenzyl)-6-(4-chlorophenyl)-1-methyl-4H-2,3,4,5,10b-pentaazabenz[e]azulene and 6-(4-chlorophenyl)-4(4-formylaminobenzyl)-1-methyl-4H-2,3,4,5,10b-pentaazabenz[e]azulene

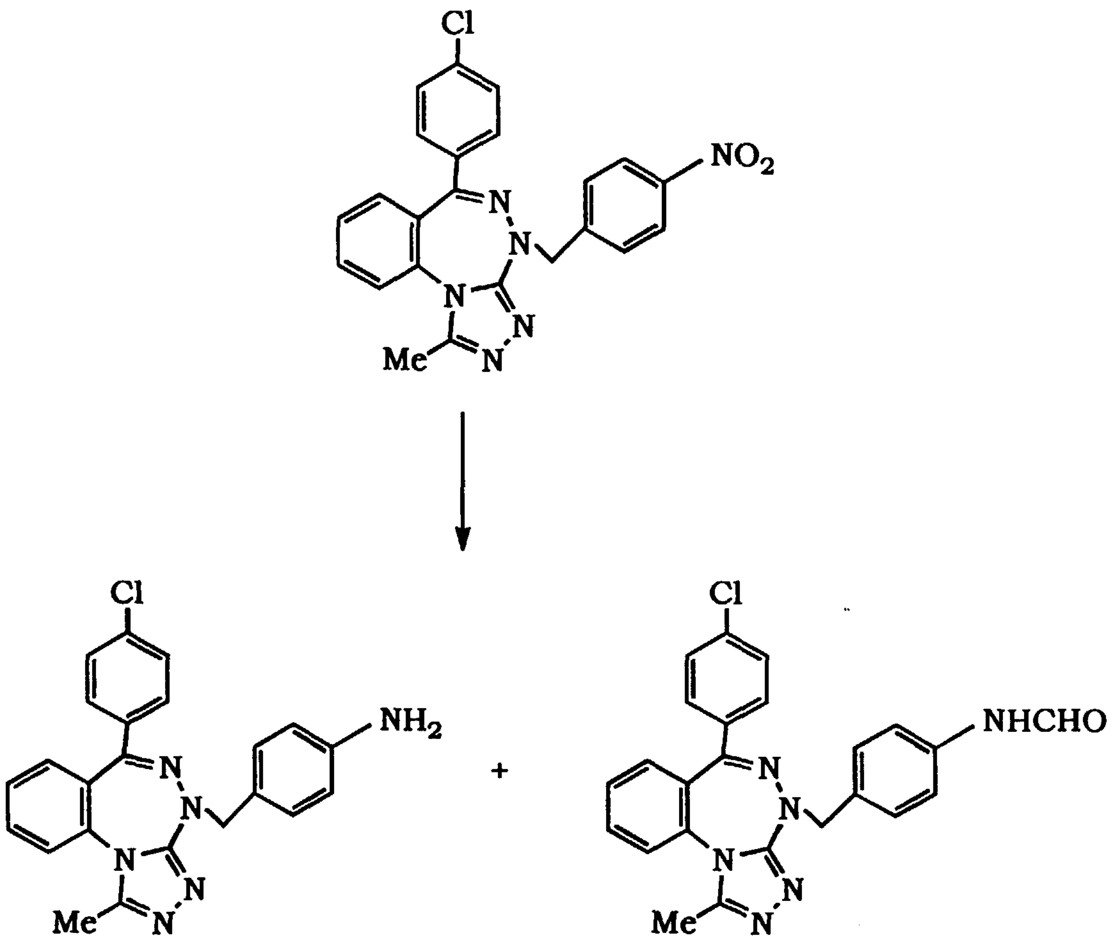

6-(4-Chlorophenyl)-1-methyl-4-(4-nitrobenzyl)-4H-2,3,4,5,10b-pentaazabenz[e]azulene (680 mg) obtained in Synthetic Example 88 was dissolved in methanol (20 ml) and formic and (6 ml). Palladium black (68 mg) was added and the mixture was stirred at room temperature for 1 hour. The catalyst was filtered off and the filtrate was concentrated under reduced pressure. The residue was dissolved in ethyl acetate and water was added. The organic layer was separated and washed successively with a saturated aqueous sodium hydrogencarbonate solution and water, dried and filtered. The filtrate was concentrated under reduced pressure. The residue was purified by silica gel column chromatography and crystallized from diethyl ether to give 120 mg of 4-(4-aminobenzyl)-6-(4-chlorophenyl)-1-methyl-4H-2,3,4,5,10b-pentaazabenz[e]azulene (m.p. 234-237°C) and 340 mg of 6-(4-chlorophenyl)-4-(4-formylaminobenzyl)-1-methyl-4H-2,3,4,5,10b-pentaazabenz[e]azulene m.p. 215-216°C).

Synthetic Example 173 (Step 10-13)

4-(4-Acetylaminobenzyl)-6-(4-chlorophenyl)-1-methyl-4H-2,3,4,5,10b-pentaazabenz[e]azulene

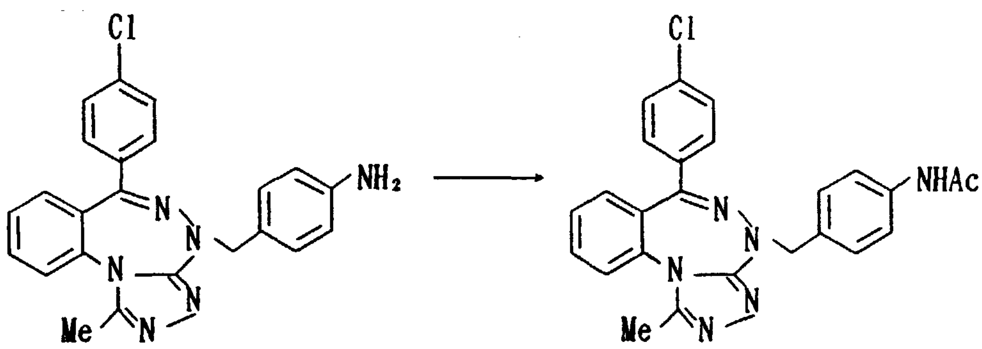

4-(4-Aminobenzyl)-6-(4-chlorophenyl)-1-methyl-4H-2,3,4,5,10b-pentaazabenz[e]azulene (12.4 mg) obtained in Synthetic Example 172 was dissolved in dichloromethane (1 ml) and the mixture was ice-cooled. Acetyl chloride (2.4 μl) was added and the mixture was stirred at said temperature for 10 minutes and at room temperature for 20 minutes. After the completion of the reaction, dichloromethane was distilled away under reduced pressure. The residue obtained was crystallized from ethyl acetate-diethyl ether to give 11.1 mg of the title compound.

Melting point : 245-249°C

Synthetic Example 174 (Step 10-13)

6-(4-Chlorophenyl)-4-(4-methylsulfonylaminobenzyl)-1-methyl-4H-2,3,4,5,10b-pentaazabenz[e]azulene and 4-(4-bis(methylsulfonyl)aminobenzyl]-6-(4-chlorophenyl)-4H-2,3,4,5,10b-pentaazabenz[e]azulene

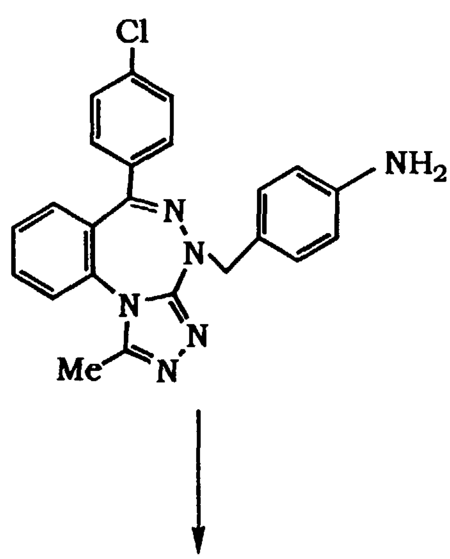

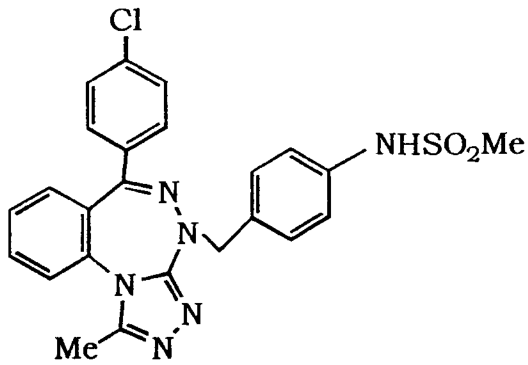

+

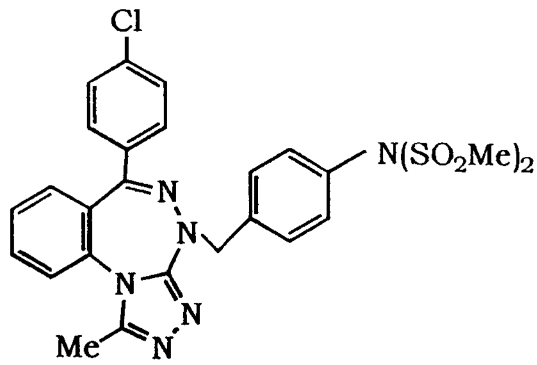

4-(4-Aminobenzyl)-6-(4-chlorophenyl)-1-methyl-4H-2,3,4,5,10b-pentaazabenz[e]azulene (24.8 mg) obtained in Synthetic Example 172 was dissolved in dichloromethane (2 ml) and triethylamine (30 μl) was added. The mixture was ice-cooled and methanesulfonyl chloride (7 μl) was added. The mixture was stirred under ice-cooling for 10 minutes and at room temperature for 2 hours. The reaction mixture was concentrated under reduced pressure and a 5% aqueous citric acid solution and ethyl acetate were added. The organic layer was separated, and successively washed with a saturated aqueous sodium hydrogencarbonate solution and water. The organic layer was dried, filtered and concentrated under reduced pressure. The residue obtained was separated and purified by preparative thin-layer chromatography. Both compounds were individually crystallized from ethyl acetate-diethyl ether to give 8 mg of 6-(4-chlorophenyl)-4-(4-methylsulfonylaminobenzyl)-1-methyl-4H-2,3,4,5,10b-pentaazabenz[e]azulene (m.p. 273-275°C) and 16 mg of-4-[4-bis(methylsulfonyl)aminobenzyl]-6-(4-chlorophenyl)-4H-2,3,4,5,10b-pentaazabenz[e]azulene (m.p. 220-221°C).

Synthetic Example 175 (Step 10-14)

6-(4-Chlorophenyl)-4-(4-dimethylaminobenzyl)-1-methyl-4H-2,3,4,5,10b-pentaazabenz[e]azulene hydrochloride

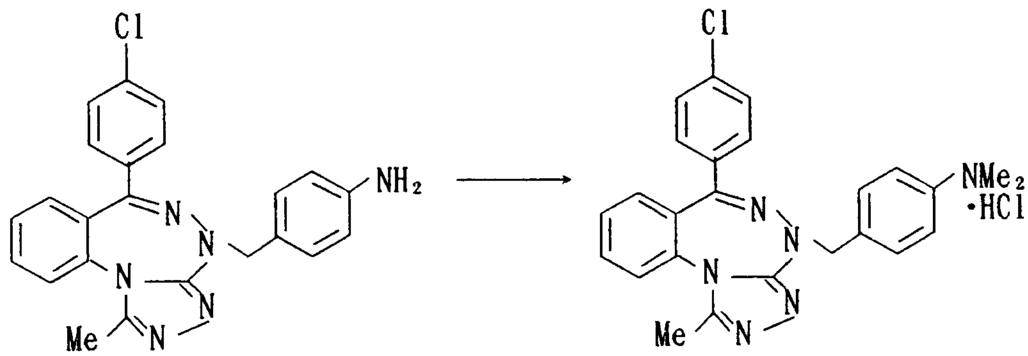

4-(4-Aminobenzyl)-6-(4-chlorophenyl)-1-methyl-4H-2,3,4,5,10b-pentaazabenz[e]azulene (124 mg) obtained in Synthetic Example 172 was dissolved in ethanol (5 ml). A 25% aqueous formaldehyde solution (0.15 ml) and palladium black (10 mg) were added and the mixture was stirred under a hydrogen atmosphere for 2 days. The catalyst was filtered off and the filtrate was concentrated under reduced pressure. The residue obtained was purified by preparative thin-layer chromatography (chloroform:acetone=3:1) and the obtained solid was dissolved in diethyl ether. A solution (0.1 ml) of 1N hydrogen chloride-diethyl ether was added to allow precipitation of crystals. The crystals were collected by filtration and washed with diethyl ether to give 34 mg of the title compound.

Melting point : 169-175°C

Synthetic Example 176 (Step 10-6)

6-(4-Chlorophenyl)-4-(2-hydroxy-2-phenylethyl)-1-methyl-4H-2,3,4,5,10b-pentaazabenz[e]azulene

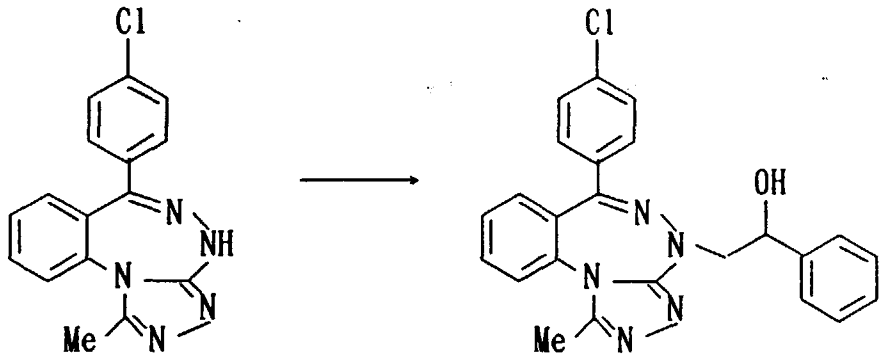

6-(4-Chlorophenyl)-1-methyl-4H-2,3,4,5,10b-pentaazabenz[e]azulene (103 mg) obtained in Preparative Example 89 was dissolved in N,N-dimethylformamide (10 ml) and the mixture was ice-cooled. Sodium hydride (60% in oil, 15.9 mg) was added and the mixture was stirred under ice-cooling for 5 minutes and at room temperature for 10 minutes. Then, styrene oxide (46 μl) was added and the mixture was stirred at 50°C for one hour. The reaction mixture was cooled to room temperature and water was added. The mixture was extracted with ethyl acetate, washed with water, dried over anhydrous magnesium sulfate and filtered. Purification by silica gel column chromatography gave 62 mg of the title compound as an amorphous solid.

$^1H$ NMR (300MHz, δ ppm, $CDC1_3$)
2.61(3H,s), 4.08(2H,m), 5.34(1H,m), 7.27-7.72(13H,m)

Synthetic Example 177 (Step 10-8)

6-(4-Chlorophenyl)-4-(2-oxo-2-phenylethyl)-1-methyl-4H-2,3,4,5,10b-pentaazabenz[e]azulene

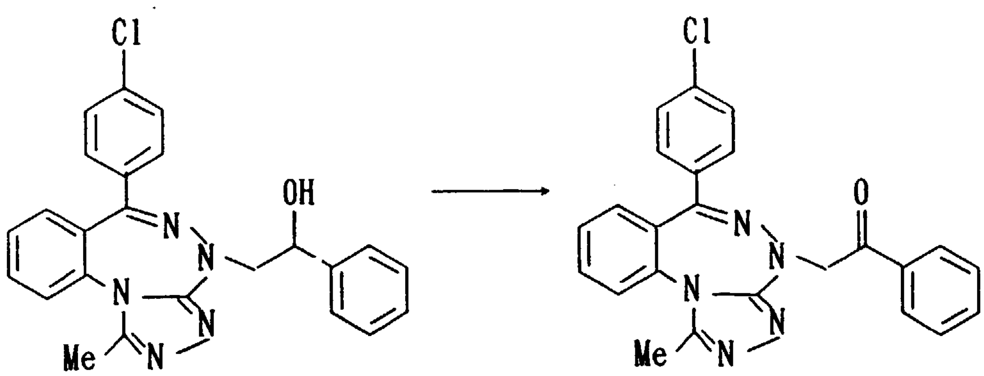

6-(4-Chlorophenyl)-4-(2-hydroxy-2-phenylethyl)-1-methyl-4H-2,3,4,5,10b-pentaazabenz[e]azulene (31 mg) obtained in Synthetic Example 176 was dissolved in anhydrous dichloromethane (0.3 ml) and pyridinium dichromate

(54.4 mg) was added, which was followed by stirring at room temperature. The reaction mixture was filtered through Celite, washed with water and dried over anhydrous sodium sulfate. The residue obtained by concentration under reduced pressure was purified by preparative thin-layer chromatography to give 2.0 mg of the title compound as an amorphous solid.

$^1$H NMR (300MHz, δ ppm, CDC1$_3$)
2.63(3H,s), 5.40(2H,s, 7.28-7.67(11H,m), 8.03-8.07(2H,m)

Synthetic Example 178 (Step 10-1)

6-(4-Chlorophenyl)-1-methyl-4-[3-phenyl-2-(tetrahydropyran-2-yloxy)propyl]-4H-2,3,4,5,10b-pentaazabenz[e]azulene

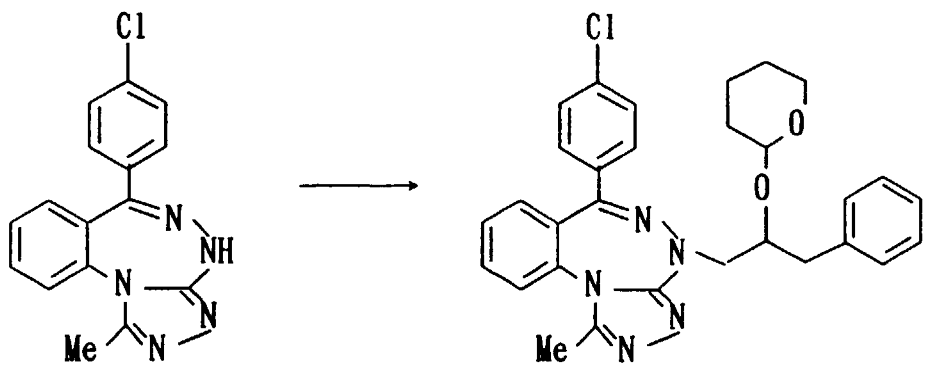

In the same manner as in Synthetic Example 21, 156 mg of the title compound was obtained from 6-(4-chlorophenyl)-1-methyl-4H-2,3,4,5,10b-pentaazabenz[e]azulene(100 mg) obtained in Preparative Example 89. Note that 2-(1-benzyl-2-bromoethoxy)tetrahydropyran was used instead of 3-(chloromethyl)benzonitrile.

$^1$H NMR (300MHz, δ ppm, CDC1$_3$)
0.96-1.66(7H,m), 2.57(3H,s), 2.88-3.47(3H,m), 3.95-4.78(4H,m), 7.13-7.65(13H,m)

Synthetic Example 179 (Step 10-1)

6-(4-Chlorophenyl)-4-[2-(2-methoxyphenyl)-2-(tetrahydropyran-2-yloxy)ethyl]-1-methyl-4H-2,3,4,5,10b-pentaazabenz[e]azulene

In the same manner as in Synthetic Example 178, the compound of Synthetic Example 179 was obtained from the compound of Preparative Example 89. The compound is shown in Table 56.

**Table 56**

| Syn. Ex. | Structural formula | $^1H$ NMR (300MHz, δppm, $CDCl_3$) |
|---|---|---|
| 179 | 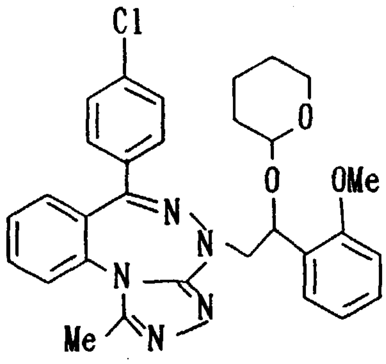 | 1.10-1.71(5H, m), 2.57(3H, s), 3.20(1H, m), 3.54(1H, m), 3.74-3.83(4H, m), 4.04-4.08(2H, m), 4.52-4.59(1H, m), 5.42(1H, m), 6.80-7.59 (12H, m). |

Synthetic Example 180 (Step 10-7)

6-(4-Chlorophenyl)-4-(2-hydroxy-3-phenylpropyl)-1-methyl-4H-2,3,4,5,10b-pentaazabenz[e]azulene

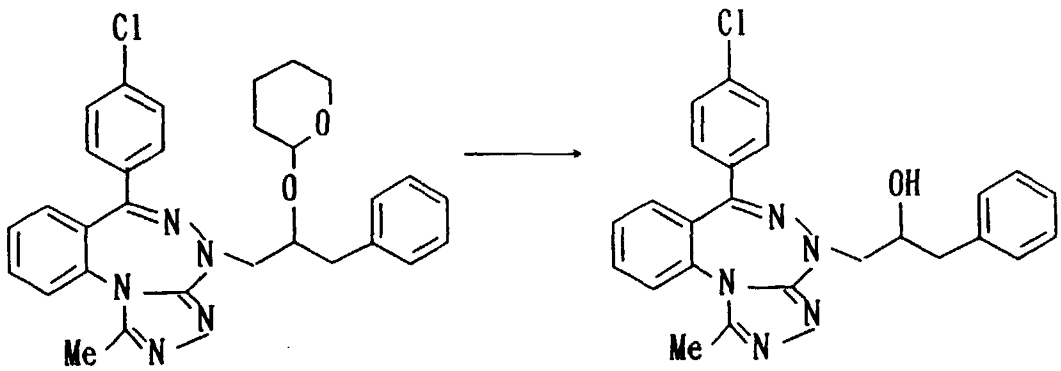

6-(4-Chlorophenyl)-1-methyl-4-[3-phenyl-2-(tetrahydropyran-2-yloxy)propyl]-4H-2,3,4,5,10b-pentaazabenz[e]azulene (146 mg) obtained in Synthetic Example 178 was dissolved in ethanol (0.7 ml) and pyridinium-p-toluenesulfonic acid (218 mg) was added, which was followed by string for 18 hours at 55°C. The mixture was allowed to cool to room temperature, and water (30 ml) was added. The mixture was extracted with ethyl acetate (30 ml). The organic layer was washed with water, dried over anhydrous sodium sulfate and filtrated. The residue obtained by concentration under reduced pressure was purified by silica gel column chromatograhy to give 112 mg of the title compound as an amorphous solid.

$^1H$ NMR (300MHz, δ ppm, $CDC1_3$)
2.59(3H,s) 2.73-2.88(2H,m), 3.81-3.98(2H,m), 4.44-4.61(1H,m), 7.22-7.44(12H,m), 7.60-7.65(1H,t,J=7. 5Hz)

Synthetic Example 181 (Step 10-8)

6-(4-Chloropbenyl)-4-(2-oxo-3-phenylpropyl)-1-methyl-4H-2,3,4,5,10b-pentaazabenz[e]azulene

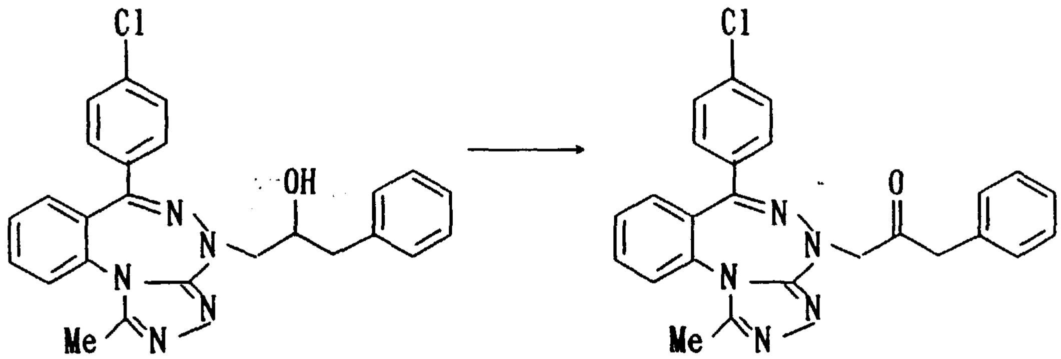

6-(4-Chlorophenyl)-4-(2-hydroxy-3-phenylpropyl)-1-methyl-4H-2,3,4,5,10b-pentaazabenz[e]azulene (89mg) obtained in Synthetic Example 180 was dissolved in anhydrous dichloromethane (1 ml) and pyridinium chlorochromate (130 mg) was added, which was followed by stirring at room temperature for 12 hours. After the completion of the reaction, the reaction mixture was purified by silica gel column chromatagraphy and crystallized from diethyl ether to give 23 mg of the title compound as white crystals.

Melting point : 81-84°C

Synthetic Examples 182-187 (Step 10-8)

In the same manner as in Synthetic Examples 176-181, the compounds of Synthetic Examples 182-187 were obtained from the compound of Preparative Example 89, which are shown in Table 57.

Synthetic Example 182

6-(4-Chlorophenyl)-4-[2-(4-chlorophenyl)-2-oxoethyl]-1-methyl-4H-2,3,4,5,10b-pentaazabenz[e]azulene

Synthetic Example 183

6-(4-Chlorophenyl)-1-methyl-4-[2-(4-methylphenyl)-2-oxoethyl]-4H-2,3,4,5,10b-pentaazabenz[e]azulene

Synthetic Example 184

6-(4-Chlorophenyl)-4-[2-(2-methoxyphenyl)-2-oxoethyl-1-methyl-4H-2,3,4,5,10b-pentaazabenz[e]azulene

Synthetic Example 185

6-(4-Chlorophenyl)-4-[2-(2,5-dimethoxyphenyl)-2-oxoethyl]-1-methyl-4H-2,3,4,5,10b-pentaazabenz[e]azulene

Synthetic Example 186

6-(4-Chlorophenyl)-4-[3-(2-methoxyphenyl)-2-oxopropyl]-1-methyl-4H-2,3,4,5,10b-pentaazabenz[e]azulene

Synthetic Example 187

6-(4-Chlorophenyl)-4-[3-(2,5-dimethoxyphenyl)-2-oxopropyl]-1-methyl-4H-2,3,4,5,10b-pentaazabenz[e]azulene

**Table 57**

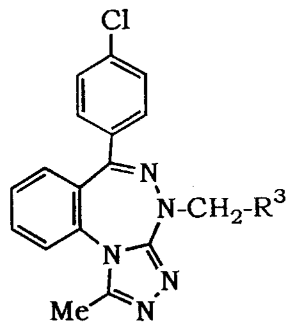

| Syn. Ex. | $R^3$ | $^1H$ NMR (300MHz, δppm, $CDCl_3$) | Melting point (°C) |
|---|---|---|---|
| 182 | 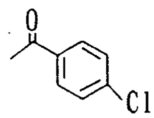 | 2.63(3H, s), 5.35(2H, s), 7.28-7.46(9H, m), 7.65(1H, t, J=7.5Hz), 7.98(2H, t, J=9.0Hz). | 222-223 |
| 183 | 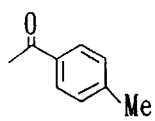 | 2.42(3H, s), 2.62(3H, s), 5.37(2H, s), 7.29-7.46(9H, m), 7.61-7.66(1H, m), 7.95(2H, d, J=6.0Hz). | 228-230 |
| 184 | 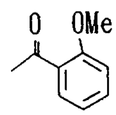 | | 224-225 |
| 185 | 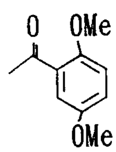 | 2.61(3H, s), 3.77(3H, s), 3.89(3H, s), 5.34(2H, broad s), 6.88(1H, d, J=6.0Hz), 7.04(1H, dd, J=3.0 and 9.0Hz), 7.23-7.47(8H, m), 7.59-7.65(1H, m). | Amorphous solid |
| 186 | 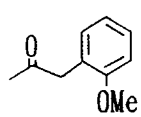 | 2.60(3H, s), 3.74(2H, s), 3.83(3H, s), 3.85(2H, s), 6.71-6.80(3H, m), 7.25-7.41(7H, m), 7.60-7.66(1H, m). | Amorphous solid |
| 187 | 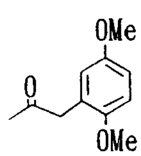 | 2.60(3H, s), 3.72-3.74(8H, m), 4.78(2H, s), 6.71-6.77(3H, m), 7.24-7.64(8H, m). | Amorphous solid |

Preparative Example 106 (Step 11)

2-[(4-Chlorophenyl)hydrazonomethyl]phenylamine

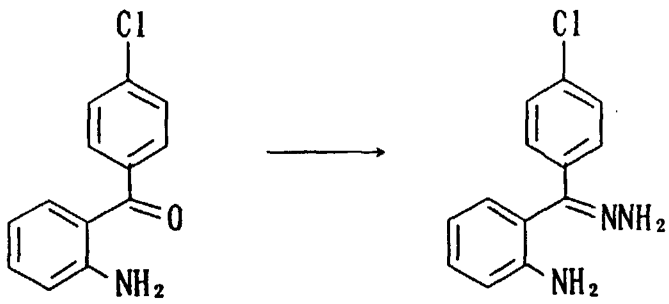

2-Aminophenyl 4-chlorophenyl ketone (27.2 g) was suspended in diethylene glycol (170 ml) and 100% hydrazine hydrate (23 ml) was added, which was followed by refluxing under heating for 7 hours. The reaction mixture was allowed to cool to room temperature and water (400 ml) was added. The mixture was extracted with ethyl acetate. The organic layer was washed with water and dried over sodium sulfate. After filtration, the residue obtained by concentration under reduced pressure was subjected to silica gel column chromatography. The obtained solid was crystallized from petroleum ether-diethyl ether to give 20.25 g of the title compound.

Melting point : 85-86°C

Preparative Example 107 (Step 12)

2-(4-Chlorobenzyl)phenylamine

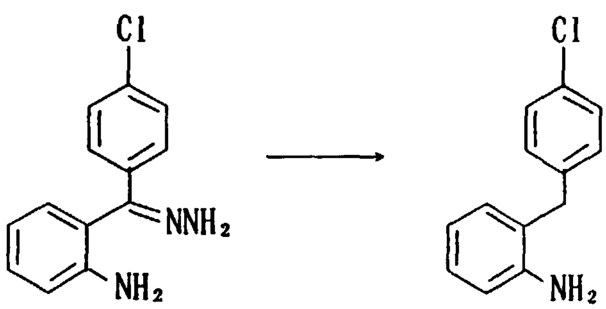

Potassium hydroxide (12.15 g) was dissolved in diethylene glycol (128 ml) and the volatile substance was distilled until the mixture reached 200°C. This solution was allowed to cool to room temperature and 2-[(4-chlorophenyl)hydrazonomethyl]phenylamine (20.25 g) obtained in Preparative Example 106 was added, which was followed by gradual heating to 150°C. The mixture was heated for 90 minutes at said temperature until generation of gases stopped. The solution was cooled to 120°C, poured into ice water and extracted with ethyl acetate. The organic layer was washed with water and dried over anhydrous magnesium sulfate. After filtration, the residue obtained by concentration under reduced pressure was subjected to silica gel column chromatography to give 17 g of the title compound as an oil.

$^1H$ NMR (300MHz, δ ppm, $CDC1_3$)

3.47(2H,s), 3.86(2H,s), 6.66-7.27(8H,m)

Preparative Example 108 (Step 13)

Acetic acid [2-(4-chlorobenzyl)phenylaminomethylene]hydrazide

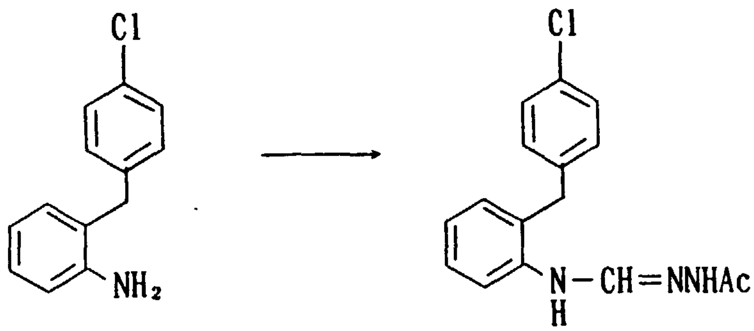

A mixture of 2-(4-chlorobenzyl)phenylamine (17 g) obtained in Preparative Example 107 and triethyl orthoformate (27.9 g) was refluxed under heating for 5 hours. The reaction mixture was allowed to cool to room temperature, and absolute ethanol (160 ml) and acetohydrazide (11.6 g) were added,which was followed by stirring for 13 hours. The precipitated crystals were collected by filtration, washed with hexane-ethanol and dried to give 25 g of the title compound as colorless crystals.

Melting point: 163-165°C

Preparative Example 109 (Step 14)

4-[2-(4-Chlorobenzyl)phenyl]-3-methyl-4H-[1,2,4]triazole

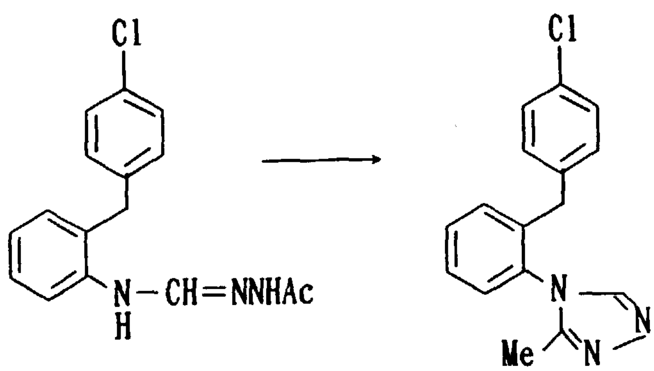

Acetic acid [2-(4-chlorobenzyl)phenylaminomethylene]hydrazide (11.6 g) obtained in Preparative Example 108 was dissolved in diethylene glycol dimethyl ether (250 ml) and the mixture was refluxed under heating for 14 hours. The mixture was cooled to room temperature and the solvent was distilled away under reduced pressure. The resulting crystals were collected by filtration, washed with diethyl ether and dried to give 6 g of the title compound as colorless crystals.

Melting point : 137-139°C

Preparative Example 110 (Step 15)

4-Chlorophenyl 2-(3-methyl[1,2,4]triazol-4-yl)phenyl ketone

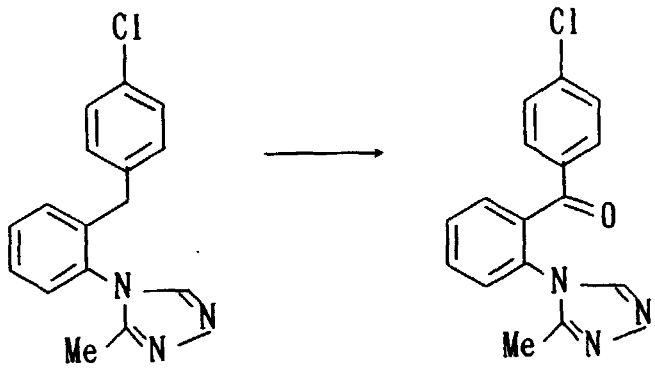

4-[2-(4-Chlorobenzl)phenyl]-3-methyl-4H-[1,2,4]triazole (290 mg) obtained in Preparative Example 109 was dissolved in glacial acetic acid (1 ml) and Jones reagent (1.2 ml) was dropwise added under ice-cooling, which was followed by reflux under heating for 4 hours. The Jones reagent (0.2 ml) was further added and the mixture was refluxed under heating for 2 hours. After the completion of the reaction, it was poured into a 5% aqueous sodium hydroxide solution and extracted with chloroform. The organic layer was dried over anhydrous sodium sulfate. After filtration, the residue obtained by concentration under reduced pressure was subjected to silica gel column chromatography to give 185 mg of the title compound as crystals.

Melting point : 140-141°C

Preparative Example 111 (Step 16)

2-(3-Bromo-5-methyl-[1,2,4]triazol-4-yl)phenyl 4-chlorophenyl ketone

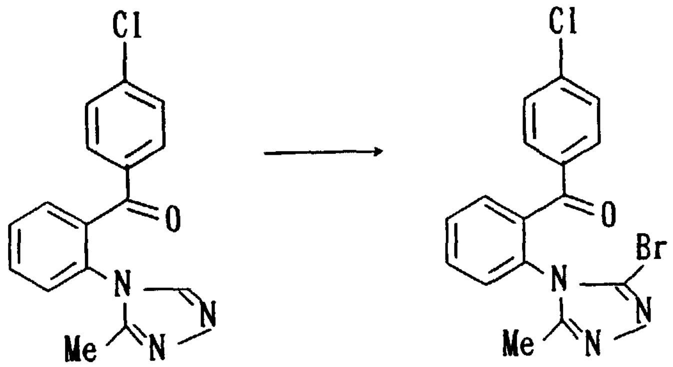

4-Chlorophenyl 2-(3-methyl-[1,2,4]triazol-4-yl)phenyl ketone (165 mg) obtained in Preparative Example 110

was dissolved in carbon tetrachloride and N-bromosuccinimide (110 mg) was added, which was followed by reflux under heating under a nitrogen atmosphere for 3 hours. After the completion of the reaction, the mixture was allowed to cool to room temperature and chloroform was added to dissolve the insoluble oil, followed by washing with water. The organic layer washed was dried over anhydrous magnesium sulfate. After filtration, the residue obtained by concentration under reduced pressure was subjected to silica gel column chromatography to give 65 mg of the title compound as crystals.

Melting point : 177-179°C

Preparative Example 112 (Step 17)

6-(4-Chlorophenyl)-1-methyl-4H-2,3,4,5,10b-pentaazabenz[e]azulene

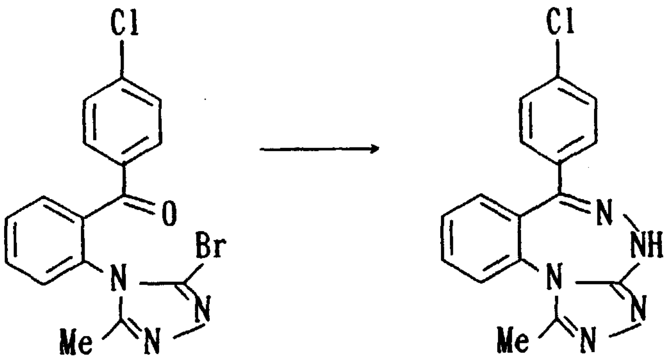

2-(3-Bromo-5-methyl-[1,2,4]triazol-4-yl)phenyl 4-chlorophenyl ketone (1.55 g) obtained in Preparative Example 111 was dissolved in absolute ethanol (30 ml) and hydrazine sulfate (1.95 g) and sodium acetate (2.87 g) were added, which was followed by reflux under heating under a nitrogen atmosphere for 160 hours. After the completion of the reaction, the mixture was allowed to cool to room temperature. The reaction mixture was concentrated under reduced pressure and an aqueous solution of 5% sodium hydrogencarbonate was added, which was followed by extraction with ethyl acetate. The organic layer was washed with saturated aqueous sodium chloride solution, dried over anhydrous magnesium sulfate and filtrated. The residue obtained by concentration under reduced pressure was subjected to silica gel column chromatography. The resulting product was crystallized from diethyl ether to give 670 mg of the title compound as needle crystals.

Melting point : 231-233°C

Preparative Example 113 (Step 18)

2-[(4-Chlorophenyl)carbonmethoxhydrazonmethyl]phenylamine

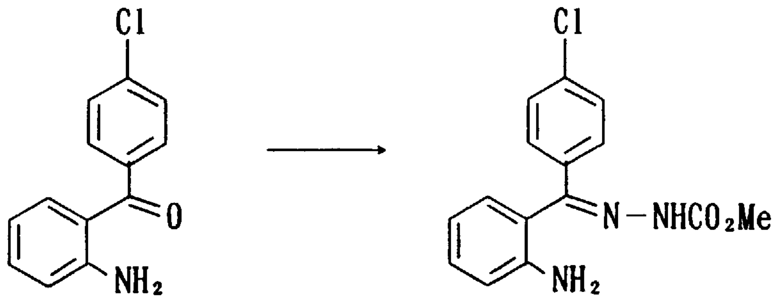

2-Aminophenyl 4-chlorophenyl ketone (25 g) was dissolved in ethanol (300 ml) and methyl carbazate (19.44 g) and p-toluenesulfonic acid monohydrate (6.16 g) were added, which was followed by reflux under heating for 12 hours. The reaction mixture was allowed to cool to room temperature, and the resulting crystals were collected by filtration and washed with ethanol to give 32 g of the title compound as white crystals.

Melting point : 217-218°C

Preparative Example 114 (Step 19)

5-(4-Chlorophenyl)-1,3-dihydrobenzo[e][1,2,4]triazepin-2-one

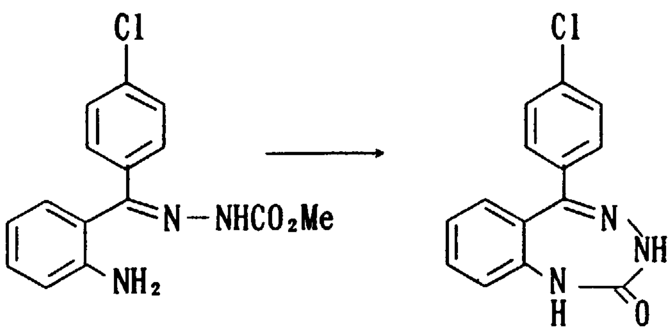

2-[(4-Chlorophenyl)carbomethoxyhydrazonomethyl]phenylamine (32 g) obtained in Preparative Example 113 was dissolved in dimethyl sulfoxide (100ml) and the mixture was heated at 180°C for 1.5 hours. The reaction mixture was cooled to room temperature and poured into water (1 L). The resulting crystals were collected by filtration and washed with water to give 26 g of the title compound as yellow crystals.

Melting point : 257-259°C

Preparative Example 115 (Step 18')

5-(4-Chlorophenyl)-1,3-dihydrobenzo[e][1,2,4]triazepin-2-one

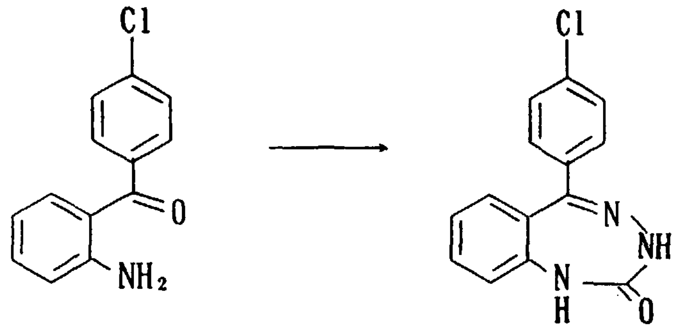

2-Aminophenyl 4-chlorophenyl ketone (25 g) was dissolved in dimethyl sulfoxide (100 ml) and methyl carbazate (22.4 g) was added, which was followed by heating with stirring at 180°C for 18 hours. The reaction mixture was cooled to room temperature and poured into water (3 L). The resulting crystals were collected by filtration and washed with water to give 24 g of the title compound as yellow crystals.

The melting point of this compound was identical with that obtained in Preparative Example 95.

Preparative Example 116 (Step 20 )

5-(4-Chlorophenyl)-1-methoxymethyl-1,3-dihydrobenzo[e][1,2,4]triazepin-2-one

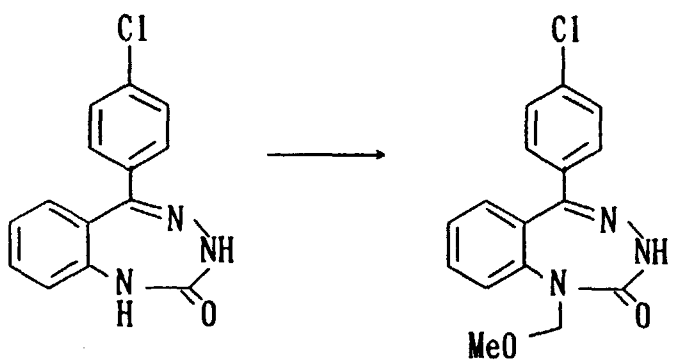

5-(4-Chlorophenyl)-1,3-dihydrobenzo[e][1,2,4]triazepin-2-one (1 g) obtained in Preparative Example 114 or Preparative Example 115 was suspended in N,N-dimethylformamide (20 ml) and potassium hydroxide (413 mg) was added, which was followed by stirring at room temperature for 15 minutes. Chloromethyl methyl ether (419 μl) was added to the reaction mixture and the mixture was stirred at room temperature for 30 minutes. The reaction mixture was poured into water (50 ml) and extracted with ethyl acetate. The organic layer was washed with water and dried over anhydrous magnesium sulfate. After filtration, the residue obtained by concentration under reduced pressure was crystallized from ethyl acetate-diethyl ether to give 350 mg of the title compound as yellow crystals.

Melting point : 177-180°C

Preparative Example 117 (Step 21)

3-Benzyl-5-(4-chlorophenyl)-1-methoxymethyl-1,3-dihydrobenzo[e][1,2,4]triazepin-2-one

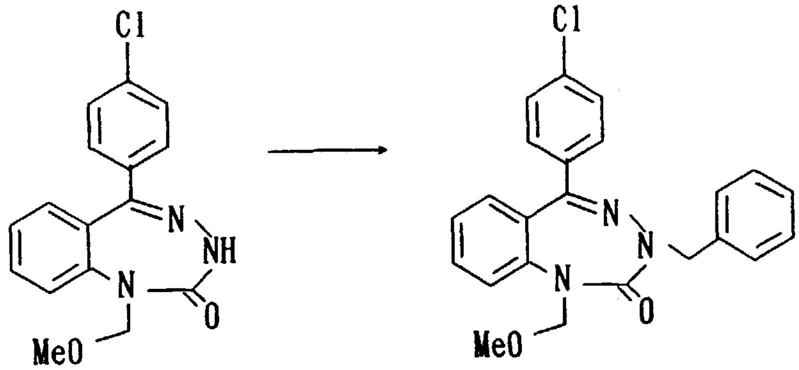

5-(4-Chlorophenyl)-1-methoxymethyl-1,3-dihydrobenzo[e][1,2,4]triazepin-2-one (350 mg) obtained in Preparative Example 116 was dissolved in dimethyl sulfoxide (3.5 ml) and sodium hydride (60% in oil, 53.2 mg) was added, which was followed by stirring at room temperature for 30 minutes. Benzyl bromide (158 μl) was added and the mixture was stirred for 1 hour. The reaction mixture was poured into water, neutralized with glacial acetic acid and extracted with ethyl acetate. The organic layer was dried over anhydrous sodium sulfate. After filtration, the residue obtained by concentration under reduced pressure was subjected to silica gel column chromatography to give 374 mg of the title compound as an oil.

$^1$H NMR (300MHz, δ ppm, $CDC1_3$)
3.47(3H,s), 4.62-5.17(4H,m), 7.04-7.57(13H,m)

Preparative Example 118 (Step 22)

3-Benzyl-5-(4-chlorophenyl)-1,3-dihydrobenzo[e][1,2,4]triazepin-2-one

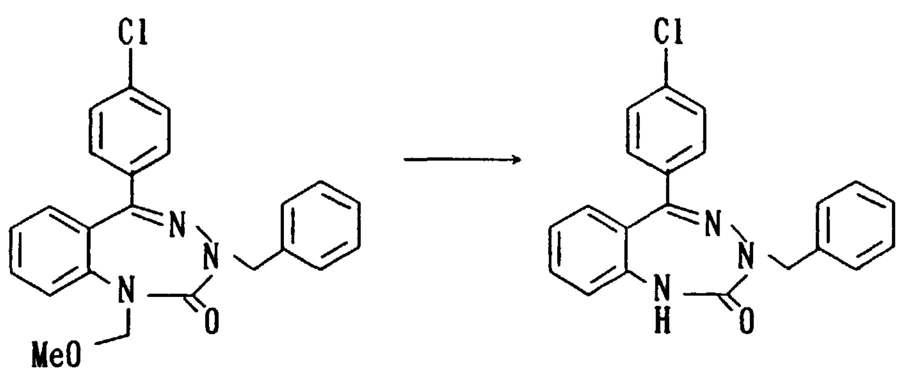

triazepin-2-one (370 mg) obtained in Preparative Example 117 was dissolved in ethanol (1 ml) and 5N hydrochloric acid (2 ml) was added, which was followed by reflux under heating for 3 hours. The reaction mixture was cooled to room temperature, added with water and extracted with ethyl acetate. The extract was washed successively with a 5% aqueous sodium hydrogencarbonate solution and water and dried over anhydrous magnesium sulfate. After filtration, the residue

obtained by concentration under reduced pressure was washed with diethyl ether to give 260 mg of the title compound as white crystals.

Melting point : 200-202°C

Preparative Example 119 (Step 23)

3-Benzyl-5-(4-chlorophenyl)-1,3-dihydrobenzo[e][1,2,4]triazepine-2-thione

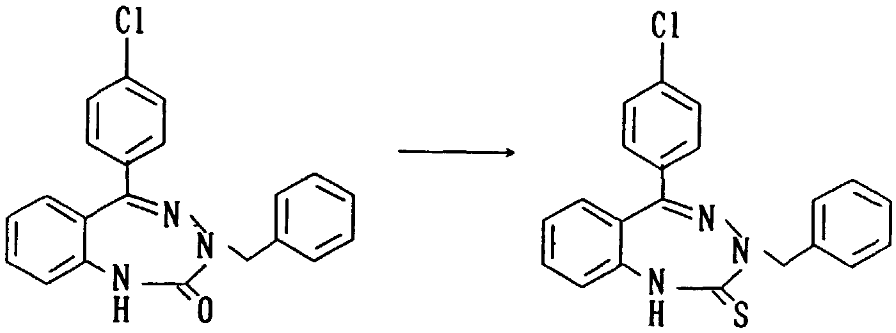

3-Benzyl-5-(4-chlorophenyl)-1,3-dihydrobenzo[e][1,2,4]triazepin-2-one (100 mg) obtained in Preparative Example 118 was suspended in diethylene glycol dimethyl ether (2 ml) and diphosphorus pentasulfide (74 mg) and sodium hydrogencarbonate (74 mg) were added, which was followed by stirring with heating at 100°C for 22 hours. Diphosphorus pentasulfide (64 mg) was added and the mixture was stirred with heating at 100°C for 24 hours. The reaction mixture was cooled to room temperature and poured into a 5% aqueous sodium hydrogencarbonate solution. The mixture was extracted with ethyl acetate. The organic layer was washed with a saturated aqueous sodium chloride solution and dried over anhydrous magnesium sulfate. After filtration, the residue obtained by concentration under reduced pressure was purified by preparative thin-layer chromatography to give 60 mg of the title compound as yellow crystals.

Melting point : 159-160°C

Preparative Example 120 (Step 4)

3-Benzyl-5-(4-chlorophenyl)-3H-benzo[e][1,2,4]triazepin-2-ylhydrazine

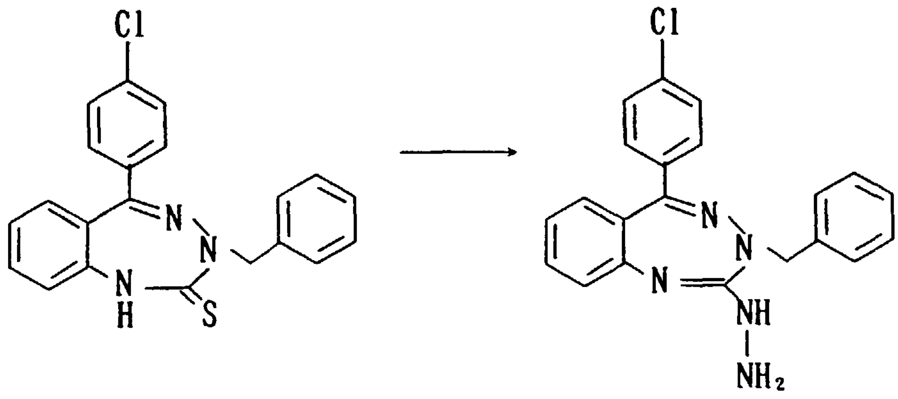

3-Benzyl-5-(4-chlorophenyl)-1,3-dihydrobenzo[e][1,2,4]triazepine-2-thione (40 mg) obtained in Preparative Example 119 was dissolved in anhydrous tetrahydrofuran (0.4 ml) and 100% hydrazine hydrate (10 μl) was added, which was followed by stirring at room temperature for 4 hours. The reaction mixture was heated to 40°C and stirred for 4 hours. The mixture was cooled to room temperature and poured into a saturated aqueous sodium chloride solution. The organic layer was extracted with ethyl acetate and dried over anhydrous magnesium sulfate. After filtration, the residue obtained by concentration under reduced pressure was purified by preparative thin-layer chromatography to give 17 mg of the title compound as a yellow powder.

$^1H$ NMR (300MHz, δ ppm, $CDC1_3$)
4.79(2H,s), 6.99-7.50(16H,m)

Synthetic Example 188 (Step 5)

4-Benzyl-6-(4-chlorophenyl)-1-methyl-4H-2,3,4,5,10b-pentaazabenz[e]azulene

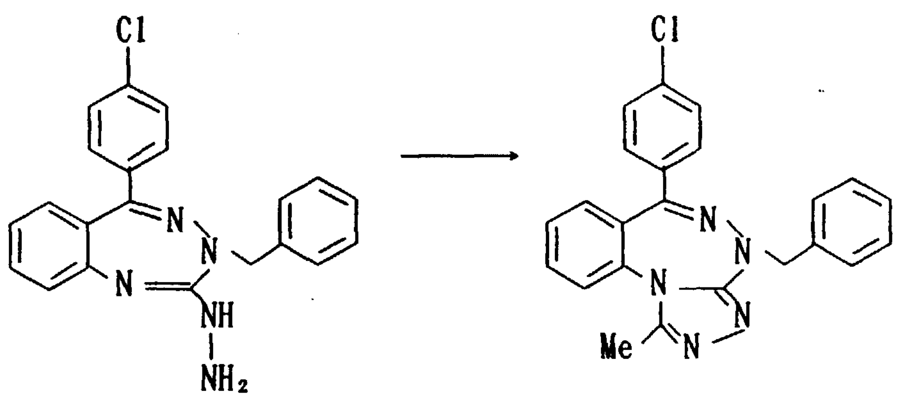

3-Benzyl-5-(4-chlorophenyl)-3H-benzo[e][1,2,4]triazepin-2-ylhydrazine (14 mg) obtained in Preparative Example 120 was dissolved in toluene (1 ml) and ethyl orthoacetate (14 μl) was added, which was followed by reflux under heating for 2 days. The reaction mixture was cooled to room temperature, poured into water(10 ml) and extracted with

ethyl acetate. The organic layer was washed with a saturated aqueous sodium chloride solution and dried over anhydrous sodium sulfate. After filtration, the residue obtained by concentration under reduced pressure was purified by preparative thin-layer chromatography to give 5 mg of the title compound as white crystals.

Melting point : 234-238°C

Preparative Example 121 (Step 26)

5-(4-Chlorophenyl)-2-(2,2-dimethoxyethylamino)-3-(4-methoxybenzyl)-3H-benzo[e][1,2,4]triazepine

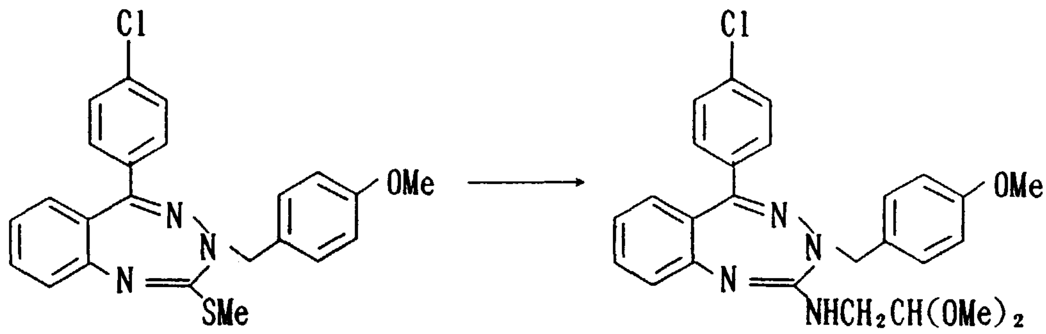

5-(4-Chlorophenyl)-3-(4-methoxybenzyl)-2-methylthio-3H-benzo[e][1,2,4]triazepine (300 mg) obtained in Preparative Example 84 was dissolved in 2-ethoxyethanol (1 ml) and aminoacetaldehyde dimethyl acetal (85.2 μl) and p-toluenesulfonic acid (14 mg) were added. The reaction mixture was heated from 80°C to refluxing temperature over 3 hours and refluxed under heating for 1 hour. After the completion of the reaction, the mixture was allowed to cool to room temperature and added with water (20 ml), followed by extraction with ethyl acetate (30 ml). The organic layer was washed successively with water, a 5% sodium hydrogencarbonate solution and water and dried over anhydrous sodium sulfate. After filtration, the residue obtained by concentration under reduced pressure was crystallized from n-hexane:diethyl ether (5:1) solvent to give 160 mg of the title compound as white crystals.

Melting point : 114-115°C

Synthetic Example 189 (Step 27)

6-(4-Chlorophenyl)-4-(4-methoxbenzyl)-4H-3,4,5,10b-tetraazabenz[e]azulene

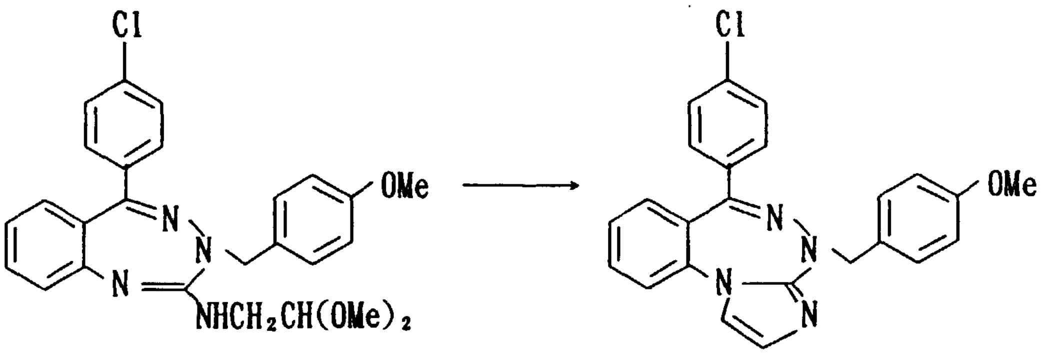

5-(4-Chlorophenyl)-2-(2,2-dimethoxyethylamino)-3-(4-methoxybenzyl)-3H-benzo[e][1,2,4]triazepine (100 mg) obtained in Preparative Example 121 was dissolved in a solution of 1N hydrochloric acid-acetic acid and refluxed under heating for 7 hours. The reaction mixture was allowed to cool and concentrated under reduced pressure. Water (20 ml) was added to the residue and the mixture was extracted with ethyl acetate (20 ml). The organic layer was washed successively with a saturated aqueous sodium hydrogencarbonate solution and water, and dried over anhydrous sodium sulfate. After filtration, the residue obtained by concentration under reduced pressure was purified by preparative thin-layer chromatography to give 32 mg of the title compound as an amorphous solid.

$^1H$ NMR (300MHz, δ ppm, $CDC1_3$)
3.79(3H,s), 4.92(2H,s), 6.83-6.87(2H,m), 6.97(1H,d,J=1.8Hz), 7.06-7.41(9H,m), 7.51-7.57(1H,m)

Example 1 (Step 10-17)

3-[6-(4-Chlorophenyl)-1-methyl-4H-2,3,4,5,10b-pentaazabenz[e]azulen-4-ylmethyl]pyridine-1-oxide

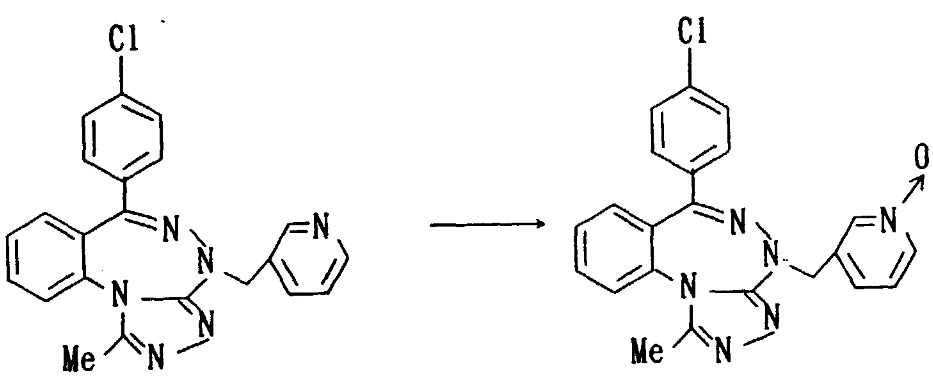

6-(4-Chlorophenyl)-1-methyl-4-(pyridin-3-ylmethyl)-4H-2,3,4,5,10b-pentaazabenz[e]azulene (20.06 g) obtained in Synthetic Example 63 was dissolved in anhydrous dichloromethane (300ml) and m-chloroperbenzoic acid (9.07g) was added under ice-cooling. The mixture was stirred at room temperature for one hour. The reaction mixture was ice-cooled and m-chloroperbenzoic acid was added in two portions (1.5 g and 0.8 g). The mixture was stirred a

room temperature for one hour. The solvent was evaporated under reduced pressure, and the obtained residue was extracted with ethyl acetate. The organic layer was washed successively with water, saturated aqueous sodium hydrogencarbonate solution and water, and dried over anhydrous sodium sulfate. The drying agent was filtered off and the solvent was evaporated under reduced pressure. The obtained residue was subjected to silica gel column chromatography. The residue obtained from the fraction eluted with chloroform:methanol=50:1- 20:1 was dissolved in ethanol (50 ml) and diethyl ether (50 ml × 2) was added gradually to allow crystallization, whereby 7.94 g of the title compound was obtained as colorless needles.

Melting point : 239-240°C
$^1$H NMR (300MHz, δ ppm, $CDC1_3$)
2.62(3H,s), 4.91(1H,m), 5.11(1H,m), 7.20-7.44(9H,m), 7.67(1H,m), 8.19(1H,d,J=6.3Hz), 8.23(1H,s)

Examples 2-5 (Step 10-17)

In the same manner as in Example 1, compounds of Examples 2, 3, 4 and 5 were obtained from the compounds of Synthetic Examples 105, 114, 118 and 121, respectively, wherein the compounds of Examples 2, 3 and 4 were crystallized from ethyl acetate/diethyl ether. The compounds are shown in Table 58.

Example 2

3-[8-Chloro-6-(2-chlorophenyl)-1-methyl-4H-2,3,4,5,10b-pentaazabenz[e]azulen-4-ylmethyl]-pyridine-1-oxide

Example 3

4-[4-(4-Chlorophenyl)-2,3,9-trimethyl-6H-1-thia-5,6,7,8,9a-pentaazacyclopent[e]azulen-6-ylmethyl]-pyridine-1-oxide

Example 4

4-[4-(4-Chlorophenyl)-2-ethyl-9-methyl-6H-1-thia-5,6,7,8,9a-pentaazacyclopent[e]azulen-6-ylmethyl]-pyridine-1-oxide

Example 5

4-[2,9-Dimethyl-4-phenyl-6H-1-thia-5,6,7,8,9a-pentaazacyclopent[e]azulen-6-ylmethyl]-pyridine-1-oxide

Table 58

| Ex. | Structural formula | $^1$H NMR (300MHz, δppm, $CDCl_3$) | Melting point (℃) |
|---|---|---|---|
| 2 | 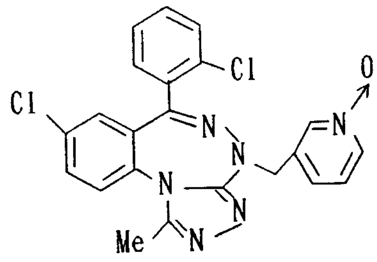 | 2.57(3H, s), 5.00(2H, m), 6.97 (1H, d, J=2.7Hz), 7.22-7.44(7H, m), 7.58 (1H, dd, J=9.0 and 2.4Hz), 8.13 (1H, d, J=6.0Hz), 8.26(1H, s). | 196-197 |
| 3 | 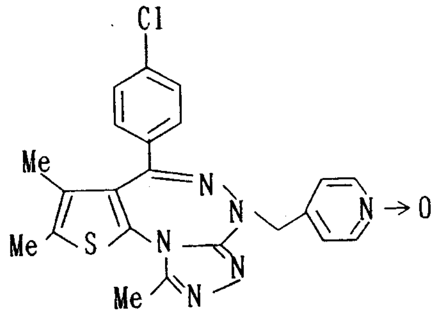 | 1.57(3H, s), 2.40(3H, s), 2.65(3H, s), 4.93(1H, m), 5.10(1H, m), 7.23-7.43(3H, m), 7.53(1H, m), 7.96(1H, m), 8.06(1H, m), 8.33 (2H, d, J=6.9Hz). | 159-162 |
| 4 | 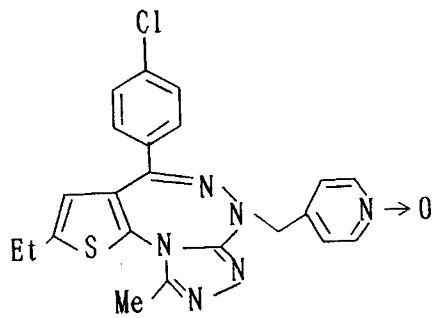 | 1.35 (3H, t, J=7.5Hz), 2.86 (2H, q, J=7.5Hz), 5.03(2H, s), 6.48(1H, s), 7.26-7.54(4H, m), 7.96 (1H, dd, J=7.7 and 1.1Hz), 8.06 (1H, d, J=1.6Hz), 8.33 (2H, d, J=7.0Hz). | 147-150 |
| 5 | 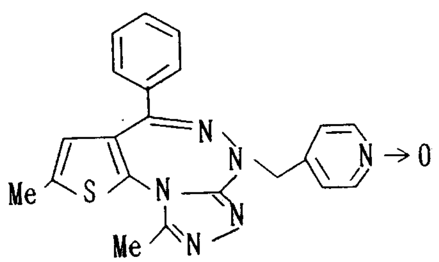 | 2.50(3H, s), 2.63(3H, s), 6.47(1H, s), 7.32-7.44(7H, m), 8.20 (2H, d, J=4.8Hz). | Amorphous solid |

Preparative Example 122 (Step 9)

4-(1-Methyl-4H-2,3,4,5,10b-pentaazabenz[e]azulen-6-yl)benzoic acid

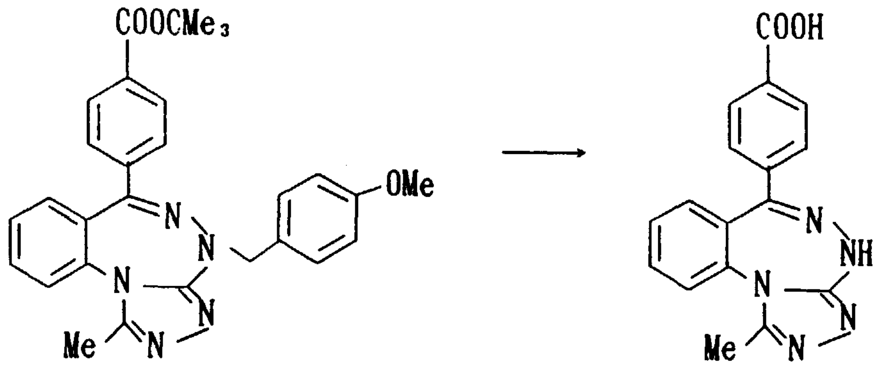

A solution of tert-butyl 4-[4-(4-methoxybenzyl)-1-methyl-4H-2,3,4,5,10b-pentaazabenz[e]azulene-6-yl]benzoate (100 mg) obtained in Synthetic Example 11 in methanesulfonic acid (223 μl) was stirred at room temperature for 3 days and at 70°C for 3 hours. The reaction mixture was allowed to cool and water (2 ml) was added, which was followed by vigorous stirring for 1 hour. The resulting precipitate was collected by filtration, and washed with water and diethyl ether and dried under reduced pressure to give 60 mg of the title compound as a colorless amorphous solid.

$^1$H NMR (300MHz δ ppm, DMSO-$d_6$)
2.56(3H,s), 7.15(1H,d,J=7.5Hz), 7.45(1H,t,J=7.1Hz), 7.63(2H,d,J=8.1Hz), 7.68-7.72(2H,m), 7.90(2H,d,J=8.1Hz), 10.1(1H,s)

Preparative Example 123 (Step 9)

Methyl 4-(1-methyl-4H-2,3,4,5,10b-pentaazabenz[e]azulen-6-yl]benzoate

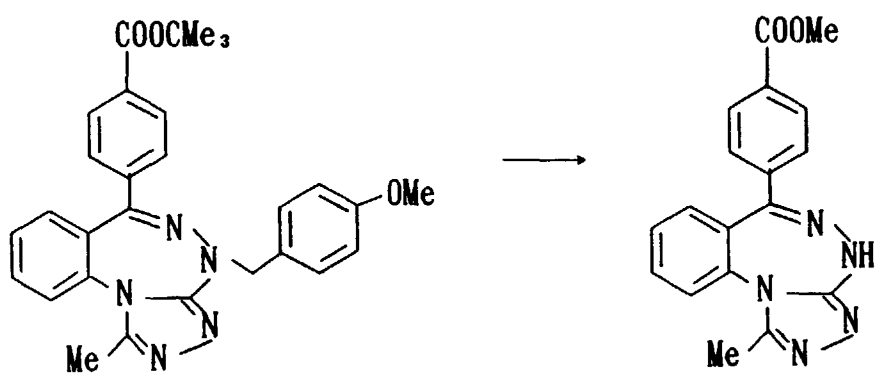

A solution of tert-butyl 4-[4-(4-methoxybenzyl)-1-methyl-4H-2,3,4,5,10b-pentaazabenz[e]azulen-6-yl]benzoate (300 mg) obtained in Synthetic Example 11 in methanesulfonic acid (0.6 ml) was stirred at 70°C for one hour. Methanol (1 ml) was added to the reaction mixture and the mixture was refluxed under heating for 1 hour. After cooling, saturated aqueous sodium hydrogencarbonate solution (25 ml) and chloroform (25 ml) were added to the reaction mixture. The separated chloroform layer was washed successively with saturated aqueous sodium hydrogencarbonate solution and

saturated brine, and dried. This solution was evaporated under reduced pressure to give a residue, which was crystallized from chloroform-diethyl ether to give 134 mg of the title compound as colorless crystals.

Melting point : 245.5-247°C

Example 6 (Step 10-1)

Methyl 4-[4-(4-chlorobenzyl)-1-methyl-4H-2,3,4,5,10b-pentaazabenz[e]azulen-6-yl]benzoate

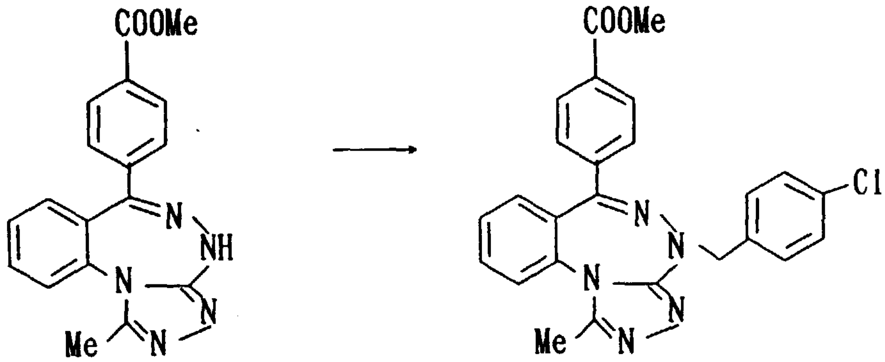

Methyl 4-(1-methyl-4H-2,3,4,5,10b-pentaazabenz[e]azulen-6-yl]benzoate obtained in Preparative Example 123 was reacted in the same manner as in Synthetic Example 86 to give the title compound.

Melting point : 186-187.5°C

Example 7 (Step 10-1)

Methyl 4-[4-(4-cyanobenzyl)-1-methyl-4H-2,3,4,5,10b-pentaazabenz[e]azulen-6-yl]benzoate

In the same manner as in Example 6, the title compound was obtained from the compound of Preparative Example 123. This compound is shown in Table 59.

**Table 59**

| Ex. | Structural formula | Melting point (°C) |
|---|---|---|
| 7 | 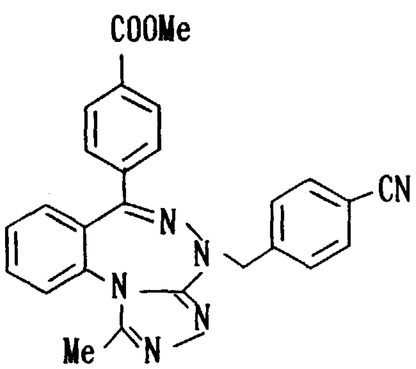 | 239.5-241 |

Example 8 (Step 10-1)

Methyl 4-[1-methyl-4-(4-nitrobenzyl)-4H-2,3,4,5,10b-pentaazabenz[e]azulen-6-yl]benzoate

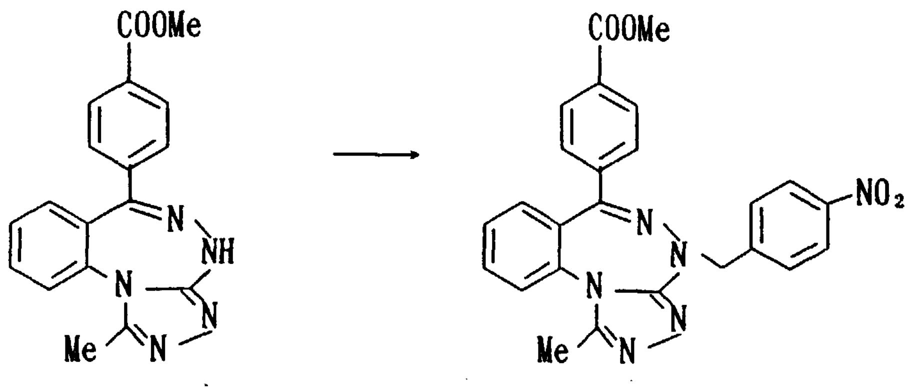

Methyl 4-(1-methyl-4H-2,3,4,5,10b-pentaazabenz[e]azulen-6-yl]benzoate (1.20 g) obtained in Preparative Example 123 was dissolved in N,N-dimethylformamide (20 ml) and ice-cooled. Thereto was added lithium bis(trimethylsilyl)amide 1M tetrahydrofuran solution (3.78 ml) and the mixture was stirred at 0°C for 40 minutes under an argon atmosphere. Then, p-nitrobenzyl bromide (855 mg) was added and the mixture was stirred at 0°C for 2.5 hours under an argon atmosphere. The solution was partitioned between ethyl acetate and aqueous 5% potassium hydrogensulfate solution. The organic layer was washed successively with saturated aqueous sodium hydrogencarbonate solution and brine, dried and concentrated. The residue was recrystallized from ethyl acetate/diethyl ether to give 1.22 g of the title compound as pale-yellow crystals.

Melting point : 205-206°C
$^1H$ NMR (300MHz, δ ppm, $CDCl_3$)
2.64(3H,s), 4.98-5.18(1H,m), 5.18-5.34(1H,m), 7.20-7.24(1H,m), 7.35-7.50(4H,m), 7.56(2H,d,J=8.7Hz), 7.65-7.75(1H,m), 8.00(2H,d,J=8.5Hz), 8.18(2H,d,J=8.8Hz)

Synthetic Example 190 (Step 10-1)

6-(4-Chlorophenyl)-1-methyl-4-(4-nitrobenzyl)-4H-2,3,4,5,10,10b-hexaazabenz[e]azulene

In the same manner as in Example 8, the title compound was obtained from the compound of Preparative Example 97. This compound is shown in Table 60.

**Table 60**

| Syn. Ex. | Structural formula | Melting point (°C) |
|---|---|---|
| 190 | 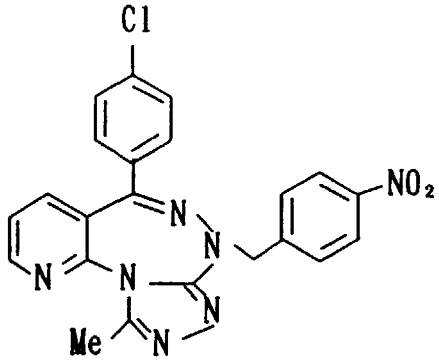 | 224-226 |

Example 9 (Step 10-18)

[4-(4-Chlorobenzyl)-1-methyl-4H-2,3,4,5,10b-pentaazabenz[e]azulen-6-yl]benzoic acid

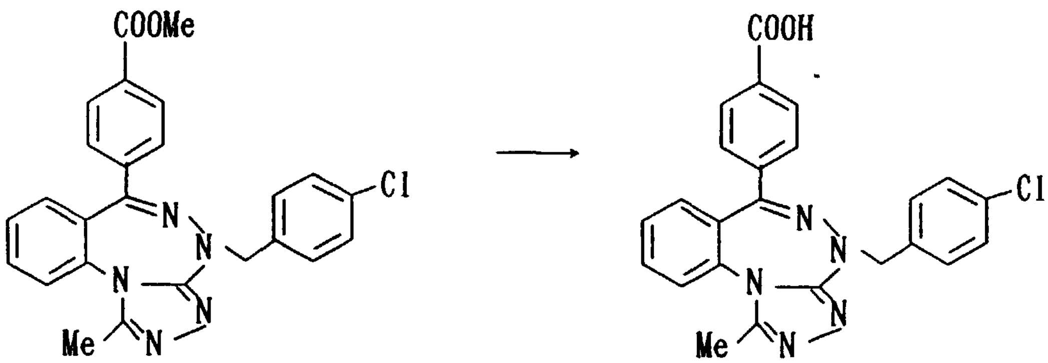

In the same manner as in Protective Groups in Organic Synthesis (John Wiley & Sons Inc. (1991)), 1N aqueous sodium hydroxide solution (9.8 ml) was added to a suspension of methyl 4-[4-(4-chlorobenzyl)-1-methyl-4H-2,3,4,5,10b-pentaazabenz[e]azulen-6-yl]benzoate (2.24 g) obtained Example 6 in methanol (45 ml). The mixture was stirred at 50°C for 1.5 hours. The reaction mixture was allowed to cool and the solvent was evaporated under reduced pressure. Diethyl ether and water were added to the residue and the aqueous layer was separated. The obtained aqueous layer was acidified (pH=2) with aqueous 5% potassium hydrogensulfate solution (50 ml) and the precipitate was extracted twice with chloroform. This chloroform layer was washed 3 times with half-saturated brine and dried. The solvent was evaporated under reduced pressure and the obtained residue was crystallized from chloroform-diethyl ether to give 2.09 g of the title compound as colorless crystals.

Melting point : 247-248°C

Example 10 (Step 10-18)

[4-(4-Cyanobenzyl)-1-methyl-4H-2,3,4,5,10b-pentaazabenz[e]azulene-6-yl]benzoic acid

In the sane manner as in Example 9, the title compound was obtained from the compound of Example 7. This compound is shown in Table 61.

**Table 61**

| Syn. Ex. | Structural formula | Melting point (°C) |
|---|---|---|
| 10 | 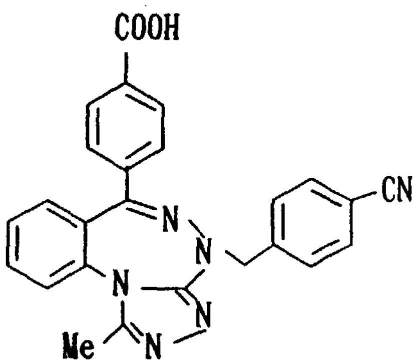 | 190-192 |

Example 11 (Step 10-18)

4-[1-Methyl-4-(4-nitrobenzyl)-4H-2,3,4,5,10b-pentaazabenz[e]azulen-6-yl]benzoic acid

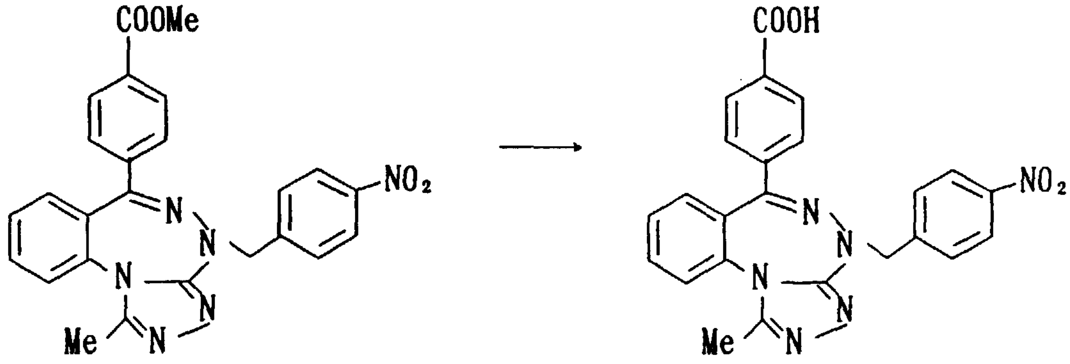

In the same manner as in Protective Groups in Organic Synthesis (John Wiley & Sons Inc. (1991)), methyl 4-[1-methyl-4-(4-nitrobenzyl)-4H-2,3,4,5,10b-pentaazabenz[e]azulen-6-yl]benzoate (97.1 mg) obtained in Example 8 was dissolved in 1,4-dioxane (0.5 ml), and conc. hydrochloric acid (0.8 ml) was added, which was followed by heating at 100°C for 7 hours. The solvent was evaporated under reduced pressure, and aqueous 1N sodium hydroxide solution was added to the residue to give a basic aqueous solution, which was washed with ethyl acetate. The basic solution

was acidified with 1N hydrochloric acid to give an insoluble solid. This solid was recrystallized from methanol/ethyl acetate/diethyl ether to give 79.4 mg of the title compound as pale-yellow crystals.

Melting point : 290°C
$^1$H NMR(300MHz,δ ppm, DMSO-$d_6$)
2.54(3H,s), 4.97(1H,d,J=15.6Hz), 5.17(1H,d,J=15.6Hz), 7.19(1H,d,J=7.8Hz), 7.40-7.60(3H,m), 7.64(2H,d,J=8.4Hz), 7.70-7.80(2H,m), 7.94(2H,d,J=8.1Hz), 8.19(2H,d,J=8.7Hz), 13.10(1H,s)

Example 12 (Step 10-19)

tert-Butyl 4-[4-(4-chlorobenzyl)-1-methyl-4H-2,3,4,5,10b-pentaazabenz[e]azulene-6-yl]phenylcarbamate

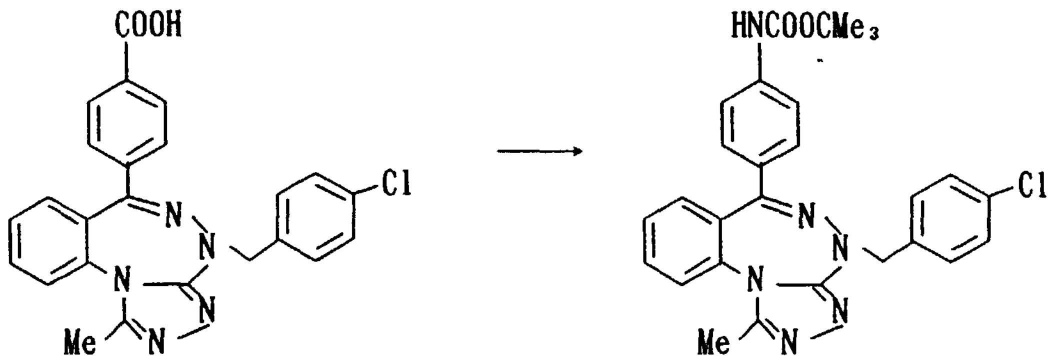

In the same manner as in T. Shioiri et al. [Journal of American Chemical Society, 94, 6203 (1972)], a solution of [4-(4-chlorobenzyl)-1-methyl-4H-2,3,4,5,10b-pentaazabenz[e]azulen-6-yl]benzoic acid (350 mg) obtained in Example 9, diphenylphosphoryl azide (DPPA, 0.18 ml) and triethylamine (0.12 ml) m tert-butyl alcohol (7 ml) was refluxed under heating for 18 hours. The reaction mixture was allowed to cool and the solvent was evaporated under reduced pressure. An aqueous 5% potassium hydogensulfate solution and ethyl acetate were added to the obtained residue, and the organic layer was separated. The organic layer was washed with saturated aqueous sodium hydrogencarbonate solution and water, and dried. This was concentrated under reduced pressure, and the obtained residue was purified by silica gel column chromatography (chloroform:methanol=50:1). The fraction containing the objective compound was concentrated under reduced pressure to give 395 mg of the title compound as a pale-yellow amorphous solid.

$^1$H NMR (300MHz δ ppm, $CDC1_3$)
1.52(9H,s), 2.61(3H,s), 4,86-5.09(2H,m), 6.63(1H,s), 7.24-7.38(11H,m), 7.59-7.62(1H,m)

Example 13, 14 (Step 10-19)

In the same manner as in Example 12, the compound of Example 13 was obtained from the compound of Example 10 and the compound of Example 14 was obtained from the compound of Example 11. These compounds are shown in Table 62.

Example 13

tert-Butyl 4-[4-(4-cyanobenzyl)-1-methyl-4H-2,3,4,5,10b-pentaazabenz[e]azulen-6-yl]phenylcarbamate

Example 14

tert-Butyl 4-[1 methyl-4-(4-nitrobenzyl)-4H-2,3,4,5,10b-pentaazabenz[e]azulen-6-yl]phenylcarbamate

**Table 62**

| Ex. | Structural formula | $^1$H NMR (300MHz, δppm, $CDCl_3$) | Melting point (°C) |
|---|---|---|---|
| 13 | 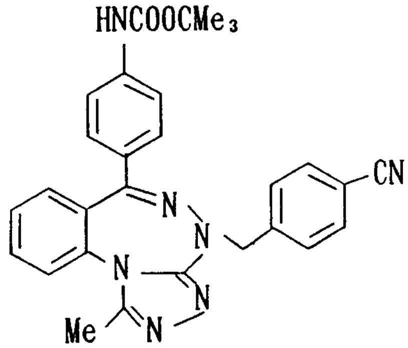 | 1.52(9H, s), 2.62(3H, s), 4.95-5.19(2H, m), 6.59(1H, s), 7.27-7.40(7H, m), 7.48 (2H, d, J=8.4Hz), 7.59 (2H, d, J=8.7Hz), 7.62-7.67(1H, m) | Amorphous solid |
| 14 | 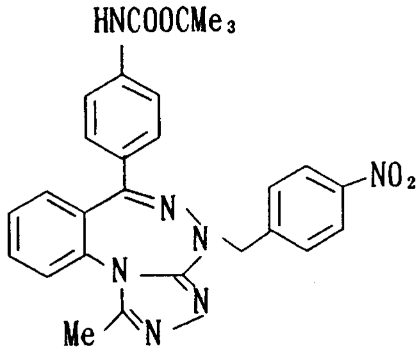 | 1.51(9H, s), 2.63(3H, s), 5.03 (1H, d, J=14.0Hz), 5.15 (1H, d, J=14.0Hz), 6.61(1H, s), 7.20-7.42(7H, m), 7.54 (2H, d, J=8.7Hz), 7.60-7.69(1H, m), 8.16 (2H, d, J=8.7Hz) | |

Synthetic Example 191 (Step 10-20)

4-[4-(4-Chlorobenzyl)-1-methyl-4H-2,3,4,5,10b-pentaazabenz[e]azulen-6-yl]phenylamine hydrochloride

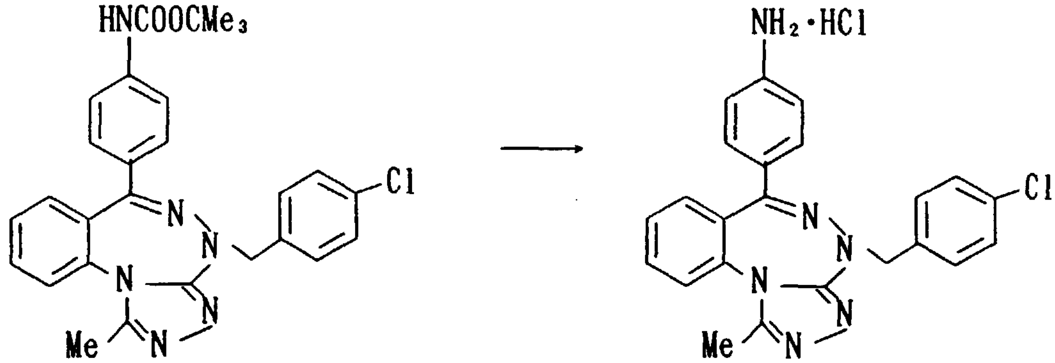

In the same manner as in Protective Groups in Organic Synthesis (John Wiley & Sons Inc. (1991)), tert-butyl 4-[4-(4-chlorobenzyl)-1-methyl-4H-2,3,4,5,10b-pentaazabenz[e]azulen-6-yl]phenylcarbamate (100 mg) obtained in Example 12 was dissolved in 4N hydrochloric acid/ 1,4-dioxane solution (1.5 ml) and the mixture was stirred at room temperature for 1 hour. The solvent was evaporated under reduced pressure, and the obtained residue was crystallized from ethanol to give 62 mg of the title compound as pale-yellow crystals.

Melting point : 183-185°C

Synthetic Example 192 (Step 10-20)

4-[4-(4-Cyanobenzyl)-1-methyl-4H-2,3,4,5,10b-pentaazabenz[e]azulen-6-yl]phenylamine hydrochloride

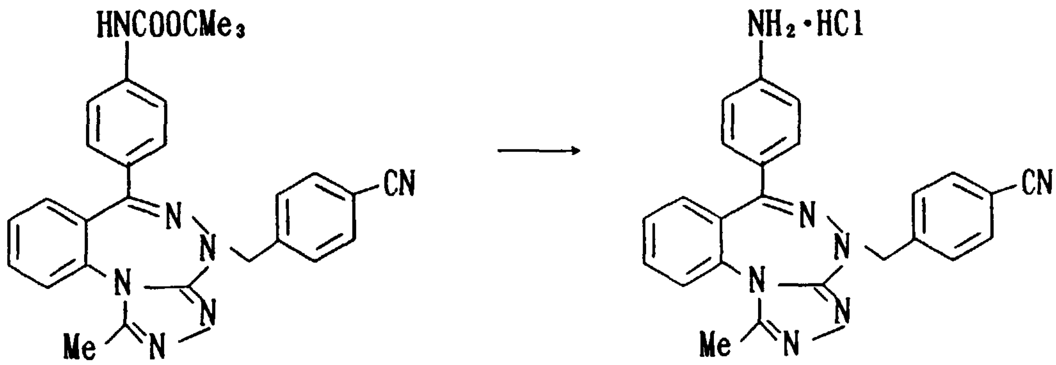

tert-Butyl 4-[4-(4-cyanobenzyl)-1-methyl-4H-2,3,4,5,10b-pentaazabenz[e]azulen-6-yl]phenylcarbamate (73 mg) obtained in Example 13 was dissolved in 4N hydrochloric acid/ 1,4-dioxane solution and the mixture was stirred at room temperature for 2 hours. The solvent was evaporated under reduced pressure, and the obtained residue was crystallized from a mixed solvent of isopropanol / ethanol to give 55 mg of the title compound as pale-yellow crystals.

Melting point : 180-190°C (decomposition)
$^1$H NMR (300MHz, $\delta$ ppm, DMSO-$d_6$)

2.61(3H,s), 4.86-5.10(2H,m), 7.04(2H,d,J=8.7Hz), 7.26-7.33(3H,m), 7.50-7.55(3H,m), 7.74-7.81(4H,m)

Synthetic Example 193 (Step 10-20)

4-[1-Methyl-4-(4-nitrobenzyl)-4H-2,3,4,5,10b-pentaazabenz[e]azulen-6-yl]phenylamine hydrochloride

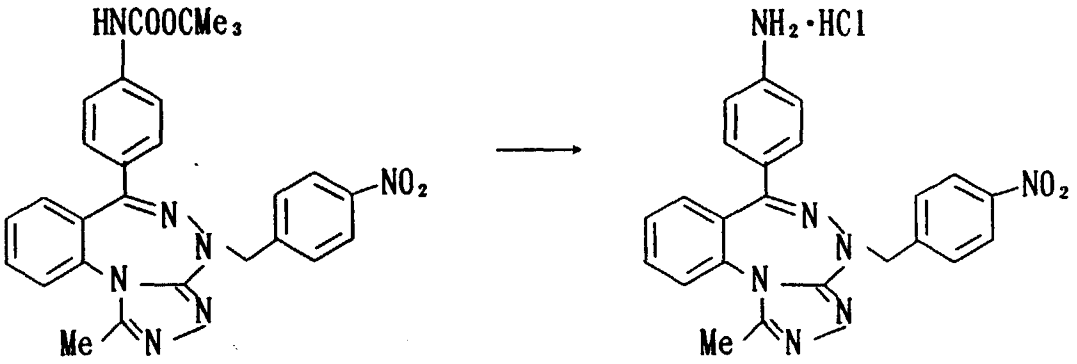

tert-Butyl 4-[1-methyl-4-(4-nitrobenzyl)-4H-2,3,4,5,10b-pentaazabenz[e]azulene-6-yl]phenylcarbamate (88.5 mg) obtained in Example 14 was dissolved in 4N hydrochloric acid /1,4-dioxane solution (3.0 ml) and the mixture was stirred at room temperature for 2.5 hours. The solvent was evaporated under reduced pressure, and the obtained residue was recrystallized from methanol/diethyl ether to give 69.6 mg of the title compound as yellow crystals.

Melting point : 236-237°C
$^1H$ NMR (300MHz, δ ppm, DMSO-$d_6$)
2.58(3H,s), 4.91(1H,d,J=15.0Hz), 5.10(1H,d,J=15.0Hz), 5.80(2H,m), 6.92(2H,d,J=8.1Hz), 7.20-7.34(3H,m), 7.44-7.56(1H,m), 7.61(2H,d,J=8.4Hz), 7.70-7.80(2H,m), 8.18(2H,d,J=8.4Hz)

Synthetic Example 194 (Step 10-21)

4-(4-Chlorobenzyl)-1-methyl-6-(4-nitrophenyl)-4H-2,3,4,5,10b-pentaazabenz[e]azulene

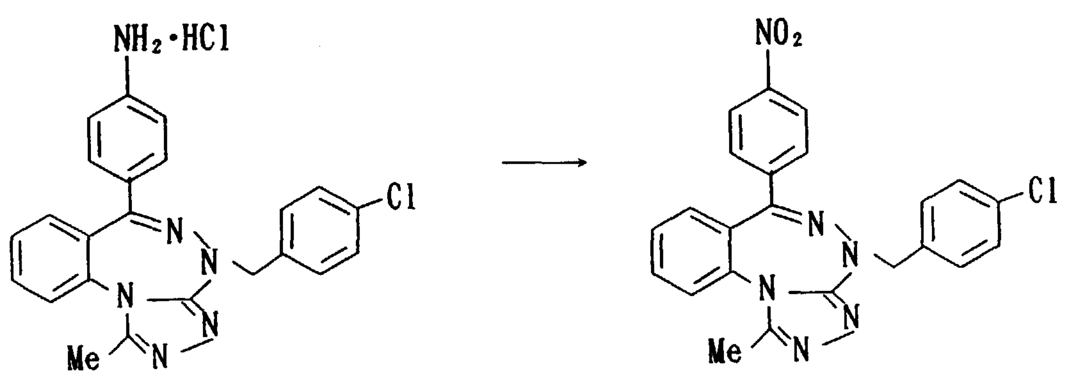

4-[4-(4-Chlorobenzyl)-1-methyl-4H-2,3,4,5,10b-pentaazabenz[e]azulen-6-yl]phenylamine hydrochloride (135 mg) obtained in Synthetic Example 191 was partitioned between saturated aqueous sodium hydrogencarbonate solution and chloroform. The organic layer was washed with saturated brine and dried. The solvent was concentrated under

reduced pressure and the obtained residue (100 mg) was dissolved in acetic acid (2 ml). In the same manner as in Oxidations in Organic Chemistry (American Chemical Society (1990)), sodium peroxoborate tetrahydrate (185 mg) was added and the mixture was stirred at 50°C for 7 hours. The reaction mixture was allowed to cool, and water (20 ml) and chloroform (20 ml) were added. The separated organic layer was washed with saturated aqueous sodium hydrogencarbonate solution and water, and dried. The solvent was evaporated under reduced pressure, and the obtained residue was purified by silica gel column chromatography (chloroform:methanol=98:2). Pure fractions were collected and concentrated under reduced pressure. The resulting solid was crystallized from ethyl acetate to give 68 mg of the title compound as a pale-yellow solid.

Melting point : 240-242°C

Example 15 (Step 10-22)

2,9-Dimethyl-4-phenyl-6-[4-(1H-tetrazol-5-yl)benzyl]-6H-5,6,7,8,9a-pentaazathieno[2,3-e]azulene

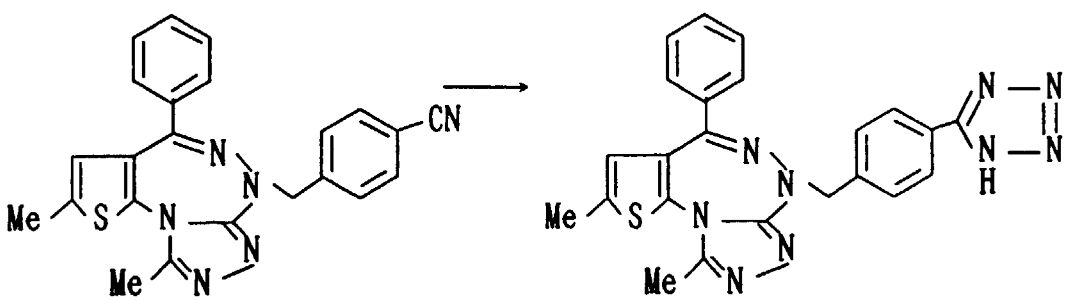

In the same manner as in journal of Medicinal Chemistry, 27, 1565 (1984), a mixture of 6-(4-cyanbenzyl)-2,9-dimethyl-4-phenyl-6H-5,6,7,8,9a-pentaazathieno[2,3-e]azulene (243 mg) obtained in Synthetic Example 122, sodium azide (116 mg) and ammonium chloride (97 mg) was dissolved in N,N-dimethyformamide (2.4 ml), and the mixture was heated at 150°C for 3 hours. The solvent was evaporated under reduced pressure, and the residue-was dissotuedm. tetrahydrofuran (5 ml). The organic layer was washed with brine and dried. Thereto was added ethyl acetate to give 242 mg of the title compound as yellow crystals.

Melting point : 264-265°C (decomposition)
$^1$H NMR (300MHz, $\delta$ ppm DMSO-$d_6$)
2.47(3H,s), 2.55(3H,s), 4.99(2H,s), 6.57(1H,m), 7.35-7.48(5H,m), 7.65(2H,d,J=8.3Hz), 8.02(2H,d,J=8.3Hz)

Example 16,17 (Step 10-23)

2,9-Dimethyl-4-phenyl-6-[4-methyl-1H-tetrazol-5-yl)benzyl]-6H-5,6,7,8,9a-pentaazathieno[2,3-e]azulene (Example 16) and 2,9-dimethyl-4-phenyl-6-[4-(2-methyl-2H-tetrazol-5-yl)benzyl]-6H-5,6,7,8,9a-pentaazathieno[2,3-e]azulene (Example 17)

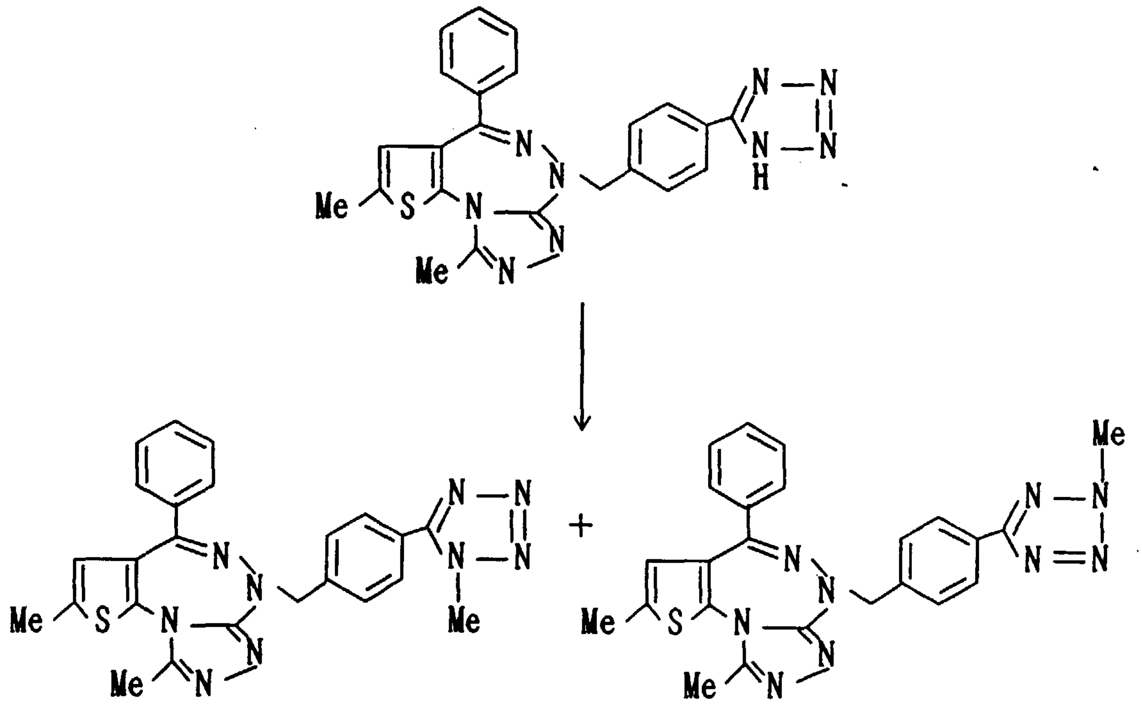

2,9-Dimethyl-4-phenyl-6-[4-(1H-tetrazol-5-yl)benzyl]-6H-5,6,7,8,9a-pentaazathieno[2,3-e]azulene (183 mg) obtained in Example 15 was dissolved in a mixed solvent of tetrahydrofuran (5 ml) and methanol (1 ml) and the mixture was ice-cooled. Thereto was added a solutioin of diazomethane in diethyl ether until the reaction mixture turned yellow. The solvent was evaporated under reduced pressure, and the residue was purified by silica gel column chromatography (developing solvent chloroform:acetone=10:1). The fractions eluted earlier were collected and concentrated to dryness. The obtained residue was crystallized from a mixed solvent of ethyl acetate/diethyl ether to give 2,9-dimethyl-4-phenyl-6-[4-(1-methyl-1H-tetrazol-5-yl)benzyl]-6H-5,6,7,8,9a-pentaazathieno[2,3-e]azulene (123 mg) as pale-yellow crystals.

Melting point : 199.5-200.5°C
$^1$H NMR (300MHz, δ ppm, $CDCl_3$)
2.49(3H,s), 2.63(3H,s), 4.39(3H,s), 5.10(2H,s), 6.44(1H,s), 7.26-7.42(5H,m), 7.58(2H,m), 8.10(2H,m)

The fractions eluted later were collected and concentrated to dryness. The obtained residue was crystallized from a mixed solvent of ethyl acetate/diethyl ether to give 2,9-dimethyl-4-phenyl-6-[4-(2-methyl-2H-tetrazol-5-yl)benzyl]-6H-5,6,7,8,9a-pentaazathieno[2,3-e]azulene (17 mg) as yellow crystals.

Melting point : 180- 182°C

Synthetic Examples 195-210

In the same manner as in WO93/07129, the compounds of Synthetic Examples 195-210 were obtained. These compounds are shown in Tables 63 and 64.

Synthetic Example 195

2-[6-(4-Chlorophenyl)-1-methyl-4H-[1,2,4]triazolo[4,3-a][1,4]benzodiazepin-4-yl]-N phenyl-acetamide

Synthetic Example 196

6-(4-Chlorophenyl)-1-methyl-4H-[1,2,4]triazolo[4,3-a][1,4]benzodiazepine-4-spiro-5'-[3'-(2,5-dimethoxyphenyl)-2',4'-dioxoimidazolidine]

Synthetic Example 197

6-(4-Chlorophenyl)-1-methyl-4H-[1,2,4]triazolo[4,3-a][1,4]benzodiazpine-4-spiro-5'-(3'-phenyl-2',4'-dioxoimidazolidine)

Synthetic Example 198

1-(3-Methylphenyl)-3-[1-methyl-6-(thiophen-2yl)-4H-[1,2,4]triazolo[4,3-a][1,4]benzodiazepin-4-yl]urea

Synthetic Example 199

1-[6-(4-Chlorophenyl)-4-ethoxycarbonyl-1-methyl-4H-[1,2,4]triazolo[4,3-a][1,4]benzodiazepin-4-yl]-3-(2,5-dimethoxyphenyl)urea

Synthetic Example 200

Benzyl [6-(4-chlorophenyl)-1-methyl-4H-[1,2,4]triazolo[4,3-a][1,4]benzodiazepin-4-yl]carbamate

Synthetic Example 201

1-[6-(4-Chlorophenyl)-1-methyl-4H-[1,2,4]triazolo[4,3-a][1,4]benzodiazepin-4-yl]methyl-3-(3-methylphenyl)urea

Synthetic Example 202

1-[6-(4-Chlorophenyl)-1-methyl-4H-[1,2,4]triazolo[4,3-a][1,4]benzodiazepin-4-yl]-3-(3-pyridyl)urea

Synthetic Example 203

1-[6-(4-Chlorophenyl)-1-methyl-4H-[1,2,4]triazolo[4,3-a][1,4]benzodiazepin-4-yl]-3-cyclohexylurea

Synthetic Example 204

1-[6-(4-Chloromphenyl)-1-methyl-4H-[1,2,4]triazolo[4,3-a][1,4]benzodiazepin-4-yl]-3-(2,5-dimethoxyphenyl)urea

Synthetic Example 205

N-[6-(4-chlorophenyl)-1-methyl-4H-[1,2,4]triazolo[4,3-a[1,4]benzodiazepin-4-yl]indole-2-carboxamide

Synthetic Example 206

6-(4-Chlorophenyl)-4-(indol-3-ylmethyl)-1-methyl-4H-[1,2,4]triazolo[4,3-a][1,4]benzodiazepine

Synthetic Example 207

2-[6-(4-Chlorophenyl)-1-methyl-4H-[1,2,4]triazolo[4,3-a[1,4]benzodiazepin-4-yl]-N-pyridin-2-yl-acetamide

Synthetic Example 208

2-[6-(4-Chlorophenyl)-1-methyl-4H-[1,2,4]triazolo[4,3-a][1,4]benzodiazepin-4-yl]-N-pyridin-3-yl-acetamilde

Synthetic Example 209

2-[6-(4-Chlorophenyl)-1-methyl-4H-[1,2,4]triazolo[4,3-a[1,4]benzodiazepin-4-yl]-N-pyridin-4-yl-acetamide

Synthetic Example 210

2-[6-(4-Chlorophenyl)-1-methyl-4H-[1,2,4]triazolo[4,3-a][1,4]benzodiazepin-4-yl]-N-(2,5-dimethoxyphenyl)-acetamide

**Table 63**

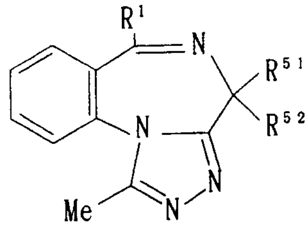

| Syn. Ex. | $R^1$ | $R^{51}$ | $R^{52}$ | Melting point (°C) |
|---|---|---|---|---|
| 195 | 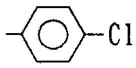 | 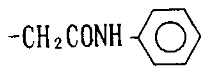 | H | 156-160 (decomposition) |
| 196 | 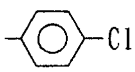 | 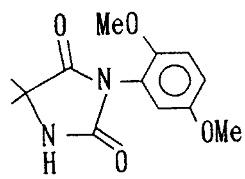 | | 257-259 |
| 197 | 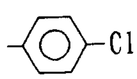 | 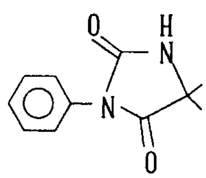 | | 249-251 |
| 198 | 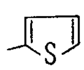 | 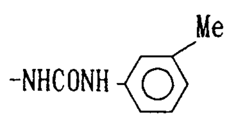 | H | 263-265 |
| 199 | 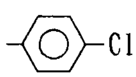 | 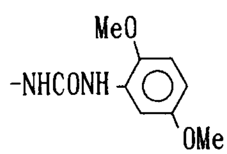 | -COOEt | 163-166 (decomposition) |
| 200 | 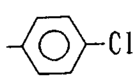 | 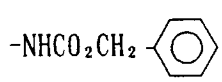 | H | 212-215 |
| 201 | 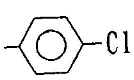 | 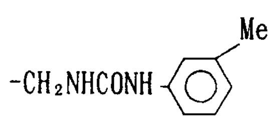 | H | 271-273 |

**Table 64**

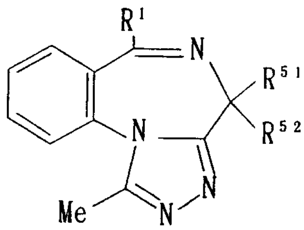

| Syn. Ex. | $R^1$ | $R^{51}$ | $R^{52}$ | Melting point (°C) |
|---|---|---|---|---|
| 202 | 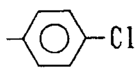 | 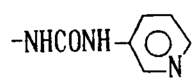 | H | 272-274 |
| 203 | 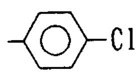 | 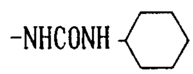 | H | 257-259 |
| 204 | 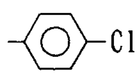 | 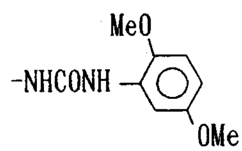 | H | 248-249 |
| 205 | 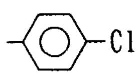 | 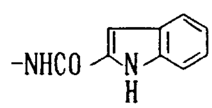 | H | 287-290 |
| 206 | 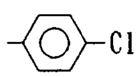 | 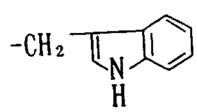 | H | 275-279 |
| 207 | 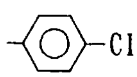 | 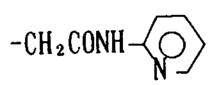 | H | 160-163 (decomp.) |
| 208 | 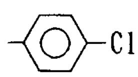 | 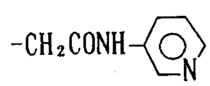 | H | 177-180 (decomp.) |
| 209 | 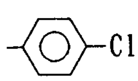 | 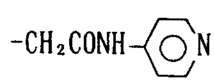 | H | 268-273 (decomp.) |
| 210 | 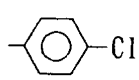 | 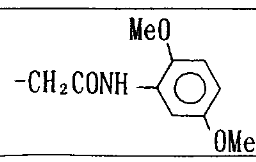 | H | 134-137 |

Formulation Examples for using the compound of the present invention as a medicament are shown in the following.

**Formulation Example 1** (example of tablet)

| | |
|---|---|
| (1) Compound of Synthetic Example 1 | 10 g |
| (2) Lactose | 50 g |
| (3) Corn starch | 15 g |
| (4) Sodium carboxymethylcellulose | 44 g |
| (5) Magnesium stearate | 1 g |

The entire amounts of (1), (2) and (3) and (4) (30 g) were kneaded with water, dried in vacuo and granulated. The granules were mixed with 14 g of (4) and 1 g of (5), and the mixture was tableted to give 1000 tablets containing 10 mg per tablet.

**Formulation Example 2** (example of injection)

Mannitol (5 g) was dissolved in water for injection (100 ml) and the compound of Synthetic Example 1(100 mg) was dissolved therein. After sterilization by filtration through a 0.22 μm filter, 1 ml of the solution was packed in sterile ampoules to give an injection containing 1 mg per ampoule.

**Experimental Examples**

The results of the tests with respect to the cytokine production inhibitory action of the compounds of the above-mentioned formula [I] of the present invention are shown in the following.

**Experimental Example 1**

Inhibitory activity on cytokine production from human PBMC (peripheral blood mononuclear cell)

(1) Isolation and culture of human PBMC

PBMC fractions were isolated from human blood using a mono-poly resolving medium. The cells (PBMC) were cultured in 5% FCS (fetal calf serum)/RPMI1640 medium. Thereafter, the test compound was added to the final concentration of 0.001-10 μM, and LPS (lipopolysaccharide) was added to the final concentration of 10 μg/ml. After incubatioin for 24 hours, the supernatant was separated and freeze stocked at -80°C.

(2) Assay of cytokine

On the day of experiment, the supernatant was thawed and the production amount of cytokine was measured by ELISA. IL-6 was assayed using [(h)IL-6] human, ELISA system (Amersham); TNF-α was assayed using [(h)TNF-α] human, ELISA system (Amersham); GM-CSF was assayed using human GM-CSF immunoassay (R&D systems); and IL-8 was assayed using human IL-8 immunoassay (R&D systems). The determination method followed the method of each kit. The results are expressed in $IC_{50}$.
As shown in Table 65 to Table 68, the compounds of the present invention suppressed the production of IL-6, TNF-α, GM-CSF and -IL-8.

Table 65

| Compound No. (Synthetic Example) | IL-6 $IC_{50}$ (μM) | TNF-α $IC_{50}$ (μM) |
|---|---|---|
| 1 | 1.3 | 0.1 |

Table 65 (continued)

| Compound No. (Synthetic Example) | IL-6 $IC_{50}$ (μM) | TNF-α $IC_{50}$ (μM) |
|---|---|---|
| 4 | | 0.03 |
| 5 | 1.5 | 1.9 |
| 7 | 0.23 | 0.5 |
| 13 | 0.02 | <0.01 |
| 14 | 0.02 | <0.01 |
| 16 | 0.6 | 0.08 |
| 17 | | 0.12 |
| 18 | 0.008 | <0.01 |
| 20 | 0.8 | 0.06 |
| 39 | <0.01 | <0.01 |
| 54 | 0.01 | 0.02 |
| 86 | 0.08 | 0.01 |
| 92 | 0.15 | 0.007 |
| 95 | 0.34 | 0.03 |
| 97 | 0.22 | 0.007 |
| 102 | 1.15 | 0.05 |
| 103 | 0.11 | 0.001 |
| 108 | <0.01 | <0.01 |
| 109 | <0.01 | 0.07 |
| 111 | 0.4 | 0.07 |

Table 66

| Compound No. (Synthetic Example) | IL-6 $IC_{50}$ (μM) | TNF-α $IC_{50}$ (μM) |
|---|---|---|
| 114 | 0.01 | <0.01 |
| 115 | 0.06 | 0.01 |
| 119 | <0.01 | <0.01 |
| 121 | 0.04 | <0.01 |
| 122 | <0.01 | <0.01 |
| 125 | 0.02 | <0.01 |
| 127 | 0.08 | <0.01 |
| 129 | 0.06 | 0.014 |
| 195 | 3.4 | 0.6 |
| 196 | 2.5 | 0.09 |
| 197 | >10 | 0.3 |
| 198 | >10 | 0.08 |

Table 66 (continued)

| Compound No. (Synthetic Example) | IL-6 $IC_{50}$ (μM) | TNF-α $IC_{50}$ (μM) |
|---|---|---|
| 199 | 1.6 | 0.01 |
| 200 | 1.0 | <0.01 |
| 201 | >10 | 0.3 |
| 202 | >10 | 0.09 |
| 203 | 5.7 | 0.2 |
| 204 | >10 | 0.4 |
| 205 | 3.9 | 0.01 |
| 206 | 6.7 | 0.3 |

Table 67

| Compound No. | IL-6 $IC_{50}$ (μM) | TNF-α $IC_{50}$ (μM) |
|---|---|---|
| Example 1 | 0.62 | 0.13 |
| Example 3 | 0.2 | 0.05 |
| Example 4 | 2 | <0.01 |
| Example 5 | 0.3 | 0.3 |
| Synthetic Example 191 | 0.001 | 0.001 |
| Synthetic Example 192 | 0.03 | 0.02 |
| Synthetic Example 193 | <0.001 | 0.001 |
| Example 16 | 0.3 | 0.06 |

Table 68

| Compound No. (Synthetic Example ) | IL-8 $IC_{50}$ (μM) | GM-CSF $IC_{50}$ (μM) |
|---|---|---|
| 19 | 0.26 | 0.05 |

**Experimental Example 2**

Inhibitory activity on *in vivo* IL-6 production by LPS stimulation

LFS (0.3 mg/kg) was intravenously administered to male ICR mice (6-week-old) fasted for 24 hours. At the same time, the test compound suspended in 0.5% CMC-Na ( carboxymethylcellulose sodium) was orally administered at the dose of 10 mg/kg.

Two hours later, blood was taken from eyegrounds. The blood was centrifuged, and the plasma fraction was diluted and quantitatively assayed for IL-6 in blood by ELISA (Endogen EM-IL6). The inhibitory percentage of the group administered with the test compound was expressed relative to the amount of IL-6 in blood of the cup administered with 0.5% CMC-Na. The results are shown in Table 69.

As shown in Table 69, the compounds of the present invention suppressed the in vivo production of IL-6.

Table 69

| Compound No. | Inhibtion (%) of plasma IL-6 production (10 mg/kg, p.o.) |
|---|---|
| Synthetic Example 54 | 56 |
| Synthetic Example 86 | 89 |
| Synthetic Example 95 | 78 |
| Synthetic Example 97 | 98 |
| Synthetic Example 102 | 78 |
| Synthetic Example 103 | 75 |
| Synthetic Example 108 | 70 |
| Synthetic Example 109 | 51 |
| Synthetic Example 111 | 62 |
| Synthetic Example 115 | 75 |
| Synthetic Example 118 | 75 |
| Synthetic Example 121 | 91 |
| Synthetic Example 122 | 71 |
| Synthetic Example 125 | 84 |
| Synthetic Example 127 | 83 |
| Example 1 | 73 |
| Synthetic Example 191 | 62 |
| Synthetic Example 192 | 67 |
| Synthetic Example 193 | 67 |
| Example 16 | 84 |

**Experimental Example 3**

Inhibitory activity on *in vivo* TNF-α production by LPS stimulation

The test compound suspended in 0.5% CMC-Na (carboxymethylcellulose sodium) was orally administered at the dose of 30 mg/kg to male ICR mice (6-week-old) fasted for 24 hours. At 30 minutes after oral administration, LPS was intravenously administered at the dose of 0.3 mg/kg. At one hour after LSP administration, blood was taken from the heart. The TNF-α in plasma was assayed by ELISA (Genzyme). The suppression of TNF-α production of the group administered with test compounds was calculated by the following formula.

$$\text{Inhibition (\%)} = \left[1-\frac{\text{test compound administered group}}{\text{0.5\% CMC-Na administered group}}\right] \times 100$$

The results are shown in Table 70. As shown in Table 70, the compound of the present invention suppressed the *in vivo* production of TNF-α.

Table 70

| Compound No. (Synthetic Example) | Inhibition (%) of plasma TNF-$\alpha$ production (30 mg/kg, p.o.) |
|---|---|
| 19 | 51 |

**Experimental Example 4**

Inhibitory activity on IL-2 and IFN$\gamma$ production

The spleen cells obtained from 9-week-old Balb/c mice (Charles River Japan, Inc.) was suspended in RPMI-1640 medium supplemented with 10% FCS, at $4 \times 10^6$ cells/ml. The suspended cells were incubated in a 96 well plate (0.25 ml per well) in the presence of 5 $\mu$g/ml concanavalin A. The cells were cultured with or without addition of various concentrations of test compounds. After 24 hours' incubation, the culture broth was taken for assay of cytokine and preserved at -80°C. The concentrations of IL-2 and IFN$\gamma$ in the culture broth were measured using an EIA kit (perSeptive Diagnostic) according to the attached protocol. The results are expressed in $IC_{50}$.

As shown in Table 71, the compound of the present invention suppressed the production of IL-2 and IFN$\gamma$.

Table 71

| Compound No. (Synthetic Example) | IL-2 $IC_{50}$ ($\mu$M) | IFN$\gamma$ $IC_{50}$ ($\mu$M) |
|---|---|---|
| 19 | 0.24 | 0.12 |

**Experimental Example 5**

Effects on rat adjuvant arthritis

Lewis rats (5-week-old, Charles River Japan, Inc.) were used for the test. Adjuvant arthritis (AIA) was induced by injection (0.5 mg) of Mycobacterium butyricum (Difco) suspended in paraffin oil, from the root of the tail at day 0. The volume of the hind limb was measured with time after the injection of adjuvant. The test compound was suspended in 0.5% HPMC (hydroxypropylmethycellulose) and orally administered at the dose of 10 mg/kg once a day for 28 days from day 0. The suppression of edema of hind limb at day 28 was calculated as follows.

$$\text{Suppression (\%)} = 100 \times \frac{\text{(AIA treated group-AIA control group)}}{\text{(normal control group-AIA control group)}}$$

The results are shown in mean±standard error and tested by Student-test. The result was significant when p value was less than 0.05.

As shown In Table 72, the compound of the present invention significantly suppressed the edema of hind limb.

Table 72

| Compound No. (Synthetic Example) | Suppression (%) of edema |
|---|---|
| 19 | 72.7±3.3** |

**p < 0.01

**Industrial Applicability**

The compounds of the above-mentioned formula [I] of the present invention have superior cytokine production

inhibitory action and are low toxic. In paticular, the compounds of the present invention have superior antiinflammatory action. Therefore, these compounds are useful as cytokine production inhibitors, particularly for the suppression of the production of IL-6, TNF-α, IL-8, IFNγ, IL-2 and GM-CSF, and as antiinflammatory drug. The cytokine production inhibitor of the present invention can be used for the prophylaxis and/or treatment of diseases wherein cytokine is responsible for the pathogenesis or formation of the disease state. The diseases wherein cytokine is responsible for the pathogenesis or formation of the disease state is exemplified by chronic inflammation, autoimmune diseases, viral diseases and cancer. Specific examples include autoimmune diseases such as chronic rheumatoid arthritis and SLE, atrial myxoma, Castleman's disease, myeloma, Lennert's T lymphoma, mesangial proliferative nephritis, cachexia caused by terminal cancer or AIDS, ARDS, viral infections such as viral hapatitis, acute myocardial infarction, gout, psoriasis, asthma, fulminant hepatitis, malignant tumor and the like.

The compounds of the formulas [II'] and [III'] of the present invention are useful as intermediates for the production of the triazepine compound of the formula [I''']. The compounds of the formulas [II''] and [III''] of the present invention are useful as intermediates for the production of the triazepine compound of the formula [I'''']. Using these intermediates and following the above-explained production methods, the objective compounds of the formulas [I'''] and [I''''] can be respectively produced.

This application is based on application Nos. 174268/1996 and 95237/1997 filed in Japan, the contents of which are incorporated hereinto by reference.

## Claims

1. A cytokine production inhibitor comprising, as an active ingredient, a compound of the formula [I]

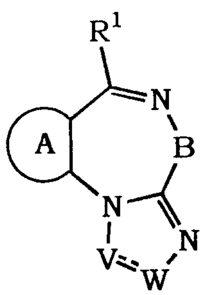

[I]

wherein

$R^1$ is an optionally substituted aryl or an optionally substituted heteroaryl;

B is a group of the formula

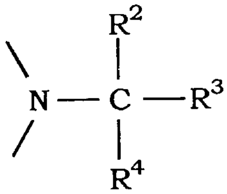 or 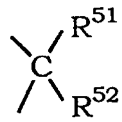

wherein,

$R^2$ is hydrogen atom, hydroxy, halogen atom or lower alkyl,

$R^4$ is hydrogen atom or halogen atom,

or $R^2$ and $R^4$ in combination form carbonyl with the carbon atom they bind with,

$R^3$ is hydrogen atom, lower alkyl, lower alkoxy, cycloalkyl, optionally substituted aryl, optionally substituted heteroaryl or a group of the formula:

$-CR^5=CR^6R^7$, $-OR^{18}$, 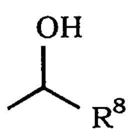, 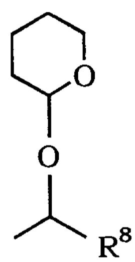, $-COOR^8$,

$-CONHR^8$, 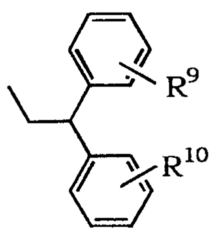, 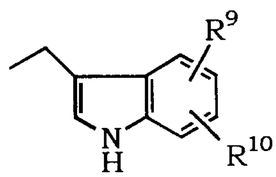 or -X-Y

wherein $R^5$, $R^6$ and $R^7$ are the same or different and each is hydrogen atom, halogen atom, lower alkyl, or optionally substituted aryl, $R^8$ is hydrogen atom, lower alkyl, cycloalkyl, optionally substituted aryl, aralkyl or optionally substituted heteroaryl, $R^9$ and $R^{10}$ are the same or different and each is hydrogen atom, lower alkyl, lower alkoxy, hydroxy, halogen atom, nitro or amino, $R^{18}$ is optionally substituted aryl, X is $-(CH_2)_m-$, $-CO-$, $-COCH_2-$, $-NH-$, $-NHCH_2-$, $-CH_2NH-$, $-CH_2NHCO-$, $-OCH_2-$, $-(CH_2)_nO-$ or $-CH_2S-$, Y is halogen atom, cycloalkyl, optionally substituted aryl or optionally substituted heteroaryl, m is an integer of 1 to 4 and n is an integer of 1 to 4,

$R^{51}$ is hydrogen atom, lower alkyl, haloalkyl, optionally substituted aryl, optionally substituted heteroaryl, aralkyl, heteroarylalkyl or a group selected from the groups of the following formulas:

$—(CH_2)_bN(R^{53})(R^{54})$ (1)

$—(CH_2)_bOR^{55}$ (2)

$—(CH_2)_bN(R^{55})\overset{\overset{Z}{\|}}{C}N(R^{56})(R^{57})$ (3)

$—(CH_2)_bN(R^{55})CORa^{56}$ (4)

$—(CH_2)_bN(R^{55})SO_2R^{58}$ (5)

$—(CH_2)_bN(R^{55})COOR^{59}$ (6)

$—(CH_2)_bO\overset{\overset{Z}{\|}}{C}N(R^{56})(R^{57})$ (7)

$—(CH_2)_bOCOR^{60}$ (8)

$—(CH_2)_bCON(R^{61})(R^{62})$ (9)

$—(CH_2)_bOSO_2R^{58}$ (10)

$—(CH_2)_bCOR^{63}$ (11)

$—(CH_2)_bS(O)_pR^{56}$ (12)

$—CON(R^{55})OR^{53}$ (13)

$—CON(R^{55})N(R^{55})\overset{\overset{Z}{\|}}{C}Ra^{56}$ (14)

$—CON(R^{55})N(R^{55})SO_2Ra^{56}$ (15)

$$-N(R^{55})\overset{\overset{\displaystyle Z}{\|}}{C}N(R^{55})CORa^{56} \qquad (16)$$

$$-N(R^{55})\overset{\overset{\displaystyle Z}{\|}}{C}N(R^{55})SO_2Ra^{56} \qquad (17)$$

$$-CON(R^{55})N(R^{55})(R^{56}) \qquad (18)$$

$$-(CH_2)_bN(R^{55})COCON(R^{56})(R^{57}) \qquad (19) \text{ and}$$

$$-(CH_2)_aCOOR^{64} \qquad (20)$$

wherein b is 0 or an integer of 1 to 6, Z is oxygen atom or sulfur atom, $R^{53}$ and $R^{54}$ are the same or different and each is hydrogen atom, lower alkyl, optionally substituted aryl or aralkyl, $R^{55}$ is hydrogen atom, lower alkyl or aralkyl, $R^{56}$ and $R^{57}$ are the same or different and each is hydrogen atom, lower alkyl, cycloalkyl, cycloalkylalkyl, optionally substituted aryl, aralkyl, optionally substituted heteroaryl or heteroarylalkyl, $Ra^{56}$ is lower alkyl, cycloalkyl, cycloalkylalkyl, optionally substituted aryl, aralkyl, optionally substituted heteroaryl or heteroarylalkyl, $R^{58}$ is lower alkyl, optionally substituted aryl, aralkyl, cycloalkyl, or optionally substituted heteroaryl, $R^{59}$ is lower alkyl, optionally substituted aryl or aralkyl, $R^{60}$ is lower alkyl, lower alkenyl, lower alkynyl, cycloalkyl, cycloalkylalkyl, optionally substituted aryl, aralkyl, optionally substituted heteroaryl or heteroarylalkyl, $R^{61}$ and $R^{62}$ are the same or different and each is hydrogen atom, lower alkyl, acyl, optionally substituted aryl or aralkyl, $R^{63}$ is lower alkyl, optionally substituted aryl, aralkyl, optionally substituted heteroaryl or heteroarylalkyl, p is 0, 1 or 2, a is an integer of 1 to 6, and $R^{64}$ is hydrogen atom, lower alkyl, optionally substituted aryl or aralkyl, and

$R^{52}$ is hydrogen atom or $-COOR^{53}$ wherein $R^{53}$ is hydrogen atom, lower alkyl, optionally substituted aryl or aralkyl, or $R^{51}$ and $R^{52}$ in combination form, together with the carbon atom they bind with, a spiro ring of the formula:

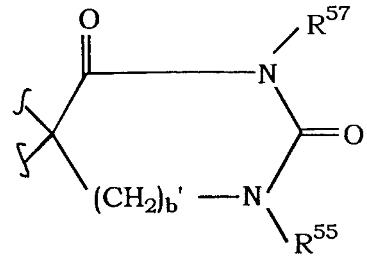

wherein b' is 0 or 1, $R^{55}$ is hydrogen atom, lower alkyl or aralkyl, and $R^{57}$ is hydrogen atom, lower alkyl, cycloalkyl, cycloalkylalkyl, optionally substituted aryl, aralkyl, optionally substituted heteroaryl or heteroarylalkyl; and

ring A is a ring selected from the following rings:

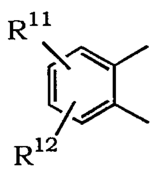 , 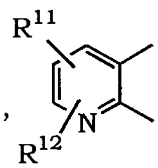 , 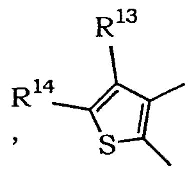 and 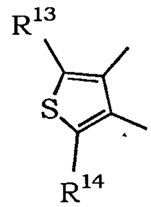

wherein $R^{11}$ and $R^{12}$ are the same or different and each is hydrogen atom, halogen atom, lower alkyl, said lower alkyl being optionally substituted by halogen atom, lower alkoxy, nitro, amino, amino substituted by lower alkyl, cyclic amino, hydroxy, acyloxy, cyano, carbamoyl, carbamoyl substituted by lower alkyl, cyclic aminocarbonyl, carboxy, lower alkoxycarbonyl or aralkyloxycarbonyl, lower alkenyl, aralkyl, aralkyl substituted by lower alkyl, lower alkoxy, nitro, amino, amino substituted by lower alkyl, cyclic amino, hydroxy, acyloxy, cyano, carbamoyl, carbamoyl substituted by lower alkyl, cyclic aminocarbonyl, carboxy, lower alkoxycarbonyl or aralkyloxycarbonyl, and $R^{13}$ and $R^{14}$ are the same or different and each is hydrogen atom, halogen atom, lower alkyl, lower alkenyl, lower alkynyl, haloalkyl, lower alkoxy, nitro, amino, amino substituted by lower alkyl, cyclic amino, hydroxy, acyloxy, cyano, carbamoyl, carbamoyl substituted by lower alkyl, cyclic aminocarbonyl, carboxy, lower alkoxycarbonyl, aralkyloxycarbonyl, cycloalkyl, or lower alkylcarbonyl; and

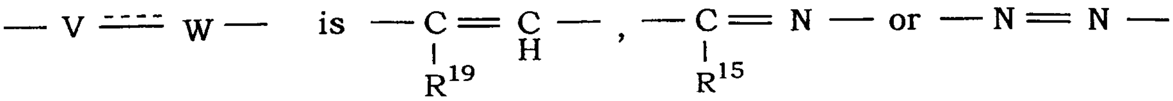
is , or

wherein $R^{15}$ is lower alkyl and $R^{19}$ is hydrogen atom or lower alkyl, or a pharmaceutically acceptable salt thereof.

2. The cytokine production inhibitor of claim 1, comprising, as an active ingredient, a compound

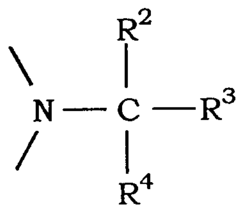

of the formula [I] wherein B is namely, a compound of the formula [I']

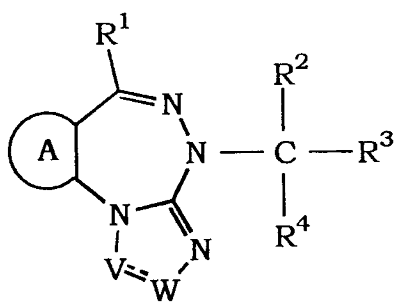
[I']

wherein $R^1$, $R^2$, $R^3$, $R^4$, A, V and W are as defined in claim 1,
or a pharmaceutically acceptable salt thereof.

3. The cytokine production inhibitor of claim 2, wherein, in the formula [I'], the ring A is

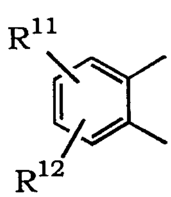
or
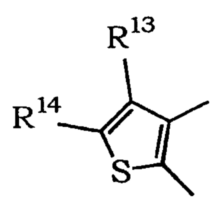

wherein $R^{11}$, $R^{12}$, $R^{13}$ and $R^{14}$ are as defined in claim 1,

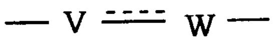 is 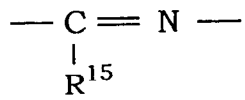

wherein $R^{15}$ is lower alkyl,
$R^1$ is optionally substituted phenyl, and
$R^2$ and $R^4$ are both hydrogen atoms.

**4.** The cytokine production inhibitor of claim 2, comprising, as an active ingredient, a compound selected from the group consisting of:

6-(4-chlorophenyl)-4-(4-methoxybenzyl)-1-methyl-4H-2,3,4,5,10b-pentaazabenz[e]azulene,
6-(4-chlorophenyl)-4-(3,4-dimethoxybenzyl)-1-methyl-4H-2,3,4,5,10b-pentaazabenz[e]azulene,
4-(4-methoxybenzyl)-1-methyl-6-phenyl-4H-2,3,4,5,10b-pentaazabenz[e]azulene,
4-(4-methoxybenzyl)-1,9-dimethyl-6-phenyl-4H-2,3,4,5,10b-pentaazabenz[e]azulene,
8-chloro-4-(4-methoxybenzyl)-1-methyl-6-phenyl-4H-2,3,4,5,10b-pentaazabenz[e]azulene,
4-(4-methoxybenzyl)-1-methyl-8-nitro-6-phenyl-4H-2,3,4,5,10b-pentaazabenz[e]azulene,
4-(4-methoxybenzyl)-1-methyl-6-(4-methylphenyl)-4H-2,3,4,5,10b-pentaazabenz[e]azulene,
8-chloro-6-(2-chlorophenyl)-4-(4-methoxybenzyl)-1-methyl-4H-2,3,4,5,10b-pentaazabenz[e]azulene,
6-(4-chlorophenyl)-4-(4-methoxybenzyl)-1,9-dimethyl-4H-2,3,4,5,10b-pentaazabenz[e]azulene,
6-(4-bromophenyl)-4-(4-methoxybenzyl)-1-methyl-4H-2,3,4,5,10b-pentaazabenz[e]azulene,
tert-butyl 4-[4-(4-methoxybenzyl)-1-methyl-4H-2,3,4,5,10b-pentaazabenz[e]azulen-6-yl]benzoate,
6-(4-chlorophenyl)-4-(4-methoxybenzyl)-1-methyl-4H-2,3,4,5,10,10b-hexaazabenz[e]azulene,
4-(4-chlorophenyl)-6-(4-methoxybenzyl)-2,3,9-trimethyl-6H-5,6,7,8,9a-pentaazathieno[2,3-e]azulene,
4-(4-chlorophenyl)-2-ethyl-6-(4-methoxybenzyl)-9-methyl-6H-5,6,7,8,9a-pentaazathieno[2,3-e]azulene,
6-(4-methoxybenzyl)-2,9-dimethyl-4-phenyl-6H-5,6,7,8,9a-pentaazathieno[2,3-e]azulene,
6-(4-methoxybenzyl)-2,3,9-trimethyl-4-phenyl-6H-5,6,7,8,9a-pentaazathieno[2,3-e]azulene,
2-ethyl-6-(4-methoxybenzyl)-9-methyl-4-phenyl-6H-5,6,7,8,9a-pentaazathieno[2,3-e]azulene,
6-(4-methoxybenzyl)-4-(4-methoxyphenyl)-2,3,9-trimethyl-6H-5,6,7,8,9a-pentaazathieno[2,3-e]azulene,
6-(4-chlorophenyl)-1-methyl-4-(pyridin-3-ylmethyl)-4H-2,3,4,5,10b-pentaazabenz[e]azulene,
4-(2-chlorophenyl)-2,3,9-trimethyl-6-(pyridin-4-ylmethyl)-6H-5,6,7,8,9a-pentaazathieno[2,3-e]azulene,
6-(4-chlorophenyl)-4-(3-cyanobenzyl)-1-methyl-4H-2,3,4,5,10b-pentaazabenz[e]azulene,
6-(4-chlorophenyl)-4-(2-fluorobenzyl)-1-methyl-4H-2,3,4,5,10b-pentaazabenz[e]azulene,
6-[4-chlorophenyl)-4-(3-fluorobenzyl)-1-methyl-4H-2,3,4,5,10b-pentaazabenz[e]azulene,
6-(4-chlorophenyl)-4-(4-fluorobenzyl)-1-methyl-4H-2,3,4,5,10b-pentaazabenz[e]azulene,
6-(4-chlorophenyl)-4-(2,4-difluorobenzyl)-1-methyl-4H-2,3,4,5,10b-pentaazabenz[e]azulene,
6-(4-chlorophenyl)-4-(2,5-difluorobenzyl)-1-methyl-4H-2,3,4,5,10b-pentaazabenz[e]azulene,
6-(4-chlorophenyl)-4-(3,5-difluorobenzyl)-1-methyl-4H-2,3,4,5,10b-pentaazabenz[e]azulene,
6-(4-chlorophenyl)-4-(3,4-difluorobenzyl)-1-methyl-4H-2,3,4,5,10b-pentaazabenz[e]azulene,
6-(4-chlorophenyl)-1-methyl-4-(2-trifluoromethylbenzyl)-4H-2,3,4,5,10b-pentaazabenz[e]azulene,
6-(4-chlorophenyl)-1-methyl-4-(4-trifluoromethylbenzyl)-4H-2,3,4,5,10b-pentaazabenz[e]azulene,
6-(4-chlorophenyl)-1-methyl-4-(3-trifluoromethylbenzyl)-4H-2,3,4,5,10b-pentaazabenz[e]azulene,
6-(4-chlorophenyl)-1-methyl-4-(4-trifluoromethoxybenzyl)-4H-2,3,4,5,10b-pentaazabenz[e]azulene,
6-(4-chlorophenyl)-1-methyl-4-(3-nitrobenzyl)-4H-2,3,4,5,10b-pentaazabenz[e]azulene,
4-(2-chlorobenzyl)-6-(4-chlorophenyl)-1-methyl-4H-2,3,4,5,10b-pentaazabenz[e]azulene,
4-(3-chlorobenzyl)-6-(4-chlorophenyl)-1-methyl-4H-2,3,4,5,10b-pentaazabenz[e]azulene,
4-(4-chlorobenzyl)-6-(4-chlorophenyl)-1-methyl-4H-2,3,4,5,10b-pentaazabenz[e]azulene,
6-(4-chlorophenyl)-4-(2-cyanobenzyl)-1-methyl-4H-2,3,4,5,10b-pentaazabenz[e]azulene,
6-(4-chlorophenyl)-4-(4-cyanobenzyl)-1-methyl-4H-2,3,4,5,10b-pentaazabenz[e]azulene,
6-(4-chlorophenyl)-4-(2-methoxybenzyl)-1-methyl-4H-2,3,4,5,10b-pentaazabenz[e]azulene,
6-(4-chlorophenyl)-4-(3-methoxybenzyl)-1-methyl-4H-2,3,4,5,10b-pentaazabenz[e]azulene,
6-(4-chlorophenyl)-4-(4-methoxybenzyl)-1-methyl-4H-2,3,4,5,10b-pentaazabenz[e]azulene,
6-(4-chlorophenyl)-4-(2,5-dimethoxybenzyl)-1-methyl-4H-2,3,4,5,10b-pentaazabenz[e]azulene,
6-(4-chlorophenyl)-1-methyl-4-(3,4,5-trimethoxybenzyl)-4H-2,3,4,5,10b-pentaazabenz[e]azulene,
4-(5-acetyl-2-methoxbenzyl)-6-(4-chlorophenyl)-1-methyl-4H-2,3,4,5,10b-pentaazabenz[e]azulene,

6-(4-chlorophenyl)-1-methyl-4-(3,4-methylenedioxybenzyl)-4H-2,3,4,5,10b-pentaazabenz[e]azulene,
4-(2-chloro-4,5-methylenedioxybenzyl)-6-(4-chlorophenyl)-1-methyl-4H-2,3,4,5,10b-pentaazabenz[e]azulene,
6-(4-chlorophenyl)-4-(2-methoxy-5-nitrobenzyl)-1-methyl-4H-2,3,4,5,10b-pentaazabenz[e]azulene,
6-(4-chlorophenyl)-4-(4-methoxy-3-nitrobenzyl)-1-methyl-4H-2,3,4,5,10b-pentaazabenz[e]azulene,
4-(3-chloro-4-methoxybenzyl)-6-(4-chlorophenyl)-1-methyl-4H-2,3,4,5,10b-pentaazabenz[e]azulene,
6-(4-chlorophenyl)-4-(3,5-dichloro-4-methoxybenzyl)-1-methyl-4H-2,3,4,5,10b-pentaazabenz[e]azulene,
6-(4-chlorophenyl)-1-methyl-4-(2-methylbenzyl)-4H-2,3,4,5,10b-pentaazabenz[e]azulene,
6-(4-chlorophenyl)-1-methyl-4-(3-methylbenzyl)-4H-2,3,4,5,10b-pentaazabenz[e]azulene,
6-(4-chlorophenyl)-1-methyl-4-(4-methylbenzyl)-4H-2,3,4,5,10b-pentaazabenz[e]azulene,
4-(4-tert-butylbenzyl)-6-(4-chlorophenyl)-1-methyl-4H-2,3,4,5,10b-pentaazabenz[e]azulene,
6-(4-chlorophenyl)-1-methyl-4-(naphthalen-1-ylmethyl)-4H-2,3,4,5,10b-pentaazabenz[e]azulene,
6-(4-chlorophenyl)-1-methyl-4-(naphthalen-2-ylmethyl)-4H-2,3,4,5,10b-pentaazabenz[e]azulene,
4-(4-benzyloxybenzyl)-6-(4-chlorophenyl)-1-methyl-4H-2,3,4,5,10b-pentaazabenz[e]azulene,
4-benzyl-6-(4-chlorophenyl)-1-methyl-4H-2,3,4,5,10b-pentaazabenz[e]azulene,
6-(4-chlorophenyl)-1-methyl-4-(4-phenylbenzyl)-4H-2,3,4,5,10b-pentaazabenz[e]azulene,
4-(4-chlorophenoxymethyl)-6-(4-chlorophenyl)-1-methyl-4H-2,3,4,5,10b-pentaazabenz[e]azulene,
6-(4-chlorophenyl)-1-methyl-4-(pyridin-2-ylmethyl)-4H-2,3,4,5,10b-pentaazabenz[e]azulene,
6-(4-chlorophenyl)-4-[2-(indol-3-yl)ethyl]-1-methyl-4H-2,3,4,5,10b-pentaazabenz[e]azulene,
6-(4-chlorophenyl)-4-(2-methyl-1,3-thiazol-4-ylmethyl)-1-methyl-4H-2,3,4,5,10b-pentaazabenz[e]azulene,
6-(4-chlorophenyl)-4-(5-chlorothiophen-2-ylmethyl)-1-methyl-4H-2,3,4,5,10b-pentaazabenz[e]azulene,
6-(4-chlorophenyl)-1-methyl-4-(3,5-dimethylisoxazol-4-ylmethyl)-4H-2,3,4,5,10b-pentaazabenz[e]azulene,
6-(4-chlorophenyl)-1-methyl-4-phenethyl-4H-2,3,4,5,10b-pentaazabenz[e]azulene,
6-(4-chlorophenyl)-1-methyl-4-(3-phenylpropyl)-4H-2,3,4,5,10b-pentaazabenz[e]azulene,
6-(4-chlorophenyl)-4-(3,3-diphenylpropyl)-1-methyl-4H-2,3,4,5,10b-pentaazabenz[e]azulene,
6-(4-chlorophenyl)-4-cyclopropylmethyl-1-methyl-4H-2,3,4,5,10b-pentaazabenz[e]azulene,
6-(4-chlorophenyl)-4-cyclohexylmethyl-1-methyl-4H-2,3,4,5,10b-pentaazabenz[e]azulene,
6-(4-chlorophenyl)-4-(2-cyclohexylethyl)-1-methyl-4H-2,3,4,5,10b-pentaazabenz[e]azulene,
6-(4-chlorophenyl)-1-methyl-4-(3-phenyl-2-propenyl)-4H-2,3,4,5,10b-pentaazabenz[e]azulene,
4-allyl-6-(4-chlorophenyl)-1-methyl-4H-2,3,4,5,10b-pentaazabenz[e]azulene,
6-(4-chlorophenyl)-1-methyl-4-(2-methyl-2-propenyl)-4H-2,3,4,5,10b-pentaazabenz[e]azulene,
6-(4-chlorophenyl)-4-(2-chloro-2-propenyl)-1-methyl-4H-2,3,4,5,10b-pentaazabenz[e]azulene,
4-(2-bromo-2-propenyl)-6-(4-chlorophenyl)-1-methyl-4H-2,3,4,5,10b-pentaazabenz[e]azulene,
6-(4-chlorophenyl)-4-(2,3-dichloro-2-propenyl)-1-methyl-4H-2,3,4,5,10b-pentaazabenz[e]azulene,
6-(4-chlorophenyl)-4-(3,4-dibenzyloxybenzyl)-1-methyl-4H-2,3,4,5,10b-pentaazabenz[e]azulene,
4-benzyloxymethyl-6-(4-chlorophenyl)-1-methyl-4H-2,3,4,5,10b-pentaazabenz[e]azulene,
6-(4-chlorophenyl)-1-methyl-4-(3-phenoxypropyl)-4H-2,3,4,5,10b-pentaazabenz[e]azulene,
6-(4-chlorophenyl)-4-(3,3-dichloro-2-propenyl)-1-methyl-4H-2,3,4,5,10b-pentaazabenz[e]azulene,
6-(4-chlorophenyl)-4-(4-methoxy-3-methylbenzyl)-1-methyl-4H-2,3,4,5,10b-pentaazabenz[e]azulene,
6-(4-chlorophenyl)-4-(3,4-dichlorobenzyl)-1-methyl-4H-2,3,4,5,10b-pentaazabenz[e]azulene,
6-(4-chlorophenyl)-1-methyl-4-(pyridin-4-ylmethyl)-4H-2,3,4,5,10b-pentaazabenz[e]azulene,
6-(4-chlorophenyl)-1-methyl-4-(4-methylsulfonylbenzyl)-4H-2,3,4,5,10b-pentaazabenz[e]azulene,
6-(4-chlorophenyl)-1-methyl-4-(4-nitrobenzyl)-4H-2,3,4,5,10b-pentaazabenz[e]azulene,
6-(4-chlorophenyl)-4-(2,6-dichloropyridin-4-ylmethyl)-1-methyl-4H-2,3,4,5,10b-pentaazabenz[e]azulene,
6-(4-chlorophenyl)-4-(2,2,2-trifluoroethyl)-1-methyl-4H-2,3,4,5,10b-pentaazabenz[e]azulene,
6-(4-chlorophenyl)-4-(3,5-dinitrobenzyl)-1-methyl-4H-2,3,4,5,10b-pentaazabenz[e]azulene,
8-chloro-1-methyl-6-phenyl-4-(pyridin-4-ylmethyl)-4H-2,3,4,5,10b-pentaazabenz[e]azulene,
8-chloro-1-methyl-6-phenyl-4-(pyridin-3-ylmethyl)-4H-2,3,4,5,10b-pentaazabenz[e]azulene,
1-methyl-6-phenyl-4-(pyridin-3-ylmethyl)-4H-2,3,4,5,10b-pentaazabenz[e]azulene,
1-methyl-6-phenyl-4-(pyridin-4-ylmethyl)-4H-2,3,4,5, 10b-pentaazabenz[e]azulene,
1,9-dimethyl-6-phenyl-4-(pyridin-3-ylmethyl)-4H-2,3,4,5,10b-pentaazabenz[e]azulene,
1,9-dimethyl-6-phenyl-4-(pyridin-4-ylmethyl)-4H-2,3,4,5,10b-pentaazabenz[e]azulene,
4-(3-cyanobenzyl)-1,9-dimethyl-6-phenyl-4H-2,3,4,5,10b-pentaazabenz[e]azulene,
4-(4-cyanobenzyl)-1,9-dimethyl-6-phenyl-4H-2,3,4,5,10b-pentaazabenz[e]azulene,
4-(3,4-dichlorobenzyl)-1,9-dimethyl-6-phenyl-4H-2,3,4,5,10b-pentaazabenz[e]azulene,
1-methy1-8-nitro-6-phenyl-4-(pyridin-3-ylmethyl)-4H-2,3,4,5,10b-pentaazabenz[e]azulene,
1-methyl-8-nitro-6-phenyl-4-(pyridin-4-ylmethyl)-4H-2,3,4,5,10b-pentaazabenz[e]azulene,
1-methyl-6-(4-methylphenyl)-4-(pyridin-4-ylmethyl)-4H-2,3,4,5,10b-pentaazabenz[e]azulene,
4-(3-cyanobenzyl)-1-methyl-6-(4-methylphenyl)-4H-2,3,4,5,10b-pentaazabenz[e]azulene,

8-chloro-6-(2-chlorophenyl)-1-methyl-4-(pyridin-3-ylmethyl)-4H-2,3,4,5,10b-pentaazabenz[e]azulene,
8-chloro-6-(2-chlorophenyl)-1-methyl-4-(pyridin-4-ylmethyl)-4H-2,3,4,5,10b-pentaazabenz[e]azulene,
methyl 4-[6-(4-chlorophenyl)-1-methyl-4H-2,3,4,5,10b-pentaazabenz[e]azulen-4-ylmethyl]benzoate,
6-(4-chlorophenyl)-4-(4-cyanobenzyl)-1,9-dimethyl-4H-2,3,4,5,10b-pentaazabenz[e]azulene,
4-(4-bromobenzyl)-6-(4-bromophenyl)-1-methyl-4H-2,3,4,5,10b-pentaazabenz[e]azulene,
4-(4-cyanobenzyl)-1-methyl-6-phenyl-4H-2,3,4,5,10b-pentaazabenz[e]azulene,
4-[6-(4-chlorophenyl)-1-methyl-4H-2,3,4,5,10b-pentaazabenz[e]azulen-4-ylmethyl]benzoic acid,
4-(4-chlorophenyl)-2,3,9-trimethyl-6-(pyridin-3-ylmethyl)-6H-5,6,7,8,9a-pentaazathieno[2,3-e]azulene,
4-(4-chlorophenyl)-2,3,9-trimethyl-6-(pyridin-4-ylmethyl)-6H-5,6,7,8,9a-pentaazathieno[2,3-e]azulene,
4-(4-chlorophenyl)-6-(4-cyanobenzyl)-2,3,9-trimethyl-6H-5,6,7,8,9a-pentaazathieno[2,3-e]azulene,
4-(4-chlorophenyl)-6-(3,4-difluorobenzyl)-2,3,9-trimethyl-6H-5,6,7,8,9a-pentaazathieno[2,3-e]azulene,
4-(4-cholorophenyl)-2-ethyl-9-methyl-6-(pyridin-3-ylmethyl)-6H-5,6,7,8,9a-pentaazathieno[2,3-e]azulene,
4-(4-chlorophenyl)-2-ethyl-9-methyl-6-(pyridin-4-ylmethyl)-6H-5,6,7,8,9a-pentaazathieno[2,3-e]azulene,
4-(4-chlorophenyl)-6-(4-cyanobenzyl)-2-ethyl-9-methyl-6H-5,6,7,8,9a-pentaazathieno[2,3-e]azulene,
2,9-dimethyl-4-phenyl-6-(pyridin-3-ylmethyl)-6H-5,6,7,8,9a-pentaazathieno[2,3-e]azulene,
2,9-dimethyl-4-phenyl-6-(pyridin-4-ylmethyl)-6H-5,6,7,8,9a-pentaazathieno[2,3-e]azulene,
6-(4-cyanobenzyl)-2,9-dimethyl-4-phenyl-6H-5,6,7,8,9a-pentaazathieno[2,3-e]azulene,
6-(4-chlorobenzyl)-2,9-dimethyl-4-phenyl-6H-5,6,7,8,9a-pentaazathieno[2,3-e]azulene,
2,3,9-trimethyl-4-phenyl-6-(pyridin-3-ylmethyl)-6H-5,6,7,8,9a-pentaazathieno[2,3-e]azulene,
2,3,9-trimethyl-4-phenyl-6-(pyridin-4-ylmethyl)-6H-5,6,7,8,9a-pentaazathieno[2,3-e]azulene,
2-ethyl-9-methyl-4-phenyl-6-(pyridin-3-ylmethyl)-6H-5,6,7,8,9a-pentaazathieno[2,3-e]azulene,
2-ethyl-9-methyl-4-phenyl-6-(pyridin-4-ylmethyl)-6H-5,6,7,8,9a-pentaazathieno[2,3-e]azulene,
4-(4-methoxyphenyl)-2,3,9-trimethyl-6-(pyridin-3-ylmethyl)-6H-5,6,7,8,9a-pentaazathieno[2,3-e]azulene,
4-(4-methoxyphenyl)-2,3,9-trimethyl-6-(pyridin-4-ylmethyl)-6H-5,6,7,8,9a-pentaazathieno[2,3-e]azulene,
6-(4-cyanobenzyl)-4-(4-methoxyphenyl)-2,3,9-trimethyl-6H-5,6,7,8,9a-pentaazathieno[2,3-e]azulene,
4-(4-chlorophenyl)-6-(4-fluorobenzyl)-2,3,9-trimethyl-6H-5,6,7,8,9a-pentaazathieno[2,3-e]azulene,
6-(4-chlorobenzyl)-4-(4-chlorophenyl)-2,3,9-trimethyl-6H-5,6,7,8,9a-pentaazathieno[2,3-e]azulene,
4-(4-chlorophenyl)-6-(3,4-dichlorobenzyl)-2,3,9-trimethyl-6H-5,6,7,8,9a-pentaazathieno[2,3-e]azulene,
4-(4-chlorophenyl)-6-(3,4-dichlorobenzyl)-2-ethyl-9-methyl-6H-5,6,7,8,9a-pentaazathieno[2,3-e]azulene,
6-(4-chlorophenyl)-1-methyl-4-(2-nitrobenzyl)-4H-2,3,4,5,10b-pentaazabenz[e]azulene,
6-(4-chlorophenyl)-4-ethoxycarbonylmethyl-1-methyl-4H-2,3,4,5,10b-pentaazabenz[e]azulene,
[6-(4-chlorophenyl)-1-methyl-4H-2,3,4,5,10b-pentaazabenz[e]azulen-4-yl]acetic acid,
6-(4-chlorophenyl)-1-methyl-4-phenylcarbamoylmethyl-4H-2,3,4,5,10b-pentaazabenz[e]azulene,
6-(4-chlorophenyl)-1-methyl-4-(4-methylphenylcarbamoylmethyl)-4H-2,3,4,5,10b-pentaazabenz[e]azulene,
6-(4-chlorophenyl)-4-(2-methoxyphenylcarbamoylmethyl)-1-methyl-4H-2,3,4,5,10b-pentaazabenz[e]azulene,
6-(4-chlorophenyl)-4-(2,5-dimethoxyphenylcarbamoylmethyl)-1-methyl-4H-2,3,4,5,10b-pentaazabenz[e]azulene,
4-(4-chloro-2,5-dimethoxyphenylcarbamoylmethyl)-6-(4-chlorophenyl)-1-methyl-4H-2,3,4,5,10b-pentaazabenz[e]azulene,
6-(4-chlorophenyl)-1-methyl-4-(naphthalen-1-ylcarbamoylmethyl)-4H-2,3,4,5,10b-pentaazabenz[e]azulene,
6-(4-chlorophenyl)-1-methyl-4-(pyridin-3-ylcarbamoylmethyl)-4H-2,3,4,5,10b-pentaazabenz[e]azulene,
6-(4-chlorophenyl)-4-(cyclohexylcarbamoylmethyl)-1-methyl-4H-2,3,4,5,10b-pentaazabenz[e]azulene,
6-(4-chlorophenyl)-1-methyl-4-n-propylcarbamoylmethyl-4H-2,3,4,5,10b-pentaazabenz[e]azulene,
4-bromoacetyl-6-(4-chlorophenyl)-1-methyl-4H-2,3,4,5,10b-pentaazabenz[e]azulene,
6-(4-chlorophenyl)-4-(2-methoxphenylaminoacetyl)-1-methyl-4H-2,3,4,5,10b-pentaazabenz[e]azulene,
6-(4-chlorophenyl)-1-methyl-4-phenylaminoacetyl-4H-2,3,4,5,10b-pentaazabenz[e]azulene,
6-(4-chlorophenyl)-1-methyl-4-(4-methylphenylaminoacetyl)-4H-2,3,4,5,10b-pentaazabenz[e]azulene,
6-(4-chlorophenyl)-4-(3-fluorophenylaminoacetyl)-1-methyl-4H-2,3,4,5,10b-pentaazabenz[e]azulene,
6-(4-chlorophenyl)-4-(2,5-dimethoxyphenylaminoacetyl)-1-methyl-4H-2,3,4,5,10b-pentaazabenz[e]azulene,
6-(4-chlorophenyl)-1-methyl-4-phenylthioacetyl-4H-2,3,4,5,10b-pentaazabenz[e]azulene,
6-(4-chlorophenyl)-1-methyl-4-phenylacetyl-4H-2,3,4,5,10b-pentaazabenz[e]azulene,
6-(4-chlorophenyl)-1-methyl-4-phenyloxalyl-4H-2,3,4,5,10b-pentaazabenz[e]azulene,
6-(4-chlorophenyl)-4-ethoxymethyl-1-methyl-4H-2,3,4,5,10b-pentaazabenz[e]azulene,
N-[6-(4-chlorophenyl)-1-methyl-4H-2,3,4,5,10b-pentaaabenz[e]azulen-4-ylmethyl]-N-phenyl-amine,
4-benzylcarbamoyl-6-(4-chlorophenyl)-1-methyl-4H-2,3,4,5,10b-pentaazabenz[e]azulene,
6-(4-chlorophenyl)-1-methyl-4-(3-methylphenylcarbamoyl)-4H-2,3,4,5,10b-pentaazabenz[e]azulene,
6-(4-chlorophenyl)-4-(4-hydroxybenzyl)-1-methyl-4H-2,3,4,5,10b-pentaazabenz[e]azulene,
6-(4-chlorophenyl)-4-(3,4-dihydroxybenzyl)-1-methyl-4H-2,3,4,5,10b-pentaazabenz[e]azulene,

6-(4-chlorophenyl)-4-(4-ethoxybenzyl)-1-methyl-4H-2,3,4,5,10b-pentaazabenz[e]azulene,
6-(4-chlorophenyl)-1-methyl-4-(4-methylsulfonylphenyl)hydroxymethyl-4H-2,3,4,5,10b-pentaazabenz[e]azulene,
4-(4-aminobenzyl)-6-(4-chlorophenyl)-1-methyl-4H-2,3,4,5,10b-pentaazabenz[e]azulene,
6-(4-chlorophenyl)-4-(4-formylaminobenzyl)-1-methyl-4H-2,3,4,5,10b-pentaazabenz[e]azulene,
4-(4-acetylaminobenzyl)-6-(4-chlorophenyl)-1-methyl-4H-2,3,4,5,10b-pentaazabenz[e]azulene,
6-(4-chlorophenyl)-4-(4-methylsulfonylaminobenzyl)-1-methyl-4H-2,3,4,5,10b-pentaazabenz[e]azulene,
4-[4-bis(methylsulfonyl)aminobenzyl]-6-(4-chlorophenyl)-4H-2,3,4,5,10b-pentaazabenz[e]azulene,
6-(4-chlorophenyl)-4-(4-dimethylaminobenzyl)-1-methyl-4H-2,3,4,5,10b-pentaazabenz[e]azulene,
6-(4-chlorophenyl)-4-(2-hydroxy-2-phenylethyl)-1-methyl-4H-2,3,4,5,10b-pentaazabenz[e]azulene,
6-(4-chlorophenyl)-4-(2-oxo-2-phenylethyl)-1-methyl-4H-2,3,4,5,10b-pentaazabenz[e]azulene,
6-(4-chlorophenyl)-1-methyl-4-[3-phenyl-2-(tetrahydropyran-2-yloxy)propyl]-4H-2,3,4,5,10b-pentaazabenz[e]azulene,
6-(4-chlorophenyl)-4-[2-(2-methoxyphenyl)-2-(tetrahydropyran-2-yloxy)ethyl]-1-methyl-4H-2,3,4,5,10b-pentaazabenz[e]azulene,
6-(4-chlorophenyl)-4-(2-hydroxy-3-phenylpropyl)-1-methyl-4H-2,3,4,5,10b-pentaazabenz[e]azulene,
6-(4-chlorophenyl)-4-(2-oxo-3-phenylpropyl)-1-methyl-4H-2,3,4,5,10b-pentaazabenz[e]azulene,
6-(4-chlorophenyl)-4-[2-(4-chlorophenyl)-2-oxoethyl]-1-methyl-4H-2,3,4,5,10b-pentaazabenz[e]azulene,
6-(4-chlorophenyl)-1-methyl-4-[2-(4-methylphenyl)-2-oxoethyl]-4H-2,3,4,5,10b-pentaazabenz[e]azulene,
6-(4-chlorophenyl)-4-[2-(2-methoxyphenyl)-2-oxoethyl]-1-methyl-4H-2,3,4,5,10b-pentaazabenz[e]azulene,
6-(4-chlorophenyl)-4-[2-(2,5-dimethoxyphenyl)-2-oxoethyl]-1-methyl-4H-2,3,4,5,10b-pentaazabenz[e]azulene,
6-(4-chlorophenyl)-4-[3-(2-methoxyphenyl)-2-oxopropyl]-1-methyl-4H-2,3,4,5,10b-pentaazabenz[e]azulene,
6-(4-chlorophenyl)-4-[3-(2,5-dimethoxyphenyl)-2-oxopropyl]-1-methyl-4H-2,3,4,5,10b-pentaazabenz[e]azulene,
4-benzyl-6-(4-chlorophenyl)-1-methyl-4H-2,3,4,5,10b-pentaazabenz[e]azulene,
6-(4-chlorophenyl)-4-(4-methoxybenzyl)-4H-3,4,5,10b-tetraazabenz[e]azulene,
3-[6-(4-chlorophenyl)-1-methyl-4H-2,3,4,5,10b-pentaazabenz[e]azulen-4-ylmethyl]-pyridine-1-oxide,
3-[8-chloro-6-(2-chlorophenyl)-1-methyl-4H-2,3,4,5,10b-pentaazabenz[e]azulen-4-ylmethyl]-pyridine-1-oxide,
4-[4-(4-chlorophenyl)-2,3,9-trimethyl-6H-1-thia-5,6,7,8,9a-pentaazacyclopent[e]azulen-6-ylmethyl]-pyridine-1-oxide,
4-[4-(4-chlorophenyl)-2-ethyl-9-methyl-6H-1-thia-5,6,7,8,9a-pentaazacyclopent[e]azulen-6-ylmethyl]-pyridine-1-oxide,
4-[2,9-dimethyl-4-phenyl-6H-1-thia-5,6,7,8,9a-pentaazacyclopent[e]azulen-6-ylmethyl]-pyridine-1-oxide,
methyl 4-[4-(4-chlorobenzyl)-1-methyl-4H-2,3,4,5,10b-pentaazabenz[e]azulen-6-yl]benzoate,
methyl 4-[4-(4-cyanobenzyl)-1-methyl-4H-2,3,4,5,10b-pentaazabenz[e]azulen-6-yl]benzoate,
methyl 4-[1-methyl-4-(4-nitrobenzyl)-4H-2,3,4,5,10b-pentaazabenz[e]azulen-6-yl]benzoate,
6-(4-chlorophenyl)-1-methyl-4-(4-nitrobenzyl)-4H-2,3,4,5,10,10b-hexaazabenz[e]azulene,
[4-(4-chlorobenzyl)-1-methyl-4H-2,3,4,5,10b-pentaazabenz[e]azulen-6-yl]benzoic acid,
[4-(4-cyanobenzyl)-1-methyl-4H-2,3,4,5,10b-pentaazabenz[e]azulen-6-yl]benzoic acid,
4-[1-methyl-4-(4-nitrobenzyl)-4H-2,3,4,5,10b-pentaazabenz[e]azulen-6-yl]benzoic acid,
tert-butyl 4-[4-(4-chlorobenzyl)-1-methyl-4H-2,3,4,5,10b-pentaazabenz[e]azulen-6-yl]phenylcarbamate,
tert-butyl 4-[4-[4-cyanobenzyl)-1-methyl-4H-2,3,4,5,10b-pentaazabenz[e]azulen-6-yl]phenylcarbamate,
tert-butyl 4-[1-methyl-4-(4-nitrobenzyl)-4H-2,3,4,5,10b-pentaazabenz[e]azulen-6-yl]phenylcarbamate,
4-[4-(4-chlorobenzyl)-1-methyl-4H-2,3,4,5,10b-pentaazabenz[e]azulen-6-yl]phenylamine,
4-[4-(4-cyanobenzyl)-1-methyl-4H-2,3,4,5,10b-pentaazabenz[e]azulen-6-yl]phenylamine,
4-[1-methyl-4-(4-nitrobenzyl)-4H-2,3,4,5,10b-pentaazabenz[e]azulen-6-yl]phenylamine,
4-(4-chlorobenzyl)-1-methyl-6-(4-nitrophenyl)-4H-2,3,4,5,10b-pentaazabenz[e]azulene,
2,9-dimethyl-4-phenyl-6-[4-(1H-tetrazol-5-yl)benzyl]-6H-5,6,7,8,9a-pentaazathieno[2,3-e]azulene,
2,9-dimethyl-4-phenyl-6-[4-(1-methyl-1H-tetrazol-5-yl)benzyl]-6H-5,6,7,8,9a-pentaazathieno[2,3-e]azulene,
and
2,9-dimethyl-4-phenyl-6-[4-(2-methyl-2H-tetrazol-5-yl)benzyl]-6H-5,6,7,8,9a-pentaazathieno[2,3-e]azulene,

or a pharmaceutically acceptable salt thereof.

**5.** The cytokine production inhibitor of claim 1, comprising, as an active ingredient, a compound of the formula [I] wherein B is

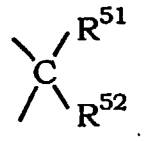

namely, a compound of the formula [I"]

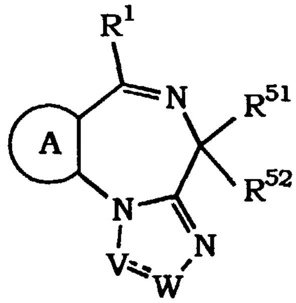

[I"]

wherein $R^1$, $R^{51}$, $R^{52}$, A, V and W are as defined in claim 1, or a pharmaceutically acceptable salt thereof.

6. The cytokine production inhibitor of claim 5, wherein, in the formula [I"], the ring A is

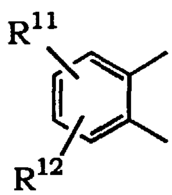

or

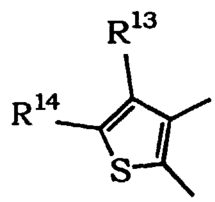

wherein $R^{11}$, $R^{12}$, $R^{13}$ and $R^{14}$ are as defined in claim 1,

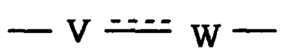

is

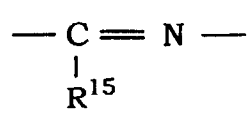

wherein $R^{15}$ is lower alkyl,
$R^{51}$ is hydrogen atom, optionally substituted aryl, optionally substituted heteroaryl, aralkyl, heteroarylalkyl or a group of the formula:

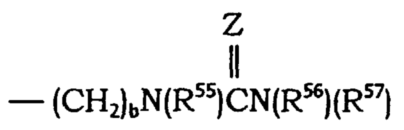 (3)

$-(CH_2)_bN(R^{55})CORa^{56}$ (4)

$-(CH_2)_bN(R^{55})COOR^{59}$ (6) or

$-(CH_2)_bCON(R^{61})(R^{62})$ (9)

wherein b, Z, $R^{55}$, $R^{56}$, $Ra^{56}$,$R^{57}$, $R^{59}$, $R^{61}$ and $R^{62}$ are as defined in claim 1, and $R^{52}$ is hydrogen atom or -$COOR^{53}$ wherein $R^{53}$ is as defined in claim 1, or $R^{51}$ and $R^{52}$ incombination form, together with the carbon atom they bind with, a spiro ring of the formula

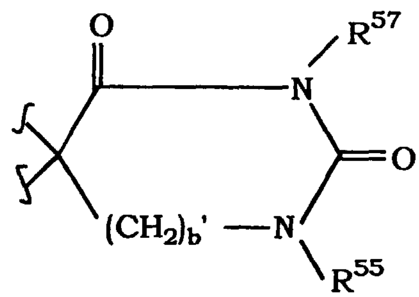

wherein b', $R^{55}$ and $R^{57}$ are as defined in claim 1.

7. The cytokine production inhibitor of claim 5, comprising, as an active ingredient, a compound selected from the group consisting of:

 2-[6-(4-chlorophenyl)-1-methyl-4H-[1,2,4]triazolo[4,3-a][1,4]benzodiazepin-4-yl]-N-phenyl-acetamide,
 6-(4-chlorophenyl)-1-methyl-4H-[1,2,4]triazolo[4,3-a][1,4]benozodiazepine-4-spiro-5'-[3'-(2,5-dimethoxyphenyl)-2',4'-dioxoimidazolidine],
 6-(4-chlorophenyl)-1-methyl-4H-[1,2,4]triazolo[4,3-a][1,4]benzodiazepine-4-spiro-5'-(3'-phenyl-2',4'-dioxoimidazolidine),
 1-(3-methylphenyl)-3-[1-methyl-6-(thiophen-2-yl)-4H-[1,2,4]triazolo[4,3-a][1,4]benzodiazepin-4-yl]urea,
 1-[6-(4-chlorophenyl)-4-ethoxycarbonyl-1-methyl-4H-[1,2,4]triazolo[4,3-a][1,4]benzodiazepin-4-yl]-3-(2,5-dimethoxyphenyl)urea,
 benzyl [6-[4-chlorophenyl)-1-methyl-4H-[1,2,4]triazolo[4,3-a][1,4]benzodiazepin-4-yl]carbamate,
 1-[6-(4-chlorophenyl)-1-methyl-4H-[1,2,4]triazolo[4,3-a][1,4]benzodiazepin-4-yl]methyl-3-(3-methylphenyl)urea,
 1-[6-(4-chlorophenyl)-1-methyl-4H-[1,2,4]triazolo[4,3-a][1,4]benzodiazepin-4-yl]-3-(3-pyridyl)urea,
 1-[6-(4-chlorophenyl)-1-methyl-4H-[1,2,4]triazolo[4,3-a][1,4]benzodiazepin-4-yl]-3-cyclohexylurea,
 1-[6-(4-chlorophenyl)-1-methyl-4H-[1,2,4]triazolo[4,3-a][1,4]benzodiazepin-4-yl]-3-(2,5-dimethoxyphenyl)urea,
 N-[6-(4-chlorophenyl)-1-methyl-4H-[1,2,4]triazolo[4,3-a][1,4]benzodiazepin-4-yl]indole-2-carboxamide,
 6-(4-chlorophenyl)-4-(indol-3-ylmethyl)-1-methyl-4H-[1,2,4]triazolo[4,3-a][1,4]benzodiazepine,
 2-[6-(4-chlorophenyl)-1-methyl-4H-[1,2,4]triazolo[4,3-a][1,4]benzodiazepin-4-yl]-N-pyridin-2-yl-acetamide,
 2-[6-(4-chlorophenyl)-1-methyl-4H-[1,2,4]triazolo[4,3-a][1,4]benzodiazepin-4-yl]-N-pyridin-3-yl-acetamide,
 2-[6-(4-chlorophenyl)-1-methyl-4H-[1,2,4]triazolo[4,3-a][1,4]benzodiazepin-4-yl]-N-pyridin-4-yl-acetamide, and
 2-[6-(4-chlorophenyl)-1-methyl-4H-[1,2,4]triazolo[4,3-a][1,4]benzodiazepin-4-yl]-N-(2,5-dimethoxyphenyl)-acetamide,

or a pharmaceutically acceptable salt thereof.

8. The cytokine production inhibitor of any of claims 1 to 7, wherein the cytokine is interleukin 6.

**9.** The cytokine production inhibitor of any of claims 1 to 7, wherein the cytokine is TNF-$\alpha$.

**10.** The cytokine production inhibitor of any of claims 1 to 7, wherein the cytokine is interleukin 8.

**11.** The cytokine production inhibitor of any of claims 1 to 7, wherein the cytokine is interferon $\gamma$.

**12.** The cytokine production inhibitor of any of claims 1 to 7, wherein the cytokine is interleukin 2.

**13.** The cytokine production inhibitor of any of claims 1 to 7, wherein the cytokine is GM-CSF.

**14.** An antiinflammatory agent comprising a compound of any of claims 1 to 7, or a pharmaceutically acceptable salt thereof, as an active ingredient.

**15.** A triazepine compound of the formula [I''']

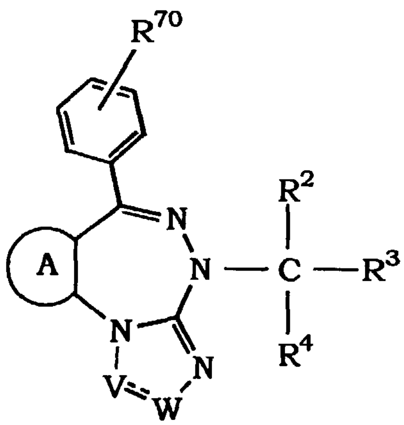

[I''']

wherein $R^{70}$ is -$COOR^{71}$ wherein $R^{71}$ is hydrogen atom or lower alkyl, or -$NHCOOR^{72}$ wherein $R^{72}$ is lower alkyl, and $R^2$, $R^3$, $R^4$, ring A, V and W are as defined in claim 1,
or a pharmaceutically acceptable salt thereof.

**16.** The triazepine compound of claim 15, wherein, in the formula [I'''], the ring A is

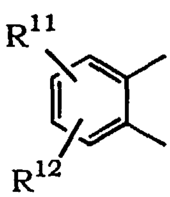

or

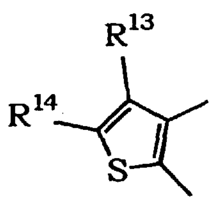

wherein, $R^{11}$, $R^{12}$, $R^{13}$ and $R^{14}$ are as defined in claim 1,

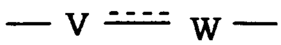

is

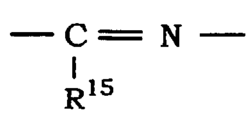

wherein $R^{15}$ is lower alkyl, and $R^2$ and $R^4$ are both hydrogen atoms,
or a pharmaceutically acceptable salt thereof.

**17.** A triazepine compound of the formula [I'''']

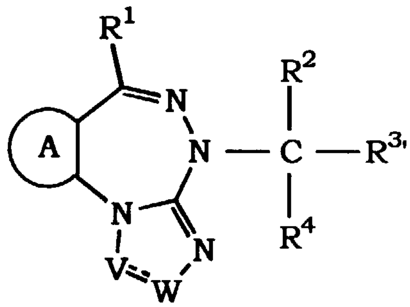

[I'''']

wherein $R^{3'}$ is pyridine-1-oxide or phenyl substituted by tetrazolyl or alkyltetrazolyl, and $R^1$, $R^2$, $R^4$, ring A, V and W are as defined in claim 1,
or a pharmaceutically acceptable salt thereof.

**18.** The triazepine compound of claim 17, wherein, in the formula [I''''], the ring A is

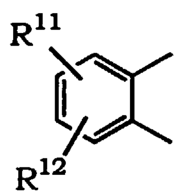

or

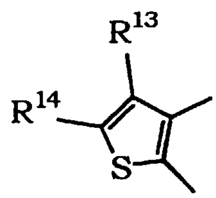

wherein, $R^{11}$, $R^{12}$, $R^{13}$ and $R^{14}$ are as defined in claim 1,

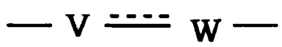

is

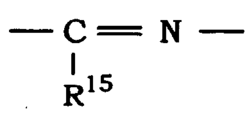

wherein $R^{15}$ is lower alkyl, and $R^2$ and $R^4$ are both hydrogen atoms,
or a pharmaceutically acceptable salt thereof.

**19.** A pharmaceutical composition comprising the triazepine compound of claim 15 or 16 or a pharmaceutically acceptable salt thereof, and a pharmaceutically acceptable carrier.

**20.** A pharmaceutical composition comprising the triazepine compound of claim 17 or 18 or a pharmaceutically acceptable salt thereof, and a pharmaceutically acceptable carrier.

**21.** A triazepinethione compound of the formula [II']

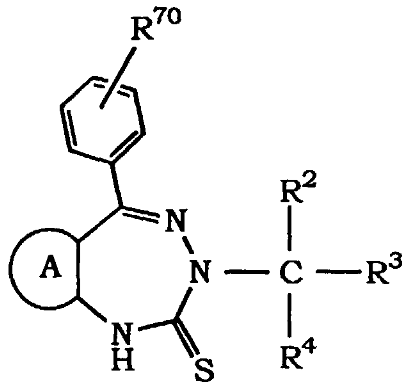

[II']

wherein $R^{70}$ is $-COOR^{71}$ wherein $R^{71}$ is hydrogen atom or lower alkyl, or $-NHCOOR^{72}$ wherein $R^{72}$ is lower alkyl, and $R^2$, $R^3$, $R^4$ and ring A are as defined in claim 1,
or a salt thereof.

**22.** The triazepinethione compound of claim 21, wherein the ring A is

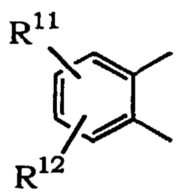

or

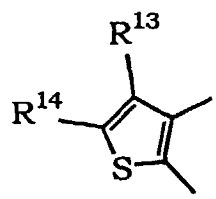

wherein, $R^{11}$, $R^{12}$, $R^{13}$ and $R^{14}$ are as defined in claim 1, and $R^2$ and $R^4$ are both hydrogen atoms,
or a salt thereof.

**23.** An alkylthiotriazepine compound of the formula [III']

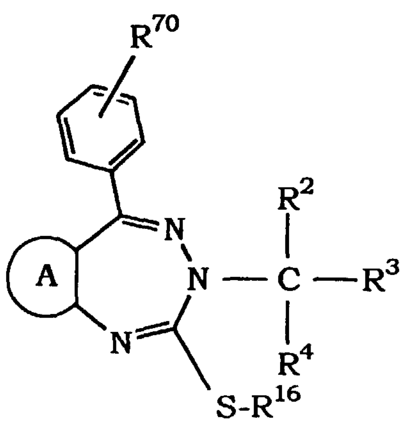

[III']

wherein $R^{16}$ is lower alkyl, $R^{70}$ is - $COOR^{71}$ wherein $R^{71}$ is hydrogen atom or lower alkyl, or $-NHCOOR^{72}$ wherein $R^{72}$ is lower alkyl, and $R^2$, $R^3$, $R^4$ and ring A are as defined in claim 1,
or a salt thereof.

**24.** The alkylthiotriazepine compound of claim 23, wherein the ring A is

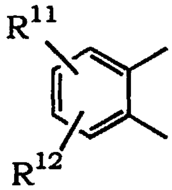

or

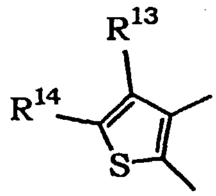

wherein, $R^{11}$, $R^{12}$, $R^{13}$ and $R^{14}$ are as defined in claim 1, and $R^2$ and $R^4$ are both hydrogen atoms, or a salt thereof.

**25.** A triazepinethione compound of the formula [II"]

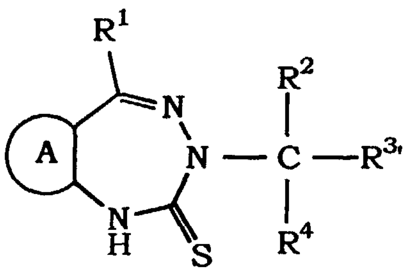

[II"]

wherein $R^{3'}$ is pyridine-1-oxide or phenyl substituted by tetrazolyl or alkyltetrazolyl, and $R^1$, $R^2$, $R^4$ and ring A are as defined in claim 1,
or a salt thereof.

**26.** The triazepinethione compound of claim 25, wherein the ring A is

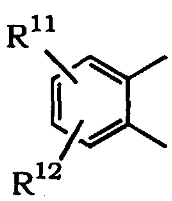

or

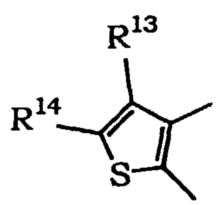

wherein, $R^{11}$, $R^{12}$, $R^{13}$ and $R^{14}$ are as defined in claim 1, and $R^2$ and $R^4$ are both hydrogen atoms, or a salt thereof.

**27.** An alkylthiotriazepine compound of the formula [III"]

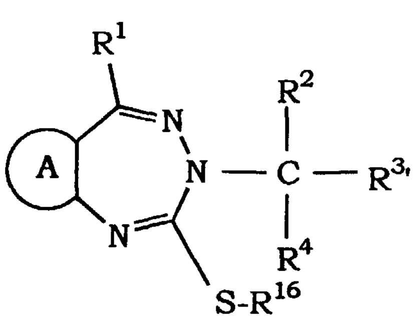

[III"]

wherein $R^{3'}$ is pyridine-1-oxide or phenyl substituted by tetrazolyl or alkyltetrazolyl, $R^{16}$ is lower alkyl, and $R^1$, $R^2$, $R^4$ and ring A are as defined in claim 1,
or a salt thereof.

**28.** The alkylthiotriazepine compound of claim 27, wherein the ring A is

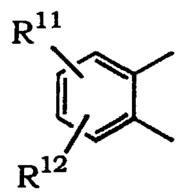

or

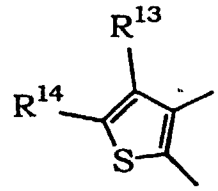

wherein, $R^{11}$, $R^{12}$, $R^{13}$ and $R^{14}$ are as defined in claim 1, and $R^2$ and $R^4$ are both hydrogen atoms,
or a salt thereof.

## INTERNATIONAL SEARCH REPORT

International application No.
PCT/JP97/02016

**A. CLASSIFICATION OF SUBJECT MATTER**

Int. Cl$^6$ C07D487/04, 487/14, 495/12, A61K31/55

According to International Patent Classification (IPC) or to both national classification and IPC

**B. FIELDS SEARCHED**

Minimum documentation searched (classification system followed by classification symbols)

Int. Cl$^6$ C07D487/04, 487/14, 495/12, A61K31/55

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| X<br><br><br>A | International Journal of Immunopharmacology, Vol. 14, No. 2, pp. 227-237, 1992, M.P. Chang et al. "Mechanism of Immunosuppressive Effect of Alprazolam: Alprazolam Suppresses T-Cèll Proliferation by Selectively Inhibiting the Production of IL2 but not Acquisition of IL2 Receptor" | 1-5, 7, 12<br><br><br>6, 8-11, 13 |
| X | JP, 51-115498, A (The Upjohn Co.), October 12, 1976 (12. 10. 76), Page 10, upper right column & DE, 2601400, A & FR, 2297622, A & GB, 1489980, A | 14 |
| X | US, 4144233, A (The Dow Chemical Co., Midland, Mich.), March 13, 1979 (13. 03. 79) & US, 4163104, A & US, 4189588, A | 15 - 28 |
| X | WO, 96/16062, A (Japan Tobacco Inc.), May 30, 1996 (30. 05. 96) | 15 - 28 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| September 9, 1997 (09. 09. 97) | September 24, 1997 (24. 09. 97) |
| Name and mailing address of the ISA/<br>Japanese Patent Office<br>Facsimile No. | Authorized officer<br><br>Telephone No. |

Form PCT/ISA/210 (second sheet) (July 1992)

**INTERNATIONAL SEARCH REPORT**

International application No.
PCT/JP97/02016

C (Continuation). DOCUMENTS CONSIDERED TO BE RELEVANT

| Category* | Citation of document, with indication, where appropriate, of the relevant passages | Relevant to claim No. |
|---|---|---|
| A | & JP, 08-225546, A | 1 - 14 |

Form PCT/ISA/210 (continuation of second sheet) (July 1992)